# EUROPEAN PATENT APPLICATION

(11) **EP 4 567 035 A1**
(43) Date of publication of application: **11.06.2025**
(21) Application number: 23850500.2
(22) Date of filing: 04.08.2023
(51) Int. Cl.: C07D 473/00, A61K 31/519, A61P 27/02

(54) **NOVEL COMPOUND AS HYPOXIA-INDUCIBLE FACTOR 1(HIF-1) INHIBITOR OR VASCULAR ENDOTHELIAL GROWTH FACTOR (VEGF) INHIBITOR, AND PHARMACEUTICAL COMPOSITION COMPRISING SAME**

(30) Priority: 05.08.2022 KR 20220098183
(71) Applicant: Nextgen Bioscience Co., Ltd., Seongnam-si, Gyeonggi-do 13487 (KR)
(72) Inventor: KIM, Shin Ae, Suwon-si, Gyeonggi-do 16507 (KR); LEE, Jae Sang, Yongin-si, Gyeonggi-do 16836 (KR); LEE, Jae Un, Seongnam-si, Gyeonggi-do 13505 (KR); SHIN, Soo Jeong, Yongin-si, Gyeonggi-do 16944 (KR); KIM, A Reum, Seoul 01913 (KR); LEE, Bong Yong, Seoul 06625 (KR); HAN, Na Ra, Yongin-si, Gyeonggi-do 17064 (KR)
(74) Representative: Graf von Stosch Patentanwaltsgesellschaft mbH
(86) International application number: PCT/KR2023/011528
(87) International publication number: WO 2024/030000

(57) **Abstract**

The present invention provides a compound as a HIF-1α inhibitor or an angiogenesis inducer VEGF inhibitor, and a pharmaceutical composition comprising the same for the prevention or treatment of a disease related to HIF-1α or VEGF. The novel compound of the present invention exhibits an excellent inhibitory effect on the expression of HIF-1α and angiogenesis inducer VEGF, and thus can be usefully used for the prevention or treatment of a disease related to HIF-1α and angiogenesis inducer VEGF.

## Description

### FIELD

The present invention relates to a novel compound as a HIF-1 protein inhibitor or a VEGF inhibitor, a hydrate thereof, a solvate thereof, a prodrug thereof, an isomer thereof or a pharmaceutically acceptable salt thereof, and a pharmaceutical composition comprising the same.

### BACKGROUND

Hypoxia, a pathological condition in which adequate oxygen supply is not achieved, is a typical feature of various incurable diseases such as cancer, heart disease, ischemic disease, and vascular diseases. HIF-1 (Hypoxia Inducible Factor-1) is a transcription factor induced by hypoxia. HIF-1 includes HIF-1α, which is degraded in an oxygen-dependent manner, and HIF-1α induces the expression of genes such as hexokinase 2, glucose transporter 1, erythropoietin, IGF-2, endoglin, VEGFA, MMP-2, uPAR, and MDR1, thereby acquiring traits such as resistance to apoptosis, increased angiogenic potential, increased cell proliferation potential, and increased cell migration (metastasis) and invasion potential.

In particular, HIF-1α can be utilized as a target for the development of therapeutic agents for diseases in which the activation of angiogenesis is associated with the aggravation of the disease, and HIF-1α is a factor, one of the angiogenesis regulatory proteins, and controls angiogenesis in terms of vascular destruction and vascular dysfunction by inducing the expression of VEGF (vascular endothelial growth factor). Angiogenesis is the process in which new capillaries are formed from existing microvessels. Angiogenesis normally occurs during embryonic development, tissue regeneration and wound healing, and the development of the corpus luteum, which is a periodic change in the female reproductive system, and it is also strictly regulated in these cases. In adults, vascular endothelial cells grow very slowly and do not divide relatively well compared to other types of cells. The process of angiogenesis generally consists of the reorganization of blood vessels and the creation of new capillaries through the decomposition of the vascular basement membrane by proteases, the migration and proliferation of vascular endothelial cells, and the formation of lumen by vascular endothelial cell differentiation due to stimulation of angiogenesis-promoting factors. However, there are diseases caused by angiogenesis not being autonomously regulated and growing pathologically. Excessive angiogenesis can cause diseases such as hemangiomas, angiofibromas, vascular malformations, and cardiovascular diseases such as arteriosclerosis, vascular adhesions, and edematous sclerosis. In particular, it has been revealed that excessive angiogenesis plays a critical role in diseases such as macular degeneration.

Although various hypotheses have been put forward regarding the cause and developmental mechanism of macular degeneration, including neovascularization, it has not yet been clearly identified, and since various studies are in progress regarding diagnosis and treatment, there is a continuous demand for the development of new substances for application in inhibiting neovascularization.

### OBJECT OF THE INVENTION

The problem to be addressed by the present invention is provide a novel structural compound exhibiting excellent inhibitory activity against HIF-1α or angiogenesis inducer VEGF.

Further, the problem to be addressed by the present invention is to provide a pharmaceutical composition for preventing or treating a disease associated with HIF-1α or angiogenesis inducer VEGF, which comprises a compound having the novel structure.

Further, the problem to be addressed by the present invention is to provide a method for preventing or treating a disease associated with HIF-1α or angiogenesis inducer VEGF, which comprises a step of administering a compound having the novel structure to a subject or individual in need thereof.

Further, the problem to be addressed by the present invention is to provide a use of the compound having the novel structure for preparing a medicament for use in the prevention or treatment of diseases associated with HIF-1α or angiogenesis inducer VEGF.

The problems to be addressed by the present invention are not limited to the problems mentioned above, and other technical problems not mentioned can be clearly understood by a person having ordinary skill in the technical field to which the present invention belongs from the description below.

### SUMMARY

In order to address the above problem, according to one aspect of the present invention, there is provided a compound represented by the following Formula I, a solvate thereof, a prodrug thereof, an isomer thereof, or a pharmaceutically acceptable salt thereof, wherein: 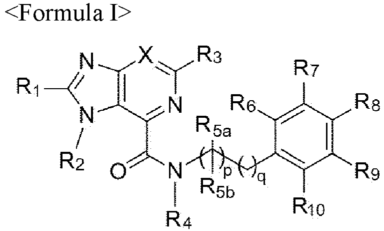
X is C or N,
R₁ is hydrogen, amino-C₁₋₃ alkyl, cycloalkyl of 1 to 2 rings of C₃₋₈ (wherein hydrogen is protium or deuterium), or Rₛ, R₁ is substituted with one or more independent R₁ₐ or not,
wherein R₁ₐ is C₁₋₅ alkyl, halogen, oxo, or -C(O)OR_{1b}, and R_{1b} is hydrogen, C₁₋₃ alkyl, or arylalkyl,
R₂ is hydrogen, C₁₋₃ alkyl or triC₁₋₃ alkyl-silyl-C₁₋₃ alkoxy-C₁₋₃ alkyl,
R₃ is hydrogen, C₁₋₃ alkyl or halogen,
R₄ is hydrogen or C₁₋₃ alkyl,
p and q are independently integers 0 or 1,
when p is 1, R₅ₐ and R_{5b} is independently hydrogen or C₁₋₃ alkyl, or R₅ₐ and R_{5b} are connected to each other to form a C₃₋₆ ring,
R₆ is hydrogen or halogen,
R₇ is hydrogen, halogen, Rₛ, Rᵤ, -NH-aryl, -NH-cycloC₃₋₇alkyl, -NHRₛ, -NHRᵤ or - NHC(O)Rᵤ,
R₈ is hydrogen, halogen, or a 4- to 6-membered heteroaryl having 1 to 3 of N,
R₇ and R₈ are independently substituted with one or more independent Rₘ or not,
Rₘ is C₁₋₃ alkyl (wherein hydrogen is protium or deuterium), C₃₋₁₀ cycloalkyl, C₁₋₃ alkoxy, sulfonyl, amino, halogen, cyano, haloalkyl, carbamoyl, carboxyl, aryl (wherein hydrogen is protium or deuterium), arylalkyl, Rᵤ, or Rₛ, Rₘ is substituted with one or more independent Rₙ or not,
Rₙ is C₁₋₃ alkyl, C₁₋₃ alkylsulfonyl, C₁₋₃ alkoxy, halogen, cyano, haloalkyl, carbamoyl, carboxyl, acetyl, aryl, arylalkyl, oxido, or alkylcarboxyl-(8-cycloalkyl-7H-carboxamido)-alkyl,
R₉ and R₁₀ are independently hydrogen or halogen,
Rₛ is a 4- to 8-membered saturated heterocycloalkyl having 1 to 2 heteroatoms selected from N and O,
Rᵤ is a 4- to 6-membered heteroaryl having 1 to 3 heteroatoms selected from N, O and S.

According to another aspect of the present invention, there is provided a pharmaceutical composition for preventing or treating a disease associated with HIF-1α or angiogenesis inducer VEGF, comprising a compound represented by the Formula I, a hydrate thereof, a solvate thereof, a prodrug thereof, an isomer thereof, or a pharmaceutically acceptable salt thereof as an active ingredient.

According to another aspect of the present invention, there is provided a method for preventing or treating a disease associated with HIF-1α or angiogenesis inducer VEGF, comprising a step of administering to a subject or individual in need thereof a compound represented by the Formula I, a hydrate thereof, a solvate thereof, a prodrug thereof, an isomer thereof, or a pharmaceutically acceptable salt thereof.

According to another aspect of the present invention, there is provided a use of a compound represented by the Formula I, a hydrate thereof, a solvate thereof, a prodrug thereof, an isomer thereof, or a pharmaceutically acceptable salt thereof, for preparing a medicament for use in the prevention or treatment of a disease associated with HIF-1α or angiogenesis inducer VEGF.

According to another aspect of the present invention, there is provided a formulation comprising a compound represented by the Formula I, a hydrate thereof, a solvate thereof, a prodrug thereof, an isomer thereof, or a pharmaceutically acceptable salt thereof, and a pharmaceutically acceptable additive.

It was confirmed that the novel compound of the present invention exhibits an excellent inhibitory effect on the expression of HIF-1α and its downstream gene, the angiogenesis inducer VEGF, and thus it was revealed that it can be usefully used for the prevention or treatment of diseases associated with HIF-1α and the angiogenesis inducer VEGF.

The effects of the present invention are not limited to the effects described above, and should be understood to include all effects that can be inferred from the composition of the invention described in the description or claims of the present invention.

### DETAILED DESCRIPTION OF THE INVENTION

The present invention provides a compound represented by the following Formula I, a hydrate thereof, a solvate thereof, a prodrug thereof, an isomer thereof, or a pharmaceutically acceptable salt thereof, wherein: 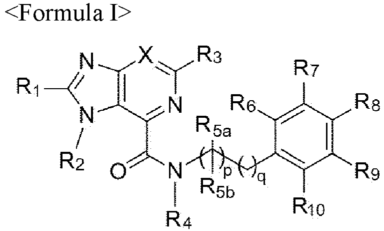
X is C or N,
R₁ is hydrogen, amino-C₁₋₃ alkyl, cycloalkyl of 1 to 2 rings of C₃₋₈ (wherein hydrogen is protium or deuterium), or Rₛ, R₁ is substituted with one or more independent R₁ₐ or not,
wherein R₁ₐ is C₁₋₅ alkyl, halogen, oxo, or -C(O)OR_{1b}, and R_{1b} is hydrogen, C₁₋₃ alkyl, or arylalkyl,
R₂ is hydrogen, C₁₋₃ alkyl or triC₁₋₃ alkyl-silyl-C₁₋₃ alkoxy-C₁₋₃ alkyl,
R₃ is hydrogen, C₁₋₃ alkyl or halogen,
R₄ is hydrogen or C₁₋₃ alkyl,
p and q are independently integers 0 or 1,
when p is 1, R₅ₐ and R_{5b} is independently hydrogen or C₁₋₃ alkyl, or R₅ₐ and R_{5b} are connected to each other to form a C₃₋₆ ring,
R₆ is hydrogen or halogen,
R₇ is hydrogen, halogen, Rₛ, Rᵤ, -NH-aryl, -NH-cycloC₃₋₇alkyl, -NHRₛ, -NHRᵤ or - NHC(O)Rᵤ,
R₈ is hydrogen, halogen, or a 4- to 6-membered heteroaryl having 1 to 3 of N,
R₇ and R₈ are independently substituted with one or more independent Rₘ or not,
Rₘ is C₁₋₃ alkyl (wherein hydrogen is protium or deuterium), C₃₋₁₀ cycloalkyl, C₁₋₃ alkoxy, sulfonyl, amino, halogen, cyano, haloalkyl, carbamoyl, carboxyl, aryl (wherein hydrogen is protium or deuterium), arylalkyl, Rᵤ, or Rₛ, Rₘ is substituted with one or more independent Rₙ or not,
Rₙ is C₁₋₃ alkyl, C₁₋₃ alkylsulfonyl, C₁₋₃ alkoxy, halogen, cyano, haloalkyl, carbamoyl, carboxyl, acetyl, aryl, arylalkyl, oxido, or alkylcarboxyl-(8-cycloalkyl-7H-carboxamido)-alkyl,
R₉ and R₁₀ are independently hydrogen or halogen,
Rₛ is a 4- to 8-membered saturated heterocycloalkyl having 1 to 2 heteroatoms selected from N and O,
Rᵤ is a 4- to 6-membered heteroaryl having 1 to 3 heteroatoms selected from N, O and S.

In one embodiment, R₁ may be hydrogen, aminomethyl, cyclobutyl, cyclopentyl (wherein hydrogen is protium or deuterium), spiro[2.3]hexanyl, spiro[3.3]heptanyl, bicyclo[1.1.1]pentanyl, bicyclo[3.1.0]hexanyl, bicyclo[2.1.1]hexanyl, bicyclo[2.2.1]heptanyl, pyrrolidinyl, tetrahydrofuranyl, or piperidinyl, and R₁ₐ may be methyl, propyl, isopropyl, fluoro, oxo, -C(O)OH, -C(O)O-methyl, or -C(O)O-benzyl.

In one embodiment, R₂ may be hydrogen, methyl or trimethyl-silyl-ethoxy-methyl.

In one embodiment, R₃ may be hydrogen, methyl, ethyl or chloro.

In one embodiment, R₄ may be hydrogen or methyl.

In one embodiment, R₅ₐ and R_{5b} may be independently hydrogen or methyl, or R₅ₐ and R_{5b} may be connected to each other to form cyclopropyl.

In one embodiment, R₆ may be hydrogen or fluoro.

In one embodiment, R₇ may be hydrogen, Br, piperazinyl, diazepanyl, pyrrolyl, furanyl, thiophenyl, pyrazolyl, isothiazolyl, triazolyl, oxadiazolyl, -NH-phenyl, -NH-cyclopentyl, -NH-piperidinyl, -NH-pyrrolyl, -NH-thiophenyl, -NH-pyrazolyl, -NH-isoxazolyl, -NH-oxadiazolyl, -NH-thiadiazolyl, -NH-pyridinyl, or -NHC(O)-pyrazolyl.

In one embodiment, R₈ may be hydrogen, fluoro, chloro, or pyrazolyl.

In one embodiment, Rₘ may be methyl (wherein hydrogen is protium or deuterium), methoxy, sulfonyl, amino, fluoro, chloro, cyano, trifluoromethyl, difluoroethyl, trifluoroethyl, carbamoyl, carboxyl, cyclopentyl, cyclohexyl, phenyl (wherein hydrogen is protium or deuterium), benzyl, thiophenyl, furanyl, pyrazolyl, thiazolyl, pyridinyl, pyrimidinyl, or tetrahydrofuranyl, and Rₙ may be methyl, ethyl, methylsulfonyl, methoxy, fluoro, chloro, iodo, bromo, cyano, trifluoromethyl, carbamoyl, carboxyl, acetyl, phenyl, benzyl, oxido, or methylcarboxyl-(8-cyclopentyl-7H-purine-6-carboxamido)-ethyl.

In one embodiment, R₉ may be hydrogen, fluoro or chloro.

In one embodiment, R₁₀ may be hydrogen or fluoro.

This specification uses the following definitions when defining the compound of Formula I unless specifically defined.

The term "alkyl" as used herein refers to a straight-chain or branched-chain saturated hydrocarbon, preferably C ₁₋₁₀ alkyl. Examples of alkyl include, but are not limited to, methyl, ethyl, *n*-propyl, *iso*-propyl, *n*-butyl, *iso*-butyl, *tert-*butyl, *n*-pentyl, *iso-*pentyl, *n*-hexyl, 3-methylhexyl, 2,2-dimethylpentyl, 2,3-dimethylpentyl, *n*-heptyl, *n*-octyl, *n*-nonyl and *n*-decyl, etc..

The term "cycloalkyl" as used herein refers to a partially or fully saturated single or fused ring hydrocarbon, and a C ₃₋₁₀ monocyclic, bicyclic or tricyclic functional group. Examples of monocyclic functional groups in cycloalkyl include cyclopropyl, cyclobutyl, cyclopentyl, cyclohexyl, cyclohexynyl and the like. Bicyclic functional groups in cycloalkyl include bicycloalkyl and spiro functional groups, and specific examples include bicyclo[1.1.1]pentanyl, bicyclo[3.1.0]hexanyl, bicyclo[2.1.1]hexanyl, bicyclo[2.2.1]heptanyl, spiro[2.3]hexanyl, spiro[3.3]heptanyl and the like.

The term "hydroxy" or "hydroxyl" as used herein is defined as -OH, and the term "alkoxy", unless otherwise defined, means alkyloxy, a radical in which the hydrogen atoms of the hydroxy group are replaced by 1 to 10 alkyl atoms.

The term "halogen" or "halo" as used herein refers to fluorine (F), chlorine (Cl), bromine (Br), or iodine (I).

The terms "haloalkyl" and "haloalkoxy" as used herein mean alkyl or alkoxy substituted with one or more halogen atoms.

The term "heteroatom" as used herein means N, O or S.

The term "aryl" as used herein means an aromatic hydrocarbon, and includes a polycyclic aromatic ring system in which a carbocyclic aromatic ring or a heteroaryl ring is fused with one or more other rings. Preferably, it is C₅₋₁₂ aryl, and more preferably, C₅₋₁₀ aryl. For example, the aryl includes, but is not limited to, phenyl, naphthyl, tetrahydronaphthyl, and the like. Also included are groups in which a heteroaryl ring is fused to a cycloalkyl or a non-aromatic heterocyclic ring, for example, indanyl or tetrahydrobenzopyranyl.

The term "heteroaryl" or "aromatic heterocycle" as used herein means a 3- to 12-membered, more preferably 5- to 10-membered aromatic hydrocarbon forming a single or fused ring that contains one or more heteroatoms selected from N, O and S as ring atoms, and that can be fused with a benzo or C₃₋₈ cycloalkyl. For example, heteroaryl includes, but are not limited to, pyrrolyl, imidazolyl, pyrazolyl, triazolyl, pyridyl, pyrazinyl, pyrimidyl, pyridazinyl, triazinyl, oxadiazolyl, isoxadiazolyl, tetrazolyl, indolyl, indazolyl, isoxazolyl, oxazolyl, thiazolyl, isothiazolyl, furanyl, benzofuranyl, thiophenyl, benzothiazolyl, benzooxazolyl, benzimidazolyl, quinolinyl, isoquinolinyl, etc..

The term "heterocycloalkyl" as used herein refers to a non-aromatic alkyl ring, which is a non-aromatic carbocyclic ring containing one or more heteroatoms, such as N, O or S, within the ring. The ring may be 5, 6, 7 or 8 membered and/or may be fused to another ring, such as a cycloalkyl or an aromatic ring. Examples of such compounds include pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, oxathiolanyl, dithiolanyl, piperidinyl, tetrahydropyranyl, thianyl, piperazinyl, morpholino, and dioxanyl.

Arylalkyl, alkylaryl and heteroarylalkyl refer to groups formed by combining aryl and alkyl or heteroaryl and alkyl as defined above, and examples thereof include, but are not limited to, benzyl, thiopheneethyl, and pyrimidinemethyl.

The term "Cbz" as used herein means carbobenzyloxy.

Representative examples of the compound of Formula I according to the present invention are as follows:
[1] 8-cyclopentyl-N-(3-fluoro-5-(1-(trifluoromethyl)-1H-pyrazol-4-yl)benzyl)-7H-purine-6-carboxamide,
[2] 8-cyclopentyl-N-(3-(1-(2,2-difluoroethyl)-1H-pyrazol-4-yl)-5-fluorobenzyl)-7H-purine-6-carboxamide,
[3] 8-cyclopentyl-N-(3-fluoro-5-(1-(2,2,2-trifluoroethyl)-1H-pyrazol-4-yl)benzyl)-7H-purine-6-carboxamide,
[4] (S)-8-cyclopentyl-N-(1-(3-(1-methyl-1H-pyrazol-4-yl)phenyl)ethyl)-7H-purine-6-carboxamide,
[5] 8-cyclopentyl-N-(3-fluoro-5-(1-methyl-1H-pyrazole-4-carboxamido)benzyl)-7H-purine-6-carboxamide,
[6] 8-cyclopentyl-N-(1-(3-(1-methyl-1H-pyrazol-4-yl)phenyl)cyclopropyl)-7H-purine-6-carboxamide,
[7] 8-cyclopentyl-N-(3-fluoro-5-(1-phenyl-1H-pyrazol-4-yl)benzyl)-7H-purine-6-carboxamide,
[8] 8-cyclopentyl-N-(3-fluoro-5-((1-methyl-1H-pyrazol-4-yl)amino)benzyl)-7H-purine-6-carboxamide,
[9] N-(3-(1-benzyl-1H-pyrazol-4-yl)-5-fluorobenzyl)-8-cyclopentyl-7H-purine-6-carboxamide,
[10] 8-cyclopentyl-N-(3-fluoro-5-(pyridin-3-ylamino)benzyl)-7H-purine-6-carboxamide,
[11] 8-cyclopentyl-N-(3-fluoro-5-(1H-pyrazol-4-yl)benzyl)-7H-purine-6-carboxamide,
[12] 8-cyclopentyl-N-(3-fluoro-5-(1-(pyridin-4-yl)-1H-pyrazol-4-yl)benzyl)-7H-purine-6-carboxamide,
[13] 8-cyclopentyl-N-(3-fluorobenzyl)-7H-purine-6-carboxamide,
[14] N-(3-bromo-5-fluorobenzyl)-8-cyclopentyl-7H-purine-6-carboxamide,
[15] 8-cyclopentyl-N-(3-fluoro-5-(phenylamino)benzyl)-7H-purine-6-carboxamide,
[16] 8-cyclopentyl-N-(3-fluoro-5-(pyridin-4-ylamino)benzyl)-7H-purine-6-carboxamide,
[17] 8-cyclopentyl-N-(3-fluoro-5-((3-(trifluoromethyl)phenyl)amino)benzyl)-7H-purine-6-carboxamide,
[18] 8-cyclopentyl-N-(3-((4-(dimethylamino)phenyl)amino)-5-fluorobenzyl)-7H-purine-6-carboxamide,
[19] 8-cyclopentyl-N-(3-fluoro-5-((4-(methylsulfonyl)phenyl)amino)benzyl)-7H-purine-6-carboxamide,
[20] N-(3-((4-chloro-2-fluorophenyl)amino)-5-fluorobenzyl)-8-cyclopentyl-7H-purine-6-carboxamide,
[21] 8-cyclopentyl-N-(3-fluoro-5-((4-fluorophenyl)amino)benzyl)-7H-purine-6-carboxamide,
[22] N-(3-(1-(3-cyanophenyl)-1H-pyrazol-4-yl)-5-fluorobenzyl)-8-cyclopentyl-7-((2-(trimethylsilyl)ethoxy)methyl)-7H-purine-6-carboxamide,
[23] N-(3-(1-(3-cyanophenyl)-1H-pyrazol-4-yl)-5-fluorobenzyl)-8-cyclopentyl-7H-purine-6-carboxamide,
[24] 8-cyclopentyl-N-(3-fluoro-5-(1-(4-(methylsulfonyl)benzyl)-1H-pyrazol-4-yl)benzyl)-7H-purine-6-carboxamide,
[25] 8-cyclopentyl-N-(3-fluoro-5-(1-(4-methoxyphenyl)-1H-pyrazol-4-yl)benzyl)-7H-purine-6-carboxamide,
[26] 8-cyclopentyl-N-(3-fluoro-5-(1-(4-(trifluoromethyl)phenyl)-1H-pyrazol-4-yl)benzyl)-7H-purine-6-carboxamide,
[27] 8-cyclopentyl-N-(3-fluoro-5-(1-(4-fluorophenyl)-1H-pyrazol-4-yl)benzyl)-7H-purine-6-carboxamide,
[28] 8-cyclopentyl-N-(3-fluoro-5-(1-(4-(methylsulfonyl)phenyl)-1H-pyrazol-4-yl)benzyl)-7H-purine-6-carboxamide,
[29] 8-cyclopentyl-N-(3-fluoro-5-(1-(3-(trifluoromethyl)benzyl)-1H-pyrazol-4-yl)benzyl)-7H-purine-6-carboxamide,
[30] 8-cyclopentyl-N-(3-fluoro-5-(1-(pyridin-2-yl)-1H-pyrazol-4-yl)benzyl)-7H-purine-6-carboxamide,
[31] 8-cyclopentyl-N-(3-fluoro-5-(1-(pyridin-3-yl)-1H-pyrazol-4-yl)benzyl)-7H-purine-6-carboxamide,
[32] 8-cyclopentyl-N-(3-fluoro-5-(1-(pyrimidin-5-yl)-1H-pyrazol-4-yl)benzyl)-7H-purine-6-carboxamide,
[33] 8-cyclopentyl-N-(3-fluoro-5-((2-methoxyphenyl)amino)benzyl)-7H-purine-6-carboxamide,
[34] 8-cyclopentyl-N-(3-fluoro-5-((3-methoxyphenyl)amino)benzyl)-7H-purine-6-carboxamide,
[35] 8-cyclopentyl-N-(3-fluoro-5-((4-methoxyphenyl)amino)benzyl)-7H-purine-6-carboxamide,
[36] 8-cyclopentyl-N-(3-fluoro-5-((4-methoxyphenyl)amino)benzyl)-7-methyl-7H-purine-6-carboxamide,
[37] N-(3-(1-(3-carbamoylphenyl)-1H-pyrazol-4-yl)-5-fluorobenzyl)-8-cyclopentyl-7H-purine-6-carboxamide,
[38] 8-cyclopentyl-N-(3-fluoro-5-(isothiazol-4-yl)benzyl)-7H-purine-6-carboxamide,
[39] 8-cyclopentyl-N-(3-fluoro-5-(furan-2-yl)benzyl)-7H-purine-6-carboxamide,
[40] 8-cyclopentyl-N-(3-fluoro-5-(thiophen-3-yl)benzyl)-7H-purine-6-carboxamide,
[41] 8-cyclopentyl-N-(3-fluoro-5-((4-(trifluoromethyl)phenyl)amino)benzyl)-7H-purine-6-carboxamide,
[42] 8-cyclopentyl-N-(3-fluoro-5-(1-(2-methylpyridin-4-yl)-1H-pyrazol-4-yl)benzyl)-7H-purine-6-carboxamide,
[43] 8-cyclopentyl-N-(3-fluoro-5-(1-(6-methoxypyridin-3-yl)-1H-pyrazol-4-yl)benzyl)-7H-purine-6-carboxamide,
[44] 8-cyclopentyl-N-(3-fluoro-5-(1-(6-(trifluoromethyl)pyridin-3-yl)-1H-pyrazol-4-yl)benzyl)-7H-purine-6-carboxamide,
[45] 8-cyclopentyl-N-(3-fluoro-5-(1-(3-(methylsulfonyl)phenyl)-1H-pyrazol-4-yl)benzyl)-7H-purine-6-carboxamide,
[46] 8-cyclopentyl-N-(3-fluoro-5-(1-(3-fluorophenyl)-1H-pyrazol-4-yl)benzyl)-7H-purine-6-carboxamide,
[47] 8-cyclopentyl-N-(3-fluoro-5-(1-(6-fluoropyridin-3-yl)-1H-pyrazol-4-yl)benzyl)-7H-purine-6-carboxamide,
[48] 8-cyclopentyl-N-(3-fluoro-5-(1-(5-iodopyridin-2-yl)-1H-pyrazol-4-yl)benzyl)-7H-purine-6-carboxamide,
[49] 3-(4-(3-((8-cyclopentyl-7H-purine-6-carboxamido)methyl)-5-fluorophenyl)-1H-pyrazol-1-yl)benzoic acid,
[50] N-(3-(1-(4-cyanophenyl)-1H-pyrazol-4-yl)-5-fluorobenzyl)-8-cyclopentyl-7H-purine-6-carboxamide,
[51] 8-cyclopentyl-N-(3-fluoro-5-(1-(4-fluorophenyl)-1H-pyrazol-3-yl)benzyl)-7H-purine-6-carboxamide,
[52] 8-cyclopentyl-N-(3-fluoro-5-(1-(4-fluorophenyl)-1H-pyrrol-3-yl)benzyl)-7H-purine-6-carboxamide,
[53] N-(3-(1-(4-carbamoylphenyl)-1H-pyrazol-4-yl)-5-fluorobenzyl)-8-cyclopentyl-7H-purine-6-carboxamide,
[54] 4-(4-(3-((8-cyclopentyl-7H-purine-6-carboxamido)methyl)-5-fluorophenyl)-1H-pyrazol-1-yl)benzoic acid,
[55] 8-cyclopentyl-N-(3-fluoro-5-(1-(4-fluorophenyl)-1H-pyrazol-4-yl)benzyl)-7-methyl-7H-purine-6-carboxamide,
[56] 8-cyclopentyl-N-(2-fluoro-5-(1-(4-fluorophenyl)-1H-pyrazol-4-yl)benzyl)-7H-purine-6-carboxamide,
[57] 8-cyclopentyl-N-(3-(1-(4-fluorophenyl)-1H-pyrazol-4-yl)benzyl)-7H-purine-6-carboxamide,
[58] N-(3-(1-(4-chlorophenyl)-1H-pyrazol-4-yl)-5-fluorobenzyl)-8-cyclopentyl-7H-purine-6-carboxamide,
[59] N-(3-(1-(4-bromo-2-(methylsulfonyl)phenyl)-1H-pyrazol-4-yl)-5-fluorobenzyl)-8-cyclopentyl-7H-purine-6-carboxamide,
[60] 8-cyclopentyl-N-(3-(1-(3,4-difluorophenyl)-1H-pyrazol-4-yl)-5-fluorobenzyl)-7H-purine-6-carboxamide,
[61] 2-chloro-8-cyclopentyl-N-(3-fluoro-5-(1-(4-fluorophenyl)-1H-pyrazol-4-yl)benzyl)-7H-purine-6-carboxamide,
[62] (S)-N-(3-fluoro-5-(1-methyl-1H-pyrazol-4-yl)benzyl)-8-(pyrrolidin-2-yl)-7H-purine-6-carboxamide hydrochloride,
[63] (S)-N-(3-fluoro-5-(1-methyl-1H-pyrazol-4-yl)benzyl)-8-(1-methylpyrrolidin-2-yl)-7H-purine-6-carboxamide,
[64] benzyl (R)-2-(6-((3-fluoro-5-(1-methyl-1H-pyrazol-4-yl)benzyl)carbamoyl)-7H-purin-8-yl)pyrrolidine-1-carboxylate,
[65] (R)-N-(3-fluoro-5-(1-methyl-1H-pyrazol-4-yl)benzyl)-8-(pyrrolidin-2-yl)-7H-purine-6-carboxamide hydrochloride,
[66] (R)-N-(3-fluoro-5-(1-methyl-1H-pyrazol-4-yl)benzyl)-8-(1-methylpyrrolidin-2-yl)-7H-purine-6-carboxamide,
[67] (S)-N-(3-fluoro-5-(1-methyl-1H-pyrazol-4-yl)benzyl)-8-(1-isopropylpyrrolidin-2-yl)-7H-purine-6-carboxamide,
[68] (R)-N-(3-fluorobenzyl)-8-(1-methylpyrrolidin-2-yl)-7H-purine-6-carboxamide,
[69] benzyl 4-(6-((3-fluoro-5-(1-methyl-1H-pyrazol-4-yl)benzyl)carbamoyl)-7H-purin-8-yl)piperidine-1-carboxylate,
[70] N-(3-fluoro-5-(1-methyl-1H-pyrazol-4-yl)benzyl)-8-(piperidin-4-yl)-7H-purine-6-carboxamide hydrochloride,
[71] N-(3-fluoro-5-(1-methyl-1H-pyrazol-4-yl)benzyl)-8-(1-methylpiperidin-4-yl)-7H-purine-6-carboxamide,
[72] benzyl ((6-((3-fluoro-5-(1-methyl-1H-pyrazol-4-yl)benzyl)carbamoyl)-7H-purin-8-yl)methyl)carbamate,
[73] 8-(aminomethyl)-N-(3-fluoro-5-(1-methyl-1H-pyrazol-4-yl)benzyl)-7H-purine-6-carboxamide,
[74] N-(3-fluoro-5-(1-methyl-1H-pyrazol-4-yl)benzyl)-7H-purine-6-carboxamide,
[75] benzyl 3-(6-((3-fluoro-5-(1-methyl-1H-pyrazol-4-yl)benzyl)carbamoyl)-7H-purin-8-yl)pyrrolidine-1-carboxylate,
[76] N-(3-fluoro-5-(1-methyl-1H-pyrazol-4-yl)benzyl)-8-(pyrrolidin-3-yl)-7H-purine-6-carboxamide hydrochloride,
[77] 8-(6,6-difluorobicyclo[3.1.0]hexan-3-yl)-N-(3-fluoro-5-(1-methyl-1H-pyrazol-4-yl)benzyl)-7H-purine-6-carboxamide,
[78] 8-(bicyclo[3.1.0]hexan-3-yl)-N-(3-fluoro-5-(1-methyl-1H-pyrazol-4-yl)benzyl)-7H-purine-6-carboxamide,
[79] N-(3-fluoro-5-(1-methyl-1H-pyrazol-4-yl)benzyl)-8-(1-methylpyrrolidin-3-yl)-7H-purine-6-carboxamide,
[80] 8-(3,3-difluorocyclopentyl)-N-(3-fluoro-5-(1-(4-fluorophenyl)-1H-pyrazol-4-yl)benzyl)-7H-purine-6-carboxamide,
[81] 8-(3,3-difluorocyclopentyl)-N-(3-fluoro-5-(1-(3-fluorophenyl)-1H-pyrazol-4-yl)benzyl)-7H-purine-6-carboxamide,
[82] 8-(3,3-difluorocyclopentyl)-N-(3-fluoro-5-(1-(6-fluoropyridin-3-yl)-1H-pyrazol-4-yl)benzyl)-7H-purine-6-carboxamide,
[83] 8-(3,3-difluorocyclopentyl)-N-(3-fluoro-5-(1-(pyridin-3-yl)-1H-pyrazol-4-yl)benzyl)-7H-purine-6-carboxamide,
[84] (E1, E2) 8-(3,3-difluorocyclopentyl)-N-(3-fluoro-5-(1-(4-fluorophenyl)-1H-pyrazol-4-yl)benzyl)-7H-purine-6-carboxamide,
[85] (R)-N-(3-fluoro-5-(1-(4-fluorophenyl)-1H-pyrazol-4-yl)benzyl)-8-(tetrahydrofuran-2-yl)-7H-purine-6-carboxamide,
[86] (R)-N-(3-fluoro-5-(1-(4-fluorophenyl)-1H-pyrazol-4-yl)benzyl)-8-(3-oxocyclopentyl)-7H-purine-6-carboxamide,
[87] N-(3-(1-(3-chloro-4-fluorophenyl)-1H-pyrazol-4-yl)-5-fluorobenzyl)-8-cyclopentyl-7H-purine-6-carboxamide,
[88] (S)-N-(3-fluoro-5-(1-(4-fluorophenyl)-1H-pyrazol-4-yl)benzyl)-8-(3-oxocyclopentyl)-7H-purine-6-carboxamide,
[89] N-(3-fluoro-5-(1-(4-fluorophenyl)-1H-pyrazol-4-yl)benzyl)-8-(tetrahydrofuran-3-yl)-7H-purine-6-carboxamide,
[90] (R)-8-cyclopentyl-N-(1-(3-(1-(4-fluorophenyl)-1H-pyrazol-4-yl)phenyl)ethyl)-7H-purine-6-carboxamide,
[91] (S)-8-cyclopentyl-N-(1-(3-(1-(4-fluorophenyl)-1H-pyrazol-4-yl)phenyl)ethyl)-7H-purine-6-carboxamide
[92] 8-(3,3-difluorocyclobutyl)-N-(3-fluoro-5-(1-(4-fluorophenyl)-1H-pyrazol-4-yl)benzyl)-7H-purine-6-carboxamide,
[93] 2-cyclopentyl-N-(3-fluoro-5-(1-(4-fluorophenyl)-1H-pyrazol-4-yl)benzyl)-3H-imidazo[4,5-c]pyridine-4-carboxamide,
[94] 8-cyclopentyl-N-(3-(1-(4-fluorophenyl)-1H-pyrazol-4-yl)phenethyl)-7H-purine-6-carboxamide,
[95] 8-cyclopentyl-N-(2-fluoro-3-(1-(4-fluorophenyl)-1H-pyrazol-4-yl)benzyl)-7H-purine-6-carboxamide,
[96] 8-cyclopentyl-N-(4-fluoro-3-(1-(4-fluorophenyl)-1H-pyrazol-4-yl)benzyl)-7H-purine-6-carboxamide,
[97] 8-(3,3-dimethylcyclobutyl)-N-(3-fluoro-5-(1-(4-fluorophenyl)-1H-pyrazol-4-yl)benzyl)-7H-purine-6-carboxamide,
[98] 8-(6,6-difluorospiro[3.3]heptan-2-yl)-N-(3-fluoro-5-(1-(4-fluorophenyl)-1H-pyrazol-4-yl)benzyl)-7H-purine-6-carboxamide,
[99] 8-(bicyclo[1.1.1]pentan-1-yl)-N-(3-fluoro-5-(1-(4-fluorophenyl)-1H-pyrazol-4-yl)benzyl)-7H-purine-6-carboxamide,
[100] N-(3-fluoro-5-(1-(4-fluorophenyl)-1H-pyrazol-4-yl)benzyl)-8-(spiro[3.3]heptan-2-yl)-7H-purine-6-carboxamide,
[101] N-(3-fluoro-5-(1-(4-fluorophenyl)-1H-pyrazol-4-yl)benzyl)-8-(3-fluorobicyclo[1.1.1]pentan-1-yl)-7H-purine-6-carboxamide,
[102] (A, B) N-(3-fluoro-5-(1-(4-fluorophenyl)-1H-pyrazol-4-yl)benzyl)-8-(3-fluorocyclobutyl)-7H-purine-6-carboxamide,
[103] (A, B) methyl 3-(6-((3-fluoro-5-(1-(4-fluorophenyl)-1H-pyrazol-4-yl)benzyl)carbamoyl)-7H-purin-8-yl)cyclobutanecarboxylate,
[104] N-(3-chloro-5-(1-(4-fluorophenyl)-1H-pyrazol-4-yl)benzyl)-8-cyclopentyl-7H-purine-6-carboxamide,
[105] 8-cyclopentyl-N-(3-fluoro-5-(1-(4-fluorophenyl)-1H-pyrazol-4-yl)phenyl)-7H-purine-6-carboxamide,
[106] 3-(6-((3-fluoro-5-(1-(4-fluorophenyl)-1H-pyrazol-4-yl)benzyl)carbamoyl)-7H-purin-8-yl)cyclobutanecarboxylic acid,
[107] 2-cyclopentyl-N-(3-fluoro-5-(1-(4-fluorophenyl)-1H-pyrazol-4-yl)benzyl)-1H-imidazo[4,5-b]pyridine-7-carboxamide,
[108] 8-cyclopentyl-N-(4-(1-(4-fluorophenyl)-1H-pyrazol-4-yl)benzyl)-7H-purine-6-carboxamide,
[109] 8-cyclopentyl-N-(3-fluoro-4-(1-(4-fluorophenyl)-1H-pyrazol-4-yl)benzyl)-7H-purine-6-carboxamide,
[110] (A, B) N-(3-fluoro-5-(1-(4-fluorophenyl)-1H-pyrazol-4-yl)benzyl)-8-(3-fluorocyclopentyl)-7H-purine-6-carboxamide,
[111] N-(3-fluoro-5-(1-(4-fluorophenyl)-1H-pyrazol-4-yl)benzyl)-8-(spiro[2.3]hexan-5-yl)-7H-purine-6-carboxamide,
[112] 8-cyclopentyl-N-(3-fluoro-5-(1-(4-fluorophenyl)-1H-pyrazol-4-yl)benzyl)-N-methyl-7H-purine-6-carboxamide,
[113] 8-(bicyclo[3.1.0]hexan-3-yl)-N-(3-fluoro-5-(1-(4-fluorophenyl)-1H-pyrazol-4-yl)benzyl)-7H-purine-6-carboxamide,
[114] 8-(bicyclo[2.2.1]heptan-2-yl)-N-(3-fluoro-5-(1-(4-fluorophenyl)-1H-pyrazol-4-yl)benzyl)-7H-purine-6-carboxamide,
[115] 8-(bicyclo[2.1.1]hexan-1-yl)-N-(3-fluoro-5-(1-(4-fluorophenyl)-1H-pyrazol-4-yl)benzyl)-7H-purine-6-carboxamide,
[116] 8-cyclopentyl-N-(3-fluoro-5-(4-(4-fluorophenyl)-1H-1,2,3-triazol-1-yl)benzyl)-7H-purine-6-carboxamide,
[117] 8-cyclopentyl-N-(3-fluoro-5-(1-(thiophen-3-yl)-1H-pyrazol-4-yl)benzyl)-7H-purine-6-carboxamide,
[118] 8-cyclopentyl-N-(3-fluoro-5-((1-methyl-1H-pyrazol-3-yl)amino)benzyl)-7H-purine-6-carboxamide,
[119] 8-cyclopentyl-N-(3-fluoro-5-(1-(4-fluorophenyl)-1H-1,2,3-triazol-4-yl)benzyl)-7H-purine-6-carboxamide,
[120] N-(3-((1H-pyrazol-3-yl)amino)-5-fluorobenzyl)-8-cyclopentyl-7H-purine-6-carboxamide,
[121] 8-cyclopentyl-N-(3-fluoro-5-(isoxazol-3-ylamino)benzyl)-7H-purine-6-carboxamide,
[122] N-(3-((4-cyanophenyl)amino)-5-fluorobenzyl)-8-cyclopentyl-7H-purine-6-carboxamide,
[123] N-(3-(1-(4-acetylphenyl)-1H-pyrazol-4-yl)-5-fluorobenzyl)-8-cyclopentyl-7H-purine-6-carboxamide,
[124] 8-cyclopentyl-N-(3-fluoro-5-(1-(furan-3-yl)-1H-pyrazol-4-yl)benzyl)-7H-purine-6-carboxamide,
[125] N-(3-(1'-benzyl-1'H-[1,4'-bipyrazol]-4-yl)-5-fluorobenzyl)-8-cyclopentyl-7H-purine-6-carboxamide,
[126] N-(3-((4-carbamoylphenyl)amino)-5-fluorobenzyl)-8-cyclopentyl-7H-purine-6-carboxamide,
[127] ethyl 4-((3-((8-cyclopentyl-7H-purine-6-carboxamido)methyl)-5-fluorophenyl)amino)benzoate,
[128] 8-cyclopentyl-N-(3-fluoro-5-(1'-methyl-1'H-[1,4'-bipyrazol]-4-yl)benzyl)-7H-purine-6-carboxamide,
[129] 8-cyclopentyl-N-(3-fluoro-5-((1-methylpiperidin-4-yl)amino)benzyl)-7H-purine-6-carboxamide,
[130] 8-cyclopentyl-N-(3-fluoro-5-(thiophen-3-ylamino)benzyl)-7H-purine-6-carboxamide,
[131] 8-cyclopentyl-N-(3-fluoro-5-(4-(4-fluorophenyl)piperazin-1-yl)benzyl)-7H-purine-6-carboxamide,
[132] 8-cyclopentyl-N-(3-fluoro-5-(5-(4-fluorophenyl)-1,3,4-oxadiazol-2-yl)benzyl)-7H-purine-6-carboxamide,
[133] 4-((3-((8-cyclopentyl-7H-purine-6-carboxamido)methyl)-5-fluorophenyl)amino)benzoic acid,
[134] N-(3-((1,3,4-oxadiazol-2-yl)amino)-5-fluorobenzyl)-8-cyclopentyl-7H-purine-6-carboxamide,
[135] 8-cyclopentyl-N-(3-(cyclopentylamino)-5-fluorobenzyl)-7H-purine-6-carboxamide,
[136] N-(3-((1,3,4-thiadiazol-2-yl)amino)-5-fluorobenzyl)-8-cyclopentyl-7H-purine-6-carboxamide,
[137] 8-cyclopentyl-N-(3-fluoro-5-(4-(4-fluorophenyl)-1,4-diazepan-1-yl)benzyl)-7H-purine-6-carboxamide,
[138] 8-cyclopentyl-N-(3-(1-cyclopentyl-1H-pyrazol-4-yl)-5-fluorobenzyl)-7H-purine-6-carboxamide,
[139] N-(3-(1-cyclohexyl-1H-pyrazol-4-yl)-5-fluorobenzyl)-8-cyclopentyl-7H-purine-6-carboxamide,
[140] 8-cyclopentyl-N-(3-fluoro-5-(1-(tetrahydrofuran-3-yl)-1H-pyrazol-4-yl)benzyl)-7H-purine-6-carboxamide,
[141] 8-cyclopentyl-N-(3-fluoro-5-(1-(thiazol-4-yl)-1H-pyrazol-4-yl)benzyl)-7H-purine-6-carboxamide,
[142] 8-cyclopentyl-N-(3-fluoro-5-((1-methyl-1H-pyrrol-3-yl)amino)benzyl)-7H-purine-6-carboxamide,
[143] 8-cyclopentyl-N-(3-fluoro-5-(1-(1-oxidothiophen-3-yl)-1H-pyrazol-4-yl)benzyl)-7H-purine-6-carboxamide,
[144] 8-(3,3-difluorocyclopentyl)-N-(3-fluoro-5-((1-methyl-1H-pyrazol-4-yl)amino)benzyl)-7H-purine-6-carboxamide,
[145] 8-cyclopentyl-N-(3-fluoro-5-(1-phenyl-*d*₅-1H-pyrazol-4-yl)benzyl)-7H-purine-6-carboxamide,
[146] 8-cyclopentyl-N-(3-fluoro-5-(1-(4-fluorophenyl)-3-methyl-1H-pyrazol-4-yl)benzyl)-7H-purine-6-carboxamide,
[147] 8-cyclopentyl-N-(3-fluoro-5-(1-(4-fluorophenyl)-5-methyl-1H-pyrazol-4-yl)benzyl)-7H-purine-6-carboxamide,
[148] 8-(3,3-difluorocyclopentyl)-N-(3-fluoro-5-(1-(phenyl-*d*₅)-1H-pyrazol-4-yl)benzyl)-7H-purine-6-carboxamide,
[149] 8-cyclopentyl-N-(3-fluoro-5-(4-(4-fluorophenyl)-1H-pyrazol-1-yl)benzyl)-7H-purine-6-carboxamide,
[150] methyl 2-(8-cyclopentyl-7H-purine-6-carboxamido)-3-(4-(4-(3-((8-cyclopentyl-7H-purine-6-carboxamido)methyl)-5-fluorophenyl)-1H-pyrazol-1-yl)phenyl)propanoate,
[151] 8-cyclopentyl-N-(3-fluoro-5-((1-(methyl-*d*₃)-1H-pyrazol-4-yl)amino)benzyl)-7H-purine-6-carboxamide,
[152] 8-(3,3-difluorocyclopentyl)-N-(3-fluoro-5-((1-(methyl-*d*₃)-1H-pyrazol-4-yl)amino)benzyl)-7H-purine-6-carboxamide, and
[153] 8-(cyclopentyl-2,2,3,3,4,4,5,5-*d*₈)-N-(3-fluoro-5-(1-(4-fluorophenyl)-1H-pyrazol-4-yl)benzyl)-7H-purine-6-carboxamide.

The compound represented by the above Formula I according to the present invention can be prepared and used in the form of a prodrug, a hydrate, a solvate, and a pharmaceutically acceptable salt in order to promote *in vivo* absorption or increase solubility, and therefore the above prodrug, hydrate, solvate, and pharmaceutically acceptable salt also fall within the scope of the present invention.

The term "prodrug" as used herein refers to a substance that is transformed *in vivo* into the parent drug. Prodrugs are often used because, in some cases, they are easier to administer than the parent drug. For example, they may be bioactive by oral administration, whereas the parent drug may not be. Prodrugs may also have improved solubility in pharmaceutical compositions than the parent drug. For example, prodrugs may be *in vivo* hydrolysable esters of compounds according to the invention and pharmaceutically acceptable salts thereof. Another example of a prodrug may be a short peptide (polyamino acid) that is linked to an acid group that is metabolized to reveal the active site of the peptide.

The term "hydrate" as used herein means a compound of the present invention or a salt thereof having a stoichiometric or non-stoichiometric amount of water bound by non-covalent intermolecular forces.

The term "solvate" as used herein means a compound of the present invention or a salt thereof having a stoichiometric or non-stoichiometric amount of solvent bound by non-covalent intermolecular forces. Preferred solvents therefor include solvents that are volatile, non-toxic, and/or suitable for administration to humans.

The term "isomer" as used herein means a compound of the present invention or a salt thereof which has the same chemical formula or molecular formula but is structurally or sterically different. Such isomers include structural isomers such as tautomers, and stereoisomers such as R or S isomers having an asymmetric carbon center, geometric isomers (trans, cis), and diastereomers. All of these isomers and mixtures thereof are also included in the scope of the present invention. In particular, when the compound of Formula I has optical isomers, stereoisomers, regioisomers, or rotamers, these are also included in the compound of Formula I and can be obtained as a single product according to synthetic and separation methods known per se. For example, when the compound of Formula I includes optical isomers, optical isomers resolved from this compound are also included in the compound of formula I. Optical isomers can be prepared by methods known per se.

The term "pharmaceutically acceptable salt" as used herein means a salt form of a compound which does not cause serious irritation to an organism to which the compound is administered and does not impair the biological activity and physical properties of the compound. The pharmaceutical salt includes acid addition salts formed by acids which form non-toxic acid addition salts containing pharmaceutically acceptable anions, for example, inorganic acids such as hydrochloric acid, sulfuric acid, nitric acid, phosphoric acid, hydrobromic acid, hydroiodic acid, etc., organic carboxylic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc.; amino acid salts such as lysine, arginine, guanidine, etc.; organic salts such as dicyclohexylamine, N-methyl-D-glucamine, tris(hydroxymethyl)methylamine, diethanolamine, choline, and triethylamine. The compound of formula 1 according to the present invention can be converted into its salt by a conventional method.

The synthetic methods of Examples 1 to 153 are exemplified as a method for preparing a compound of Formula I of the present invention, and these synthetic methods of Examples 1 to 153 do not limit the method for preparing a compound of Formula I according to the present invention. The synthetic methods of Examples 1 to 153 are merely exemplified, and it is obvious that they can be easily modified by those skilled in the art depending on a specific substituent.

The present invention also provides a pharmaceutical composition for preventing or treating a disease associated with HIF-1α (Hypoxia-inducible factor 1 alpha) or VEGF (Vascular endothelial growth factor), comprising the compound represented by the Formula I, a hydrate thereof, a solvate thereof, a prodrug thereof, an isomer thereof, or a pharmaceutically acceptable salt thereof as an active ingredient.

The present invention also provides a method for preventing or treating a disease associated with HIF-1α or angiogenesis inducer VEGF, comprising a step of administering to a subject or individual in need thereof a compound represented by the Formula I, a hydrate thereof, a solvate thereof, a prodrug thereof, an isomer thereof, or a pharmaceutically acceptable salt thereof.

The present invention also provides a use of a compound represented by the Formula I, a hydrate thereof, a solvate thereof, a prodrug thereof, an isomer thereof, or a pharmaceutically acceptable salt thereof, for preparing a medicament for use in the prevention or treatment of a disease associated with HIF-1α or angiogenesis inducer VEGF.

In one embodiment, preventing or treating a disease associated with HIF-1α or VEGF may be via an inhibition of angiogenesis.

In one embodiment, the inhibition of angiogenesis may via inhibition of HIF-1α expression or inhibition of VEGF expression.

In one embodiment, the disease associated with HIF-1α or VEGF is at least one selected from the group consisting of stroke, brainstem glioma, cerebellar astrocytoma, cerebral astrocytoma, ependymoma, liver cancer, stomach cancer, breast cancer, colon cancer, bone cancer, pancreatic cancer, head or neck cancer, ovarian cancer, rectal cancer, esophageal cancer, small intestine cancer, anal cancer, skin cancer, fallopian tube carcinoma, endometrial carcinoma, uterine cancer, vaginal carcinoma, vulvar carcinoma, Hodgkin's disease, oral cancer, prostate cancer, testicular cancer, bladder cancer, kidney cancer, ureteral cancer, spleen cell carcinoma, renal pelvic carcinoma, medulloblastoma, neuroblastoma, brain tumor, central nervous system tumor, melanoma, non-small cell lung cancer, lymphoma, non-Hodgkin's lymphoma, cancer, cervical cancer, thyroid cancer, blood cancer, renal cell carcinoma, hepatocellular carcinoma, metastatic cancer, hemangioma, pyogenic granuloma, Kaposi's sarcoma, hemangioendothelioma, solid tumor, follicular cyst, endometriosis, uterine sclerosis, ovarian hypertension, ovarian hyperactivity, uterine dysfunction, warts, scar keloids, allergic edema, atherosclerosis, peritoneal sclerosis, cardiac dysfunction, amyotrophic lateral sclerosis, obesity, rheumatoid arthritis, synovitis, bone and cartilage destruction, malignant fibrous histiocytoma of bone, osteomyelitis, pannus growth, liver fibrosis, pulmonary fibrosis, fibrosis, NASH (non-alcoholic steato hepatitis), pneumonia, asthma, rhinitis, pulmonary hypertension, ischemic acute kidney injury, glomerulonephritis, diabetic nephropathy, malignant nephrosclerosis, thrombotic microvascular lesions, organ transplant rejection, glomerulopathy, inflammatory neurodegenerative diseases, nicotine addiction-related vascular diseases, chronic kidney disease, retinopathy of prematurity, diabetic retinopathy, corneal transplant rejection, ischemic retinopathy, erythroderma, proliferative retinopathy, psoriasis, hemophilic joints, keloids, wound granulation, vascular adhesion, autoimmune diseases, angiofibromas, retrolental fibroplasia, cataracts, glaucoma, macular degeneration, age-related macular degeneration, corneal neovascularization, retinal neovascularization, choroidal neovascularization, intraocular neovascularization, neovascular glaucoma, neovascular macular degeneration, retinal artery occlusion, retinal vein occlusion, retinocytoma, visual pathway and hypothalamic glioma, rhabdomyosarcoma, histosarcoma, inflammation, corneal ulcer, proliferative vitreoretinopathy, cirrhosis, asthma, periodontal disease, allergic dermatitis, Alzheimer's disease, Parkinson's disease, Huntington's disease, thyroiditis, Graves' orbitopathy, leukomalacia, leukemia, acute lymphoblastic leukemia, chronic lymphocytic leukemia, chronic myelogenous leukemia, acute myeloid leukemia, multiple myeloma, inflammatory bowel disease, Crohn's disease, ulcerative colitis, and Behcet's enteritis.

In one embodiment, the angiogenesis-related disease is at least one selected from the group consisting of cancer, cervical cancer, blood cancer, renal cell cancer, hepatocellular cancer, metastatic cancer, hemangioma, pyogenic granuloma, Kaposi's sarcoma, hemangioendothelioma, follicular cyst, endometriosis, uterine cirrhosis, ovarian hypertension, ovarian hyperactivity, uterine dysfunction, warts, scar keloids, allergic edema, atherosclerosis, peritoneal sclerosis, cardiac dysfunction, amyotrophic lateral sclerosis, obesity, rheumatoid arthritis, synovitis, bone and cartilage destruction, osteomyelitis, pannus growth, liver fibrosis, NASH (Non-alcoholic steato hepatitis), pneumonia, asthma, rhinitis, pulmonary hypertension, ischemic acute kidney injury, glomerulonephritis, chronic kidney disease, retinopathy of prematurity, diabetic retinopathy, glaucoma, macular degeneration, corneal neovascularization, retinal neovascularization, choroidal neovascularization, intraocular neovascularization, Alzheimer's disease, Parkinson's disease, Huntington's disease, thyroiditis, Graves' orbitopathy and leukomalacia. The angiogenesis-related disease may be a neovascular eye disease, for example, retinopathy of prematurity, proliferative diabetic retinopathy, neovascular glaucoma, macular degeneration, corneal neovascularization, retinal neovascularization, choroidal neovascularization or intraocular neovascularization. The angiogenesis-related disease may be macular degeneration.

In one embodiment, the HIF-1α or angiogenesis inducer VEGF related disease may be a neovascular eye disease, and the neovascular eye disease may be at least one selected from the group consisting of corneal neovascularization, retinal neovascularization, choroidal neovascularization, intraocular neovascularization, neovascular glaucoma, proliferative diabetic retinopathy, neovascular macular degeneration, and retinopathy of prematurity.

The above neovascular eye disease may be macular degeneration. The macular degeneration may be neovascular age-related macular degeneration (Neovascular AMD) or non-neovascular age-related macular degeneration (Non-neovascular AMD).

The present invention also provides a formulation comprising a compound represented by the Fformula I, a hydrate thereof, a solvate thereof, a prodrug thereof, an isomer thereof, or a pharmaceutically acceptable salt thereof, and a pharmaceutically acceptable additive.

The above additives may include pharmaceutically acceptable carriers such as excipients, disintegrants, sweeteners, lubricants or flavoring agents that are commonly used, and may be formulated into oral preparations such as tablets, capsules, powders, granules, suspensions, emulsions or syrups; or parenteral preparations such as external solutions, external suspensions, external emulsions, gels (ointments, etc.), inhalants, sprays and injections. In the case of oral tablets, carriers such as lactose and corn starch, and lubricants such as magnesium stearate may be commonly added. In the case of oral capsules, lactose and/or dried corn starch may be used as diluents. When an oral aqueous suspension is required, the active ingredient may be combined with an emulsifier and/or a suspending agent. If necessary, a specific sweetener and/or flavoring agent may be added. For intramuscular, intraperitoneal, subcutaneous and intravenous administration, sterile solutions of the active ingredient are usually prepared, the pH of the solution being suitably adjusted and buffered. For intravenous administration, the total concentration of the solutes should be adjusted so as to impart isotonicity to the preparation. The composition according to the present invention may be in the form of an aqueous solution containing a pharmaceutically acceptable carrier, such as saline having a pH of 7.4. The solution may be introduced into the intramuscular bloodstream of the patient by local injection. The preparation may be formulated in various forms, for example, as a single-dose or multiple-dose dosage form.

The subject to which the pharmaceutical composition of the present invention is administered may be, for example, a human, a monkey, a cow, a horse, a sheep, a pig, a chicken, a turkey, a cat, a dog, a mouse, a rat, a rabbit or a guinea pig, and may be, for example, a mammal, for example, a human, but is not limited thereto.

In addition, the pharmaceutical composition of the present invention can be administered orally or parenterally, and in case of parenteral administration, it can be administered by routes such as skin, transdermally, ocularly, intraperitoneally, rectal, or intravenous, intramuscular, subcutaneous, intrauterine epidural, intracerebroventricular injection, or topical administration. Ocular topical administration includes, for example, direct administration into the eye, or administration around the eye, behind the eye, subretinal, central retinal, outside the fovea, subconjunctival, intravitreous, intracameral, or suprachoroidal. The pharmaceutical composition can be administered through an insertion device.

The dosage of the active ingredient contained in the pharmaceutical composition of the present invention varies depending on the patient's condition and weight, the degree of the disease, the form of the active ingredient, the route and period of administration, and can be appropriately adjusted depending on the patient. For example, the active ingredient may be administered at a dosage of 0.0001 to 1000 mg/kg per day, preferably 0.001 to 100 mg/kg, and the administration may be administered once a day or divided into several times. In addition, the pharmaceutical composition of the present invention may contain the active ingredient at a weight percentage of 0.001 to 90% based on the total weight of the composition.

Hereinafter, the present invention will be described in more detail through Preparation examples, Examples, and Experimental examples. However, the following Preparation examples, examples, and experimental examples are intended to illustrate the present invention, and the scope of the present invention is not limited thereto.

### Preparation example 1

### 1.1 Preparation of 8-cyclopentyl-7H-purine-6-carbonitrile

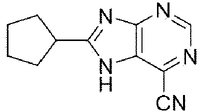

6-Chloro-8-cyclopentyl-7H-purine (1.5 g, 6.73 mmol), KCN (1.32 g, 20.3 mmol), and sodium p-toluenesulfinate (1.2 g, 6.75 mmol) were dissolved in sulfolane (25 mL) and stirred at 160-163 °C for 3 h. After the reaction mixture was cooled to room temperature, silica gel was added to absorb all of the sulfolane. The silica gel was loaded in a solid state, and the residue was purified by silica gel column chromatography (0-5% MeOH in DCM) to obtain the title compound (5.43 g, 71%) as a yellow solid.
LC-MS, *m*/*z* 214 [M+H]⁺

### 1.2 Preparation of methyl 8-cyclopentyl-7H-purine-6-carboxylate

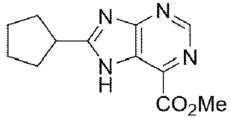

8-Cyclopentyl-7H-purine-6-carbonitrile (5.40 g, 25.3 mmol) obtained in the above Preparation example 1.1 was dissolved in MeOH (220 mL), and TMSCl (chlorotrimethylsilane, 33.5 mL) was added. The mixture was stirred at 70°C for 4.5 hours and quenched with saturated NaHCO₃ aqueous solution. The aqueous layer was extracted with DCM, and the organic layer was separated and dried over MgSO₄. After concentration, the obtained residue was purified by silica gel column chromatography (0-5% MeOH in DCM) to obtain the title compound (3.66 g, 59%) as a white solid.
LC-MS, *m*/*z* 247 [M+H]⁺

### 1.3. Preparation of 8-cyclopentyl-7H-purine-6-carboxylic acid

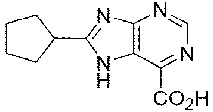

Methyl 8-cyclopentyl-7H-purine-6-carboxylate (3.65 g, 14.8 mmol) obtained in the above Preparation example 1.2 was dissolved in MeOH (111 mL), 2 M NaOH (47.0 mL) was added, and the mixture was stirred at room temperature. After 3.5 hours, the reaction mixture was acidified with 1 M HCl (~pH 2), and the resulting solid was filtered and washed with hexane. The solid was dried under vacuum to obtain the title compound (3.02 g, 88%) as a white solid.
¹H NMR (400 MHz, CD₃OD) *δ* 9.00 (s, 1H), 3.58 - 3.44 (m, 1H), 2.26 - 2.16 (m, 2H), 2.09 - 1.98 (m, 2H), 1.98 - 1.88 (m, 2H), 1.85 - 1.72 (m, 2H).
LC-MS, *m*/*z* 233 [M+H]⁺

### Example 1

### 1.1 3-Fluoro-5-(1-(trifluoromethyl)-1H-pyrazol-4-yl)benzonitrile

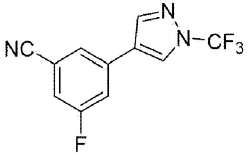

3-Bromo-5-fluorobenzonitrile (200 mg, 1.00 mmol), 4-(4,4,5,5-tetramethyl-1,3,2-dioxaborolan-2-yl)-1-(trifluoromethyl)-1H-pyrazole (393 mg, 1.50 mm), and Pd(dppf)Cl₂ ([1,1'-Bis(diphenylphosphino)ferrocene]dichloropalladium(II), 36.6 mg, 0.05 mmol) were dissolved in dioxane (3.4 mL), and 2 M K₂CO₃ (1.25 mL) was added and degassed with N₂ for 10 minutes The reaction mixture was stirred at 80°C for 2 hours and then cooled to room temperature. The reaction mixture was extracted with EtOAc, and the organic layer was dried over MgSO₄ and concentrated. The obtained residue was purified by silica gel column chromatography (0-5% MeOH in CH₂Cl₂) to obtain the title compound (100 mg, 39%) as a white solid.
LC-MS, *m*/*z* 256 [M+H]⁺

### 1.2 (3-Fluoro-5-(1-(trifluoromethyl)-1H-pyrazol-4-yl)phenyl)methanamine

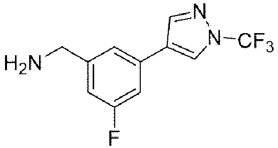

3-Fluoro-5-(1-(trifluoromethyl)-1*H*-pyrazol-4-yl)benzonitrile (90 mg, 0.35 mmol) obtained in the above Example 1.1 was dissolved in THF (5 mL), cooled to 0°C, LAH solution (2.0 M in THF; 0.35 mL) was added, and stirred at room temperature for 2 hours. The reaction mixture was cooled to 0°C, quenched by adding H₂O (2 mL), and adjusted to pH 10 with 2 M NaOH solution. EtOAc (5 mL) was added, stirred for 1 hour, and filtered to remove the solid. The filtrate was concentrated, dissolved again in EtOAc, dried over MgSO₄, and the organic layer was concentrated. The obtained residue was purified by silica gel column chromatography (0-10% MeOH in CH₂Cl₂) to obtain the title compound (44 mg, 49%) as an oil.
LC-MS, *m*/*z* 260 [M+H]⁺

### 1.3 8-Cyclopentyl-N-(3-fluoro-5-(1-(trifluoromethyl)-1H-pyrazol-4-yl)benzyl)-7H-purine-6-carboxamide

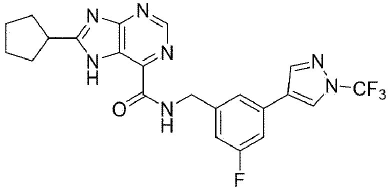

8-Cyclopentyl-7H-purine-6-carboxylic acid (15 mg) obtained in the above Preparation example 1.3 0.07 mmol), (3-Fluoro-5-(1-(trifluoromethyl)-1H-pyrazol-4-yl)phenyl)methanamine (17 mg, 0.07 mmol) and T3P (Propylphosphonic anhydride, 50 wt.% solution in EtOAc, 128 mg) were dissolved in ethyl acetate (3 mL), DIPEA (N,N-Diisopropylethylamine, 30 µL) was added, and the mixture was stirred at 50°C. After 12 h, ethyl acetate and H₂O were added to the reaction mixture, extracted, and the organic layer was dried over Na₂SO₄ and concentrated. The obtained residue was purified by silica gel column chromatography (0-5% MeOH in DCM) to obtain the title compound (25 mg, 81%) as a white solid.
¹H NMR (400 MHz, CD₃OD) *δ* 8.95 (s, 1H), 8.62 (s, 1H), 8.22 (s, 1H), 7.53 (s, 1H), 7.34 (d, *J* = 12 Hz, 1H), 7.11 (d, *J* = 8.0 Hz, 1H), 4.70 (s, 2H), 3.50 - 3.45 (m, 1H), 2.18 (m, 2H), 2.03 - 2.01 (m, 2H), 1.91 (m, 2H), 1.77 - 1.74 (m, 2H).
LC-MS, *m*/*z* 474 [M+H]⁺

### Example 2

### 2.1 3-(1-(2,2-Difluoroethyl)-1H-pyrazol-4-yl)-5-fluorobenzonitrile

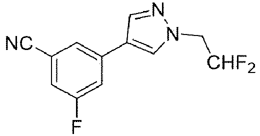

By the same method as in Example 1.1, 3-bromo-5-fluorobenzonitrile (200 mg, 1.00 mmol), 1-(2,2-difluoroethyl)-4-(4,4,5,5-tetramethyl-1,3,2-dioxaborolan-2-yl)-1H-pyrazole (387 mg, 1.50 mmol), Pd(dppf)Cl₂ (([1,1'-Bis(diphenylphosphino)ferrocene]dichloropalladium(II), 36.6 mg, 0.05 mmol), 2M K₂CO₃ (1.25 mL) and dioxane (3.4 mL) were used to obtain the title compound (251 mg, 100%) as a white solid.
LC-MS, *m*/*z* 252 [M+H]⁺

### 2.2 (3-(1-(2,2-Difluoroethyl)-1H-pyrazol-4-yl)-5-fluorophenyl)methanamine

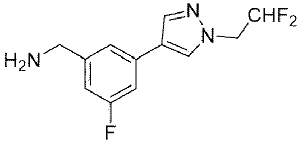

By the same method as in Example 1.2, 3-(1-(2,2-difluoroethyl)-1H-pyrazol-4-yl)-5-fluorobenzonitrile (251 mg, 1.00 mmol) and LAH solution (2.0 M in THF; 1.0 mL) were used to obtain the title compound (193 mg, 76%) as a yellow syrup.
LC-MS, *m*/*z* 256 [M+H]⁺

### 2.3 8-Cyclopentyl-N-(3-(1-(2,2-difluoroethyl)-1H-pyrazol-4-yl)-5-fluorobenzyl)-7H-purine-6-carboxamide

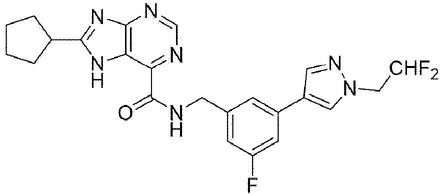

By the same method as in Example 1.3, 8-cyclopentyl-7H-purine-6-carboxylic acid (15.0 mg, 0.07 mmol), (3-(1-(2,2-difluoroethyl)-1H-pyrazol-4-yl)-5-fluorophenyl)methanamine (16.6 mg, 0.07 mmol), T3P (50 wt.% solution in EtOAc, 355 mg) and DIPEA (76 µL) were used to obtain the title compound (3.4 mg, 11%) as a white solid.
¹H NMR (400 MHz, CDCl₃) *δ* 10.38 (s, 1H), 9.03 (s, 1H), 8.45 (s, 1H), 7.82 (s, 1H), 7.72 (s, 1H), 7.11 (d, *J* = 9.6 Hz, 1H), 6.97 (d, *J* = 9.0 Hz, 1H), 6.29 - 5.92 (m, 1H), 4.71 (d, *J* = 6.2 Hz, 2H), 4.49 (td, *J =* 13.5, 4.1 Hz, 2H), 3.49 - 3.37 (m, 1H), 2.30 - 2.17 (m, 2H), 2.11 - 2.00 (m, 2H), 1.97 - 1.85 (m, 2H), 1.83 - 1.72 (m, 2H).
LC-MS, *m*/*z* 470 [M+H]⁺

### Example 3

### 3.1 3-Fluoro-5-(1-(2,2,2-trifluoroethyl)-1H-pyrazol-4-yl)benzonitrile

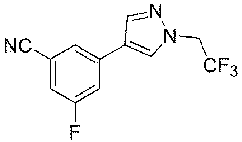

By the same method as in Example 1.1, 3-bromo-5-fluorobenzonitrile (200 mg, 1.00 mmol), 4-(4,4,5,5-tetramethyl-1,3,2-dioxaborolan-2-yl)-1-(2,2,2-trifluoroethyl)-1H-pyrazole (414 mg, 1.50 mmol), Pd(dppf)Cl₂ (36.6 mg, 0.05 mmol), 2M K₂CO₃ (1.25 mL) and dioxane (3.4 mL) were used to obtain the title compound (269 mg, 100%) as a white solid.
LC-MS, *m*/*z* 270 [M+H]⁺

### 3.2 (3-Fluoro-5-(1-(2,2,2-trifluoroethyl)-1H-pyrazol-4-yl)phenyl)methanamine

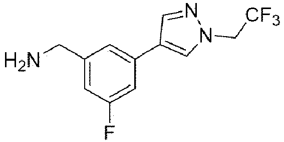

By the same method as in Example 1.2, 3-fluoro-5-(1-(2,2,2-trifluoroethyl)-1H-pyrazol-4-yl)benzonitrile (269 mg, 1.00 mmol) and LAH solution (2.0 M in THF; 1.0 mL) were used to obtain the title compound (222 mg, 81%) as a yellowish syrup.
LC-MS, *m*/*z* 274 [M+H]⁺

### 3.3 8-Cyclopentyl-N-(3-fluoro-5-(1-(2,2,2-trifluoroethyl)-1H-pyrazol-4-yl)benzyl)-7H-purine-6-carboxamide

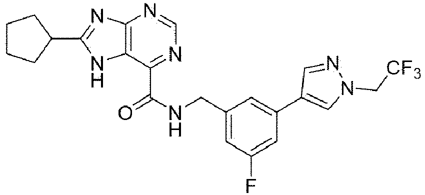

By the same method as in Example 1.3, 8-cyclopentyl-7H-purine-6- carboxylic acid (15.0 mg, 0.07 mmol), (3-fluoro-5-(1-(2,2,2-trifluoroethyl)-1H-pyrazol-4-yl)phenyl)methanamine (17.8 mg, 0.07 mmol), T3P (50 wt.% solution in EtOAc, 128 mg) and DIPEA (30 µL) were used to obtain the title compound (8.7 mg, 27%) as a white solid.
¹H NMR (400 MHz, CDCl₃) *δ* 10.35 (s, 1H), 9.03 (s, 1H), 8.45 (s, 1H), 7.84 (s, 1H), 7.75 (s, 1H), 7.26 (s, 1H), 7.12 (d, *J* = 9.1 Hz, 1H), 6.99 (d, *J* = 8.8 Hz, 1H), 4.76 - 4.69 (m, 4H), 3.47 - 3.38 (m, 1H), 2.29 - 2.19 (m, 2H), 2.11 - 2.00 (m, 2H), 1.97 - 1.85 (m, 2H), 1.83 - 1.71 (m, 2H).
LC-MS, *m*/*z* 488 [M+H]⁺

### Example 4

### 4.1 (S)-tert-butyl (1-(3-(1-methyl-1H-pyrazol-4-yl)phenyl)ethyl)carbamate

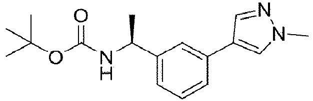

(S)-tert-butyl (1-(3-bromophenyl)ethyl)carbamate (200 mg, 0.70 mmol) in 1,4-dioxane (3 mL) was added 1-methyl-4-(4,4,5,5-tetramethyl-1,3,2-dioxaborolan-2-yl)-1H-pyrazole (220 mg, 1.0 mmol) and 2 M K₂CO₃ aqueous solution (1.2 mL, 2.4 mmol). The mixture was degassed with N₂ gas for 10 min, PdCl₂ (dppf) (26 mg, 0.035 mmol) was added, and the reaction was performed at 80 °C. After 3 h, the reaction solution was diluted with H₂O, extracted three times with EtOAc, and the organic layer was dried over MgSO₄ and concentrated. The obtained residue was purified by silica gel column chromatography (0-3% MeOH in DCM) to obtain the title compound (60 mg, 30%) as a light-colored solid.
LC-MS, *m*/*z* 302 [M+H]⁺

### 4.2 (S)-1-(3-(1-methyl-1H-pyrazol-4-yl)phenyl)ethan-1-amine

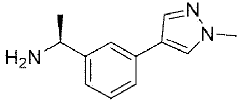

To a solution of 1-(3-(1-methyl-1H-pyrazol-4-yl)phenyl)ethyl)carbamate(60 mg, 0.20 mmol) in DCM(1 mL) was added dropwise TFA(0.2 mL) at 0 °C. The reaction temperature was gradually raised to room temperature and stirred for 1 hours. After completion of the reaction, the mixture was quenched with saturated aq. Na₂CO₃ and extracted three times with DCM (1% MeOH). The filtrate was concentrated and purified by silica gel column chromatography (0-3% MeOH in DCM) to obtain the title compound (20 mg, 50%).

¹H NMR (400 MHz, CDCl₃) *δ* 7.77 (s, 1H), 7.63 (s, 1H), 7.47 (s, 1H), 7.33 (dt, *J =* 15.2, 7.6 Hz, 2H), 7.20 (d, *J =* 7.0 Hz, 1H), 4.15 (q, *J =* 6.6 Hz, 1H), 3.94 (s, 3H), 1.43 (d, *J =* 6.6 Hz, 3H).

### 4.3 (S)-8-cyclopentyl-N-(1-(3-(1-methyl-1H-pyrazol-4-yl)phenyl)ethyl)-7H-purine-6-carboxamide

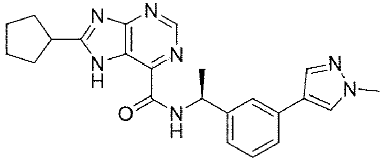

By the same method as in Example 1.3, (S)-1-(3-(1-methyl-1H-pyrazol-4-yl)phenyl)ethanamine (10 mg, 0.050 mmol), 8-cyclopentyl-7H-purine-6-carboxylic acid (12 mg, 0.050 mmol), T3P (50 wt.% solution in EtOAc, 89 µL, 0.15 mmol), and DIPEA (34 µL, 0.20 mmol) were used to obtain the title compound (13 mg, 63%).
¹H NMR (400 MHz, DMSO-*d*₆) *δ* 13.12 (s, 1H), 9.38 (s, 1H), 8.94 (s, 1H), 8.07 (s, 1H), 7.81 (s, 1H), 7.66 (s, 1H), 7.39 (s, 1H), 7.27 (d, *J =* 6.4 Hz, 2H), 5.23 - 5.17 (m, 1H), 3.83 (s, 3H), 3.45 (s, 1H), 2.04 (s, 2H), 1.90 (s, 2H), 1.77 (s, 2H), 1.62 (d, *J =* 4.8 Hz, 2H), 1.58 (d, *J* = 6.8 Hz, 3H).
LC-MS, *m*/*z* 416 [M+H]⁺

### Example 5

### 5.1 N-(3-cyano-5-fluorophenyl)-1-methyl-1H-pyrazole-4-carboxamide

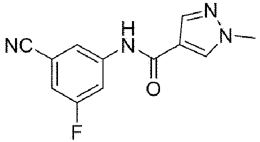

3-Amino-5-fluorobenzonitrile (119 mg, 0.87 mmol), 1-methyl-1H-pyrazole-4-carboxylic acid (100 mg, 0.79 mmol) and T3P (50 wt.% solution in EtOAc, 1.52 g, 2.39 mmol) were dissolved in ethyl acetate (15 mL), DIPEA (333 µL, 1.91 mmol) was added and stirred at 50°C. After 12 h, ethyl acetate and H₂O were added to the reaction mixture, extracted, and the organic layer was dried over Na₂SO₄ and concentrated. The obtained residue was purified by silica gel column chromatography (0-5% MeOH in DCM) to obtain the title compound (85 mg, 44%) as a yellow solid.
LC-MS, *m*/*z* 245 [M+H]⁺

### 5.2 N-(3-(aminomethyl)-5-fluorophenyl)-1-methyl-1H-pyrazole-4-carboxamide

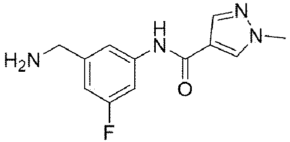

To a solution of N-(3-cyano-5-fluorophenyl)-1-methyl-1H-pyrazole-4-carboxamide (55 mg, 0.23 mmol) in EtOH (1 mL) was added a 10% palladium on carbon (Pd/C; 50 mg) and AcOH (50% in water; 0.5 mL). The mixture was stirred under an atmosphere of H₂ at room temperature overnight. The reaction mixture was filtered through a pad of Celite and concentrated to obtain the title compound (56 mg, crude).
LC-MS, *m*/*z* 249 [M+H]⁺

### 5.3 8-Cyclopentyl-N-(3-fluoro-5-(1-methyl-1H-pyrazole-4-carboxamido)benzyl)-7H-purine-6-carboxamide

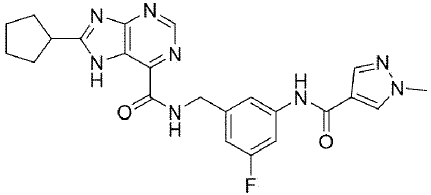

By the same method as in Example 1.3, N-(3-(aminomethyl)-5-fluorophenyl)-1-methyl-1H-pyrazole-4-carboxamide (22 mg, 0.086 mmol), 8-cyclopentyl-7H-purine-6-carboxylic acid (20 mg, 0.086 mmol), T3P (50 wt.% solution in EtOAc, 154 µL, 0.26 mmol), DIPEA (58 µL, 0.34 mmol) was used to obtain the title compound (5 mg, 13%).
¹H NMR (400 MHz, DMSO-*d*₆) *δ* 13.16 (s, 1H), 9.94 (s, 1H), 9.75 (s, 1H), 8.94 (s, 1H), 8.26 (s, 1H), 7.96 (s, 1H), 7.61 (d, *J=* 11.4 Hz, 1H), 7.40 (s, 1H), 6.89 (d, *J =* 9.8 Hz, 1H), 4.53 (d, *J =* 6.0 Hz, 2H), 3.86 (d, *J =* 7.8 Hz, 3H), 3.47 (s, 1H), 2.05 (m, 2H), 1.91 (m, 2H), 1.79 (m, 2H), 1.64 (m, 2H).
LC-MS, *m*/*z* 463 [M+H]⁺

### Example 6

### 6.1 tert-Butyl (1-(3-bromophenyl)cyclopropyl)carbamate

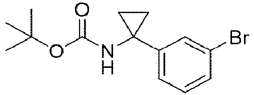

To a solution of 1-(3-Bromophenyl)cyclopropanamine (300 mg, 1.4 mmol) in THF (6 mL) was added Boc₂O (370 mg, 1.7 mmol) and Na₂CO₃ (300 mg, 2.8 mmol). The mixture was stirred at room temperature for 3 h. After the reaction was completed, the mixture was diluted with H₂O, extracted three times with DCM, and the organic layer was dried over MgSO₄ and concentrated. The obtained residue was purified by silica gel column chromatography (0-3% MeOH in DCM) to obtain the title compound (437 mg, 99%).
LC-MS, *m*/*z* 313 [M+H]⁺

### 6.2 tert-Butyl (1-(3-(1-methyl-1H-pyrazol-4-yl)phenyl)cyclopropyl)carbamate

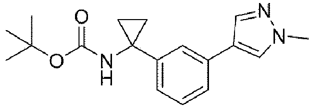

By the same method as in Example 4.1, tert-butyl (1-(3-bromophenyl)cyclopropyl)carbamate (430 mg, 1.4 mmol), 1-methyl-4- (4,4,5,5-tetramethyl-1,3,2-dioxaborolan-2-yl)-1H-pyrazole (430 mg, 2.1 mmol), 2 M K₂CO₃ aqueous solution (2.4 mL, 4.8 mmol), PdCl₂ (dppf) (50 mg, 0.069 mmol) and 1,4-dioxane (5 mL) were used to obtain the title compound (4 mg, 99%).
LC-MS, *m*/*z* 314 [M+H]⁺

### 6.3 1-(3-(1-Methyl-1H-pyrazol-4-yl)phenyl)cyclopropan-1-amine

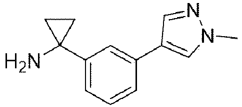

By the same method as in Example 4.2 above, tert-butyl (1-(3-(1-methyl-1H-pyrazol-4-yl)phenyl)cyclopropyl)carbamate (430 mg, 1.4 mmol) and TFA (2 mL) was used to obtain the title compound (318 mg, crude).
LC-MS, *m*/*z* 214 [M+H]⁺

### 6.4 8-Cyclopentyl-N-(1-(3-(1-methyl-1H-pyrazol-4-yl)phenyl)cyclopropyl)-7H-purine-6-carboxamide

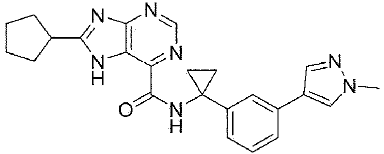

By the same method as in Example 1.3, 1-(3-(1-methyl-1H-pyrazol-4-yl)phenyl) cyclopropanamine (69 mg, 0.32 mmol), 8-cyclopentyl-7H-purine-6-carboxylic acid (50 mg, 0.22 mmol), T3P (50 wt.% solution in EtOAc, 380 µL, 0.65 mmol), DIPEA (150 µL, 0.86 mmol) and EtOAc (5 mL) were used to obtain the title compound (83 mg, 90%).
¹H NMR (400 MHz, DMSO-*d*₆) *δ* 13.16 (s, 1H), 9.84 (s, 1H), 8.96 (s, 1H), 8.08 (s, 1H), 7.80 (s, 1H), 7.43 (s, 1H), 7.36 (d, *J =* 7.6 Hz, 1H), 7.25 (t, *J =* 7.7 Hz, 1H), 7.18 (d, *J =* 7.8 Hz, 1H), 3.84 (s, 3H), 3.50 - 3.43 (m, 1H), 2.07 (d, *J =* 12.4 Hz, 2H), 1.92 (dd, *J =* 12.2, 7.4 Hz, 2H), 1.80 (s, 2H), 1.69 - 1.61 (m, 2H), 1.36 (s, 4H).
LC-MS, *m*/*z* 428 [M+H]⁺

### Example 7

### 7.1 3-Fluoro-5-(1-phenyl-1H-pyrazol-4-yl)benzonitrile

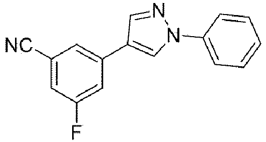

By the same method as in Example 4.1, 3-bromo-5-fluorobenzonitrile (148 mg, 0.74 mmol), 1-phenyl-4-(4,4,5,5-tetramethyl-1,3,2-dioxaborolan-2-yl)-1H-pyrazole (300 mg, 1.1 mmol), 2 M K₂CO₃ aqueous solution (1.3 mL, 2.6 mmol), PdCl₂ (dppf) (27 mg, 0.037 mmol) and 1,4- dioxane (3 mL) were used to obtain the title compound (194 mg, 99%).
LC-MS, *m*/*z* 264 [M+H]⁺

### 7.2 (3-Fluoro-5-(1-phenyl-1H-pyrazol-4-yl)phenyl)methanamine

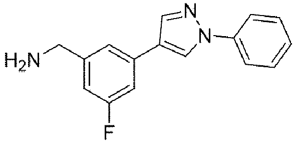

3-Fluoro-5-(1-phenyl-1H-pyrazol-4-yl)benzonitrile (100 mg, 0.38 mmol) was added to THF (3 mL), followed by LAH solution (2.0 M in THF, 380 µL, 0.76 mmol) at 0 °C. The mixture was stirred under an atmosphere of N₂ at room temperature for 1 h. After completion of the reaction, the mixture was quenched with 1 M NaOH (pH = 8~9), filtered through a Celite pad, and washed several times with EtOAc. The filtrate was dried over Na₂SO₄, and the organic layer was concentrated. The obtained residue was purified by silica gel column chromatography (0-5% MeOH in DCM) to obtain the title compound (70 mg, 70%).
LC-MS, *m*/*z* 268 [M+H]⁺

### 7.3 8-Cyclopentyl-N-(3-fluoro-5-(1-phenyl-1H-pyrazol-4-yl)benzyl)-7H-purine-6-carboxamide

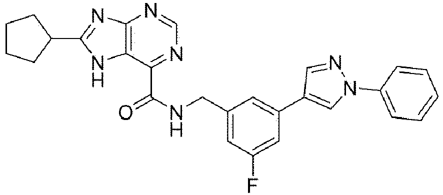

By the same method as in Example 1.3, (3-fluoro-5-(1-phenyl-1H-pyrazol-4-yl)phenyl)methanamine (10 mg, 0.037 mmol), 8-cyclopentyl-7H-purine-6-carboxylic acid (9 mg, 0.037 mmol), T3P (50 wt.% solution in EtOAc, 67 µL, 0.11 mmol), DIPEA (25 µL, 0.15 mmol) and EtOAc (1 mL) were used to obtain the title compound (13 mg, 72%).
¹H NMR (400 MHz, DMSO-*d*₆) *δ* 13.18 (s, 1H), 9.78 (s, 1H), 9.06 (s, 1H), 8.97 (s, 1H), 8.25 (s, 1H), 7.88 (d, *J =* 8.3 Hz, 2H), 7.60 (s, 1H), 7.55 - 7.48 (m, 3H), 7.34 (t, *J =* 7.2 Hz, 1H), 7.07 (d, *J =* 9.3 Hz, 1H), 4.61 (d, *J =* 5.8 Hz, 2H), 3.52 - 3.47 (m, 1H), 2.08 (d, *J =* 7.8 Hz, 2H), 1.94 (d, *J =* 7.7 Hz, 2H), 1.81 (s, 2H), 1.66 (s, 2H).
LC-MS, *m*/*z* 482 [M+H]⁺

### Example 8

### 8.1 3-Fluoro-5-((1-methyl-1H-pyrazol-4-yl)amino)benzonitrile

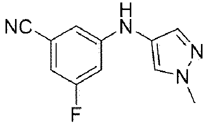

3-Bromo-5-fluorobenzonitrile (260 mg, 1.3 mmol), 1-methyl-1H-pyrazol-4-amine (100 mg, 1.0 mmol), BINAP (240 mg), Pd₂(dba)₃ (180 mg), and sodium tert-butoxide (190 mg) were added to toluene (4 mL) and stirred at 100 °C for 1 h. After the reaction was completed, the mixture was diluted with H₂O, extracted three times with EtOAc, and the organic layer was dried over Na₂SO₄ and concentrated. The obtained residue was purified by amino silica gel column chromatography (0-5% MeOH in DCM) to obtain the title compound (160 mg, 58%).
LC-MS, *m*/*z* 217 [M+H]⁺

### 8.2 N-(3-(aminomethyl)-5-fluorophenyl)-1-methyl-1H-pyrazol-4-amine

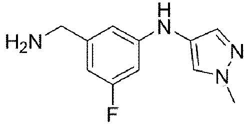

By the same method as in Example 7.2, 3-fluoro-5-((1-methyl-1H-pyrazol-4-yl)amino)benzonitrile (140 mg, 0.65 mmol), LAH solution (2.0 M in THF, 0.7 mL, 1.3 mmol) and THF (4 mL) were used to obtain the title compound (100 mg, 70%).
LC-MS, *m*/*z* 221 [M+H]⁺

### 8.3 8-Cyclopentyl-N-(3-fluoro-5-((1-methyl-1H-pyrazol-4-yl)amino)benzyl)-7H-purine-6-carboxamide

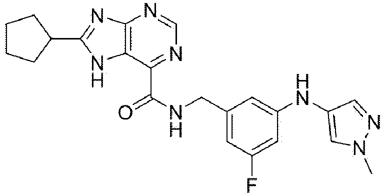

By the same method as in Example 1.3, N-(3-(aminomethyl)-5-fluorophenyl)-1-methyl-1H-pyrazol-4-amine (180 mg, 0.82 mmol), 8-cyclopentyl-7H-purine-6-carboxylic acid (190 mg, 0.82 mmol), T3P (50 wt.% solution in EtOAc, 1.5 mL, 2.5 mmol), DIPEA (560 µL, 3.3 mmol) and EtOAc (5 mL) were used to obtain the title compound (132 mg, 37%).
¹H NMR (400 MHz, DMSO-*d*₆) *δ* 13.15 (s, 1H), 9.64 (s, 1H), 8.93 (s, 1H), 7.77 (s, 1H), 7.59 (s, 1H), 7.25 (s, 1H), 6.56 (s, 1H), 6.38 (d, *J =* 9.4 Hz, 1H), 6.31 (d, *J =* 11.8 Hz, 1H), 4.40 (d, *J =* 6.3 Hz, 2H), 3.73 (s, 3H), 3.46 (s, 1H), 2.05 (d, *J =* 5.7 Hz, 2H), 1.93 - 1.87 (m, 2H), 1.77 (s, 2H), 1.67 - 1.60 (m, 2H).
LC-MS, *m*/*z* 435 [M+H]⁺

### Example 9

### 9.1 3-(1-Benzyl-1H-pyrazol-4-yl)-5-fluorobenzonitrile

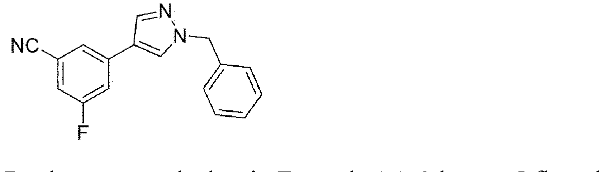

By the same method as in Example 1.1, 3-bromo-5-fluorobenzonitrile (200 mg, 1.00 mmol), 1-benzyl-4-(4,4,5,5-tetramethyl-1,3,2-dioxaborolan-2-yl)-1H-pyrazole (426 mg, 1.5 mmol), Pd(dppf)Cl₂ (36.6 mg, 0.05 mmol) and dioxane (3.4 mL) were used to obtain the title compound (276 mg, 99%) as a white solid.
LC-MS, *m*/*z* 277 [M+H]⁺

### 9.2 (3-(1-Benzyl-1H-pyrazol-4-yl)-S-fluorophenyl)methanamine

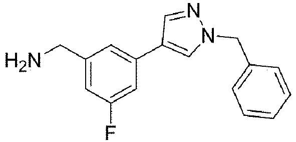

By the same method as in Example 1.2, 3-(1-benzyl-1H-pyrazol-4-yl)-5-fluorobenzonitrile (260 mg, 0.94 mmol), LAH solution (2.0 M in THF, 0.94 mL, 1.88 mmol), and THF (5 mL) were used to obtain the title compound (104 mg, 39%) as an oil.
LC-MS, *m*/*z* 282 [M+H]⁺

### 9.3 N-(3-(1-benzyl-1H-pyrazol-4-yl)-5-fluorobenzyl)-8-cyclopentyl-7H-purine-6-carboxamide

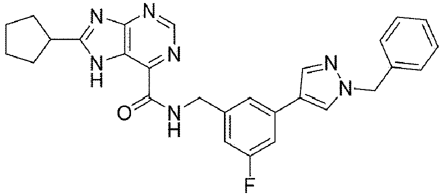

By the same method as in Example 1.3, using 8-cyclopentyl-7H-purine-6-carboxylic acid (14 mg, 0.06 mmol), (3-(1-benzyl-1H-pyrazol-4-yl)-5-fluorophenyl)methanamine (17 mg, 0.06 mmol), T3P (50 wt. % solution in EtOAc, 128 mg, 0.20 mmol), DIPEA (29 µL, 0.17 mmol) and ethyl acetate (3 mL), the title compound (17 mg, 57%) was obtained as a white solid.
¹H NMR (400 MHz, CD₃OD) *δ* 8.93 (s, 1H), 8.06 (s, 1H), 7.86 (s, 1H), 7.40 (s, 1H), 7.32 - 7.17 (m, 6H), 6.99 (d, *J* = 12 Hz, 1H), 5.32 (s, 2H), 4.65 (s, 2H), 3.48 - 3.44 (m, 1H), 2.17 (m, 2H), 2.00 (m, 2H), 1.89 (m, 2H), 1.74 (m, 2H).
LC-MS, *m*/*z* 496 [M+H]⁺

### Example 10

### 10.1 3-Fluoro-5-(pyridin-3-ylamino)benzonitrile

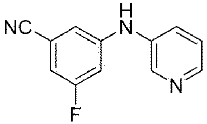

3-Bromo-5-fluorobenzonitrile (256 mg, 1.28 mmol), pyridin-3-amine (100 mg, 1.06 mmol), Xantphos (30 mg, 0.05 mmol), Pd₂(dba)₃ (15 mg, 0.016 mmol), and K₂CO₃ (318 mg, 2.3 mmol) were added to dioxane (5 mL) and stirred at 80°C. After 12 h, ethyl acetate and H₂O were added to the reaction mixture, extracted, and the organic layer was dried over Na₂SO₄ and concentrated. The obtained residue was purified by silica gel column chromatography (0-5% MeOH in DCM) to obtain the title compound (190 mg, 89%) as a white solid.
LC-MS, *m*/*z* 214 [M+H]⁺

### 10.2 N-(3-(aminomethyl)-5-fluorophenyl)pyridine-3-amine

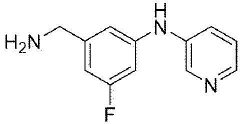

By the same method as in Example 1.2, 3-fluoro-5-(pyridin-3-ylamino)benzonitrile (180 mg, 0.83 mmol) and LAH solution (2.0 M in THF, 0.84 mL) were used to obtain the title compound (70 mg, 38%) as an oil.
LC-MS, *m*/*z* 218 [M+H]⁺

### 10.3 8-Cyclopentyl-N-(3-fluoro-5-(pyridin-3-ylamino)benzyl)-7H-purine-6-carboxamide

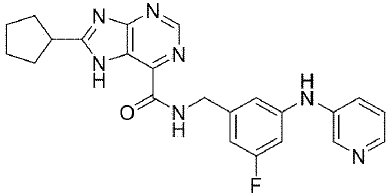

By the same method as in Example 1.3, using 8-cyclopentyl-7H-purine-6-carboxylic acid (42 mg, 0.18 mmol), N-(3-(aminomethyl)-5-fluorophenyl)pyridin-3-amine (40 mg, 0.18 mmol), T3P (50 wt.% solution in EtOAc, 355 mg, 0.56 mmol) and DIPEA (76 µL, 0.45 mmol), the title compound (25 mg, 32%) was obtained as a white solid.
¹H NMR (400 MHz, CD₃OD) *δ* 8.95 (s, 1H), 8.29 (s, 1H), 8.01 (s, 1H), 7.58 (d, *J =* 8.0 Hz, 1H), 7.24 (m, 1H), 6.96 (s, 1H), 6.69 (m, 2H), 4.61 (s, 2H), 3.48 - 3.44 (m, 1H), 2.18 (m, 2H), 2.01 (m, 2H), 1.91 (m, 2H), 1.77 (m, 2H).
LC-MS, *m*/*z* 432 [M+H]⁺

### Example 11

### 11.1 3-Fluoro-5-(1H-pyrazol-4-yl)benzonitrile

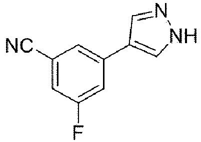

By the same method as in Example 1.1, 3-bromo-5-fluorobenzonitrile (1.3 g, 6.6 mmol), 4-(4,4,5,5-tetramethyl-1,3,2-dioxaborolan-2-yl)-1H-pyrazole (1.9 g, 10 mmol), 2 M K₂CO₃ aqueous solution (12 mL, 24 mmol), Pd(dppf)Cl₂ (240 mg, 0.33 mmol) and dioxane (24 mL) were used to obtain the title compound (360 mg, 29%).
LC-MS, *m*/*z* 188 [M+H]⁺

### 11.2 (3-Fluoro-5-(1H-pyrazol-4-yl)phenyl)methanamine

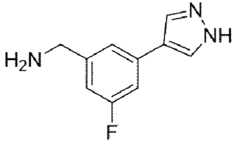

By the same method as in Example 7.2, 3-fluoro-5-(1H-pyrazol-4-yl)benzonitrile (247 mg, 1.3 mmol), LAH solution (2.0 M in THF, 1.3 mL, 2.6 mmol), and THF (4 mL) were used to obtain the title compound (364 mg, crude).
LC-MS, *m*/*z* 192 [M+H]⁺

### 11.3 8-Cyclopentyl-N-(3-fluoro-5-(1H-pyrazol-4-yl)benzyl)-7H-purine-6-carboxamide

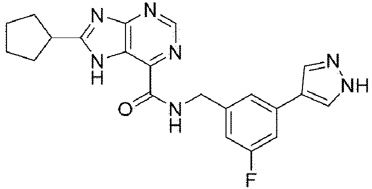

By the same method as in Example 1.3, (3-fluoro-5-(1H-pyrazol-4-yl)phenyl) methanamine (364 mg, crude), 8-cyclopentyl-7H-purine-6-carboxylic acid (364 mg, 1.6 mmol), T3P (50 wt.% solution in EtOAc, 2.8 mL, 4.8 mmol), DIPEA (1.0 mL, 6.3 mmol) and EtOAc (15 mL) were used to obtain the title compound (136 mg, 21%).
¹H NMR (400 MHz, DMSO-*d*₆) *δ* 13.14 (s, 1H), 12.98 (s, 1H), 9.72 (s, 1H), 8.94 (s, 1H), 8.21 (s, 1H), 7.92 (s, 1H), 7.45 (s, 1H), 7.33 (d, *J =* 9.8 Hz, 1H), 6.97 (d, *J =* 9.6 Hz, 1H), 4.55 (d, *J =* 6.2 Hz, 2H), 3.45 (d, *J=* 8.2 Hz, 1H), 2.05 (d, *J =* 8.3 Hz, 2H), 1.90 (dd, *J=* 12.4, 7.4 Hz, 2H), 1.78 (s, 2H), 1.68 - 1.60 (m, 2H).
LC-MS, *m*/*z* 406 [M+H]⁺

### Example 12

### 12.1 3-Fluoro-5-(1-(pyridin-4-yl)-1H-pyrazol-4-yl)benzonitrile

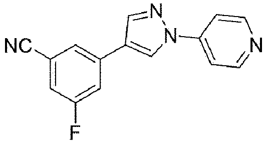

3-Fluoro-5-(1H-pyrazol-4-yl)benzonitrile (100 mg, 0.53 mmol), 4-iodopyridine (219 mg, 1.0 mmol), CuI (10 mg, 0.053 mmol), K₂CO₃ (220 mg, 1.6 mmol), and NMP (1 mL) were added in a sealed tube for microwave, and stirred at 150 °C for 4 h using a microwave. After confirming that compound 3-fluoro-5-(1H-pyrazol-4-yl)benzonitrile was completely consumed by LC-MS, the mixture was quenched with saturated NaHCO₃ aqueous solution, extracted three times with DCM (1% MeOH), and the organic layer was dried over Na₂SO₄ and concentrated. The obtained residue was purified by silica gel column chromatography (0-50% EtOAc in n-hexane) to obtain the title compound (41 mg, 29%).
¹H NMR (400 MHz, DMSO-*d*₆) *δ* 13.11 (s, 1H), 8.39 (s, 1H), 8.09 (s, 1H), 8.00 (s, 1H), 7.87 (d, *J =* 10.6 Hz, 1H), 7.58 (d, *J =* 8.1 Hz, 1H).
LC-MS, *m*/*z* 265 [M+H]⁺

### 12.2 (3-Fluoro-5-(1-(pyridin-4-yl)-1H-pyrazol-4-yl)phenyl)methanamine

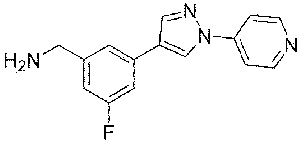

By the same method as in Example 7.2, using 3-fluoro-5-(1-(pyridin-4-yl)-1H-pyrazol-4-yl)benzonitrile (41 mg, 0.16 mmol), LAH solution (2.0 M in THF, 0.16 mL, 0.31 mmol) and THF (1 mL), the title compound (42 mg, crude) was obtained.
LC-MS, *m*/*z* 265 [M+H]⁺

### 12.3 8-Cyclopentyl-N-(3-fluoro-5-(1-(pyridin-4-yl)-1H-pyrazol-4-yl)benzyl)-7H-purine-6-carboxamide

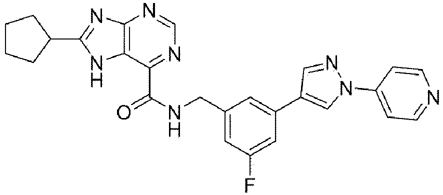

By the same method as in Example 1.3, (3-fluoro-5-(1-(pyridin-4-yl)-1H-pyrazol-4-yl)phenyl)methanamine (42 mg, crude), 8-cyclopentyl-7H-purine-6-carboxylic acid (36 mg, 0.16 mmol), T3P (50 wt.% solution in EtOAc, 280 µL, 0.47 mmol), DIPEA (110 µL, 0.63 mmol) and EtOAc (3 mL) were used to obtain the title compound (5 mg, 7%).
¹H NMR (400 MHz, DMSO-*d*₆) *δ* 13.16 (s, 1H), 9.80 (s, 1H), 9.25 (s, 1H), 8.93 (s, 1H), 8.66 (d, J = 6.2 Hz, 2H), 8.37 (s, 1H), 7.87 (d, J = 6.2 Hz, 2H), 7.60 (s, 1H), 7.49 (d, J = 9.6 Hz, 1H), 7.09 (d, J = 9.4 Hz, 1H), 4.60 (d, J = 6.4 Hz, 2H), 3.48 - 3.43 (m, 1H), 2.09 - 2.01 (m, 2H), 1.94 - 1.87 (m, 2H), 1.81 - 1.73 (m, 2H), 1.67 - 1.59 (m, 2H).
LC-MS, *m*/*z* 483 [M+H]⁺

### Example 13

### 8-Cyclopentyl-N-(3-fluorobenzyl)-7H-purine-6-carboxamide

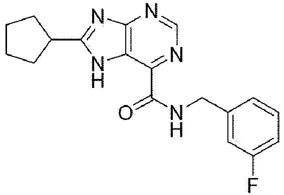

By the same method as in Example 1.3, 8-cyclopentyl-7H-purine-6-carboxylic acid (114 mg, 0.49 mmol), (3-fluorophenyl)methanamine (100 mg, 0.49 mmol), T3P (50 wt.% solution in EtOAc, 2.87 mmol), and DIPEA (206 µL, 1.18 mmol) were used to obtain the title compound (130 mg, 78%) as a white solid.
¹H NMR (400 MHz, CD₃OD) *δ* 8.95 (s, 1H), 7.36 (m, 1H), 7.22 (d, *J =* 8.0 Hz, 1H), 7.16 (d, *J =* 12 Hz, 1H), 6.99 (m, 1H), 4.68 (s, 2H), 3.49 (m, 1H), 2.19 (m, 2H), 2.03 (m, 2H), 1.92 (m, 2H), 1.77 (m, 2H).
LC-MS, *m*/*z* 340 [M+H]⁺

### Example 14

### N-(3-bromo-5-fluorobenzyl)-8-cyclopentyl-7H-purine-6-carboxamide

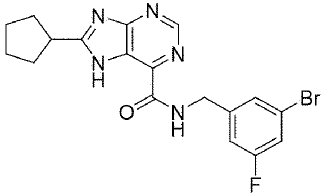

By the same method as in Example 1.3, 8-cyclopentyl-7H-purine-6-carboxylic acid (150 mg, 0.65 mmol), (3-bromo-5-fluorophenyl)methanamine (158 mg, 0.77 mmol), T3P (50 wt.% solution in EtOAc, 1.21 g, 3.81 mmol), and DIPEA (272 µL, 1.56 mmol) were used to obtain the title compound (250 mg, 92%) as a white solid.
¹H NMR (400 MHz, CD₃OD) *δ* 8.96 (s, 1H), 7.42 (m, 1H), 7.25 (d, *J* = 8.0 Hz, 1H), 7.18 (d, *J =* 12 Hz, 1H), 4.65 (s, 2H), 3.49 (m, 1H), 2.19 (m, 2H), 2.03 (m, 2H), 1.92 (m, 2H), 1.77 (m, 2H).
LC-MS, *m*/*z* 419 [M+H]⁺

### Example 15

### 15.1 3-Fluoro-5-(phenylamino)benzonitrile

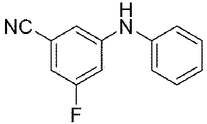

By the same method as in Example 10.1, 3-bromo-5-fluorobenzonitrile (256 mg, 1.28 mmol), aniline (100 mg, 1.07 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (200 mg, 94%) as a white solid.
LC-MS, *m*/*z* 213 [M+H]⁺

### 15.2 3-(Aminomethyl)-5-fluoro-N-phenylaniline

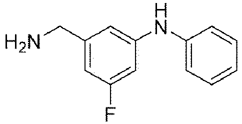

By the same method as in Example 1.2, 3-fluoro-5-(phenylamino)benzonitrile (200 mg, 0.94 mmol) and LAH solution (2.0 M in THF, 0.94 mL) were used to obtain the title compound (140 mg, 69%) as an oil.
LC-MS, *m*/*z* 217 [M+H]⁺

### 15.3 8-Cyclopentyl-N-(3-fluoro-5-(phenylamino)benzyl)-7H-purine-6-carboxamide

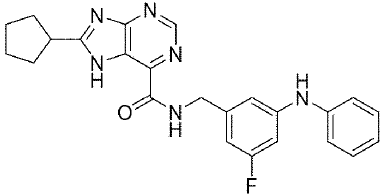

By the same method as in Example 1.3, 8-cyclopentyl-7H-purine-6-carboxylic acid (32 mg, 0.14 mmol), 3-(aminomethyl)-5-fluoro-N-phenylaniline (30 mg, 0.14 mmol), T3P (Propylphosphonic anhydride, 50 wt.% solution in EtOAc, 266 mg, 0.42 mmol) and DIPEA (N, N-Diisopropylethylamine, 57 µL, 0.34 mmol) were used to obtain the title compound (50 mg, 84%) as a white solid.
¹H NMR (400 MHz, CD₃OD) *δ* 8.95 (s, 1H), 7.19 (m, 2H), 7.08 (d, *J =* 8.0 Hz, 2H), 6.87 (m, 2H), 6.64 (d, *J =* 8.0 Hz, 1H), 6.56 (d, *J =* 12 Hz, 1H), 4.59 (s, 2H), 3.48 - 3.46 (m, 1H), 2.19 (m, 2H), 2.03 (m, 2H), 1.91 (m, 2H), 1.77 (m, 2H).
LC-MS, *m*/*z* 431 [M+H]⁺

### Example 16

### 16.1 3-Fluoro-5-(pyridin-4-ylamino)benzonitrile

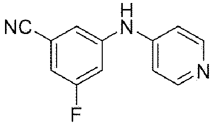

By the same method as in Example 10.1, 3-bromo-5-fluorobenzonitrile (256 mg, 1.28 mmol), pyridin-4-amine (100 mg, 1.07 mmol), Xantphos (30 mg, 0.05 mmol), Pd₂(dba)₃ (Tris(dibenzylideneacetone)dipalladium(0), 15 mg, 0.016 mmol), K₂CO₃ (318 mg, 2.3 mmol) and dioxane (5 mL) were used to obtain the title compound (182 mg, 85%) as a white solid.
LC-MS, *m*/*z* 214 [M+H]⁺

### 16.2 N-(3-(aminomethyl)-5-fluorophenyl)pyridine-4-amine

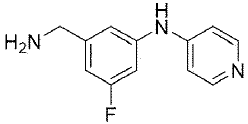

By the same method as in Example 1.2, 3-fluoro-5-(pyridin-4-ylamino)benzonitrile (160 mg, 0.75 mmol) and LAH solution (lithium aluminum hydride, 2.0 M in THF, 0.75 mL) were used to obtain the title compound (130 mg, 80%) as an oil.
LC-MS, *m*/*z* 218 [M+H]⁺

### 16.3 8-Cyclopentyl-N-(3-fluoro-5-(pyridin-4-ylamino)benzyl)-7H-purine-6-carboxamide

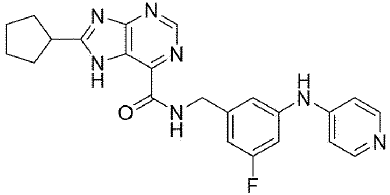

By the same method as in Example 1.3, 8-cyclopentyl-7H-purine-6-carboxylic acid (42 mg, 0.18 mmol), N-(3-(aminomethyl)-5-fluorophenyl)pyridin-4-amine (40 mg, 0.18 mmol), T3P (50 wt% solution in EtOAc, 355 mg, 0.56 mmol) and DIPEA (76 µL, 0.45 mmol) were used to obtain the title compound (40 mg, 51%) as a white solid.
¹H NMR (400 MHz, CD₃OD) *δ* 8.95 (s, 1H), 8.11 (d, *J =* 4.0 Hz, 2H), 7.09 (s, 1H), 6.98 (d, *J =* 8.0 Hz, 2H), 6.87 (d, *J =* 12 Hz, 2H), 4.66 (s, 2H), 3.48 - 3.46 (m, 1H), 2.19 (m, 2H), 2.01 (m, 2H), 1.91 (m, 2H), 1.77 (m, 2H).
LC-MS, *m*/*z* 432 [M+H]⁺

### Example 17

### 17.1 3-Fluoro-5-((3-(trifluoromethyl)phenyl)amino)benzonitrile

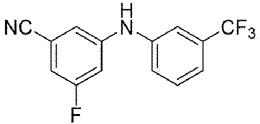

By the same method as in Example 10.1, 3-bromo-5-fluorobenzonitrile (256 mg, 1.28 mmol), 3-(trifluoromethyl)aniline (177 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (80 mg, 29%) as a white solid.
LC-MS, *m*/*z* 281 [M+H]⁺

### 17.2 3-(Aminomethyl)-5-fluoro-N-(3-(trifluoromethyl)phenyl)aniline

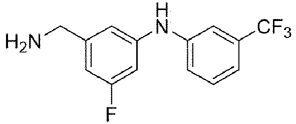

By the same method as in Example 1.2, 3-fluoro-5-((3-(trifluoromethyl)phenyl)amino)benzonitrile (80 mg, 0.29 mmol) and LAH solution (2.0 M in THF, 0.29 mL) were used to obtain the title compound (50 mg, 62%) as an oil.
LC-MS, *m*/*z* 285 [M+H]⁺

### 17.3 8-Cyclopentyl-N-(3-fluoro-5-((3-(trifluoromethyl)phenyl)amino)benzyl)-7H-purine-6-carboxamide

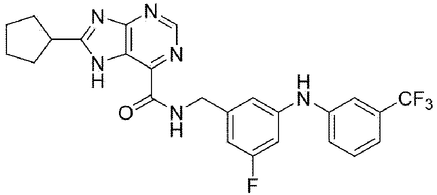

By the same method as in Example 1.3, using 8-cyclopentyl-7H-purine-6-carboxylic acid (37 mg, 0.16 mmol), 3-(aminomethyl)-5-fluoro-N-(3-(trifluoromethyl)phenyl)aniline (45 mg, 0.16 mmol), T3P (50 wt% solution in EtOAc, 305 mg, 0.48 mmol) and DIPEA (66 µL, 0.39 mmol), the title compound (30 mg, 37%) was obtained as a white solid.
¹H NMR (400 MHz, CD₃OD) *δ* 8.95 (s, 1H), 7.33 - 7.26 (m, 3H), 7.08 (d, *J =* 8.0 Hz, 1H), 6.96 (s, 1H), 6.70 - 6.66 (m, 2H), 4.62 (s, 2H), 3.50 - 3.46 (m, 1H), 2.19 (m, 2H), 2.02 (m, 2H), 1.92 (m, 2H), 1.77 (m, 2H).
LC-MS, *m*/*z* 499 [M+H]⁺

### Example 18

### 18.1 3-((4-(Dimethylamino)phenyl)amino)-5-fluorobenzonitrile

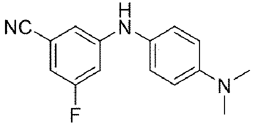

By the same method as in Example 10.1, 3-bromo-5-fluorobenzonitrile (256 mg, 1.28 mmol), N1,N1-dimethylbenzene-1,4-diamine (150 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (210 mg, 82%) as a white solid.
LC-MS, *m*/*z* 256 [M+H]⁺

### 18.2 N-1-(3-(aminomethyl)-5-fluorophenyl)-N4,N4-dimethylbenzene-1,4-diamine

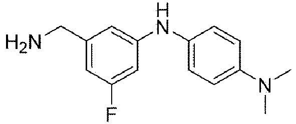

By the same method as in Example 1.2, 3-((4-(dimethylamino)phenyl)amino)-5-fluorobenzonitrile (100 mg, 0.39 mmol) and LAH solution (lithium aluminum hydride, 2.0 M in THF, 0.39 mL) were used to obtain the title compound (80 mg, 79%) as an oil.
LC-MS, *m*/*z* 260 [M+H]⁺

### 18.3 8-Cyclopentyl-N-(3-((4-(dimethylamino)phenyl)amino)-5-fluorobenzyl)-7H-purine-6-carboxamide

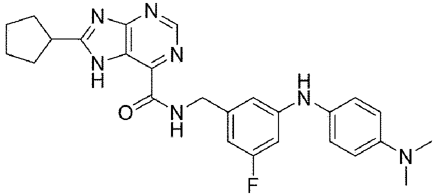

By the same method as in Example 1.3, using 8-cyclopentyl-7H-purine-6-carboxylic acid (35 mg, 0.15 mmol), N1-(3-(aminomethyl)-5-fluorophenyl)-N4,N4-dimethylbenzene-1,4-diamine (40 mg, 0.15 mmol), T3P (50 wt. % solution in EtOAc, 305 mg, 0.48 mmol), and DIPEA (N,N-Diisopropylethylamine, 66 µL, 0.39 mmol), the title compound (31 mg, 44%) was obtained as a white solid.
¹H NMR (400 MHz, CD₃OD) *δ* 8.93 (s, 1H), 7.97 (s, 1H), 6.96 (m, 2H), 6.66 (m, 2H), 6.40 (m, 2H), 4.53 (s, 2H), 3.46 - 3.44 (m, 1H), 2.98 (s, 3H), 2.85 (s, 3H), 2.19 (m, 2H), 2.01 (m, 2H), 1.90 (m, 2H), 1.76 (m, 2H).
LC-MS, *m*/*z* 474 [M+H]⁺

### Example 19

### 19.1 3-Fluoro-5-((4-(methylsulfonyl)phenyl)amino)benzonitrile

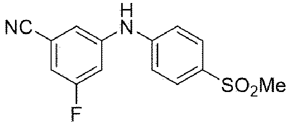

By the same method as in Example 10.1, 3-bromo-5-fluorobenzonitrile (256 mg, 1.28 mmol), 4-(methylsulfonyl)aniline (188 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (280 mg, 96%) as a white solid.
LC-MS, *m*/*z* 291 [M+H]⁺

### 19.2 3-(Aminomethyl)-5-fluoro-N-(4-(methylsulfonyl)phenyl)aniline

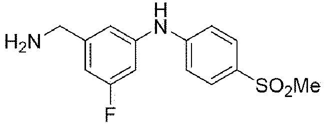

By the same method as in Example 1.2, 3-fluoro-5-((4-(methylsulfonyl)phenyl)amino)benzonitrile (100 mg, 0.34 mmol) and LAH solution (2.0 M in THF, 0.34 mL) were used to obtain the title compound (72 mg, 82%) as an oil.
LC-MS, *m*/*z* 295 [M+H]⁺

### 19.3 8-Cyclopentyl-N-(3-fluoro-5-((4-(methylsulfonyl)phenyl)amino)benzyl)-7H-purine-6-carboxamide

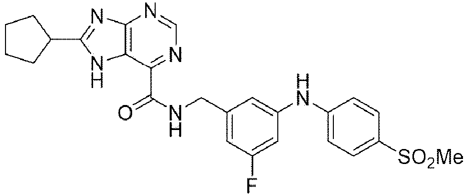

By the same method as in Example 1.3, using 8-cyclopentyl-7H-purine-6-carboxylic acid (35 mg, 0.15 mmol), 3-(aminomethyl)-5-fluoro-N-(4-(methylsulfonyl)phenyl)aniline (40 mg, 0.14 mmol), T3P (50 wt. % solution in EtOAc, 305 mg, 0.48 mmol) and DIPEA (66 µL, 0.39 mmol), the title compound (32 mg, 45%) was obtained as a white solid.
¹H NMR (400 MHz, CD₃OD) *δ* 8.94 (s, 1H), 7.69 (d, *J =* 8 Hz, 2H), 7.17 (d, *J =* 8 Hz, 2H), 7.05 (s, 1H), 6.78 (m, 2H), 4.63 (s, 2H), 3.49 - 3.45 (m, 1H), 3.02 (s, 3H), 2.18 (m, 2H), 2.00 (m, 2H), 1.90 (m, 2H), 1.76 (m, 2H).
LC-MS, *m*/*z* 509 [M+H]⁺

### Example 20

### 20.1 3-((4-Chloro-2-fluorophenyl)amino)-5-fluorobenzonitrile

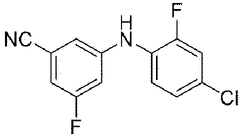

By the same method as in Example 10.1, 3-bromo-5-fluorobenzonitrile (256 mg, 1.28 mmol), 4-chloro-2-fluoroaniline (160 mg, 1.1 mmol), Xantphos (30 mg, 0.05 mmol), Pd₂(dba) ₃ (15 mg, 0.016 mmol), K₂CO₃ (318 mg, 2.3 mmol) and dioxane (5 mL) were used to obtain the title compound (100 mg, 38%) as a white solid.
LC-MS, *m*/*z* 265 [M+H]⁺

### 20.2 N-(3-(aminomethyl)-5-fluorophenyl)-4-chloro-2-fluoroaniline

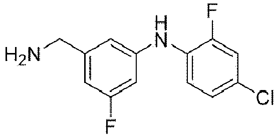

By the same method as in Example 1.2, 3-((4-chloro-2-fluorophenyl)amino)-5-fluorobenzonitrile (90 mg, 0.34 mmol) and LAH solution (2.0 M in THF, 0.34 mL) were used to obtain the title compound (50 mg, 55%) as an oil.
LC-MS, *m*/*z* 269 [M+H]⁺

### 20.3 N-(3-((4-chloro-2-fluorophenyl)amino)-5-fluorobenzyl)-8-cyclopentyl-7H-purine-6-carboxamide

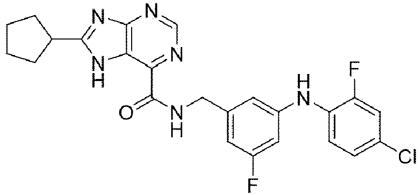

By the same method as in Example 1.3, using 8-cyclopentyl-7H-purine-6-carboxylic acid (35 mg, 0.15 mmol), N-(3-(aminomethyl)-5-fluorophenyl)-4-chloro-2-fluoroaniline (40 mg, 0.15 mmol), T3P (50 wt. % solution in EtOAc, 305 mg, 0.48 mmol) and DIPEA (66 µL, 0.39 mmol), the title compound (60 mg, 83%) was obtained as a white solid.
¹H NMR (400 MHz, CD₃OD) *δ* 8.92 (s, 1H), 7.24 (t, J = 8.0 Hz, 1H), 7.11 (d, J = 8.0 Hz, 1H), 6.96 (d, J = 8.0 Hz, 1H), 6.82 (s, 1H), 6.58 (m, 2H), 4.58 (s, 2H), 3.48 - 3.41 (m, 1H), 2.16 (m, 2H), 2.00 (m, 2H), 1.89 (m, 2H), 1.74 (m, 2H).
LC-MS, *m*/*z* 483 [M+H]⁺

### Example 21

### 21.1 3-Fluoro-5-((4-fluorophenyl)amino)benzonitrile

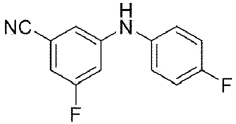

By the same method as in Example 10.1, 3-bromo-5-fluorobenzonitrile (256 mg, 1.28 mmol), 4-fluoroaniline (122 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (90 mg, 36%) as a white solid.
LC-MS, *m*/*z* 231 [M+H]⁺

### 21.2 3-(Aminomethyl)-5-fluoro-N-(4-fluorophenyl)aniline

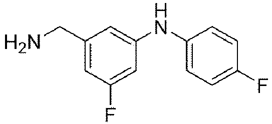

By the same method as in Example 1.2, 3-fluoro-5-((4-fluorophenyl)amino)benzonitrile (90 mg, 0.34 mmol) and LAH solution (2.0 M in THF, 0.34 mL) were used to obtain the title compound (20 mg, 22%) as an oil.
LC-MS, *m*/*z* 235 [M+H]⁺

### 21.3 8-Cyclopentyl-N-(3-fluoro-5-((4-fluorophenyl)amino)benzyl)-7H-purine-6-carboxamide

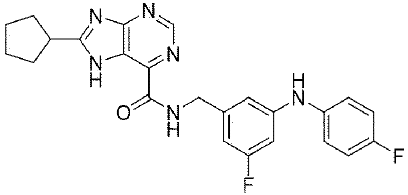

By the same method as in Example 1.3, 8-cyclopentyl-7H-purine-6-carboxylic acid (15 mg, 0.06 mmol), 3-(aminomethyl)-5-fluoro-N-(4-fluorophenyl)aniline (15 mg, 0.06 mmol), T3P (50 wt. % solution in EtOAc, 124 mg, 0.20 mmol) and DIPEA (27 µL, 0.16 mmol) were used to obtain the title compound (8 mg, 28%) as a viscous oil.
¹H NMR (400 MHz, CD₃OD) *δ* 8.95 (s, 1H), 7.07 (m, 2H), 6.93 (t, *J =* 8.0 Hz, 2H), 6.80 (s, 1H), 6.53 (t, *J =* 8.0 Hz, 2H) 4.58 (s, 2H), 3.50 - 3.46 (m, 1H), 2.19 (m, 2H), 2.01 (m, 2H), 1.92 (m, 2H), 1.77 (m, 2H).
LC-MS, *m*/*z* 449 [M+H]⁺

### Example 22

### 22.1 N-(3-bromo-5-fluorobenzyl)-8-cyclopentyl-7-((2-(trimethylsilyl)ethoxy)methyl)-7H-purine-6-carboxamide

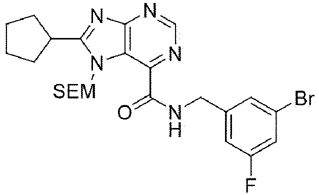

To a solution of N-(3-bromo-5-fluorobenzyl)-8-cyclopentyl-7H-purine-6-carboxamide (30 mg, 0.072 mmol) in THF (1 mL) was added NaH (60% dispersed in mineral oil) (4 mg, 0.11 mmol) at 0 °C, and the mixture was stirred at room temperature for 1 h. After cooling the reaction mixture to 0 °C, (2-(chloromethoxy)ethyl)trimethylsilane (19 µL, 0.11 mmol) was added, and the mixture was stirred at room temperature for 1 h. After the reaction was complete, the mixture was diluted with H₂O, extracted three times with EtOAc, and the organic layer was dried over MgSO₄ and concentrated. The obtained residue was purified by silica gel column chromatography (0-30% EtOAc in n-hexane) to obtain the title compound (25 mg, 64%).
¹H NMR (400 MHz, DMSO-*d*₆) *δ* 8.99 (s, 1H), 8.76 (s, 1H), 8.19 (s, 1H), 8.15 - 8.09 (m, 2H), 7.68 - 7.63 (m, 2H), 7.57 (s, 1H), 7.34 (d, J = 9.2 Hz, 1H), 7.12 (d, J = 9.7 Hz, 1H), 5.74 (s, 2H), 4.78 (s, 2H), 3.66 - 3.56 (m, 3H), 2.19 - 2.12 (m, 2H), 2.07 - 2.00 (m, 2H), 1.84 - 1.77 (m, 2H), 1.74 - 1.67 (m, 2H), 0.91 (t, J = 8.0 Hz, 2H), -0.07 (s, 9H).
LC-MS, *m*/*z* 637 [M+H]⁺

### 22.2 N-(3-(1-(3-cyanophenyl)-1H-pyrazol-4-yl)-5-fluorobenzyl)-8-cyclopentyl-7-((2-(trimethylsilyl)ethoxy)methyl)-7H-purine-6-carboxamide

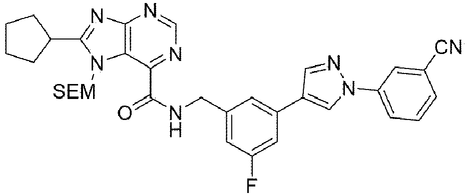

By the same method as in Example 4.1, using N-(3-bromo-5-fluorobenzyl)-8-cyclopentyl-7-((2-(trimethylsilyl)ethoxy)methyl)-7H-purine-6-carboxamide (20 mg, 0.037 mmol), 3-(4-(4,4,5,5-tetramethyl-1,3,2-dioxaborolan-2-yl)-1H-pyrazol-1-yl)benzonitrile (16 mg, 0.055 mmol), 2 M K₂CO₃ aqueous solution (0.07 mL, 0.128 mmol), PdCl₂ (dppf) (1 mg, 0.002 mmol) and 1,4-dioxane (1 mL), the title compound (12 mg, 52%) was obtained.
¹H NMR (400 MHz, CD₃OD) *δ* 8.99 (s, 1H), 8.76 (s, 1H), 8.19 (s, 1H), 8.15 - 8.09 (m, 2H), 7.68 - 7.63 (m, 2H), 7.57 (s, 1H), 7.34 (d, J = 9.2 Hz, 1H), 7.12 (d, J = 9.7 Hz, 1H), 5.74 (s, 2H), 4.78 (s, 2H), 3.66 - 3.56 (m, 3H), 2.19 - 2.12 (m, 2H), 2.07 - 2.00 (m, 2H), 1.84 - 1.77 (m, 2H), 1.74 - 1.67 (m, 2H), 0.91 (t, J = 8.0 Hz, 2H), -0.07 (s, 9H).
LC-MS, *m*/*z* 637 [M+H]⁺

### Example 23

### N-(3-(1-(3-cyanophenyl)-1H-pyrazol-4-yl)-5-fluorobenzyl)-8-cyclopentyl-7H-purine-6-carboxamide

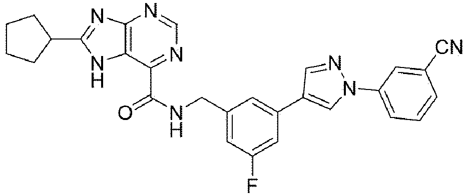

To a solution of N-(3-(1-(3-cyanophenyl)-1H-pyrazol-4-yl)-5-fluorobenzyl)-8-cyclopentyl-7-((2-(trimethylsilyl)ethoxy)methyl)-7H-purine-6-carboxamide (10 mg, 0.016 mmol) in EtOH (2 mL) was added 2N HCl aqueous solution (0.4 mL, 0.39 mmol), and the mixture was stirred at 80°C for 4 h. The reaction solution was neutralized with 2N aq. NaOH, and EtOH was evaporated. The reaction mixture was diluted with H₂O, extracted three times with EtOAc, and the organic layer was dried over MgSO₄ and concentrated. The obtained residue was purified by silica gel column chromatography (0-5% MeOH in DCM) to obtain the title compound (6 mg, 75%).
¹H NMR (400 MHz, DMSO-*d*₆) *δ* 13.15 (s, 1H), 9.76 (s, 1H), 9.18 (s, 1H), 8.95 (s, 1H), 8.33 (d, *J =* 9.9 Hz, 2H), 8.22 (d, *J =* 7.5 Hz, 1H), 7.80 - 7.71 (m, 2H), 7.58 (s, 1H), 7.46 (d, *J =* 10.1 Hz, 1H), 7.10 - 7.04 (m, 1H), 4.59 (d, *J =* 6.2 Hz, 2H), 3.51 - 3.44 (m, 1H), 2.05 (s, 2H), 1.91 (s, 2H), 1.78 (s, 2H), 1.64 (s, 2H).
LC-MS, *m*/*z* 507 [M+H]⁺

### Example 24

### 24.1 3-Fluoro-5-(1-(4-(methylsulfonyl)benzyl)-1H-pyrazol-4-yl)benzonitrile

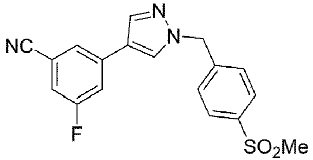

To a solution of 3-fluoro-5-(1H-pyrazol-4-yl)benzonitrile (65 mg, 0.35 mmol) in 1,4-dioxane (2 mL) was added NaH (60% dispersion in mineral oil) (17 mg, 0.70 mmol) at 0 °C, and the mixture was stirred at room temperature for 1 h. After cooling the reaction mixture to 0 °C, 1-(bromomethyl)-4-(methylsulfonyl)benzene (173 mg, 0.42 mmol) was added, and the mixture was stirred at room temperature overnight. After the reaction was complete, the mixture was diluted with H₂O, extracted three times with EtOAc, and the organic layer was dried over MgSO₄ and concentrated. The obtained residue was purified by silica gel column chromatography (0-50% EtOAc in n-hexane) to obtain the title compound (110 mg, 89%).
LC-MS, *m*/*z* 356 [M+H]⁺

### 24.2 (3-Fluoro-5-(1-(4-(methylsulfonyl)benzyl)-1H-pyrazol-4-yl)phenyl)methanamine

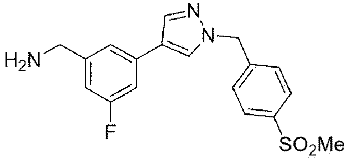

By the same method as in Example 7.2, 3-fluoro-5-(1-(4-(methylsulfonyl)benzyl)-1H-pyrazol-4-yl)benzonitrile (110 mg, 0.31 mmol), LAH solution (2.0 M in THF, 310 µL, 0.62 mmol) and THF (1 mL) were used to obtain the title compound (40 mg, 36%).
LC-MS, *m*/*z* 360 [M+H]⁺

### 24.3 8-Cyclopentyl-N-(3-fluoro-5-(1-(4-(methylsulfonyl)benzyl)-1H-pyrazol-4-yl)benzyl)-7H-purine-6-carboxamide

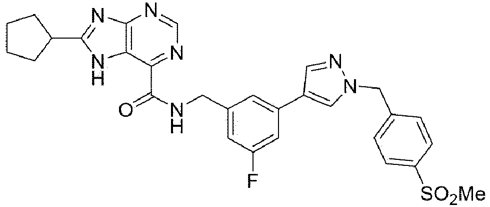

By the same method as in Example 1.3, 3-fluoro-5-(1-(4-(methylsulfonyl)benzyl)-1H-pyrazol-4-yl)benzonitrile (40 mg, 0.11 mmol), 8-cyclopentyl-7H-purine-6-carboxylic acid (22 mg, 0.093 mmol), T3P (50 wt.% solution in EtOAc, 830 µL, 0.28 mmol), DIPEA (40 µL, 0.23 mmol) and EtOAc (1 mL) were used to obtain the title compound (12 mg, 23%).
¹H NMR (400 MHz, DMSO-*d*₆) *δ* 13.15 (s, 1H), 9.74 (s, 1H), 8.94 (s, 1H), 8.36 (s, 1H), 7.97 (s, 1H), 7.89 (d, *J* = 8.2 Hz, 2H), 7.47 - 7.42 (m, 3H), 7.32 (d, *J =* 10.1 Hz, 1H), 7.00 (d, *J* = 9.2 Hz, 1H), 5.46 (s, 2H), 4.55 (d, *J=* 5.8 Hz, 2H), 3.46 (s, 1H), 3.17 (s, 3H), 2.05 (d, *J* = 10.3 Hz, 2H), 1.91 (d, *J* = 7.7 Hz, 2H), 1.78 (s, 2H), 1.63 (d, *J=* 5.1 Hz, 2H).
LC-MS, *m*/*z* 574 [M+H]⁺

### Example 25

### 25.1 tert-Butyl 3-bromo-5-fluorobenzylcarbamate

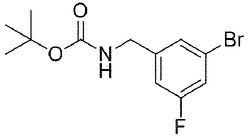

By the same method as in Example 6.1, (3-bromo-5-fluorophenyl)methanamine (2 g, 9.802 mmol), (Boc)₂O (30% in THF, 4.3 g, 19.6040 mmol), and DCM (20 mL) were used to obtain the title compound (2.95 g, 99%) as a colorless clear liquid.

¹H NMR (400 MHz, CDCl₃) *δ* 7.21 (s, 1H), 7.13 (d, *J =* 8 Hz, 1H), 6.94 (d, *J=* 8.8 Hz, 1H), 4.92 (broad, 1H), 4.29 (d, *J =* 5.6 Hz, 1H), 1.47 (s, 9H).

### 25.2 tert-Butyl (3-fluoro-5-(1H-pyrazol-4-yl)benzyl)carbamate

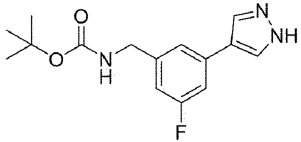

DMF (7.5 mL) and H₂O (3 mL) were added to tert-butyl 3-bromo-5-fluorobenzylcarbamate (0.5 g, 1.64 mmol), 4-(4,4,5,5-tetramethyl-1,3,2-dioxaborolan-2-yl)-1H-pyrazole (0.48 g, 2.47 mmol) and Na₂CO₃ (0.61 g, 5.85 mmol), and the mixture was degassed with N₂ for 10 min. Then, Pd(PPh₃)₄ (0.095 g, 0.082 mmol) was added, and the reaction mixture was stirred at 140 °C overnight. Ethyl acetate and H₂O were added to the reaction mixture, extracted, and the organic layer was dried over MgSO₄ and concentrated. The obtained residue was purified by silica gel column chromatography (0-65% EtOAc in n-hexane) to obtain the title compound (374 mg, 78%) as a pale yellow solid.
¹H NMR (400 MHz, CD₃OD) *δ* 7.98 (broad, 2H), 7.32 (s, 1H), 7.21 (d, *J* = 9.6 Hz, 1H), 6.85 (d, *J =* 9.2 Hz, 1H), 4.24 (s, 2H), 1.47 (s, 9H).
LC-MS, *m*/*z* 292 [M+H]⁺

### 25.3 tert-Butyl (3-fluoro-5-(1-(4-methoxyphenyl)-1H-pyrazol-4-yl)benzyl)carbamate

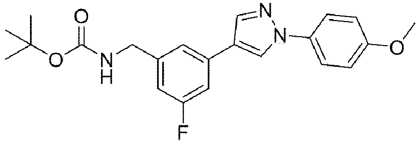

DMSO (1.5 mL) was added to tert-Butyl (3-fluoro-5-(1H-pyrazol-4-yl)benzyl)carbamate (50 mg, 0.17 mmol), 4-iodoanisole (60 mg, 0.26 mmol), CuI (3.3 mg, 0.017 mmol), L-proline (4 mg, 0.034 mmol) and K₂CO₃ (35.6 mg, 0.26 mmol), and the mixture was stirred at 110°C for 8 h. After completion of the reaction, H₂O was added to the reaction mixture, and the mixture was extracted three times with ethyl acetate. The organic layer was dried over MgSO₄ and concentrated. The obtained residue was purified by silica gel column chromatography (0-3% MeOH in DCM) to obtain the title compound (59.6 mg, 87%) as a light brown solid.

¹H NMR (400 MHz, CDCl₃) *δ* 8.06 (s, 1H), 7.94 (s, 1H), 7.62 (d, *J =* 8.8 Hz, 2H), 7.24 (s, 1H), 7.25 (s, 1H), 7.13 (d, *J =* 10 Hz, 1H), 7.0 (d, *J =* 8.8 Hz, 2H), 6.89 (d, *J =* 9.2 Hz, 2H), 4.34 (d, *J* = 5.2 Hz, 2H), 3.86 (s, 3H), 1.48 (s, 9H).

### 25.4 (3-Fluoro-5-(1-(4-methoxyphenyl)-1H-pyrazol-4-yl)phenyl)methanamine, trifluoroacetate

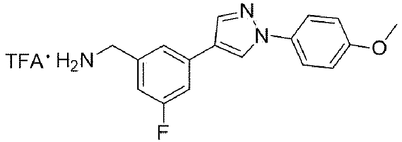

tert-Butyl (3-fluoro-5-(1-(4-methoxyphenyl)-1H-pyrazol-4-yl)benzyl)carbamate (57.5 mg, 0.14 mmol) was dissolved in DCM (4 mL), trifluoroacetic acid (1.2 mL) was added, and the mixture was stirred at room temperature for one hour. After completion of the reaction, the process of diluting with DCM and concentrating was repeated five times to obtain the title compound (56 mg, crude) as a dark brown viscous liquid.
LC-MS, *m*/*z* 298 [M+H]⁺

### 25.5 8-Cyclopentyl-N-(3-fluoro-5-(1-(4-methoxyphenyl)-1H-pyrazol-4-yl)benzyl)-7H-purine-6-carboxamide

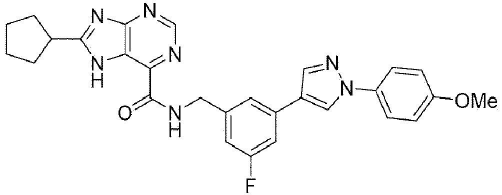

By the same method as in Example 1.3, using 8-cyclopentyl-7H-purine-6-carboxylic acid (33.6 mg, 0.14 mmol), (3-fluoro-5-(1-(4-methoxyphenyl)-1H-pyrazol-4-yl)phenyl)methanamine, trifluoroacetate (56 mg), T3P (50 wt. % solution in EtOAc, 138 mg, 0.43 mmol), DIPEA (110 µL) and DMF (2 mL), the title compound (27.1 mg, 37% yield in 2 steps) was obtained as a pale yellow foam.
¹H NMR (400 MHz, DMSO-*d*₆) *δ* 13.18 (1H, Broad), 9.77 (t, *J=* 6.8 Hz, 1H), 8.96 (d, *J =* 11.2 Hz, 2H), 8.19 (s, 1H), 7.78 (2H, *J* = 9.2 Hz, 2H), 7.58 (s, 1H), 7.47 (d, *J* = 9.6 Hz, 1H), 7.07 (m, 3H), 4.61 (d, *J =* 6.4 Hz, 2H), 3.49 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 512 [M+H]⁺

### Example 26

### 26.1 tert-Butyl (3-fluoro-5-(1-(4-(trifluoromethyl)phenyl)-1H-pyrazol-4-yl)benzyl)carbamate

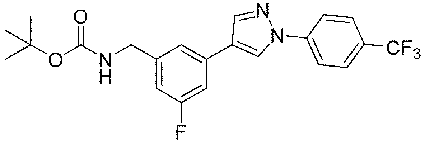

By the same method as in Example 25.3, using tert-butyl (3-fluoro-5-(1H-pyrazol-4-yl)benzyl)carbamate (50 mg, 0.17 mmol), 4-iodobenzotrifluoride (70 mg, 0.26 mmol), CuI (3.3 mg, 0.017 mmol), L-proline (4 mg, 0.034 mmol), K₂CO₃ (35.6 mg, 0.26 mmol) and DMSO (1.5 mL), the title compound (67 mg, 89%) was obtained as an off-white solid.

¹H NMR (400 MHz, CDCl₃) *δ* 8.21 (s, 1H), 8.00 (s, 1H), 7.87 (d, *J=* 8.4 Hz, 2H), 7.75 (d, *J =* 8.4 Hz, 2H), 7.26 (s, 1H), 7.14 (d, *J* = 9.6 Hz, 1H), 6.92 (d, *J =* 9.2 Hz, 1H), 4.96 (broad, 1H), 4.36 (d, *J* = 5.6 Hz, 2H), 1.48 (s, 9H).

### 26.2 (3-Fluoro-5-(1-(4-(trifluoromethyl)phenyl)-1H-pyrazol-4-yl)phenyl)methanamine, trifluoroacetate

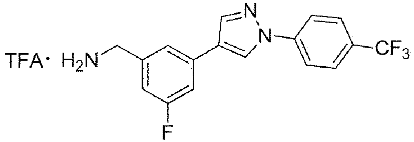

By the same method as in Example 25.4, tert-butyl (3-fluoro-5-(1-(4-(trifluoromethyl)phenyl)-1H-pyrazol-4-yl)benzyl)carbamate (62.9 mg, 0.14 mmol), trifluoroacetic acid (1.2 mL) and DCM (4 ml) were used to obtain the title compound (56 mg, crude) as a dark brown viscous liquid.
LC-MS, *m*/*z* 336 [M+H]⁺

### 26.3 8-Cyclopentyl-N-(3-fluoro-5-(1-(4-(trifluoromethyl)phenyl)-1H-pyrazol-4-yl)benzyl)-7H-purine-6-carboxamide

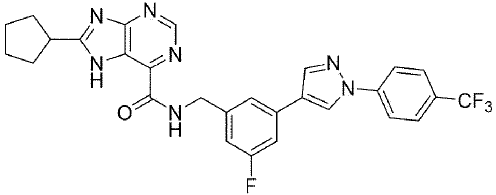

By the same method as in Example 1.3, using 8-cyclopentyl-7H-purine-6- carboxylic acid (34 mg, 0.14 mmol), (3-fluoro-5-(1-(4-(trifluoromethyl)phenyl)-1H-pyrazol-4-yl)phenyl)methanamine, trifluoroacetate (63 mg), T3P (50 wt.% solution in EtOAc, 138 mg, 0.43 mmol), DIPEA (110 µL) and DMF (2 mL), the title compound (31 mg, 39% yield in 2 steps) was obtained as a pale yellow foam.
¹H NMR (400 MHz, DMSO-*d*₆) *δ* 13.18 (1H, Broad), 9.79 (m, 1H), 9.23 (s, 1H), 8.98 (s, 1H), 8.35 (s, 1H), 8.12 (d, *J* = 8.4 Hz, 2H), 7.92 (d, *J* = 8.4 Hz, 2H), 7.62 (s, 1H), 7.52 (d, *J* = 10 Hz, 1H), 7.10 (d, *J =* 8.8 Hz, 1H), 4.62 (d, *J =* 6.0 Hz, 2H), 3.59 - 3.43 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 550 [M+H]⁺

### Example 27

### 27.1 tert-Butyl (3-fluoro-5-(1-(4-fluorophenyl)-1H-pyrazol-4-yl)benzyl)carbamate

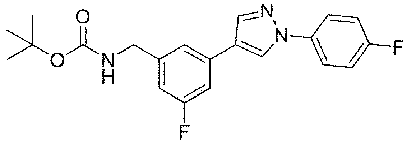

By the same method as in Example 25.3, using tert-butyl (3-fluoro-5-(1H-pyrazol-4-yl)benzyl)carbamate (300 mg, 1.03 mmol), 1-fluoro-4-iodobenzene (343 mg, 1.54 mmol), CuI (20 mg, 0.103 mmol), L-proline (24 mg, 0.206 mmol), K₂CO₃ (214 mg, 1.54 mmol) and DMSO (3 mL), the title compound (345 mg, 87%) was obtained as an off-white solid.
¹H NMR (400 MHz, CDCl₃) *δ* 8.09 (s, 1H), 7.96 (s, 1H), 7.69 (dd, *J=* 8.8, 4.4 Hz, 2H), 7.24 (s, 1H), 7.18 (t, *J =* 8 Hz, 2H), 7.13 (d, *J* = 9.6 Hz, 1H), 6.90 (d, *J =* 8.8 Hz, 1H), 4.93 (s, 1H), 4.35 (d, *J* = 5.6 Hz, 2H), 1.48 (s, 9H).
LC-MS, *m*/*z* 386 [M+H]⁺

### 27.2 (3-Fluoro-5-(1-(4-fluorophenyl)-1H-pyrazol-4-yl)phenyl)methanamine, trifluoroacetate

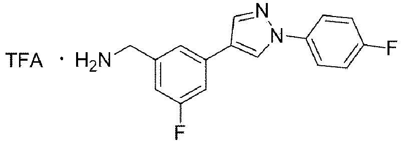

By the same method as in Example 25.4, tert-butyl (3-fluoro-5-(1-(4-(trifluoromethyl)phenyl)-1H-pyrazol-4-yl)benzyl)carbamate (345 mg, 0.89 mmol), trifluoroacetic acid (3 mL), and DCM (9 mL) were used to obtain the title compound (350 mg, crude) as an off-white solid.
LC-MS, *m*/*z* 286 [M+H]⁺

### 27.3 8-Cyclopentyl-N-(3-fluoro-5-(1-(4-fluorophenyl)-1H-pyrazol-4-yl)benzyl)-7H-purine-6-carboxamide

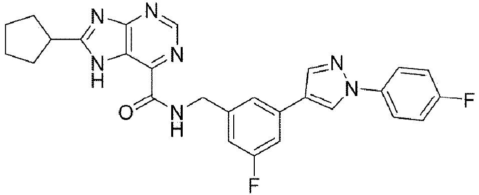

8-Cyclopentyl-7H-purine-6-carboxylic acid (208 mg, 0.89 mmol), (3-fluoro-5-(1-(4-fluorophenyl)-1H-pyrazol-4-yl)phenyl)methanamine, trifluoroacetate (350 mg, crude) and T3P (50 wt.% solution in EtOAc, 850 mg, 2.67 mmol) were dissolved in DMF (4.5 mL), DIPEA (690 µL) was added, and the mixture was stirred at room temperature overnight. Ethyl acetate and H₂O were added to the reaction mixture, extracted, and the organic layer was dried over MgSO₄ and concentrated. The obtained residue was purified by silica gel column chromatography (0-3 % MeOH in DCM) to obtain the title compound (319 mg, 71% yield in 2 steps) as a pale yellow foam.
¹H NMR (400 MHz, DMSO-*d*₆) *δ* 13.2 (1H, Broad), 9.77 (t, *J =* 5.6 Hz, 1H), 9.04 (s, 1H), 8.98 (s, 1H), 8.25 (s, 1H), 7.91 (dd, *J* = 9.2, 4.8 Hz, 2H), 7.59 (s, 1H), 7.48 (d, *J =* 9.6 Hz, 1H), 7.39 (t, *J =* 8.8 Hz, 2H), 7.08 (d, *J =* 9.6 Hz, 1H), 4.61 (d, *J =* 6.4 Hz, 2H), 3.49 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 500 [M+H]⁺

### Example 28

### 28.1 tert-Butyl (3-fluoro-5-(1-(4-(methylsulfonyl)phenyl)-1H-pyrazol-4-yl)benzyl)carbamate

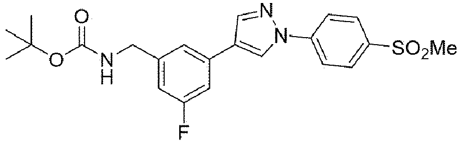

By the same method as in Example 25.3, using tert-butyl (3-fluoro-5-(1H-pyrazol-4-yl)benzyl)carbamate (50 mg, 0.17 mmol), 4-bromophenyl methyl sulfone (60.5 mg, 0.26 mmol), CuI (3.3 mg, 0.017 mmol), L-proline (4 mg, 0.034 mmol), K₂CO₃ (35.6 mg, 0.26 mmol), and DMSO (1.5 mL), the title compound (59 mg, 78%) was obtained as an off-white solid.

¹H NMR (400 MHz, CDCl₃) *δ* 8.25 (s, 1H), 8.07 (d, *J=* 8.8 Hz, 2H), 8.03 (s, 1H), 7.96 (d, *J* = 8.4 Hz, 2H), 7.15 (d, *J* = 9.2 Hz, 1H), 6.94 (d, *J =* 9.6 Hz, 1H), 4.96 (s, broad), 4.36 (d, *J= 6 Hz,* 2H), 3.11 (s, 3H), 1.49 (s, 9H).

### 28.2 (3-Fluoro-5-(1-(4-(methylsulfonyl)phenyl)-1H-pyrazol-4-yl)phenyl)methanamine, trifluoroacetate

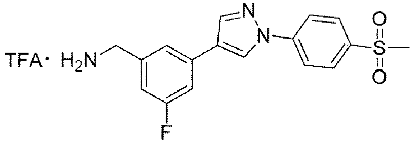

By the same method as in Example 25.4, tert-butyl (3-fluoro-5-(1-(4-(methylsulfonyl)phenyl)-1H-pyrazol-4-yl)benzyl)carbamate (56 mg, 0.13 mmol), trifluoroacetic acid (0.43 mL), and DCM (1.3 mL) were used to obtain the title compound (58 mg, crude).
LC-MS, *m*/*z* 346 [M+H]⁺

### 28.3 8-Cyclopentyl-N-(3-fluoro-5-(1-(4-(methylsulfonyl)phenyl)-1H-pyrazol-4-yl)benzyl)-7H-purine-6-carboxamide

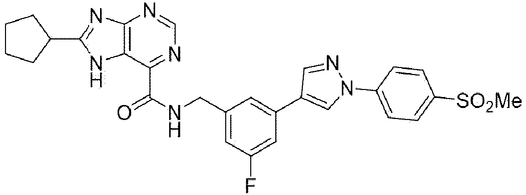

By the same method as in Example 27.3, using 8-cyclopentyl-7H-purine-6-carboxylic acid (30 mg, 0.13 mmol), T3P (50 wt. % solution in EtOAc, 120 mg, 0.38 mmol), (3-fluoro-5-(1-(4-(methylsulfonyl)phenyl)-1H-pyrazol-4-yl)phenyl)methanamine, trifluoroacetate (58 mg), DIPEA (96 µL, 0.57 mmol) and DMF (3 mL), the title compound (56 mg, 80% yield in 2 steps) was obtained as a white solid.
¹H NMR (400 MHz, DMSO-*d*₆) *δ* 13.18 (1H, brs), 9.79 (t, J = 6.4 Hz, 1H), 9.26 (s, 1H), 8.98 (s, 1H), 8.37 (s, 1H), 8.15 (d, J = 8.4 Hz, 2H), 8.09 (d, J = 8.4 Hz, 2H), 7.62 (s, 1H), 7.52 (d, J = 10 Hz, 1H), 7.10 (d, J = 10 Hz, 1H), 4.61 (d, J = 6 Hz, 2H), 3.50 (m, 1H), 3.27 (s, 3H), 2.12 - 2.04 (m, 2H), 1.97 - 1.89 (m, 2H), 1.86 - 1.76 (m, 2H), 1.71 - 1.62 (m, 2H).
LC-MS, *m*/*z* 560 [M+H]⁺

### Example 29

### 29.1 3-Fluoro-5-(1-(3-(trifluoromethyl)benzyl)-1H-pyrazol-4-yl)benzonitrile

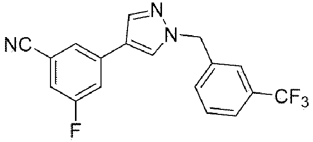

By the same method as in Example 24.1, 3-fluoro-5-(1H-pyrazol-4-yl)benzonitrile (65 mg, 0.35 mmol), NaH (60% dispersion in mineral oil) (17 mg, 0.70 mmol) and 1,4-dioxane (2 mL) were used to obtain the title compound (86 mg, 72%).
LC-MS, *m*/*z* 346 [M+H]⁺

### 29.2 (3-Fluoro-5-(1-(3-(trifluoromethyl)benzyl)-1H-pyrazol-4-yl)phenyl)methanamine

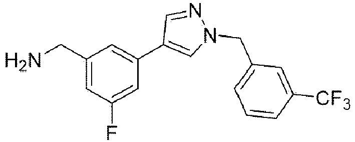

By the same method as in Example 7.2, 3-fluoro-5-(1-(3-(trifluoromethyl)benzyl)-1H-pyrazol-4-yl)benzonitrile (86 mg, 0.25 mmol), LAH solution (2.0 M in THF, 250 µL, 0.50 mmol) and THF (1 mL) were used to obtain the title compound (50 mg, 57%).
LC-MS, *m*/*z* 350 [M+H]⁺

### 29.3 8-Cyclopentyl-N-(3-fluoro-5-(1-(3-(trifluoromethyl)benzyl)-1H-pyrazol-4-yl)benzyl)-7H-purine-6-carboxamide

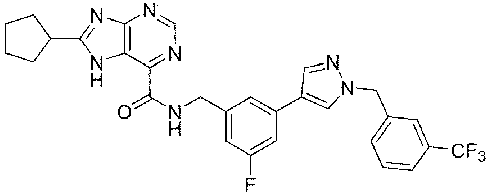

By the same method as in Example 1.3, 3-fluoro-5-(1-(3-(trifluoromethyl)benzyl)-1H-pyrazol-4-yl)phenyl)methanamine (50 mg, 0.14 mmol), 8-cyclopentyl-7H-purine-6-carboxylic acid (28 mg, 0.12 mmol), T3P (50 wt.% solution in EtOAc, 213 µL, 0.36 mmol), DIPEA (51 µL, 0.30 mmol) and DMF (2 mL) were used to obtain the title compound (5.7 mg, 8%).
¹H NMR (400 MHz, DMSO-*d*₆) *δ* 13.14 (s, 1H), 9.73 (s, 1H), 8.93 (s, 1H), 8.35 (s, 1H), 7.96 (s, 1H), 7.65 (s, 1H), 7.61 (s, 1H), 7.57 (d, *J* = 7.5 Hz, 1H), 7.52 (d, *J* = 7.8 Hz, 1H), 7.42 (s, 1H), 7.32 (d, *J =* 9.9 Hz, 1H), 6.99 (d, *J =* 9.6 Hz, 1H), 5.44 (s, 2H), 4.55 (d, *J* = 6.1 Hz, 2H), 3.48 - 3.43 (m, 1H), 2.04 (s, 2H), 1.90 (s, 2H), 1.78 (s, 2H), 1.63 (s, 2H).
LC-MS, *m*/*z* 564 [M+H]⁺

### Example 30

### 30.1 3-Fluoro-5-(1-(pyridin-2-yl)-1H-pyrazol-4-yl)benzonitrile

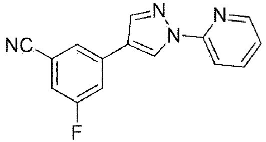

By the same method as in Example 25.3, using 3-fluoro-5-(1H-pyrazol-4-yl)benzonitrile (50 mg, 0.27 mmol), 2-iodopyridine (66 mg, 0.32 mmol), CuI (1 mg, 0.0053 mmol), (1R,2R)-N1,N2-dimethylcyclohexane-1,2-diamine (7 µL, 0.053 mmol), K₂CO₃ (78 mg, 0.56 mmol), and 1,4-dioxane (3 mL), the title compound (62 mg, 88 % ) was obtained.
LC-MS, *m*/*z* 265 [M+H]⁺

### 30.2 (3-Fluoro-5-(1-(pyridin-2-yl)-1H-pyrazol-4-yl)phenyl)methanamine

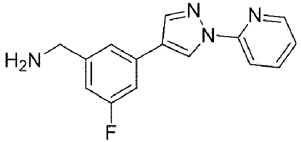

By the same method as in Example 7.2, 3-fluoro-5-(1-(pyridin-2-yl)-1H-pyrazol-4-yl)benzonitrile (62 mg, 0.24 mmol), LAH solution (2.0 M in THF, 0.24 mL, 0.47 mmol) and THF (1 mL) were used to obtain the title compound (66 mg, crude), which was used in the next reaction without separation and purification.
LC-MS, *m*/*z* 269 [M+H]⁺

### 30.3 8-Cyclopentyl-N-(3-fluoro-5-(1-(pyridin-2-yl)-1H-pyrazol-4-yl)benzyl)-7H-purine-6-carboxamide

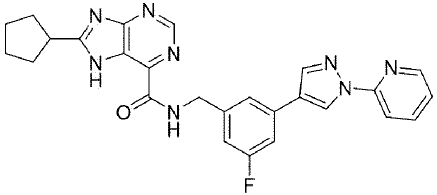

By the same method as in Example 1.3, using (3-fluoro-5-(1-(pyridin-2-yl)-1H-pyrazol-4-yl)phenyl)methanamine (66 mg), 8-cyclopentyl-7H-purine-6-carboxylic acid (57 mg, 0.25 mmol), T3P (50 wt.% solution in EtOAc, 1.8 mL, 0.74 mmol), DIPEA (110 µL, 0.62 mmol) and DMF (2 mL), the title compound (7 mg, 6%) was obtained.
¹H NMR (400 MHz, DMSO-*d*₆) *δ* 13.19 (s, 1H), 9.79 (s, 1H), 9.14 (s, 1H), 8.97 (s, 1H), 8.51 (d, *J* = 4.3 Hz, 1H), 8.35 (s, 1H), 8.05 - 7.95 (m, 2H), 7.70 (s, 1H), 7.59 (d, *J=* 10.3 Hz, 1H), 7.43 - 7.37 (m, 1H), 7.08 (d, *J =* 9.5 Hz, 1H), 4.61 (d, *J=* 6.1 Hz, 2H), 3.52 - 3.46 (m, 1H), 2.07 (m, 2H), 1.94 (m, 2H), 1.80 (m, 2H), 1.66 (m, 2H).
LC-MS, *m*/*z* 483 [M+H]⁺

### Example 31

### 31.1 tert-Butyl (3-fluoro-5-(1-(pyridin-3-yl)-1H-pyrazol-4-yl)benzyl)carbamate

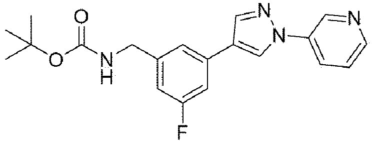

By the same method as in Example 25.3, tert-butyl 3-fluoro-5-(1H-pyrazol-4-yl)benzylcarbamate (300 mg, 1.0 mmol), 3-iodopyridine (320 mg, 1.5 mmol), CuI (20 mg, 0.10 mmol), L-proline (24 mg, 0.21 mmol), K₂CO₃ (213 mg, 1.5 mmol) and DMSO (5 mL) were used to obtain the title compound (291 mg, 77%).
LC-MS, *m*/*z* 369 [M+H]⁺

### 31.2 (3-Fluoro-5-(1-(pyridin-3-yl)-1H-pyrazol-4-yl)phenyl)methanamine, trifluoroacetate

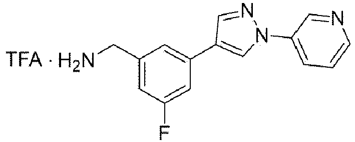

By the same method as in Example 25.4, tert-butyl 3-fluoro-5-(1-(pyridin-3-yl)-1H-pyrazol-4-yl)benzylcarbamate (291 mg, 0.79 mmol), TFA (3 mL), and DCM (7 mL) were used to obtain the title compound (300 mg), which was used in the next reaction without separation and purification.
LC-MS, *m*/*z* 269 [M+H]⁺

### 31.3 8-Cyclopentyl-N-(3-fluoro-5-(1-(pyridin-3-yl)-1H-pyrazol-4-yl)benzyl)-7H-purine-6-carboxamide

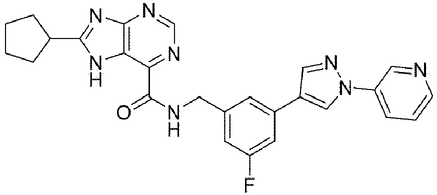

By the same method as in Example 1.3, using (3-fluoro-5-(1-(pyridin-3-yl)-1H-pyrazol-4-yl)phenyl)methanamine, trifluoroacetate (300 mg), 8-cyclopentyl-7H-purine-6-carboxylic acid (180 mg, 0.79 mmol), T3P (50 wt.% solution in EtOAc, 1.4 mL, 2.4 mmol), DIPEA (0.6 mL, 3.6 mmol) and DMF (3.5 mL), the title compound (210 mg, 56%) was obtained.
¹H NMR (400 MHz, DMSO-*d*₆) *δ* 13.19 (s, 1H), 9.80 (s, 1H), 9.20 - 9.13 (m, 2H), 8.98 (s, 1H), 8.56 (d, *J =* 4.5 Hz, 1H), 8.34 (s, 1H), 8.27 (d, *J =* 8.4 Hz, 1H), 7.64 - 7.55 (m, 2H), 7.50 (d, *J =* 10.3 Hz, 1H), 7.10 (d, *J =* 8.9 Hz, 1H), 4.62 (d, *J =* 5.9 Hz, 2H), 3.55 - 3.46 (m, 1H), 2.07 (s, 2H), 1.97 - 1.89 (m, 2H), 1.81 (s, 2H), 1.66 (s, 2H).
LC-MS, *m*/*z* 483 [M+H]⁺

### Example 32

### 32.1 tert-Butyl (3-fluoro-5-(1-(pyrimidin-5-yl)-1H-pyrazol-4-yl)benzyl)carbamate

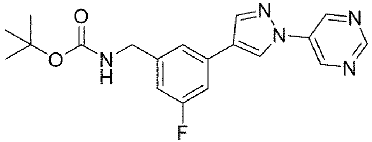

By the same method as in Example 25.3, tert-butyl 3-fluoro-5-(1H-pyrazol-4-yl)benzylcarbamate (50 mg, 0.17 mmol), 5-iodopyrimidine (41 mg, 0.26 mmol), CuI (3 mg, 0.017 mmol), L-proline (4 mg, 0.034 mmol), K₂CO₃ (36 mg, 0.26 mmol), and DMSO (3 mL) were used to obtain the title compound (59 mg, 92%).
LC-MS, *m*/*z* 370 [M+H]⁺

### 32.2 (3-Fluoro-5-(1-(pyrimidin-5-yl)-1H-pyrazol-4-yl)phenyl)methanamine, trifluoroacetate

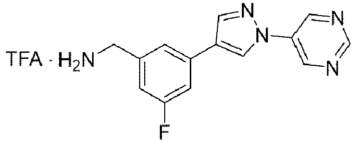

By the same method as in Example 25.4, tert-butyl 3-fluoro-5-(1-(pyrimidin-5-yl)-1H-pyrazol-4-yl)benzylcarbamate (57 mg, 0.15 mmol), TFA (0.7 mL), and DCM (2 mL) were used to obtain the title compound (61 mg, crude), which was used in the next reaction without separation and purification.
LC-MS, *m*/*z* 270 [M+H]⁺

### 32.3 8-Cyclopentyl-N-(3-fluoro-5-(1-(pyrimidin-5-yl)-1H-pyrazol-4-yl)benzyl)-7H-purine-6-carboxamide

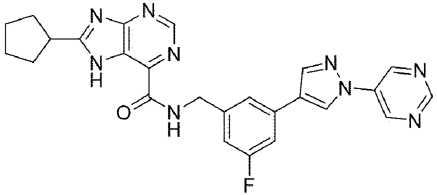

By the same method as in Example 1.3, using (3-fluoro-5-(1-(pyrimidin-5-yl)-1H-pyrazol-4-yl)phenyl)methanamine, trifluoroacetate (59 mg), 8-cyclopentyl-7H-purine-6-carboxylic acid (36 mg, 0.15 mmol), T3P (50 wt.% solution in EtOAc, 280 µL, 0.47 mmol), DIPEA (120 µL, 0.70 mmol) and DMF (2 mL), the title compound (44 mg, 59%) was obtained.
¹H NMR (400 MHz, DMSO-*d*₆) *δ* 13.15 (s, 1H), 9.77 (t, *J=* 6.1 Hz, 1H), 9.32 (s, 2H), 9.16 (d, *J* = 16.5 Hz, 2H), 8.96 (s, 1H), 8.39 (s, 1H), 7.57 (s, 1H), 7.46 (d, *J =* 9.5 Hz, 1H), 7.09 (d, *J* = 9.2 Hz, 1H), 4.60 (d, *J =* 6.1 Hz, 2H), 3.52 - 3.44 (m, 1H), 2.04 (s, 2H), 1.94 - 1.87 (m, 2H), 1.78 (s, 2H), 1.63 (d, *J=* 4.8 Hz, 2H).
LC-MS, *m*/*z* 484 [M+H]⁺

### Example 33

### 33.1 3-Fluoro-5-((2-methoxyphenyl)amino)benzonitrile

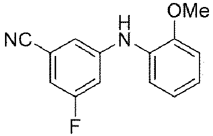

By the same method as in Example 10.1, 3-bromo-5-fluorobenzonitrile (256 mg, 1.28 mmol), 2-methoxyaniline (135 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (80 mg, 30%) as a white solid.
LC-MS, *m*/*z* 243 [M+H]⁺

### 33.2 3-(Aminomethyl)-5-fluoro-N-(2-methoxyphenyl)aniline

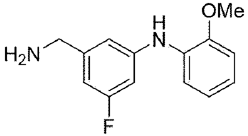

By the same method as in Example 1.2, 3-fluoro-5-((2-methoxyphenyl)amino)benzonitrile (80 mg, 0.33 mmol) and LAH solution (2.0 M in THF, 0.33 mL) were used to obtain the title compound (55 mg, 68%) as an oil.
LC-MS, *m*/*z* 247 [M+H]⁺

### 33.3 8-Cyclopentyl-N-(3-fluoro-5-((2-methoxyphenyl)amino)benzyl)-7H-purine-6-carboxamide

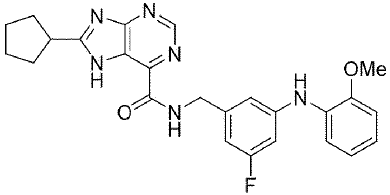

By the same method as in Example 1.3, 8-cyclopentyl-7H-purine-6-carboxylic acid (28 mg, 0.12 mmol), 3-(aminomethyl)-5-fluoro-N-(2-methoxyphenyl)aniline (30 mg, 0.12 mmol), T3P (50 wt.% solution in EtOAc, 244 mg, 0.39 mmol) and DIPEA (27 µL, 0.31 mmol) were used to obtain the title compound (40 mg, 72%) as a white solid.
¹H NMR (400 MHz, CD₃OD) *δ* 8.95 (s, 1H), 7.22 (d, *J =* 8 Hz, 1H), 6.94 - 6.86 (m, 3H), 6.75 (t, *J =* 8.0 Hz, 1H), 6.63 (d, *J =* 12 Hz, 1H), 6.56 (d, *J =* 12 Hz, 1H), 4.59 (s, 2H), 3.83 (s, 3H), 3.50 - 3.46 (m, 1H), 2.19 (m, 2H), 2.03 (m, 2H), 1.92 (m, 2H), 1.77 (m, 2H).
LC-MS, *m*/*z* 461 [M+H]⁺

### Example 34

### 34.1 3-Fluoro-5-((3-methoxyphenyl)amino)benzonitrile

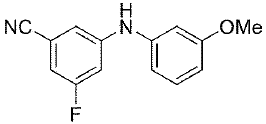

By the same method as in Example 10.1, 3-bromo-5-fluorobenzonitrile (256 mg, 1.28 mmol), 3-methoxyaniline (135 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (110 mg, 45%) as a white solid.
LC-MS, *m*/*z* 243 [M+H]⁺

### 34.2 3-(Aminomethyl)-5-fluoro-N-(3-methoxyphenyl)aniline

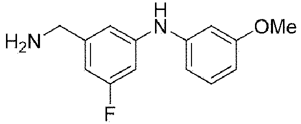

By the same method as in Example 1.2, 3-fluoro-5-((3-methoxyphenyl)amino)benzonitrile (110 mg, 0.45 mmol) and LAH solution (2.0 M in THF, 0.45 mL) were used to obtain the title compound (70 mg, 63%) as an oil.
LC-MS, *m*/*z* 247 [M+H]⁺

### 34.3 8-Cyclopentyl-N-(3-fluoro-5-((3-methoxyphenyl)amino)benzyl)-7H-purine-6-carboxamide

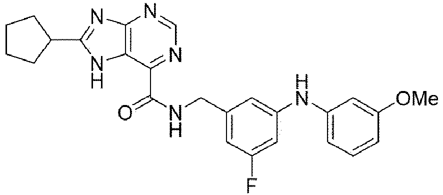

By the same method as in Example 1.3, 8-cyclopentyl-7H-purine-6-carboxylic acid (42 mg, 0.18 mmol), 3-(aminomethyl)-5-fluoro-N-(3-methoxyphenyl)aniline (45 mg, 0.18 mmol), T3P (50 wt. % solution in EtOAc, 366 mg, 0.59 mmol) and DIPEA (80 µL, 0.92 mmol) were used to obtain the title compound (50 mg, 60%) as a white solid.
¹H NMR (400 MHz, CD₃OD) *δ* 8.95 (s, 1H), 7.06 (t, *J=* 8 Hz, 1H), 6.91 (s, 1H), 6.65 - 6.61 (m, 3H), 6.57 (d, *J =* 8.0 Hz, 1H), 6.43 (d, *J =* 8.0 Hz, 1H), 4.60 (s, 2H), 3.65 (s, 3H), 3.50 - 3.46 (m, 1H), 2.19 (m, 2H), 2.03 (m, 2H), 1.92 (m, 2H), 1.77 (m, 2H).
LC-MS, *m*/*z* 461 [M+H]⁺

### Example 35

### 35.1 3-Fluoro-5-((4-methoxyphenyl)amino)benzonitrile

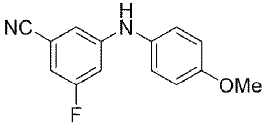

By the same method as in Example 10.1, 3-bromo-5-fluorobenzonitrile (256 mg, 1.28 mmol), 4-methoxyaniline (135 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (130 mg, 53%) as a white solid.
LC-MS, *m*/*z* 243 [M+H]⁺

### 35.2 3-(Aminomethyl)-5-fluoro-N-(4-methoxyphenyl)aniline

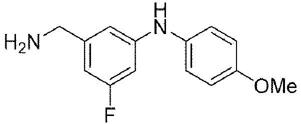

By the same method as in Example 1.2, 3-fluoro-5-((4-methoxyphenyl)amino)benzonitrile (100 mg, 0.41 mmol) and LAH solution (2.0 M in THF, 0.41 mL) were used to obtain the title compound (55 mg, 54%) as an oil.
LC-MS, *m*/*z* 247 [M+H]⁺

### 35.3 8-Cyclopentyl-N-(3-fluoro-5-((4-methoxyphenyl)amino)benzyl)-7H-purine-6-carboxamide

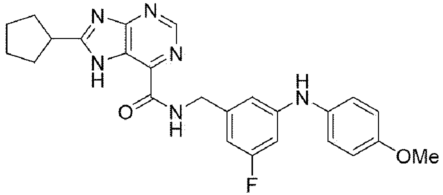

By the same method as in Example 1.3, 8-cyclopentyl-7H-purine-6-carboxylic acid (34 mg, 0.15 mmol), 4-(aminomethyl)-5-fluoro-N-(3-methoxyphenyl)aniline (37 mg, 0.15 mmol), T3P (50 wt. % solution in EtOAc, 300 mg, 0.48 mmol) and DIPEA (80 µL, 0.75 mmol) were used to obtain the title compound (45 mg, 67%) as a white solid.
¹H NMR (400 MHz, CD₃OD) *δ* 8.94 (s, 1H), 7.03 (d, *J* = 8 Hz, 2H), 6.78 (d, *J=* 12 Hz, 2H), 6.70 (s, 1H), 6.46 (d, *J =* 12 Hz, 2H), 4.56 (s, 2H), 3.71 (s, 3H), 3.50 - 3.46 (m, 1H), 2.19 (m, 2H), 2.03 (m, 2H), 1.92 (m, 2H), 1.77 (m, 2H).
LC-MS, *m*/*z* 461 [M+H]⁺

### Example 36

### 8-Cyclopentyl-N-(3-fluoro-5-((4-methoxyphenyl)amino)benzyl)-7-methyl-7H-purine-6-carboxamide

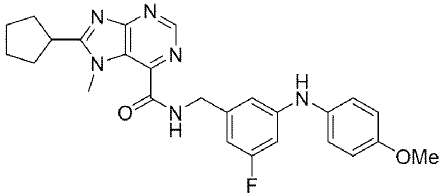

8-Cyclopentyl-N-(3-fluoro-5-((4-methoxyphenyl)amino)benzyl)-7H-purine-6-carboxamide (20 mg, 0.043 mmol) was dissolved in DMF (1 mL), CH₃I (2.7 µL, 0.043 mmol) and K₂CO₃ (30 mg, 0.22 mmol) were added, and the mixture was stirred at 40 °C for 1 h. After completion of the reaction, the mixture was cooled to room temperature and the solid was removed by filtration. After concentrating the filtrate, the obtained residue was purified by silica gel column chromatography (0-5% MeOH in CH₂Cl₂) to obtain the title compound (16 mg, 78%) as a white solid.
¹H NMR (400 MHz, CD₃OD) *δ* 8.93 (s, 1H), 7.03 (d, *J =* 8 Hz, 2H), 6.78 (m, 3H), 6.52 (m, 2H), 4.61 (s, 2H), 3.87 (s, 3H), 3.74 (s, 3H), 3.55 - 3.51 (m, 1H), 2.15 (m, 2H), 2.00 (m, 2H), 1.83 (m, 2H), 1.73 (m, 2H).
LC-MS, *m*/*z* 475 [M+H]⁺

### Example 37

### N-(3-(1-(3-carbamoylphenyl)-1H-pyrazol-4-yl)-5-fluorobenzyl)-8-cyclopentyl-7H-purine-6-carboxamide

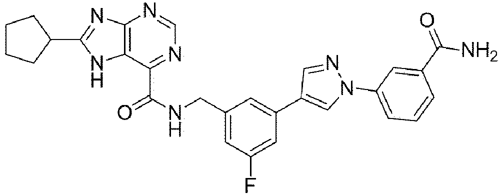

N-(3-(1-(3-Cyanophenyl)-1H-pyrazol-4-yl)-5-fluorobenzyl)-8-cyclopentyl-7H-purine-6-carboxamide (Example 23) (40 mg, 0.079 mmol) was dissolved in DMSO (2 mL), and K₂CO₃ (22 mg, 0.16 mmol) and H₂O₂ (35% aqueous solution, 105 µL) were added, and the mixture was stirred at room temperature for 1 hour. After completion of the reaction, the mixture was diluted with H₂O, extracted three times with EtOAc, and the organic layer was dried over MgSO₄ and concentrated. The obtained residue was purified by silica gel column chromatography (0-10% MeOH in DCM) to obtain the title compound (33 mg, 81%).
¹H NMR (400 MHz, DMSO-*d*₆) *δ* 13.18 (1H, Broad), 9.78 (t, *J =* 6.4 Hz, 1H), 9.14 (s, 1H). 8.98 (s, 1H), 8.37 (s, 1H), 8.29 (s, 1H), 8.12 (s, 1H), 8.03 (d, *J =* 8.4 Hz, 1H), 7.82 (d, *J =* 8 Hz, 1H), 7.63-7.50 (m, 4H), 7.07 (d, *J =* 9.2 Hz, 1H), 4.61 (d, *J =* 6.4 Hz, 2H), 3.49 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 525 [M+H]⁺

### Example 38

### 38.1 tert-Butyl 3-fluoro-5-(isothiazol-4-yl)benzylcarbamate

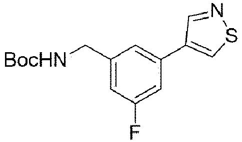

By the same method as in Example 4.1, using tert-butyl 3-bromo-5-fluorobenzylcarbamate (100 mg, 0.33 mmol), 4-(4,4,5,5-tetramethyl-1,3,2-dioxaborolan-2-yl)isothiazole (104 mg, 0.49 mmol), PdCl₂ (dppf) (12 mg, 0.016 mmol), 2 M *aq.* K₂CO₃ (0.085 mL) and 1,4-dioxane (1.5 mL), the title compound (63 mg, 63%) was obtained as a white solid.
LC-MS, *m*/*z* 309 [M+H]⁺

### 38.2 (3-Fluoro-5-(isothiazol-4-yl)phenyl)methanamine, trifluoroacetate

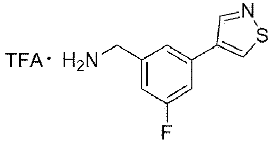

By the same method as in Example 25.4, tert-butyl 3-fluoro-5-(isothiazol-4-yl)benzylcarbamate (61 mg, 0.20 mmol), trifluoroacetic acid (0.7 mL), and DCM (2 mL) were used to obtain the title compound (94 mg, crude).

### 38.3 8-Cyclopentyl-N-(3-fluoro-5-(isothiazol-4-yl)benzyl)-7H-purine-6-carboxamide

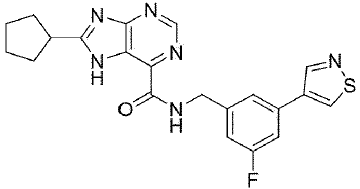

By the same method as in Example 27.3, 8-cyclopentyl-7H-purine-6-carboxylic acid (46 mg, 0.20 mmol), T3P (50 wt. % solution in EtOAc, 189 mg, 0.59 mmol), (3-fluoro-5-(isothiazol-4-yl)phenyl)methanamine, trifluoroacetate (94 mg), DIPEA (151 µL, 0.89 mmol) and DMF (2 mL) were used to obtain the title compound (56 mg, 68% yield in 2 steps) as a white solid.
¹H NMR (400 MHz, DMSO-*d*₆) *δ* 13.17 (s, 1H), 9.80 (t, *J =* 6.4 Hz, 1H), 9.42 (s, 1H), 9.07 (s, 1H), 8.98 (s, 1H), 7.68 (s, 1H), 7.60 (d, *J =* 10 Hz, 1H), 7.18 (d, *J =* 9.2 Hz, 1H), 4.63 (d, *J =* 6 Hz, 2H), 3.50 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 423 [M+H]⁺

### Example 39

### 39.1 tert-Butyl 3-fluoro-5-(furan-2-yl)benzylcarbamate

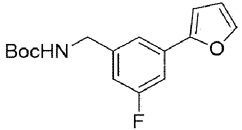

By the same method as in Example 4.1, tert-butyl 3-bromo-5-fluorobenzylcarbamate (100 mg, 0.33 mmol), 2-(furan-2-yl)-4,4,5,5-tetramethyl-1,3,2-dioxaborolane (96 mg, 0.49 mmol), PdCl₂ (dppf) (12 mg, 0.016 mmol), 2 M *aq.* K₂CO₃ (0.085 mL) and 1,4-dioxane (1.5 mL) were used to obtain the title compound (53 mg, 55%) as a yellow liquid.

### 39.2 (3-Fluoro-5-(furan-2-yl)phenyl)methanamine, trifluoroacetate

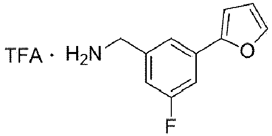

By the same method as in Example 25.4, tert-butyl 3-fluoro-5-(furan-2-yl)benzylcarbamate (51 mg, 0.18 mmol), trifluoroacetic acid (0.6 mL), and DCM (1.8 mL) were used to obtain the title compound (41 mg, crude).

### 39.3 8-Cyclopentyl-N-(3-fluoro-5-(furan-2-yl)benzyl)-7H-purine-6-carboxamide

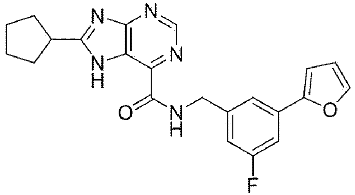

By the same method as in Example 27.3, 8-cyclopentyl-7H-purine-6-carboxylic acid (41 mg, 0.20 mmol), 3-fluoro-5-(furan-2-yl)phenyl)methanamine, trifluoroacetate (54 mg), T3P (50 wt. % solution in EtOAc, 168 mg, 0.53 mmol), DIPEA (135 µL, 0.79 mmol) and DMF (4 mL) were used to obtain the title compound (56 mg, 80% yield in 2 steps) as a white solid.
¹H NMR (400 MHz, DMSO-*d*₆) *δ* 13.19 (s, 1H), 9.82 (m, 1H), 8.97 (s, 1H), 7.78 (s, 1H), 7.58 (s, 1H), 7.43 (d, *J =* 9.6 Hz, 1H), 7.13 (d, *J =* 9.2 Hz, 1H), 7.04 (d, *J =* 3.2 Hz, 1H), 6.62-6.61 (m, 1H), 4.59 (d, *J =* 6.4 Hz, 2H), 3.50 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 406 [M+H]⁺

### Example 40

### 40.1 tert-Butyl (3-fluoro-5-(thiophen-3-yl)benzyl)carbamate

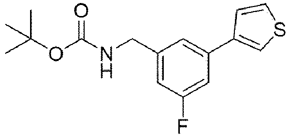

By the same method as in Example 4.1, using tert-butyl 3-bromo-5-fluorobenzylcarbamate (100 mg, 0.33 mmol), 4,4,5,5-tetramethyl-2-(thiophen-3-yl)-1,3,2-dioxaborolane (104 mg, 0.49 mmol), PdCl₂ (dppf) (12 mg, 0.016 mmol), 2 M *aq.* K₂CO₃ (0.085 mL) and 1,4-dioxane (1.5 mL), the title compound (60 mg, 59%) was obtained as a pale yellow liquid.

### 40.2 (3-Fluoro-5-(thiophen-3-yl)phenyl)methanamine, trifluoroacetate

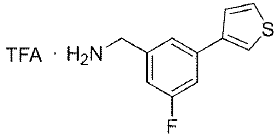

By the same method as in Example 25.4, using tert-butyl (3-fluoro-5-(thiophen-3-yl)benzyl)carbamate (60 mg, 0.20 mmol), trifluoroacetic acid (0.67 mL), and DCM (2 mL), the title compound (63 mg, crude) was obtained.

### 40.3 8-Cyclopentyl-N-(3-fluoro-5-(thiophen-3-yl)benzyl)-7H-purine-6-carboxamide

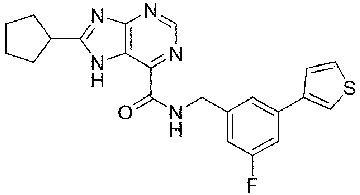

By the same method as in Example 27.3, 8-cyclopentyl-7H-purine-6-carboxylic acid (46 mg, 0.20 mmol), (3-fluoro-5-(thiophen-3-yl)phenyl)methanamine, trifluoroacetate (63 mg), T3P (50 wt. % solution in EtOAc, 188 mg, 0.59 mmol), DIPEA (151 µL, 0.89 mmol) and DMF (2 mL) were used to obtain the title compound (54 mg, 65% yield in 2 steps) as a white solid.
¹H NMR (400 MHz, DMSO-*d*₆) *δ* 13.18 (s, 1H), 9.80 (t, *J =* 6.8 Hz, 1H), 8.97 (s, 1H), 7.94 (m, 1H), 7.66 (m, 1H), 7.60 (s, 1H), 7.56 (d, *J =* 4.4 Hz, 1H), 7.47 (d, *J =* 11.2 Hz, 1H), 7.11 (d, *J =* 8.8 Hz, 1H), 4.60 (d, *J =* 6 Hz, 2H), 3.50 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 422 [M+H]⁺

### Example 41

### 41.1 3-Fluoro-5-((4-(trifluoromethyl)phenyl)amino)benzonitrile

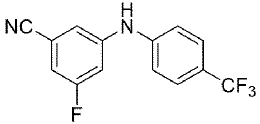

By the same method as in Example 10.1, 3-bromo-5-fluorobenzonitrile (256 mg, 1.28 mmol), 4-(trifluoromethyl)aniline (177 mg, 1.1 mmol), Xantphos (30 mg, 0.05 mmol), Pd₂(dba)₃ (15 mg, 0.016 mmol), K₂CO₃ (318 mg, 2.3 mmol) and dioxane (5 mL) were used to obtain the title compound (210 mg, 75%) as a white solid.
LC-MS, *m*/*z* 281 [M+H]⁺

### 41.2 3-(Aminomethyl)-5-fluoro-N-(4-(trifluoromethyl)phenyl)aniline

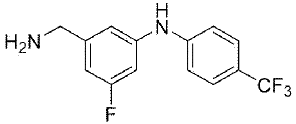

By the same method as in Example 1.2, 3-fluoro-5-((4-(trifluoromethyl)phenyl)amino)benzonitrile (200 mg, 0.71 mmol) and LAH solution (2.0 M in THF, 0.71 mL) were used to obtain the title compound (70 mg, 35%) as an oil.
LC-MS, *m*/*z* 285 [M+H]⁺

### 41.3 8-Cyclopentyl-N-(3-fluoro-5-((4-(trifluoromethyl)phenyl)amino)benzyl)-7H-purine-6-carboxamide

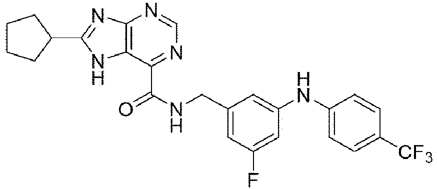

By the same method as in Example 1.3, 8-cyclopentyl-7H-purine-6-carboxylic acid (30 mg, 0.13 mmol), 3-(aminomethyl)-5-fluoro-N-(4-(trifluoromethyl)phenyl)aniline (37 mg, 0.13 mmol), T3P (50 wt. % solution in EtOAc, 300 mg, 0.48 mmol) and DIPEA (80 µL, 0.75 mmol) were used to obtain the title compound (6 mg, 9%) as a viscous oil.
¹H NMR (400 MHz, CD₃OD) *δ* 8.93 (s, 1H), 7.41 (d, *J =* 8 Hz, 2H), 7.15 (d, *J* = 8 Hz, 2H), 6.99 (s, 1H), 6.76 (d, *J =* 8 Hz, 1H), 6.70 (d, J=12 Hz, 1H), 4.61 (s, 2H), 3.48 - 3.44 (m, 1H), 2.19 (m, 2H), 2.01 (m, 2H), 1.89 (m, 2H), 1.74 (m, 2H).
LC-MS, *m*/*z* 499 [M+H]⁺

### Example 42

### 42.1 tert-Butyl (3-fluoro-5-(1-(2-methylpyridin-4-yl)-1H-pyrazol-4-yl)benzyl)carbamate

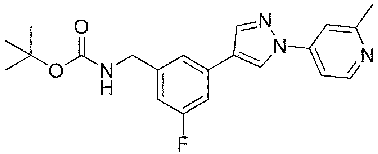

By the same method as in Example 25.3, tert-butyl 3-fluoro-5-(1H-pyrazol-4-yl)benzylcarbamate (50 mg, 0.17 mmol), 4-iodo-2-methylpyridine (44 mg, 0.26 mmol), CuI (3 mg, 0.017 mmol), L-proline (4 mg, 0.034 mmol), K₂CO₃ (36 mg, 0.26 mmol) and DMSO (3 mL) were used to obtain the title compound (45 mg, 68%).
LC-MS, *m*/*z* 383 [M+H]⁺

### 42.2 (3-Fluoro-5-(1-(2-methylpyridin-4-yl)-1H-pyrazol-4-yl)phenyl)methanamine, trifluoroacetate

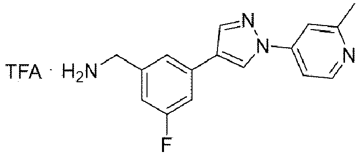

By the same method as in Example 25.4, tert-butyl 3-fluoro-5-(1-(2-methylpyridin-4-yl)-1H-pyrazol-4-yl)benzylcarbamate (45 mg, 0.12 mmol), TFA (333 µL), and DCM (1 mL) were used to obtain the title compound (47 mg, crude), which was used in the next reaction without separation and purification.
LC-MS, *m*/*z* 283 [M+H]⁺

### 42.3 8-Cyclopentyl-N-(3-fluoro-5-(1-(2-methylpyridin-4-yl)-1H-pyrazol-4-yl)benzyl)-7H-purine-6-carboxamide

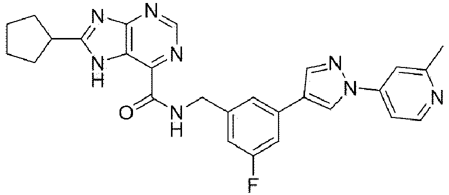

By the same method as in Example 27.3, (3-fluoro-5-(1-(2-methylpyridin-4-yl)-1H-pyrazol-4-yl)phenyl)methanamine trifluoroacetate (47 mg), 8-cyclopentyl-7H-purine-6-carboxylic acid (27 mg, 0.12 mmol), T3P (50 wt.% solution in EtOAc, 210 µL, 0.35 mmol), DIPEA (90 µL, 0.53 mmol) and DMF (2 mL) were used to obtain the title compound (44 mg, 59%).
¹H NMR (400 MHz, DMSO-*d*₆) *δ* 13.19 (s, 1H), 9.79 (s, 1H), 9.25 (s, 1H), 8.98 (s, 1H), 8.54 (d, *J* = 5.5 Hz, 1H), 8.37 (s, 1H), 7.79 (s, 1H), 7.70 (d, *J =* 5.3 Hz, 1H), 7.63 (s, 1H), 7.51 (d, *J =* 9.7 Hz, 1H), 7.12 (d, *J* = 9.0 Hz, 1H), 4.63 (d, *J=* 5.8 Hz, 2H), 3.56 - 3.45 (m, 1H), 2.55 (s, 3H), 2.07 (s, 2H), 1.98 - 1.89 (m, 2H), 1.81 (s, 2H), 1.66 (s, 2H).
LC-MS, *m*/*z* 497 [M+H]⁺

### Example 43

### 43.1 tert-Butyl (3-fluoro-5-(1-(6-methoxypyridin-3-yl)-1H-pyrazol-4-yl)benzyl)carbamate

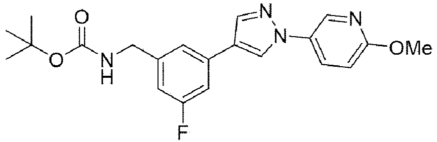

By the same method as in Example 25.3, tert-butyl 3-fluoro-5-(1H-pyrazol-4-yl)benzylcarbamate (50 mg, 0.17 mmol), 5-iodo-2-methoxypyridine (32 µL, 0.26 mmol), CuI (3 mg, 0.017 mmol), L-proline (4 mg, 0.034 mmol), K₂CO₃ (36 mg, 0.26 mmol) and DMSO (3 mL) were used to obtain the title compound (61 mg, 89%).
LC-MS, *m*/*z* 399 [M+H]⁺

### 43.2 (3-Fluoro-5-(1-(6-methoxypyridin-3-yl)-1H-pyrazol-4-yl)phenyl)methanamine, trifluoroacetate

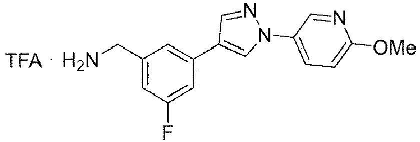

By the same method as in Example 25.4, tert-butyl 3-fluoro-5-(1-(6-methoxypyridin-3-yl)-1H-pyrazol-4-yl)benzylcarbamate (61 mg, 0.15 mmol), TFA (670 µL), and DCM (2 mL) were used to obtain the title compound (4 mg, crude), which was used in the next reaction without separation and purification.
LC-MS, *m*/*z* 299 [M+H]⁺

### 43.3 8-Cyclopentyl-N-(3-fluoro-5-(1-(6-methoxypyridin-3-yl)-1H-pyrazol-4-yl)benzyl)-7H-purine-6-carboxamide

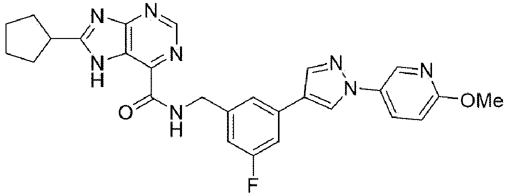

By the same method as in Example 27.3, (3-fluoro-5-(1-(6-methoxypyridin-3-yl)-1H-pyrazol-4-yl)phenyl)methanamine trifluoroacetate (64 mg), 8-cyclopentyl-7H-purine-6-carboxylic acid (36 mg, 0.15 mmol), T3P (50 wt. % solution in EtOAc, 270 µL, 0.46 mmol), DIPEA (120 µL, 0.69 mmol) and DMF (2 mL) were used to obtain the title compound (42 mg, 54%).
¹H NMR (400 MHz, DMSO-*d*₆) *δ* 13.16 (s, 1H), 9.76 (s, 1H), 8.95 (d, *J=* 3.5 Hz, 2H), 8.64 (d, *J =* 2.6 Hz, 1H), 8.23 (s, 1H), 8.16 (dd, *J=* 8.9, 2.7 Hz, 1H), 7.55 (s, 1H), 7.44 (d, *J =* 10.1 Hz, 1H), 7.05 (d, *J* = 9.2 Hz, 1H), 6.98 (d, *J =* 8.9 Hz, 1H), 4.59 (d, *J =* 6.0 Hz, 2H), 3.88 (s, 3H), 3.52 - 3.42 (m, 1H), 2.05 (d, *J =* 7.8 Hz, 2H), 1.90 (dt, *J =* 18.8, 7.0 Hz, 2H), 1.78 (s, 2H), 1.68 - 1.58 (m, 2H).
LC-MS, *m*/*z* 513 [M+H]⁺

### Example 44

### 44.1 tert-Butyl (3-fluoro-5-(1-(6-(trifluoromethyl)pyridin-3-yl)-1H-pyrazol-4-yl)benzyl)carbamate

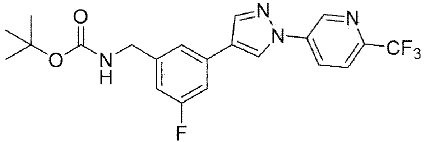

By the same method as in Example 25.3, tert-butyl 3-fluoro-5-(1H-pyrazol-4-yl)benzylcarbamate (50 mg, 0.17 mmol), 5-bromo-2-(trifluoromethyl)pyridine (58 mg, 0.26 mmol), CuI (3 mg, 0.017 mmol), L-proline (4 mg, 0.034 mmol), K₂CO₃ (36 mg, 0.26 mmol) and DMSO (3 mL) were used to obtain the title compound (57 mg, 76%).
LC-MS, *m*/*z* 437 [M+H]⁺

### 44.2 (3-Fluoro-5-(1-(6-(trifluoromethyl)pyridin-3-yl)-1H-pyrazol-4-yl)phenyl)methanamine, hydrochloride

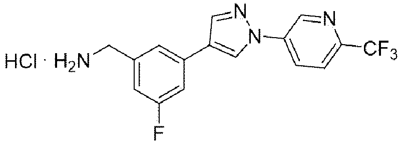

To a solution of tert-butyl 3-fluoro-5-(1-(6-(trifluoromethyl)pyridin-3-yl)-1H-pyrazol-4-yl)benzylcarbamate (57 mg, 0.13 mmol) in DCM (2 mL) was added HCl (35-37%, 670 µL) dropwise at room temperature, and the mixture was stirred at room temperature for 1 h. After the reaction was complete, the mixture was diluted with MeOH and evaporated three times to obtain the title compound (49 mg, crude), which was used in the next reaction without separation and purification.
LC-MS, *m*/*z* 337 [M+H]⁺

### 44.3 8-Cyclopentyl-N-(3-fluoro-5-(1-(6-(trifluoromethyl)pyridin-3-yl)-1H-pyrazol-4-yl)benzyl)-7H-purine-6-carboxamide

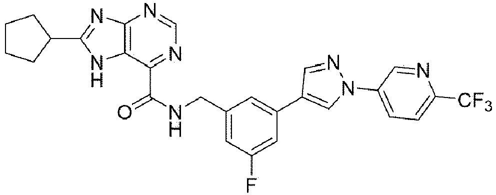

By the same method as in Example 27.3, (3-fluoro-5-(1-(6-(trifluoromethyl)pyridin-3-yl)-1H-pyrazol-4-yl)phenyl)methanamine hydrochloride (49 mg), 8-cyclopentyl-7H-purine-6-carboxylic acid (30 mg, 0.13 mmol), T3P (50 wt.% solution in EtOAc, 230 µL, 0.39 mmol), DIPEA (100 µL, 0.59 mmol) and DMF (2 mL) were used to obtain the title compound (60 mg, 83%).
¹H NMR (400 MHz, DMSO-*d*₆) *δ* 13.16 (s, 1H), 9.77 (t, *J=* 6.0 Hz, 1H), 9.31 (s, 1H), 9.27 (s, 1H), 8.95 (s, 1H), 8.50 (d, *J =* 8.6 Hz, 1H), 8.40 (s, 1H), 8.09 (d, *J =* 8.6 Hz, 1H), 7.59 (s, 1H), 7.49 (d, *J =* 9.8 Hz, 1H), 7.09 (d, *J =* 9.7 Hz, 1H), 4.60 (d, *J =* 5.8 Hz, 2H), 3.52 - 3.43 (m, 1H), 2.05 (d, *J* = 7.8 Hz, 2H), 1.90 (dd, *J =* 12.2, 7.4 Hz, 2H), 1.78 (s, 2H), 1.68 - 1.58 (m, 2H).
LC-MS, *m*/*z* 551 [M+H]⁺

### Example 45

### 45.1 tert-Butyl (3-fluoro-5-(1-(3-(methylsulfonyl)phenyl)-1H-pyrazol-4-yl)benzyl)carbamate

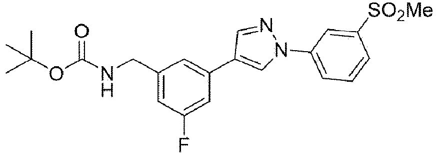

By the same method as in Example 25.3, tert-butyl (3-fluoro-5-(1H-pyrazol-4-yl)benzyl)carbamate (100 mg, 0.34 mmol), 3-bromophenyl methyl sulfone (121 mg, 0.51 mmol), CuI (6.5 mg, 0.034 mmol), L-proline (8 mg, 0.069 mmol), K₂CO₃ (71 mg, 0.51 mmol) and DMSO (2 mL) were used to obtain the title compound (104 mg, 68%) as an off-white solid.
LC-MS, *m*/*z* 446 [M+H]⁺

### 45.2 (3-Fluoro-5-(1-(3-(methylsulfonyl)phenyl)-1H-pyrazol-4-yl)phenyl)methanamine, trifluoroacetate

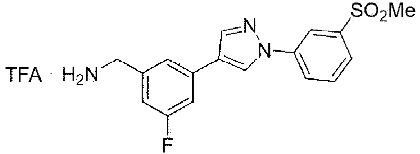

By the same method as in Example 25.4, tert-butyl (3-fluoro-5-(1-(3-(methylsulfonyl)phenyl)-1H-pyrazol-4-yl)benzyl)carbamate (104 mg, 0.23 mmol), trifluoroacetic acid (0.77 mL,) and DCM (2.3 mL) were used to obtain the title compound (350 mg, crude).
LC-MS, *m*/*z* 346 [M+H]⁺

### 45.3 8-Cyclopentyl-N-(3-fluoro-5-(1-(3-(methylsulfonyl)phenyl)-1H-pyrazol-4-yl)benzyl)-7H-purine-6-carboxamide

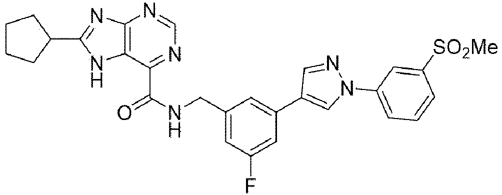

By the same method as in Example 27.3, 8-cyclopentyl-7H-purine-6-carboxylic acid (54 mg, 0.23 mmol), (3-fluoro-5-(1-(3-(methylsulfonyl)phenyl)-1H-pyrazol-4-yl)phenyl)methanamine, trifluoroacetate (107 mg), T3P (50 wt. % solution in EtOAc, 223 mg, 0.70 mmol), DIPEA (180 µL, 1.05 mmol) and DMF (2 mL) were used to obtain the title compound (66 mg, 50% yield in 2 steps) as an off-white foam.
¹H NMR (400 MHz, DMSO-*d*₆) *δ* 13.18 (s, 1H), 9.79 (t, *J =* 6.4 Hz, 1H), 9.27 (s, 1H), 8.98 (s, 1H), 8.41 (s, 1H), 8.35 (s, 1H), 8.25 (d, *J =* 8.4 Hz, 1H), 7.85 (m, 2H), 7.63 (s, 1H), 7.53 (d, *J =* 10 Hz, 1H), 7.09 (d, *J =* 9.2 Hz, 1H), 4.62 (d, *J =* 6.0 Hz, 2H), 3.50 (m, 1H), 3.32 (s, 3H), 2.12 - 2.04 (m, 2H), 1.97 - 1.89 (m, 2H), 1.86 - 1.76 (m, 2H), 1.71 - 1.62 (m, 2H).
LC-MS, *m*/*z* 560 [M+H]⁺

### Example 46

### 46.1 tert-Butyl (3-fluoro-5-(1-(3-fluorophenyl)-1H-pyrazol-4-yl)benzyl)carbamate

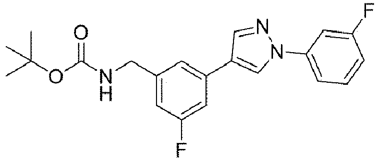

By the same method as in Example 25.3, tert-butyl (3-fluoro-5-(1H-pyrazol-4-yl)benzyl)carbamate (100 mg, 0.34 mmol), 1-fluoro-3-iodobenzene (114 mg, 0.51 mmol), CuI (6.5 mg, 0.034 mmol), L-proline (8 mg, 0.069 mmol), K₂CO₃ (71 mg, 0.51 mmol) and DMSO (2 mL) were used to obtain the title compound (100 mg, 75%) as an off-white solid.
LC-MS, *m*/*z* 386 [M+H]⁺

### 46.2 (3-Fluoro-5-(1-(3-fluorophenyl)-1H-pyrazol-4-yl)phenyl)methanamine, trifluoroacetate

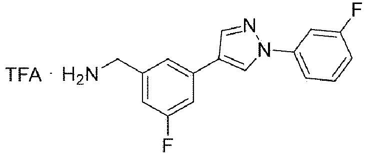

By the same method as in Example 25.4, tert-butyl (3-fluoro-5-(1-(3-fluorophenyl)-1H-pyrazol-4-yl)benzyl)carbamate (100 mg, 0.25 mmol), trifluoroacetic acid (0.8 mL), and DCM (2.5 mL) were used to obtain the title compound (100 mg, crude).
LC-MS, *m*/*z* 286 [M+H]⁺

### 46.3 8-Cyclopentyl-N-(3-fluoro-5-(1-(3-fluorophenyl)-1H-pyrazol-4-yl)benzyl)-7H-purine-6-carboxamide

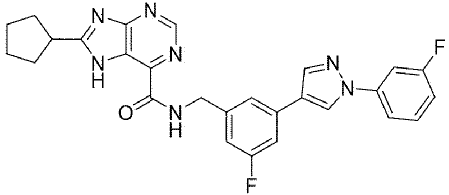

By the same method as in Example 27.3, 8-cyclopentyl-7H-purine-6-carboxylic acid (59 mg, 0.25 mmol), (3-fluoro-5-(1-(3-fluorophenyl)-1H-pyrazol-4-yl)phenyl)methanamine, trifluoroacetate (100 mg), T3P (50 wt. % solution in EtOAc, 59 mg, 0.25 mmol), DIPEA (0.2 mL, 1.15 mmol) and DMF (2 mL) were used to obtain the title compound (72 mg, 51.6% yield in 2 steps) as an off-white solid.
¹H NMR (400 MHz, DMSO-*d*₆) *δ* 13.18 (s, 1H), 9.78 (m, 1H), 9.13 (s, 1H), 8.98 (s, 1H), 8.29 (s, 1H), 7.76 (d, *J=* 9.2 Hz, 2H), 7.64 - 7.53 (m, 2H), 7.48 (d, *J=* 10.0 Hz, 1H), 7.18 (t, *J =* 8.2 Hz, 1H), 7.08 (d, *J =* 8.8 Hz, 1H), 4.61 (d, *J =* 6 Hz, 2H), 3.50 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 500 [M+H]⁺

### Example 47

### 47.1 tert-Butyl (3-fluoro-5-(1-(6-fluoropyridin-3-yl)-1H-pyrazol-4-yl)benzyl)carbamate

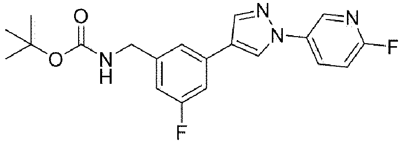

By the same method as in Example 25.3, tert-butyl 3-fluoro-5-(1H-pyrazol-4-yl)benzylcarbamate (50 mg, 0.17 mmol), 2-fluoro-5-iodopyridine (57 mg, 0.26 mmol), CuI (3 mg, 0.017 mmol), L-proline (4 mg, 0.034 mmol), K₂CO₃ (36 mg, 0.26 mmol) and DMSO (3 mL) were used to obtain the title compound (32 mg, 48%).
LC-MS, *m*/*z* 387 [M+H]⁺

### 47.2 (3-Fluoro-5-(1-(6-fluoropyridin-3-yl)-1H-pyrazol-4-yl)phenyl)methanamine, hydrochloride

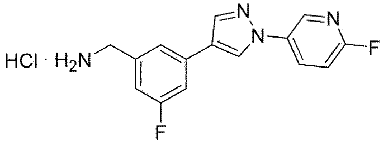

By the same method as in Example 44.2, tert-butyl 3-fluoro-5-(1-(6-fluoropyridin-3-yl)-1H-pyrazol-4-yl)benzylcarbamate (32 mg, 0.083 mmol), HCl (35-37%, 330 µL), and DCM (1 mL) were used to obtain the title compound (28 mg, crude).
LC-MS, *m*/*z* 287 [M+H]⁺

### 47.3 8-Cyclopentyl-N-(3-fluoro-5-(1-(6-fluoropyridin-3-yl)-1H-pyrazol-4-yl)benzyl)-7H-purine-6-carboxamide

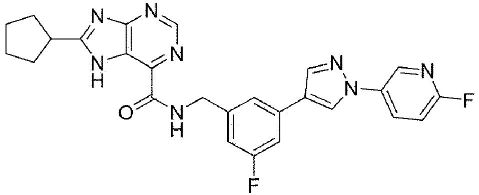

By the same method as in Example 1.3, using (3-fluoro-5-(1-(6-fluoropyridin-3-yl)-1H-pyrazol-4-yl)phenyl)methanamine hydrochloride (28 mg), 8-cyclopentyl-7H-purine-6-carboxylic acid (19 mg, 0.83 mmol), T3P (50 wt.% solution in EtOAc, 150 µL, 0.25 mmol), DIPEA (63 µL, 0.37 mmol) and DMF (2 mL), the title compound (18 mg, 43%) was obtained.
¹H NMR (400 MHz, DMSO-*d*₆) *δ* 13.19 (s, 1H), 9.80 (s, 1H), 9.13 (s, 1H), 8.97 (s, 1H), 8.77 (s, 1H), 8.46 (s, 1H), 8.34 (s, 1H), 7.59 (s, 1H), 7.48 (d, *J =* 9.9 Hz, 1H), 7.42 (d, *J =* 8.7 Hz, 1H), 7.10 (d, *J* = 9.2 Hz, 1H), 4.62 (d, *J =* 5.6 Hz, 2H), 3.48 (d, *J =* 8.2 Hz, 1H), 2.07 (s, 2H), 1.93 (d, *J =* 5.9 Hz, 2H), 1.80 (s, 2H), 1.66 (s, 2H).
LC-MS, *m*/*z* 501 [M+H]⁺

### Example 48

### 48.1 tert-Butyl (3-fluoro-5-(1-(5-iodopyridin-2-yl)-1H-pyrazol-4-yl)benzyl)carbamate

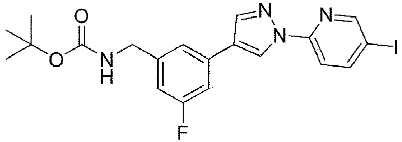

By the same method as in Example 25.3, tert-butyl 3-fluoro-5-(1H-pyrazol-4-yl)benzylcarbamate (50 mg, 0.17 mmol), 2-fluoro-5-iodopyridine (57 mg, 0.26 mmol), CuI (3 mg, 0.017 mmol), L-proline (4 mg, 0.034 mmol), K₂CO₃ (36 mg, 0.26 mmol) and DMSO (3 mL) were used to obtain the title compound (32 mg, 48%).
LC-MS, *m*/*z* 495 [M+H]⁺

### 48.2 (3-Fluoro-5-(1-(5-iodopyridin-2-yl)-1H-pyrazol-4-yl)phenyl)methanamine, hydrochloride

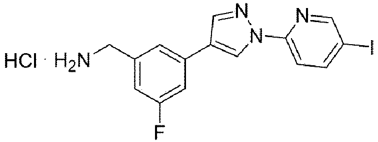

By the same method as in Example 44.2, tert-butyl 3-fluoro-5-(1-(6-iodopyridin-3-yl)-1H-pyrazol-4-yl)benzylcarbamate (20 mg, 0.041 mmol), HCl (35-37%, 333 µL), and DCM (1 mL) were used to obtain the title compound (17 mg, crude).

### 48.3 8-Cyclopentyl-N-(3-fluoro-5-(1-(5-iodopyridin-2-yl)-1H-pyrazol-4-yl)benzyl)-7H-purine-6-carboxamide

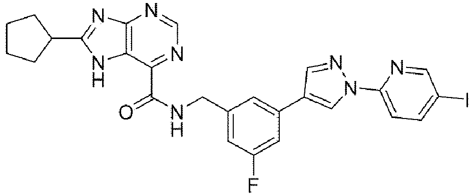

By the same method as in Example 1.3, using (3-fluoro-5-(1-(6-iodopyridin-3-yl)-1H-pyrazol-4-yl)phenyl)methanamine hydrochloride (17 mg), 8-cyclopentyl-7H-purine-6-carboxylic acid (19 mg, 0.83 mmol), T3P (50 wt.% solution in EtOAc, 150 µL, 0.25 mmol), DIPEA (63 µL, 0.37 mmol) and DMF (2 mL), the title compound (9 mg, 37%) was obtained.
¹H NMR (400 MHz, DMSO-*d*₆) *δ* 13.18 (s, 1H), 9.77 (s, 1H), 9.10 (s, 1H), 8.97 (s, 1H), 8.73 (s, 1H), 8.36 (d, *J=* 10.8 Hz, 2H), 7.80 (d, *J* = 8.6 Hz, 1H), 7.69 (s, 1H), 7.58 (d, *J =* 9.8 Hz, 1H), 7.08 (d, *J=* 9.2 Hz, 1H), 4.60 (d, *J =* 6.1 Hz, 2H), 3.49 (s, 1H), 2.06 (s, 2H), 1.93 (s, 2H), 1.80 (s, 2H), 1.66 (s, 2H).
LC-MS, *m*/*z* 609 [M+H]⁺

### Example 49

### 3-(4-(3-((8-Cyclopentyl-7H-purine-6-carboxamido)methyl)-5-fluorophenyl)-1H-pyrazol-1-yl)benzoic acid

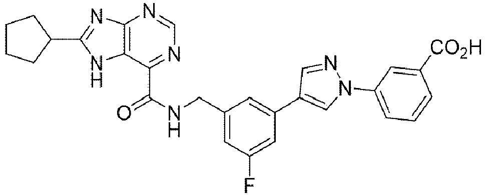

N-(3-(1-(3-Cyanophenyl)-1H-pyrazol-4-yl)-5-fluorobenzyl)-8-cyclopentyl-7H-purine-6-carboxamide (30 mg, 0.059 mmol) and NaOH (57 mg, 1.42 mmol) were added to EtOH (2 mL) and stirred at 85°C for 7 hours. After completion of the reaction, the mixture was diluted with H₂O, acidified with 1 M HCl (pH 4-5), extracted three times with EtOAc, and the organic layer was dried over MgSO₄ and concentrated. The obtained residue was purified by silica gel column chromatography (0-20% MeOH in DCM) to obtain the title compound (12.8 mg, 41%).
¹H NMR (400 MHz, DMSO-*d*₆) *δ* 13.18 (s, 1H), 9.78 (m, 1H), 9.16 (s, 1H), 8.97 (s, 1H), 8.43 (s, 1H), 8.28 (s, 1H), 8.02 (d, *J =* 8.4 Hz, 1H), 7.90 (d, *J =* 8 Hz, 1H), 7.71 - 7.44 (m, 3H), 7.07 (d, *J =* 9.2 Hz, 1H), 4.61 (d, *J =* 6.4 Hz, 2H), 3.49 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 526 [M+H]⁺

### Example 50

### 50.1 tert-Butyl (3-(1-(4-cyanophenyl)-1H-pyrazol-4-yl)-5-fluorobenzyl)carbamate

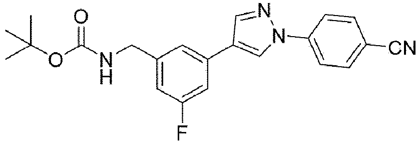

By the same method as in Example 25.3, tert-butyl (3-fluoro-5-(1H-pyrazol-4-yl)benzyl)carbamate (150 mg, 0.51 mmol), 4-iodobenzonitrile (177 mg, 0.77 mmol), CuI (10 mg, 0.051 mmol), L-proline (12 mg, 0.10 mmol), K₂CO₃ (107 mg, 0.77 mmol) and DMSO (2 mL) were used to obtain the title compound (152 mg, 75%) as an off-white solid.
LC-MS, *m*/*z* 393 [M+H]⁺

### 50.2 4-(4-(3-(Aminomethyl)-5-fluorophenyl)-1H-pyrazol-1-yl)benzonitrile, trifluoroacetate

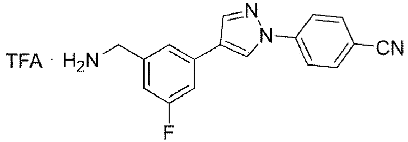

By the same method as in Example 25.4, tert-butyl (3-(1-(4-cyanophenyl)-1H-pyrazol-4-yl)-5-fluorobenzyl)carbamate (100 mg, 0.25 mmol), DCM (2.5 mL), and trifluoroacetic acid (0.8 mL) were used to obtain the title compound (154 mg, crude).
LC-MS, *m*/*z* 293 [M+H]⁺

### 50.3 N-(3-(1-(4-cyanophenyl)-1H-pyrazol-4-yl)-5-fluorobenzyl)-8-cyclopentyl-7H-purine-6-carboxamide

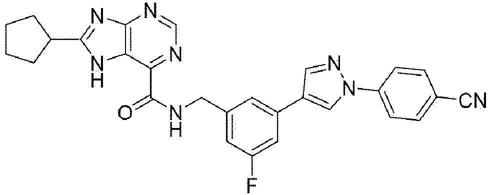

By the same method as in Example 27.3, 8-cyclopentyl-7H-purine-6-carboxylic acid (88 mg, 0.38 mmol), 4-(4-(3-(aminomethyl)-5-fluorophenyl)-1H-pyrazol-1-yl)benzonitrile, trifluoroacetate (154 mg, crude), T3P (50 wt.% solution in EtOAc, 362 mg, 1.14 mmol), DIPEA (0.29 mL, 1.71 mmol) and DMF (2 mL) were used to obtain the title compound (120 mg, 62.5% yield in 2 steps) as an off-white solid.
¹H NMR (400 MHz, DMSO-*d*₆) *δ* 13.21 (s, 1H), 9.79 (m, 1H), 9.25 (s, 1H), 8.98 (s, 1H), 8.38 (s, 1H), 8.06 (dd, *J =* 23.6, 8.8 Hz, 4H), 7.61 (s, 1H), 7.51 (d, *J =* 10.0 Hz, 1H), 7.11 (d, *J =* 10 Hz, 1H), 4.62 (d, *J =* 6 Hz, 2H), 3.50 (m, 1H), 2.12 - 2.04 (m, 2H), 1.97 - 1.89 (m, 2H), 1.86 - 1.76 (m, 2H), 1.71 - 1.62 (m, 2H).
LC-MS, *m*/*z* 507 [M+H]⁺

### Example 51

### 51.1 tert-Butyl (3-fluoro-5-(1H-pyrazol-3-yl)benzyl)carbamate

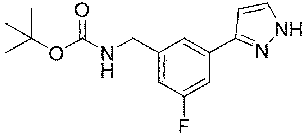

By the same method as in Example 4.1, using tert-butyl 3-bromo-5-fluorobenzylcarbamate (100 mg, 0.33 mmol), 3-(4,4,5,5-tetramethyl-1,3,2-dioxaborolan-2-yl)-1H-pyrazole (128 mg, 0.66 mmol), 2 M aq. K₂CO₃ (0.6 mL, 1.2 mmol), PdCl₂ (dppf) (12 mg, 0.017 mmol) and 1,4-dioxane (1.2 mL), the title compound (95 mg, 99%) was obtained..

¹H NMR (400 MHz, DMSO-*d*₆) *δ* 12.99 (s, 1H), 7.81 (s, 1H), 7.57 (s, 1H), 7.51 - 7.45 (m, 2H), 6.94 (d, *J* = 9.0 Hz, 1H), 6.74 (s, 1H), 4.17 (d, *J =* 6.0 Hz, 2H), 1.41 (s, 9H).

### 51.2 tert-Butyl (3-fluoro-5-(1-(4-fluorophenyl)-1H-pyrazol-3-yl)benzyl)carbamate

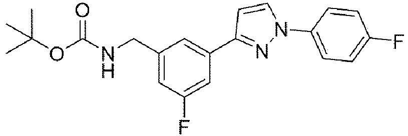

By the same method as in Example 25.3, tert-butyl 3-fluoro-5-(1H-pyrazol-3-yl)benzylcarbamate (95 mg, 0.33 mmol), 1-fluoro-4-iodobenzene (110 mg, 0.49 mmol), CuI (6 mg, 0.033 mmol), L-proline (8 mg, 0.066 mmol), K₂CO₃ (68 mg, 0.49 mmol) and DMSO (2 mL) were used to obtain the title compound (73 mg, 62%).
LC-MS, *m*/*z* 386 [M+H]⁺

### 51.3 (3-Fluoro-5-(1-(4-fluorophenyl)-1H-pyrazol-3-yl)phenyl)methanamine, hydrochloride

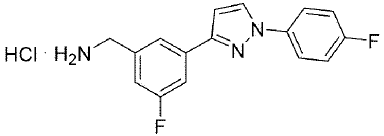

By the same method as in Example 44.2, tert-butyl 3-fluoro-5-(1-(4-fluorophenyl)-1H-pyrazol-3-yl)benzylcarbamate (73 mg, 0.19 mmol), HCl (35-37%, 700 µL), and DCM (2 mL) were used to obtain the title compound (61 mg, crude).

### 51.4 8-Cyclopentyl-N-(3-fluoro-5-(1-(4-fluorophenyl)-1H-pyrazol-3-yl)benzyl)-7H-purine-6-carboxamide

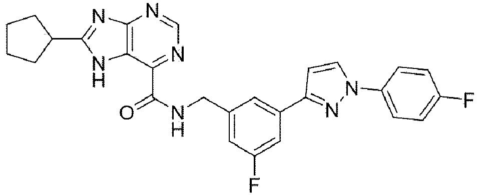

By the same method as in Example 1.3, (3-fluoro-5-(1-(4-fluorophenyl)-1H-pyrazol-3-yl)phenyl)methanamine hydrochloride (61 mg, crude), 8-cyclopentyl-7H-purine-6-carboxylic acid (44 mg, 0.19 mmol), T3P (50 wt.% solution in EtOAc, 340 µL, 0.57 mmol), DIPEA (150 µL, 0.85 mmol) and DMF (2 mL) were used to obtain the title compound (75 mg, 80%).

¹H NMR (400 MHz, DMSO-*d*₆) *δ* 13.19 (s, 1H), 9.85 (s, 1H), 8.97 (s, 1H), 8.57 (d, *J* = 2.3 Hz, 1H), 7.93 (dd, *J =* 9.0, 4.6 Hz, 2H), 7.83 (s, 1H), 7.62 (d, *J =* 10.4 Hz, 1H), 7.38 (t, *J* = 8.7 Hz, 2H), 7.20 (d, *J =* 8.6 Hz, 1H), 7.08 (d, *J =* 2.4 Hz, 1H), 4.63 (d, *J =* 6.2 Hz, 2H), 3.48 (s, 1H), 2.06 (s, 2H), 1.93 (s, 2H), 1.80 (s, 2H), 1.65 (s, 2H).
LC-MS, *m*/*z* 500 [M+H]⁺

### Example 52

### 52.1 tert-Butyl (3-fluoro-5-(1H-pyrrol-3-yl)benzyl)carbamate)

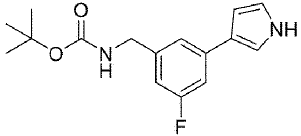

By the same method as in Example 4.1, using tert-butyl 3-bromo-5-fluorobenzylcarbamate (100 mg, 0.33 mmol), 3-(4,4,5,5-tetramethyl-1,3,2-dioxaborolan-2-yl)-1H-pyrrole (127 mg, 0.66 mmol), 2 M aq. K₂CO₃ (0.6 mL, 1.2 mmol), PdCl₂ (dppf) (12 mg, 0.017 mmol) and 1,4-dioxane (1.2 mL), the title compound (95 mg, 99%) was obtained.

¹H NMR (400 MHz, DMSO-*d*₆) *δ* 11.01 (s, 1H), 7.44 (s, 1H), 7.27 - 7.14 (m, 3H), 6.82 - 6.71 (m, *J* = 32.7 Hz, 2H), 6.43 (s, 1H), 4.12 (s, 2H), 1.40 (s, 9H).

### 52.2 tert-Butyl (3-fluoro-5-(1-(4-fluorophenyl)-1H-pyrrol-3-yl)benzyl)carbamate

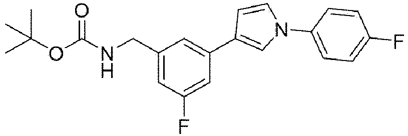

By the same method as in Example 25.3, tert-butyl 3-fluoro-5-(1H-pyrrol-3-yl)benzylcarbamate (95 mg, 0.33 mmol), 1-fluoro-4-iodobenzene (110 mg, 0.49 mmol), CuI (6 mg, 0.033 mmol), L-proline (8 mg, 0.066 mmol), K₂CO₃ (68 mg, 0.49 mmol) and DMSO (2 mL) were used to obtain the title compound (40 mg, 32%).
LC-MS, *m*/*z* 385 [M+H]⁺

### 52.3 (3-Fluoro-5-(1-(4-fluorophenyl)-1H-pyrrol-3-yl)phenyl)methanamine, hydrochloride

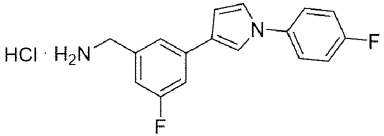

By the same method as in Example 44.2, tert-butyl 3-fluoro-5-(1-(4-fluorophenyl)-1H-pyrrol-3-yl)benzylcarbamate (40 mg, 0.10 mmol), HCl (35-37%, 300 µL), and DCM (1 mL) were used to obtain the title compound (33 mg, crude).

### 52.4 8-Cyclopentyl-N-(3-fluoro-5-(1-(4-fluorophenyl)-1H-pyrrol-3-yl)benzyl)-7H-purine-6-carboxamide

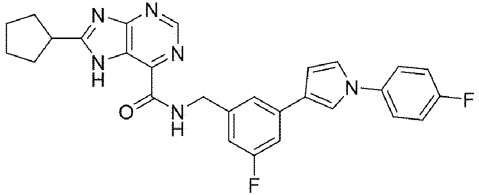

By the same method as in Example 1.3, using (3-fluoro-5-(1-(4-fluorophenyl)-1H-pyrrol-3-yl)phenyl)methanamine hydrochloride (33 mg), 8-cyclopentyl-7H-purine-6-carboxylic acid (24 mg, 0.10 mmol), T3P (50 wt.% solution in EtOAc, 190 µL, 0.31 mmol), DIPEA (80 µL, 0.47 mmol) and DMF (2 mL), the title compound (42 mg, 81%) was obtained.
¹H NMR (400 MHz, DMSO-*d*₆) *δ* 13.19 (s, 1H), 9.77 (s, 1H), 8.97 (s, 1H), 7.94 (s, 1H), 7.69 (dd, *J =* 8.8, 4.7 Hz, 2H), 7.51 (s, 1H), 7.43 (s, 1H), 7.35 (d, *J=* 8.9 Hz, 3H), 6.98 (d, *J =* 9.0 Hz, 1H), 6.72 (s, 1H), 4.58 (d, *J =* 6.2 Hz, 2H), 3.49 (s, 1H), 2.07 (s, 2H), 1.94 (d, *J =* 7.3 Hz, 2H), 1.80 (s, 2H), 1.66 (s, 2H).
LC-MS, *m*/*z* 499 [M+H]⁺

### Example 53

### N-(3-(1-(4-carbamoylphenyl)-1H-pyrazol-4-yl)-5-fluorobenzyl)-8-cyclopentyl-7H-purine-6-carboxamide

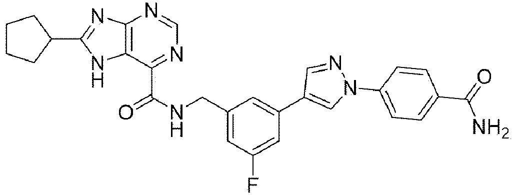

N-(3-(1-(4-cyanophenyl)-1H-pyrazol-4-yl)-5-fluorobenzyl)-8-cyclopentyl-7H-purine-6-carboxamide (30 mg, 0.059 mmol) and NaOH (57 mg, 1.42 mmol) were added to EtOH (1 mL), and stirred at 85 °C for 2 h. After completion of the reaction, the reaction mixture was diluted with H₂O, extracted twice with EtOAc, acidified with 1 M HCl (pH 4-5), and extracted again three times with EtOAc. Then the organic layer was dried over MgSO₄ and concentrated. The obtained residue was purified by silica gel column chromatography (0-20% MeOH in DCM) to obtain the title compound (4.04 mg, 13%).
¹H NMR (400 MHz, DMSO-*d*₆) *δ* 13.18 (s, 1H), 9.79 (m, 1H), 9.17 (s, 1H), 8.98 (s, 1H), 8.31 (s, 1H), 8.00 (m, 5H), 7.61 (s, 1H), 7.50 (d, *J =* 9.7 Hz, 1H), 7.43 (s, 1H), 7.08 (d, *J* = 10.3 Hz, 1H), 4.61 (d, *J =* 6.1 Hz, 2H), 3.50 (m, 1H), 2.13 - 2.03 (m, 2H), 1.98 - 1.88 (m, 2H), 1.86 - 1.76 (m, 2H), 1.71 - 1.59 (m, 2H).
LC-MS, *m*/*z* 525 [M+H]⁺

### Example 54

### 4-(4-(3-((8-Cyclopentyl-7H-purine-6-carboxamido)methyl)-5-fluorophenyl)-1H-pyrazol-1-yl)benzoic acid

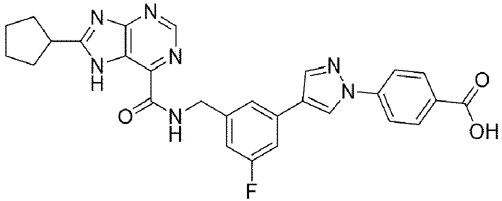

By the same method as in Example 53, N-(3-(1-(4-cyanophenyl)-1H-pyrazol-4-yl)-5-fluorobenzyl)-8-cyclopentyl-7H-purine-6-carboxamide (30 mg, 0.059 mmol), NaOH (57 mg, 1.42 mmol) and EtOH (1 mL) were used to obtain the title compound (1.91 mg, 6%).
¹H NMR (400 MHz, CD₃OD) *δ* 8.96 (s, 1H), 8.78 (s, 1H), 8.13 (m, 3H), 7.90 (d, *J =* 8 Hz, 2H), 7.57 (s, 1H), 7.35 (d, *J* = 8.8 Hz, 1H), 7.07 (d, *J =* 10.4 Hz, 1H), 4.72 (s, 2H), 3.49 (m, 1H), 2.25 - 2.15 (m, 2H), 2.09 - 1.97 (m, 2H), 1.95 - 1.86 (m, 2H), 1.81 - 1.72 (m, 2H).
LC-MS, *m*/*z* 526 [M+H]⁺

### Example 55

### 8-Cyclopentyl-N-(3-fluoro-5-(1-(4-fluorophenyl)-1H-pyrazol-4-yl)benzyl)-7-methyl-7H-purine-6-carboxamide

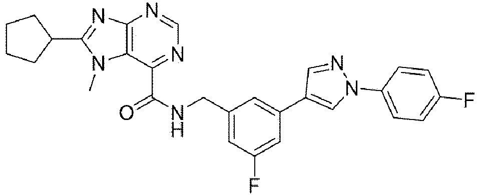

To a mixture of 8-cyclopentyl-N-(3-fluoro-5-(1-(4-fluorophenyl)-1H-pyrazol-4-yl)benzyl)-7H-purine-6-carboxamide (40 mg, 0.08 mmol) and K₂CO₃ (13.3 mg, 0.096 mmol) was added DMF (0.8 mL), stirred at room temperature for 20 min. Then MeI (13.6 mg, 0.096 mmol) was added dropwise, and the mixture was stirred at room temperature overnight. After completion of the reaction, the mixture was diluted with H₂O, extracted three times with EtOAc, and the organic layer was dried over MgSO₄ and concentrated. The obtained residue was purified by silica gel column chromatography (0-5% MeOH in DCM) to obtain the title compound (16.7 mg, yield 40.6%).
¹H NMR (400 MHz, DMSO-*d*₆) *δ* 9.56 (m, 1H), 9.06 (s, 1H), 8.96 (s, 1H), 8.26 (s, 1H), 7.91 (m, 2H), 7.62 (s, 1H), 7.50 (d, J = 10.0 Hz, 1H), 7.39 (t, J = 8.8 Hz, 2H), 7.16 (d, J = 9.6 Hz, 1H), 4.64 (d, J = 5.6 Hz, 2H), 3.82 (s, 3H), 3.55 (m, 1H), 2.14 - 2.05 (m, 2H), 2.01 - 1.91 (m, 2H), 1.79 - 1.59 (m, 4H).
LC-MS, *m*/*z* 514 [M+H]⁺

### Example 56

### 56.1 tert-Butyl 5-bromo-2-fluorobenzylcarbamate

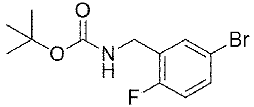

By the same method as in Example 6.1, (5-bromo-2-fluorophenyl)methanamine (200 mg, 0.98 mmol), Boc₂O (430 mg, 2.0 mmol) and DCM (5 mL) were used to obtaine the title compound (273 mg, 92%) as a colorless oil.

¹H NMR (400 MHz, DMSO-*d*₆) *δ* 7.52 - 7.36 (m, 3H), 7.15 (t, 1H), 4.13 (d, *J =* 6.0 Hz, 2H), 1.33 (s, 9H).

### 56.2 tert-Butyl 2-fluoro-5-(1H-pyrazol-4-yl)benzylcarbamate

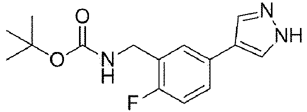

By the same method as in Example 4.1 above, tert-butyl 5-bromo-2-fluorobenzylcarbamate (270 mg, 0.90 mmol), 3-(4,4,5,5-tetramethyl-1,3, 2-Dioxaborolan-2-yl)-1H-pyrrole (260 mg, 1.3 mmol), 2M aq. K₂CO₃ (1.6 mL, 3.1 mmol), PdCl₂ (dppf) (33 mg, 0.045 mmol) and 1,4-dioxane (3.2 mL) were used to obtaine the title compound (190 mg, 66%).
LC-MS, *m*/*z* 292 [M+H]⁺

### 56.3 tert-Butyl 2-fluoro-5-(1-(4-fluorophenyl)-1H-pyrazol-4-yl)benzylcarbamate

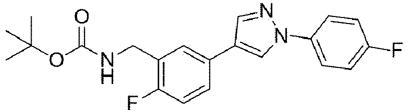

By the same method as in Example 25.3, tert-butyl 2-fluoro-5-(1H-pyrazol-4-yl)benzylcarbamate (190 mg, 0.64 mmol), 1-fluoro-4-iodobenzene (210 mg, 0.96 mmol), CuI (12 mg, 0.064 mmol), L-proline (15 mg, 0.13 mmol), K₂CO₃ (130 mg, 0.96 mmol) and DMSO (3 mL) were used to obtain the title compound (120 mg, 50%).
LC-MS, *m*/*z* 386 [M+H]⁺

### 56.4 (2-Fluoro-5-(1-(4-fluorophenyl)-1H-pyrazol-4-yl)phenyl)methanamine, hydrochloride

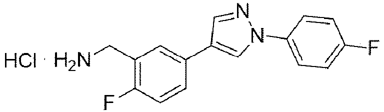

By the same method as in Example 44.2, tert-butyl 2-fluoro-5-(1-(4-fluorophenyl)-1H-pyrazol-4-yl)benzylcarbamate (20 mg, 0.052 mmol), HCl (35-37%, 0.25 mL), and DCM (0.50 mL) were used to obtain the title compound (17 mg, crude).

### 56.5 8-Cyclopentyl-N-(2-fluoro-5-(1-(4-fluorophenyl)-1H-pyrazol-4-yl)benzyl)-7H-purine-6-carboxamide

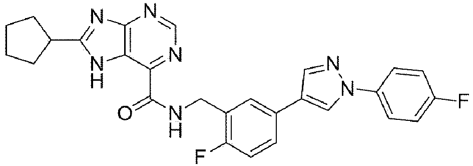

By the same method as in Example 27.3, (2-fluoro-5-(1-(4-fluorophenyl)-1H-pyrazol-4-yl)phenyl)methanamine, hydrochloride (17 mg, crude), 8-cyclopentyl-7H-purine-6-carboxylic acid (12 mg, 0.052 mmol), T3P (50 wt.% solution in EtOAc, 93 µL, 0.16 mmol), DIPEA (40 µL, 0.23 mmol) and EtOAc (1 mL) were used to obtain the title compound (22 mg, 85%).
¹H NMR (400 MHz, DMSO-*d*₆) *δ* 13.19 (s, 1H), 9.64 (t, *J=* 5.7 Hz, 1H), 8.96 (s, 1H), 8.89 (s, 1H), 8.12 (s, 1H), 7.94 - 7.83 (m, *J =* 9.0, 4.7 Hz, 2H), 7.77 (d, *J=* 7.0 Hz, 1H), 7.68 - 7.62 (m, 1H), 7.36 (t, *J =* 8.8 Hz, 2H), 7.30 - 7.22 (m, 1H), 4.66 (d, *J=* 6.0 Hz, 2H), 3.54 - 3.44 (m, 1H), 2.07 (mz, *J =* 7.6 Hz, 2H), 1.97 - 1.87 (m, *J =* 12.6, 6.6 Hz, 2H), 1.85 - 1.74 (m, 2H), 1.70 - 1.60 (m, 2H).
LC-MS, *m*/*z* 500 [M+H]⁺

### Example 57

### 57.1 tert-Butyl 3-bromobenzylcarbamate

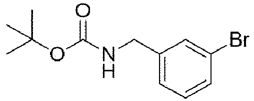

By the same method as in Example 6.1, (3-bromophenyl)methanamine (200 mg, 1.1 mmol), Boc₂O (470 mg, 2.2 mmol) and DCM (5 mL) were used to obtain the title compound (300 mg, 96%) as a colorless oil.

¹H NMR (400 MHz, DMSO-*d*₆) *δ* 7.49 - 7.36 (m, 3H), 7.30 - 7.20 (m, 2H), 4.09 (d, *J* = 6.1 Hz, 2H), 1.37 (s, 9H).

### 57.2 tert-Butyl 3-(1H-pyrazol-4-yl)benzylcarbamate

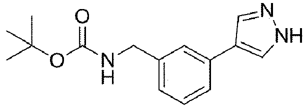

By the same method as in Example 4.1, using tert-butyl 3-bromobenzylcarbamate (300 mg, 1.1 mmol), 3-(4,4,5,5-tetramethyl-1,3,2-dioxaborolan-2-yl)-1H-pyrrole (310 mg, 1.6 mmol), 2 M aq. K₂CO₃ (1.8 mL, 3.7 mmol), PdCl₂ (dppf) (39 mg, 0.053 mmol), and 1,4-dioxane (3.6 mL), the title compound (57 mg, 20%) was obtained.
LC-MS, *m*/*z* 274 [M+H]⁺

### 57.3 tert-Butyl 3-(1-(4-fluorophenyl)-1H-pyrazol-4-yl)benzylcarbamate

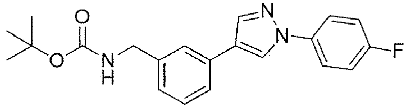

By the same method as in Example 25.3, tert-butyl 3-(1H-pyrazol-4-yl)benzylcarbamate (59 mg, 0.22 mmol), 1-fluoro-4-iodobenzene (72 mg, 0.32 mmol), CuI (4 mg, 0.022 mmol), L-proline (5 mg, 0.043 mmol), K₂CO₃ (45 mg, 0.32 mmol) and DMSO (2 mL) were used to obtain the title compound (54 mg, 68%).
LC-MS, *m*/*z* 386 [M+H]⁺

### 57.4 (3-(1-(4-Fluorophenyl)-1H-pyrazol-4-yl)phenyl)methanamine, hydrochloride

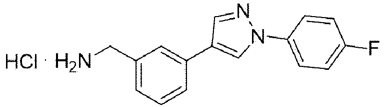

By the same method as in Example 44.2, tert-butyl 3-(1-(4-fluorophenyl)-1H-pyrazol-4-yl)benzylcarbamate (20 mg, 0.054 mmol), HCl (35-37%, 0.25 mL), and DCM (0.50 mL) were used to obtain the title compound (17 mg, crude).

### 57.5 8-Cyclopentyl-N-(3-(1-(4-fluorophenyl)-1H-pyrazol-4-yl)benzyl)-7H-purine-6-carboxamide

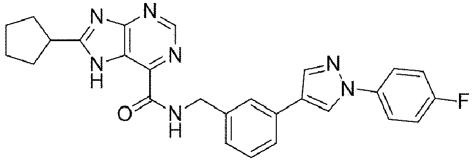

By the same method as in Example 27.3, (3-(1-(4-fluorophenyl)-1H-pyrazol-4-yl)phenyl)methanamine, hydrochloride (17 mg), 8-cyclopentyl-7H-purine-6-carboxylic acid (13 mg, 0.054 mmol), T3P (50 wt.% solution in EtOAc, 97 µL, 0.16 mmol), DIPEA (42 µL, 0.25 mmol) and EtOAc (1 mL) were used to obtain the title compound (24 mg, 92%).
¹H NMR (400 MHz, DMSO-*d*₆) *δ* 13.19 (s, 1H), 9.72 (t, *J =* 5.8 Hz, 1H), 8.96 (s, 2H), 8.18 (s, 1H), 7.97 - 7.88 (m, 2H), 7.73 (s, 1H), 7.60 (d, *J =* 7.4 Hz, 1H), 7.43 - 7.32 (m, 3H), 7.27 (d, *J =* 7.8 Hz, 1H), 4.61 (d, *J =* 6.1 Hz, 2H), 3.53 - 3.44 (m, 1H), 2.13 - 2.03 (m, 2H), 1.98 - 1.88 (m, 2H), 1.84 - 1.75 (m, 2H), 1.71 - 1.60 (m, 2H).
LC-MS, *m*/*z* 482 [M+H]⁺

### Example 58

### 58.1 tert-Butyl 3-(1-(4-chlorophenyl)-1H-pyrazol-4-yl)-5-fluorobenzylcarbamate

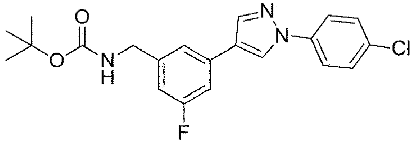

By the same method as in Example 25.3, using tert-butyl (3-fluoro-5-(1H-pyrazol-4-yl)benzyl)carbamate (50 mg, 0.17 mmol), 1-chloro-4-iodobenzene (62 mg, 0.26 mmol), CuI (3.3 mg, 0.017 mmol), L-proline (4 mg, 0.034 mmol), K₂CO₃ (36 mg, 0.26 mmol) and DMSO (1 mL), the title compound (54 mg, 78%) was obtained as an off-white solid.
LC-MS, *m*/*z* 402 [M+H]⁺

### 58.2 (3-(1-(4-Chlorophenyl)-1H-pyrazol-4-yl)-5-fluorophenyl)methanamine

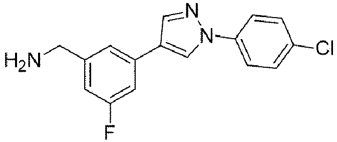

tert-Butyl 3-(1-(4-chlorophenyl)-1H-pyrazol-4-yl)-5-fluorobenzylcarbamate (54 mg, 0.13 mmol) was dissolved in DCM (1.4 mL), trifluoroacetic acid (0.47 mL) was added, and the mixture was stirred at room temperature for 1 h. After completion of the reaction, the reaction mixture was concentrated, adjusted to pH 10~11 with 1 M aq. NaOH, and extracted five times with DCM. The organic layer was dried over MgSO₄ and concentrated to obtain the title compound (20 mg, crude).
LC-MS, *m*/*z* 302 [M+H]⁺

### 58.3 N-(3-(1-(4-chlorophenyl)-1H-pyrazol-4-yl)-5-fluorobenzyl)-8-cyclopentyl-7H-purine-6-carboxamide

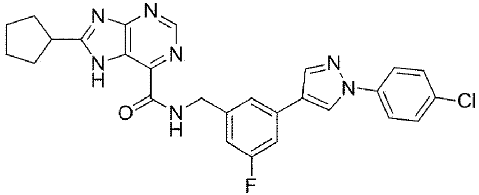

By the same method as in Example 27.3, 8-cyclopentyl-7H-purine-6-carboxylic acid (15.7 mg, 0.068 mmol), T3P (50 wt.% solution in EtOAc, 65 mg, 0.20 mmol), (3-(1-(4-chlorophenyl)-1H-pyrazol-4-yl)-5-fluorophenyl)methanamine (10 mg), DIPEA (52 µL, 0.30 mmol) and EtOAc (1 mL) were used to obtain the title compound (16 mg, 93.5% yield in 2 steps) as a white solid.
¹H NMR (400 MHz, DMSO-*d*₆) *δ* 13.18 (s, 1H), 9.79 (t, J = 5.9 Hz, 1H), 9.10 (s, 1H), 8.97 (s, 1H), 8.28 (s, 1H), 7.91 (d, J = 8.8 Hz, 2H), 7.67 - 7.55 (m, 3H), 7.48 (d, J = 9.6 Hz, 1H), 7.07 (d, J = 9.6 Hz, 1H), 4.61 (d, J = 6.1 Hz, 2H), 3.57 - 3.45 (m, 1H), 2.14 - 2.01 (m, 2H), 2.00 - 1.88 (m, 2H), 1.86 - 1.74 (m, 2H), 1.71 - 1.59 (m, 2H).
LC-MS, *m*/*z* 516 [M+H]⁺

### Example 59

### 59.1 tert-Butyl 3-(1-(4-bromo-2-(methylsulfonyl)phenyl)-1H-pyrazol-4-yl)-5-fluorobenzylcarbamate

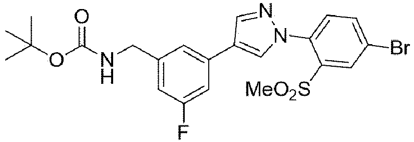

By the same method as in Example 25.3, using tert-butyl (3-fluoro-5-(1H-pyrazol-4-yl)benzyl)carbamate (50 mg, 0.17 mmol), 4-bromo-1-fluoro-2-(methylsulfonyl)benzene (65 mg, 0.26 mmol), CuI (3.3 mg, 0.017 mmol), L-proline (4 mg, 0.034 mmol), K₂CO₃ (36 mg, 0.26 mmol) and DMSO (1 mL), the title compound (76.5 mg, 96%) was obtained as an off-white solid.
LC-MS, *m*/*z* 522 [M-H]⁻

### 59.2 (3-(1-(4-Bromo-2-(methylsulfonyl)phenyl)-1H-pyrazol-4-yl)-5-fluorophenyl)methanamine, trifluoroacetate

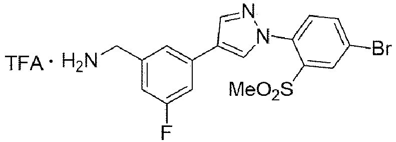

By the same method as in Example 25.4, tert-butyl 3-(1-(4-bromo-2-(methylsulfonyl)phenyl)-1H-pyrazol-4-yl)-5-fluorobenzylcarbamate (70 mg, 0.15 mmol), DCM (1.5 mL), and trifluoroacetic acid (0.5 mL) were used to obtain the title compound (71 mg, crude).
LC-MS, *m*/*z* 293 [M+H]⁺

### 59.3 N-(3-(1-(4-bromo-2-(methylsulfonyl)phenyl)-1H-pyrazol-4-yl)-5-fluorobenzyl)-8-cyclopentyl-7H-purine-6-carboxamide

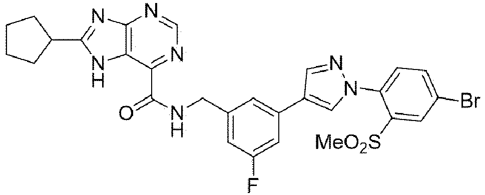

By the same method as in Example 27.3, 8-cyclopentyl-7H-purine-6-carboxylic acid (35 mg, 0.15 mmol), (3-(1-(4-bromo-2-(methylsulfonyl)phenyl)-1H-pyrazol-4-yl)-5-fluorophenyl)methanamine, trifluoroacetate (71 mg, crude), T3P (50 wt.% solution in EtOAc, 144 mg, 0.453 mmol), DIPEA (0.12 mL, 0.68 mmol) and DMF (2 mL) were used to obtain the title compound (33 mg, 39% yield in 2 steps) as an off-white solid.
¹H NMR (400 MHz, DMSO-*d*₆) *δ* 13.17 (s, 1H), 9.78 (t, J = 6.0 Hz, 1H), 8.97 (s, 1H), 8.65 (s, 1H), 8.31 (s, 1H), 8.20 (d, J = 2.2 Hz, 1H), 8.15 (dd, J = 8.5, 2.3 Hz, 1H), 7.68 (d, J = 8.3 Hz, 1H), 7.56 (s, 1H), 7.47 (d, J = 9.4 Hz, 1H), 7.07 (d, J = 9.1 Hz, 1H), 4.59 (d, J = 5.8 Hz, 2H), 3.48 (s, 3H), 2.13 - 2.00 (m, 2H), 1.98 - 1.86 (m, 2H), 1.85 - 1.74 (m, 2H), 1.72 - 1.59 (m, 2H).
LC-MS, *m*/*z* 638 [M+H]⁺

### Example 60

### 60.1 tert-Butyl 3-(1-(3,4-difluorophenyl)-1H-pyrazol-4-yl)-5-fluorobenzylcarbamate

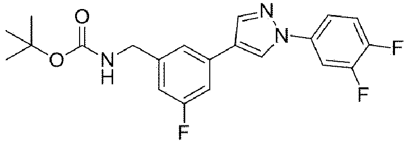

By the same method as in Example 25.3, using tert-butyl (3-fluoro-5-(1H-pyrazol-4-yl)benzyl)carbamate (33.5 mg, 0.12 mmol), 1,2-difluoro-4-iodobenzene (41.4 mg, 0.17 mmol), CuI (2.2 mg, 0.012 mmol), L-proline (2.6 mg, 0.023 mmol), K₂CO₃ (24 mg, 0.17 mmol) and DMSO (1.5 mL), the title compound (19.1 mg, 41%) was obtained as an off-white solid.
LC-MS, *m*/*z* 404 [M+H]⁺

### 60.2 (3-(1-(3,4-Difluorophenyl)-1H-pyrazol-4-yl)-5-fluorophenyl)methanamine, trifluoroacetate

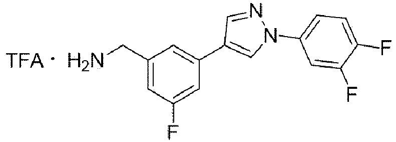

By the same method as in Example 25.4, tert-butyl 3-(1-(3,4-difluorophenyl)-1H-pyrazol-4-yl)-5-fluorobenzylcarbamate (19.1 mg, 0.047 mmol), DCM (0.5 mL), and trifluoroacetic acid (0.16 mL) were used to obtain the title compound (19 mg, crude).
LC-MS, *m*/*z* 304 [M+H]⁺

### 60.3 8-Cyclopentyl-N-(3-(1-(3,4-difluorophenyl)-1H-pyrazol-4-yl)-5-fluorobenzyl)-7H-purine-6-carboxamide

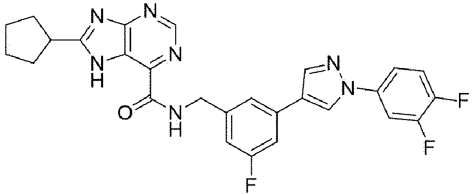

8-Cyclopentyl-7H-purine-6-carboxylic acid (9.2 mg, 0.039 mmol), (3-(1-(3,4-difluorophenyl)-1H-pyrazol-4-yl)-5-fluorophenyl)methanamine, trifluoroacetate (19 mg, crude and HATU (18 mg, 0.047 mmol) were dissolved in DCM (0.5 mL), DIPEA (10 µL, 0.059 mmol) was added, and the mixture was stirred at room temperature for 2 h. After the reaction was completed, ethyl acetate and H₂O were added to the reaction mixture, extracted, and the organic layer was dried over MgSO₄ and concentrated. The obtained residue was purified by silica gel column chromatography (0-4 % MeOH in DCM) to obtain the title compound (4.59 mg, 2 steps, yield 22.5%) as a light yellow solid.
¹H NMR (400 MHz, CD3OD) δ8.95 (s, 1H), 8.64 (s, 1H), 8.07 (s, 1H), 7.81 - 7.73 (m, 1H), 7.60 (d, J = 8.8 Hz, 1H), 7.51 (s, 1H), 7.38 (dd, J = 18.7, 9.0 Hz, 1H), 7.29 (d, J = 9.5 Hz, 1H), 7.04 (d, J = 9.1 Hz, 1H), 4.70 (s, 2H), 3.52 - 3.42 (m, 1H), 2.25 - 2.12 (m, 2H), 2.08 - 1.96 (m, 2H), 1.95 - 1.84 (m, 2H), 1.84 - 1.68 (m, 2H).
LC-MS, *m*/*z* 518 [M+H]⁺

### Example 61

### 61.1 Ethyl 2-chloro-6-((2,4-dimethoxybenzyl)amino)-5-nitropyrimidine-4-carboxylate

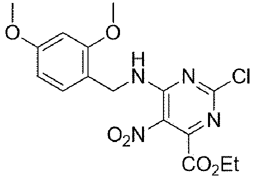

Ethyl 2,6-dichloro-5-nitropyrimidine-4-carboxylate (1.15 g, 4.3 mmol) was dissolved in THF (20 mL), and the mixture was cooled to 0°C under an atmosphere of nitrogen. NaHCO₃ (1.09 g, 12.9 mmol) was added, and a solution of (2,4-dimethoxyphenyl)methanamine (0.83 g, 4.96 mmol) in THF (3 mL) was added dropwise over 15 minutes, and the mixture was stirred at room temperature. After 1 h, the mixture was diluted with EtOAc, washed with H₂O, and the organic layer was dried over MgSO₄ and concentrated. The obtained residue was purified by silica gel column chromatography (20% EtOAc in n-hexane) to obtain the title compound (886 mg, 52%) as a yellow solid.
LC-MS, *m*/*z* 397 [M+H]⁺

### 61.2 Ethyl 5-amino-2-chloro-6-((2,4-dimethoxybenzyl)amino)pyrimidine-4-carboxylate

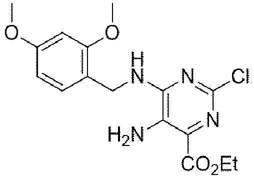

Ethyl 2-chloro-6-((2,4-dimethoxybenzyl)amino)-5-nitropyrimidine-4-carboxylate (481 mg, 1.21 mmol) was added to EtOH (17 mL), and SnCl₂ (1.16 g, 6.1 mmol) was added, and the mixture was stirred at room temperature. After 3 hours, the reaction mixture was concentrated, and H₂O of 0 °C was added, stirred, and the resulting solid was filtered. The filtered solid was added to EtOH, stirred, filtered, and dried to obtain the title compound (390 mg, 88%) as a yellow solid.
LC-MS, *m*/*z* 367 [M+H]⁺

### 61.3 5-Amino-2-chloro-6-((2,4-dimethoxybenzyl)amino)pyrimidine-4-carboxylic acid

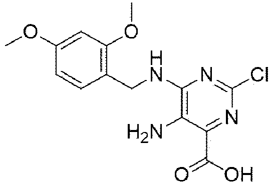

Ethyl 5-amino-2-chloro-6-((2,4-dimethoxybenzyl)amino)pyrimidine-4-carboxylate (1.17 g, 3.19 mmol) was dissolved in THF/EtOH (14 mL/26 mL), 2.0 M NaOH (9.2 mL) was added, and the mixture was stirred at room temperature for 1 h. After completion of the reaction, the mixture was acidified with 3 M HCl at 0 °C (~pH 2), and the resulting solid was filtered to obtain the title compound (850 mg, 79%) as a pale yellow solid.
LC-MS, *m*/*z* 339 [M+H]⁺

### 61.4 5-Amino-2-chloro-6-((2,4-dimethoxybenzyl)amino)-N-(3-fluoro-5-(1-(4-fluorophenyl)-1H-pyrazol-4-yl)benzyl)pyrimidine-4-carboxamide

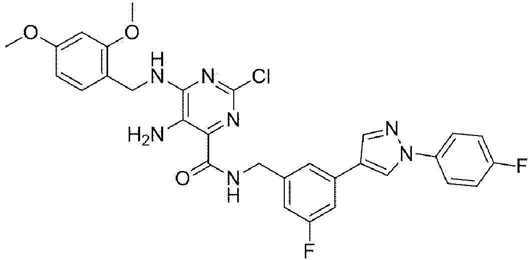

5-Amino-2-chloro-6-((2,4-dimethoxybenzyl)amino)pyrimidine-4-carboxylic acid (1 30 mg, 0.39 mmol), (3-fluoro-5-(1-(4-fluorophenyl)-1H-pyrazol-4-yl)phenyl)methanamine hydrochloride (110 mg, 0.39 mmol), T3P (50% in EtOAc, 773 mg, 1.20 mmol), and DIPEA (174 µL, 1.02 mmol) were dissolved in EtOAc (8 mL) and stirred at 50 °C. After 12 h, ethyl acetate and H₂O were added to the reaction mixture, extracted, and the organic layer was dried over Na₂SO₄ and concentrated The obtained residue was purified by silica gel column chromatography (0-5% MeOH in DCM) to obtain the title compound (13 mg, 55%) as a white solid.
LC-MS, *m*/*z* 607 [M+H]⁺

### 61.5 5,6-Diamino-2-chloro-N-(3-fluoro-5-(1-(4-fluorophenyl)-1H-pyrazol-4-yl)benzyl)pyrimidine-4-carboxamide

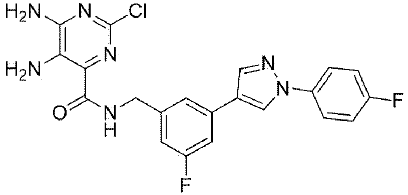

5-Amino-2-chloro-6-((2,4-dimethoxybenzyl)amino)-N-(3-fluoro-5-(1-(4-fluorophenyl)-1H-pyrazol-4-yl)benzyl)pyrimidine-4-carboxamide (120 mg, 0.20 mmol) was dissolved in CH₂Cl₂ (10 mL) solution, TFA (2 mL) was added, and the mixture was stirred at room temperature for 12 h. After completion of the reaction, the solvent was removed, neutralized with aq. NaOH, extracted with EtOAc, and the organic layer was dried over MgSO₄ and concentrated. The obtained residue was purified by silica gel column chromatography (0-5% MeOH in DCM) to obtain the title compound (85 mg, 93%) as a white solid.
LC-MS, *m*/*z* 456 [M+H]⁺

### 61.6 2-Chloro-8-cyclopentyl-N-(3-fluoro-5-(1-(4-fluorophenyl)-1H-pyrazol-4-yl)benzyl)-7H-purine-6-carboxamide

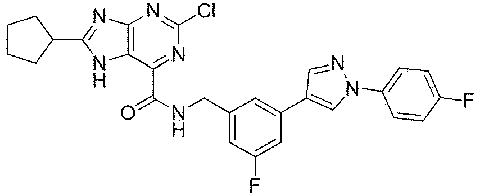

5,6-Diamino-2-chloro-N-(3-fluoro-5-(1-(4-fluorophenyl)-1H-pyrazol-4-yl)benzyl)pyrimidine-4-carboxamide (50 mg, 0.11 mmol), cyclopentanecarboxylic acid (12.5 mg, 0.11 mmol), and T3P (50 wt.% solution in EtOAc, 305 mg, 0.48 mmol) were dissolved in dioxane (3 mL). DIPEA (66 µL, 0.39 mmol) was added, and the mixture was stirred at 150 °C for 5 h using a microwave. After completion of the reaction, ethyl acetate and H₂O were added to the reaction mixture, extracted, and the organic layer was dried over Na₂SO₄ and concentrated. The obtained residue was purified by silica gel column chromatography (0-5% MeOH in DCM) to obtain the title compound (30 mg, 51%) as a white solid.
¹H NMR (400 MHz, CD₃OD) *δ* 8.61 (s, 1H), 8.08 (s, 1H), 7.81 - 7.77 (m, 2H), 7.52 (s, 1H), 7.33 (d, *J =* 12 Hz, 1H), 7.25 - 7.21 (m, 2H), 7.05 (d, *J =* 8.0 Hz, 1H), 4.67 (s, 2H), 3.46 (m, 1H), 2.17 (m, 2H), 2.00 (m, 2H), 1.90 (m, 2H), 1.76 (m, 2H).
LC-MS, *m*/*z* 534 [M+H]⁺

### Example 62

### 62.1 Benzyl (S)-2-(6-chloro-7H-purin-8-yl)pyrrolidine-1-carboxylate

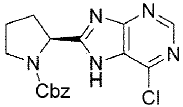

6-Chloropyrimidine-4,5-diamine (484 mg, 3.35 mmol) and benzyl (S)-2-(chlorocarbonyl)pyrrolidine-1-carboxylate (900 mg, 3.36 mmol) were added to POCl₃ (5 mL) and stirred at 110°C. After 2 hours, the reaction mixture was concentrated and neutralized with aq. NaHCO₃. The aqueous layer was extracted twice with CH₂Cl₂, and the organic layer was dried over Na₂SO₄ and concentrated. The obtained residue was recrystallized from toluene to obtain the title compound (420 mg, 35%).
¹H NMR (400 MHz, DMSO-*d*₆) *δ* 8.68 (s, 1H), 7.36 (m, 3H), 6.96 (t, *J =* 7.5 Hz, 1H), 6.81 (d, *J* = 7.4 Hz, 1H), 5.15 - 4.98 (m, 2H), 3.70 (m, 1H), 3.50 (m, 2H), 2.41 (m, 2H), 1.96 (m, 2H).
LC-MS, *m*/*z* 358 [M+H]⁺

### 62.2 Benzyl (S)-2-(6-cyano-7H-purin-8-yl)pyrrolidine-1-carboxylate

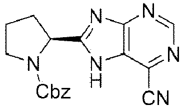

By the same method as in Preparation example 1.1, benzyl (S)-2-(6-chloro-7H-purin-8-yl)pyrrolidine-1-carboxylate (220 mg, 0.61 mmol), KCN (172 mg, 2.64 mmol) and sodium p-toluenesulfinate (110 mg, 0.62 mmol) were used to obtain the title compound (160 mg, 75%).
LC-MS, *m*/*z* 349 [M+H]⁺

### 62.3 Methyl (S)-8-(1-((benzyloxy)carbonyl)pyrrolidin-2-yl)-7H-purine-6-carboxylate

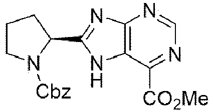

By the same method as in Preparation example 1.2, benzyl (S)-2-(6-cyano-7H-purin-8-yl)pyrrolidine-1-carboxylate (200 mg, 0.57 mmol) and TMSCl (761 µL) were used to obtain the title compound (100 mg, 46%).
LC-MS, *m*/*z* 403 [M+Na]⁺

### 62.4 (S)-8-(1-((benzyloxy)carbonyl)pyrrolidin-2-yl)-7H-purine-6-carboxylic acid

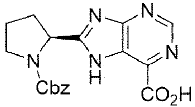

By the same method as in Preparation example 1.3, methyl (S)-8-(1-((benzyloxy)carbonyl)pyrrolidin-2-yl)-7H-purine-6-carboxylate (100 mg, 0.26 mmol) and 2 M NaOH (834 µL) were used to obtain the title compound (85 mg, 89%).
¹H NMR (400 MHz, DMSO-*d*₆) *δ* 13.21 (s, 1H), 9.02 (s, 1H), 7.36 (m, 3H), 6.91 (t, J = 7.5 Hz, 1H), 6.78 (d, J = 7.5 Hz, 1H), 5.25 (m, 1H), 5.12 - 4.93 (m, 2H), 3.70 (m, 2H), 3.54 (m, 2H), 2.47 - 2.26 (m, 2H), 2.20 - 1.80 (m, 2H).
LC-MS, *m*/*z* 368 [M+H]⁺

### 62.5 Benzyl (S)-2-(6-((3-fluoro-5-(1-methyl-1H-pyrazol-4-yl)benzyl)carbamoyl)-7H-purin-8-yl)pyrrolidine-1-carboxylate

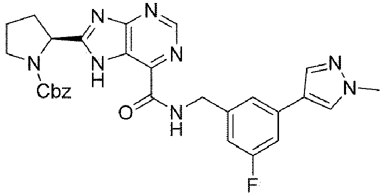

By the same method as in Example 1.3, using (S)-8-(1-((benzyloxy)carbonyl)pyrrolidine-2-yl)-7H-purine-6-carboxylic acid (85 mg, 0.23 mmol), (3-fluoro-5-(1-methyl-1H-pyrazol-4-yl)phenyl)methanamine (52 mg, 0.25 mmol), T3P (50 wt.% solution in EtOAc; 439 mg), and DIPEA (120 µl), the title compound (110 mg, 86%) was obtained.
¹H NMR (400 MHz, DMSO-*d*₆) *δ* 13.38 (s, 1H), 9.81 (s, 1H), 9.00 (s, 1H), 8.17 (s, 1H), 7.89 (s, 1H), 7.45 (d, *J* = 9.6 Hz, 1H), 7.37 (m, 3H), 7.02 (d, *J* = 9.6 Hz, 1H), 6.98 - 6.89 (m, 1H), 6.83 (t, *J* = 7.5 Hz, 1H), 6.75 (d, *J =* 7.4 Hz, 1H), 5.30 - 5.20 (m, 1H), 5.05 (m, 2H), 4.59 (d, *J =* 5.5 Hz, 2H), 3.86 (s, 3H), 3.70 (m, 2H), 2.45 - 2.26 (m, 2H), 2.04 - 1.80 (m, 2H).
LC-MS *m*/*z* 368 [M+H]⁺

### 62.6 (S)-N-(3-fluoro-5-(1-methyl-1H-pyrazol-4-yl)benzyl)-8-(pyrrolidin-2-yl)-7H-purine-6-carboxamide hydrochloride

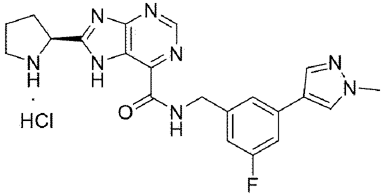

Benzyl (S)-2-(6-((3-fluoro-5-(1-methyl-1H-pyrazol-4-yl)benzyl)carbamoyl)-7H-purin-8-yl)pyrrolidine-1-carboxylate (100 mg, 0.18 mmol) was dissolved in dioxane (5 mL), HCl (35~37%, 1.82 mL) was added, and the mixture was stirred at 80°C. After 1 h, the reaction mixture was cooled to room temperature and concentrated, followed by adding EtOAc and stirring. The solid was filtered and dried to obtain the title compound (60 mg, 73%).
¹H NMR (400 MHz, DMSO-*d*₆) *δ* 10.44 (s, 1H), 9.86 (s, 1H), 9.42 (s, 1H), 9.11 (s, 1H), 8.17 (s, 1H), 7.87 (s, 1H), 7.43 (s, 1H), 7.30 (d, *J* = 10.0 Hz, 1H), 7.00 (d, *J* = 9.7 Hz, 1H), 5.09 (m, 1H), 4.59 (d, *J* = 6.3 Hz, 2H), 3.85 (s, 3H), 3.74 - 3.68 (m, 2H), 3.50 - 3.46 (m, 2H), 2.20 - 2.06 (m, 2H).
LC-MS *m*/*z* 421 [M+H]⁺

### Example 63

### (S)-N-(3-fluoro-5-(1-methyl-1H-pyrazol-4-yl)benzyl)-8-(1-methylpyrrolidin-2-yl)-7H-purine-6-carboxamide

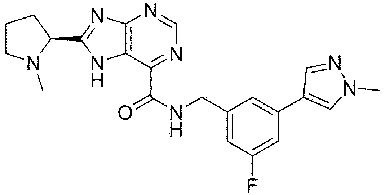

(S)-N-(3-Fluoro-5-(1-methyl-1H-pyrazol-4-yl)benzyl)-8-(pyrrolidin-2-yl)-7H-purine-6-carboxamide hydrochloride (45 mg, 0.11 mmol), paraformaldehyde (15 mg), and NaBH₄ (9 mg) were added to trifluoroethanol (3 mL) and refluxed. After 1 h, the resulting solid was filtered, and the filtrate was concentrated. The obtained residue was purified by silica gel column chromatography (0-5% MeOH in DCM) to obtain the title compound (15 mg, 31%).
¹H NMR (400 MHz, DMSO-*d*₆) δ9.75 (s, 1H), 9.00 (s, 1H), 8.17 (s, 1H), 7.89 (s, 1H), 7.43 (s, 1H), 7.31 (d, J = 10.3 Hz, 1H), 7.00 (d, J = 9.6 Hz, 1H), 4.57 (d, J = 6.3 Hz, 2H), 3.85 (s, 3H), 3.15 - 3.05 (m, 1H), 2.43 (m, 2H), 2.22 (s, 3H), 2.02 (m, 2H), 1.87 (m, 2H).
LC-MS, *m*/*z* 435 [M+H]⁺

### Example 64

### 64.1 Benzyl (R)-2-(6-chloro-7H-purin-8-yl)pyrrolidine-1-carboxylate

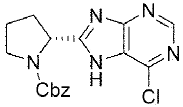

By the same method as in Example 62.1, 6-chloropyrimidine-4,5-diamine (1.16 g, 8.0 mmol) and benzyl (R)-2-(chlorocarbonyl)pyrrolidine-1-carboxylate (2.14 g, 8.0 mmol) were used to obtain the title compound (960 mg, 34%).
¹H NMR (400 MHz, DMSO-*d*₆) *δ* 8.66 (s, 1H), 7.36 (m, 3H), 7.09 (m, 1H), 6.85 (s, 1H), 5.07 (d, *J* = 10.3 Hz, 2H), 4.81 (m, 1H), 3.70 (m, 2H), 2.41 (m, 2H), 1.98 (m, 2H).
LC-MS, *m*/*z* 358 [M+H]⁺

### 64.2 Benzyl (R)-2-(6-cyano-7H-purin-8-yl)pyrrolidine-1-carboxylate

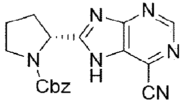

By the same method as in Preparation example 1.1, benzyl (R)-2-(6-chloro-7H-purin-8-yl)pyrrolidine-1-carboxylate (250 mg, 0.70 mmol) was used to obtain the title compound (200 mg, 82%).
¹H NMR (400 MHz, DMSO-*d*₆) *δ* 9.01 (s, 1H), 7.45 (m, 3H), 6.98 (m, 1H), 6.84 (s, 1H), 5.08 (m, 2H), 4.78 (m, H), 3.70 (m, 2H), 2.50 (m, 2H), 2.02 (m, 2H).
LC-MS, *m*/*z* 349 [M+H]⁺

### 64.3 Methyl (R)-8-(1-((benzyloxy)carbonyl)pyrrolidin-2-yl)-7H-purine-6-carboxylate

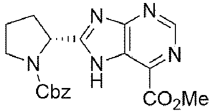

By the same method as in Preparation example 1.2, benzyl (R)-2-(6-cyano-7H-purin-8-yl)pyrrolidine-1-carboxylate (300 mg, 0.86 mmol) was used to obtain the title compound (200 mg, 61%).
¹H NMR (400 MHz, CD₃OD) *δ* 9.02(s, 1H), 7.35 (m, 3H), 5.08 (m, 2H), 4.68 (m, 1H), 3.31 (m, 2H), 2.50 (m, 2H), 2.19 (m, 2H).
LC-MS, *m*/*z* 382 [M+H]⁺

### 64.4 (R)-8-(1-((benzyloxy)carbonyl)pyrrolidin-2-yl)-7H-purine-6-carboxylic acid

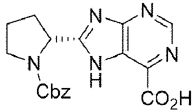

By the same method as in Preparation example 1.3, methyl (R)-8-(1-((benzyloxy)carbonyl)pyrrolidin-2-yl)-7H-purine-6-carboxylate (200 mg, 0.53 mmol) was used to obtain the title compound (140 mg, 72%).
¹H NMR (400 MHz, DMSO-*d*₆) *δ* 13.26 (s, 1H), 9.02 (s, 1H), 7.36 (m, 3H), 6.91 (t, *J* = 7.6 Hz, 1H), 6.77 (d, *J =* 7.5 Hz, 1H), 5.26 (m, 1H), 5.04 (m, 2H), 3.78 - 3.63 (m, 2H), 2.40 (m, 2H), 2.18 - 1.80 (m, 2H).
LC-MS, *m*/*z* 368 [M+H]⁺

### 64.5 Benzyl (R)-2-(6-((3-fluoro-5-(1-methyl-1H-pyrazol-4-yl)benzyl)carbamoyl)-7H-purin-8-yl)pyrrolidine-1-carboxylate

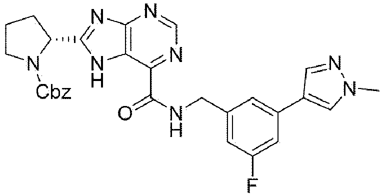

By the same method as in Example 1.3, (R)-8-(1-((benzyloxy)carbonyl)pyrrolidine-2-yl)-7H-purine-6-carboxylic acid (140 mg, 0.38 mmol), (3-fluoro-5-(1-methyl-1H-pyrazol-4-yl)phenyl)methanamine (86 mg, 0.42 mmol), T3P (50 wt. % solution in EtOAc, 724 mg, 1.14 mmol) and DIPEA (198 µ, 1.17 mmol) were used to obtain the title compound (170 mg, 81%).
¹H NMR (400 MHz, DMSO-*d*₆) *δ* 13.38 (s, 1H), 9.81 (s, 1H), 9.01 (s, 1H), 8.17 (s, 1H), 7.88 (s, 1H), 7.43 (d, *J =* 10.7 Hz, 1H), 7.36 (m, 3H), 7.00 (d, *J =* 9.6 Hz, 1H), 6.92 (m,, 1H), 6.82 (t, *J* = 7.3 Hz, 1H), 6.74 (d, *J =* 7.5 Hz, 1H), 5.25 (m, 1H), 5.04 (m, 2H), 4.58 (d, *J =* 12.7 Hz, 2H), 3.85 (s, 3H), 3.69 (m, 2H), 2.37 (m, 2H), 2.09 (m, 2H).
LC-MS, *m*/*z* 555 [M+H]⁺

### Example 65

### (R)-N-(3-fluoro-5-(1-methyl-1H-pyrazol-4-yl)benzyl)-8-(pyrrolidin-2-yl)-7H-purine-6-carboxamide hydrochloride

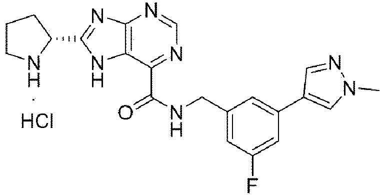

By the same method as in Example 62.6, benzyl (R)-2-(6-((3-fluoro-5-(1-methyl-1H-pyrazol-4-yl)benzyl)carbamoyl)-7H-purin-8-yl)pyrrolidine-1- carboxylate (160 mg, 0.29 mmol) and HCl (35-37%, 2.9 mL) were used to obtain the title compound (100 mg, 75%).
¹H NMR (400 MHz, DMSO-*d*₆) *δ* 10.66 (s, 1H), 9.85 (s, 1H), 9.44 (s, 1H), 9.11 (s, 1H), 8.17 (s, 1H), 7.88 (s, 1H), 7.44 (s, 1H), 7.30 (d, *J =* 10.0 Hz, 1H), 7.00 (d, *J =* 9.7 Hz, 1H), 5.17 - 5.03 (m, 1H), 4.59 (d, *J =* 6.3 Hz, 2H), 3.85 (s, 3H), 3.74 - 3.61 (m, 2H), 3.53 - 3.45 (m, 2H), 2.28 - 1.97 (m, 2H).
LC-MS, *m*/*z* 421 [M+H]⁺

### Example 66

### (R)-N-(3-fluoro-5-(1-methyl-1H-pyrazol-4-yl)benzyl)-8-(1-methylpyrrolidin-2-yl)-7H-purine-6-carboxamide

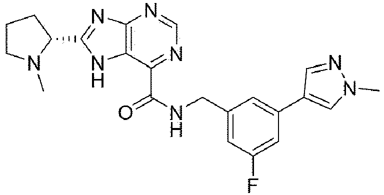

By the same method as in Example 63, (R)-N-(3-fluoro-5-(1-methyl-1H-pyrazol-4-yl)benzyl)-8-(pyrrolidin-2-yl)-7H-purine-6-carboxamide hydrochloride (45 mg, 0.1 mmol), paraformaldehyde (15 mg), NaBH₄ (9 mg) and trifluoroethanol (3 mL) were used to obtain the title compound (22 mg, 50%).
¹H NMR (400 MHz, DMSO-*d*₆) *δ* 9.74 (s, 1H), 8.99 (s, 1H), 8.17 (s, 1H), 7.89 (s, 1H), 7.43 (s, 1H), 7.31 (d, *J =* 10.4 Hz, 1H), 7.00 (d, *J =* 9.6 Hz, 1H), 4.57 (d, *J* = 6.4 Hz, 2H), 3.85 (s, 3H), 3.10 (m, 1H), 2.48 - 2.35 (m, 2H), 2.22 (s, 3H), 2.01 (m,2 H), 1.85 (. 2H).
LC-MS, *m*/*z* 435 [M+H]⁺

### Example 67

### (S)-N-(3-fluoro-5-(1-methyl-1H-pyrazol-4-yl)benzyl)-8-(1-isopropylpyrrolidin-2-yl)-7H-purine-6-carboxamide

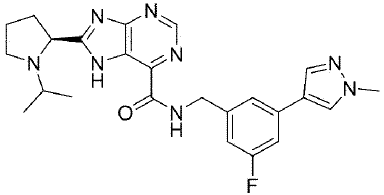

By the same method as in Example 63, (S)-N-(3-fluoro-5-(1-methyl-1H-pyrazol-4-yl)benzyl)-8-(pyrrolidin-2-yl)-7H-purine-6-carboxamide hydrochloride (20 mg, 0.05 mmol), acetone (30 µL), NaBH₄ (3.3 mg) and trifluoroethanol (1 mL) were used to obtain the title compound (11 mg, 48%).
¹H NMR (400 MHz, CDCl₃) *δ* 9.03 (s, 1H), 8.47 (s, 1H), 7.75 (s, 1H), 7.62 (s, 1H), 7.25 (s, 1H), 7.12 - 7.07 (m, 1H), 6.96 (m, 1H), 4.72 (m, 2H), 4.35 (m, 1H), 3.94 (s, 3H), 3.23 (m, 1H), 2.92 - 2.63 (m, 2H), 2.43 - 2.03 (m, 2H), 1.81 (m, 2H), 1.09 (m, 6H).
LC-MS, *m*/*z*: 463 [M+H]⁺

### Example 68

### 68.1 benzyl (R)-2-(6-((3-fluorobenzyl)carbamoyl)-7H-purin-8-yl)pyrrolidine-1-carboxylate

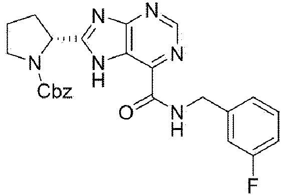

By the same method as in Example 1.3, (R)-8-(1-((benzyloxy)carbonyl)pyrrolidine-2-yl)-7H-purine-6-carboxylic acid (130 mg, 0.35 mmol), (3-fluorophenyl)methanamine (41 µL), T3P (50 wt.% solution in EtOAc, 687 mg, 1.08 mmol), and DIPEA (156 µL, 0.90 mmol) were used to obtain the title compound (155 mg, 93%).
¹H NMR (400 MHz, CDCl₃) *δ* 11.63 (s, 1H), 9.04 (s, 1H), 8.38 (s, 1H), 7.42 - 7.34 (m, 5H), 7.17 - 7.01 (m, 4H), 5.31 (s, 2H), 5.20 (m, 1H), 4.72 (d, *J* = 12 Hz, 2H), 3.55 (m, 2H), 2.25 (m, 2H), 2.11 (m, 2H).
LC-MS, *m*/*z* 475 [M+H]⁺

### 68.2 (R)-N-(3-fluorobenzyl)-8-(pyrrolidin-2-yl)-7H-purine-6-carboxamide hydrochloride

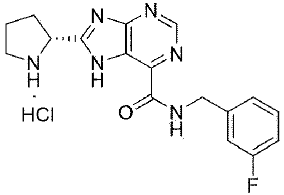

By the same method as in Example 62.6, benzyl (R)-2-(6-((3-fluorobenzyl)carbamoyl)-7H-purin-8-yl)pyrrolidine-1-carboxylate (100 mg, 0.21 mmol) and HCl (35-37%, 1.85 mL) were used to obtain the title compound (70 mg, 88%).
¹H NMR (400 MHz, DMSO-*d*₆) *δ* 10.57 (s, 1H), 9.91 (s, 1H), 9.43 (s, 1H), 9.11 (s, 1H), 7.39 (m, 1H), 7.21 (m, 2H), 7.08 (m, 1H), 5.09 (m, 1H), 4.58 (d, *J* = 4 Hz, 2H), 3.72 - 3.68 (m, 2H), 2.21 - 2.08 (m, 4H).
LC-MS, *m*/*z* 341 [M+H]⁺

### 68.3 (R)-N-(3-fluorobenzyl)-8-(1-methylpyrrolidin-2-yl)-7H-purine-6-carboxamide

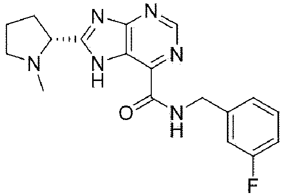

By the same method as in Example 63, (R)-N-(3-fluorobenzyl)-8-(pyrrolidin-2-yl)-7H-purine-6-carboxamide hydrochloride (70 mg, 0.19 mmol), paraformaldehyde (28 mg), NaBH₄ (17 mg) and trifluoroethanol (3 mL) were used to obtain the title compound (6.7 mg, 10%).
LC-MS, *m*/*z* 355 [M+H]⁺

### Example 69

### 69.1 Benzyl 4-(6-chloro-7H-purin-8-yl)piperidine-1-carboxylate

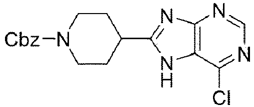

By the same method as in Example 62.6, 6-chloropyrimidine-4,5-diamine (500 mg, 3.5 mmol), benzyl 4-(chlorocarbonyl)piperidine-1-carboxylate (974 mg, 3.5 mmol) and POCl₃ (5 mL) were used to obtain the title compound (176 mg, 14%).
¹H NMR (400 MHz, CD₃OD) *δ* 8.64 (s, 1H), 7.35 (dd, *J =* 13.7, 7.0 Hz, 5H), 5.15 (s, 2H), 4.28 (d, *J =* 12.7 Hz, 2H), 3.06 (s, 3H), 2.10 (d, *J* = 12.0 Hz, 2H), 1.88 (d, *J* = 8.4 Hz, 2H).
LC-MS, *m*/*z* 372 [M+H]⁺

### 69.2 Benzyl 4-(6-cyano-7H-purin-8-yl)piperidine-1-carboxylate

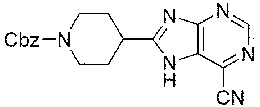

By the same method as in Preparation example 1.1, benzyl 4-(6-chloro-7H-purin-8-yl)piperidine-1-carboxylate (150 mg, 0.4 mmol), KCN (105 mg, 1.6 mmol), sodium p-toluenesulfinate (72 mg, 0.4 mmol), and sulfolane (3 mL) were used to obtain the title compound (69 mg, 35%).
¹H NMR (400 MHz, DMSO-*d*₆) *δ* 14.02 (s, 1H), 8.99 (s, 1H), 7.40 - 7.32 (m, 5H), 5.11 (s, 2H), 4.10 (d, *J =* 13.1 Hz, 2H), 3.07 (s, 3H), 2.08 (d, *J =* 10.2 Hz, 2H), 1.77 (d, *J =* 8.2 Hz, 2H).
LC-MS, *m*/*z* 363 [M+H]⁺

### 69.3 Methyl 8-(1-((benzyloxy)carbonyl)piperidin-4-yl)-7H-purine-6-carboxylate

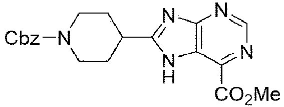

By the same method as in Preparation example 1.2, benzyl 4-(6-cyano-7H-purin-8-yl)piperidine-1-carboxylate (190 mg, 0.52 mmol) and TMSCl (680 µL, 5.4 mmol) were used to obtain the title compound (104 mg, 50%).

¹H NMR (400 MHz, DMSO-*d*₆) *δ* 13.07 (s, 1H), 9.00 (s, 1H), 7.37 (t, *J* = 11.0 Hz, 5H), 5.12 (s, 2H), 4.13 (d, *J =* 12.7 Hz, 2H), 4.00 (s, 3H), 3.02 (s, 3H), 2.03 (d, *J =* 13.9 Hz, 2H), 1.82 (s, 2H).

### 69.4 8-(1-((Benzyloxy)carbonyl)piperidin-4-yl)-7H-purine-6-carboxylic acid

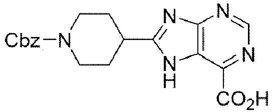

By the same method as in Preparation example 1.3, methyl 8-(1-((benzyloxy)carbonyl)piperidin-4-yl)-7H-purine-6-carboxylate (100 mg, 0.25 mmol) and 2 M NaOH (750 µL, 1.5 mmol) were used to obtain the title compound (95 mg, crude).
LC-MS, *m*/*z* 382 [M+H]⁺

### 69.5 Benzyl 4-(6-((3-fluoro-5-(1-methyl-1H-pyrazol-4-yl)benzyl)carbamoyl)-7H-purin-8-yl)piperidine-1-carboxylate

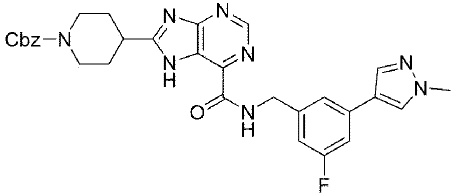

By the same method as in Example 1.3, 8-(1-((benzyloxy)carbonyl)piperidin-4-yl)-7H-purine-6-carboxylic acid (80 mg, crude), (3-fluoro-5-(1-methyl-1H-pyrazol-4-yl)phenyl)methanamine (52 mg, 0.25 mmol), T3P (50 wt.% solution in EtOAc, 187 µL, 0.63 mmol) and DIPEA (71 µL, 0.42 mmol) were used to obtain the title compound (50 mg, 42%).
¹H NMR (400 MHz, DMSO-*d*₆) *δ* 13.24 (s, 1H), 9.76 (s, 1H), 8.99 (s, 1H), 8.16 (s, 1H), 7.88 (s, 1H), 7.42 - 7.29 (m, 7H), 6.99 (d, *J =* 9.4 Hz, 1H), 5.11 (s, 2H), 4.57 (d, *J =* 6.2 Hz, 2H), 4.11 (d, *J* = 13.2 Hz, 2H), 3.85 (s, 3H), 3.00 (s, 3H), 1.99 (s, 2H), 1.81 (d, *J* = 8.9 Hz, 2H).
LC-MS, *m*/*z* 569 [M+H]⁺

### Example 70

### N-(3-fluoro-5-(1-methyl-1H-pyrazol-4-yl)benzyl)-8-(piperidin-4-yl)-7H-purine-6-carboxamide hydrochloride

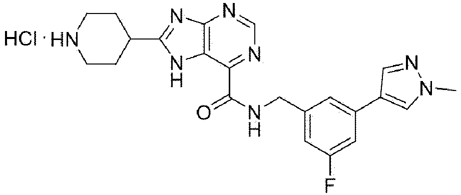

4-(6-((3-Fluoro-5-(1-methyl-1H-pyrazol-4-yl)benzyl)carbamoyl)-7H-purin-8-yl) piperidine-1-carboxylate (40 mg, 0.070 mmol) was dissolved in dioxane (4 mL), HCl (35~37%, 728 µL) was added, and the mixture was heated and stirred at 80°C. After 1 h, the reaction mixture was concentrated, co-evaporated three times with toluene, and then dried under vacuum to obtain the title compound (32 mg, 99%).
¹H NMR (400 MHz, CD₃OD) *δ* 9.03 (s, 1H), 7.99 (s, 1H), 7.83 (s, 1H), 7.42 (s, 1H), 7.20 (d, J = 10.2 Hz, 1H), 7.00 (d, J = 9.8 Hz, 1H), 4.69 (s, 2H), 3.92 (d, J = 10.2 Hz, 3H), 3.57 (d, J = 12.8 Hz, 2H), 3.48 (m, 1H), 3.25 - 3.18 (m, 2H), 2.35 (m, 2H), 2.21 (m, 2H).
LC-MS, *m*/*z* 435 [M+H]⁺

### Example 71

### N-(3-fluoro-5-(1-methyl-1H-pyrazol-4-yl)benzyl)-8-(1-methylpiperidin-4-yl)-7H-purine-6-carboxamide

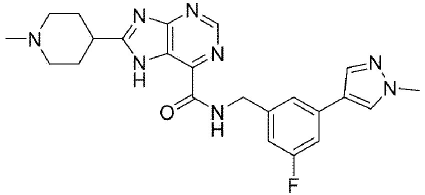

By the same method as in Example 63, N-(3-fluoro-5-(1-methyl-1H-pyrazol-4-yl)benzyl)-8-(piperidin-4-yl)-7H-purine-6-carboxamide hydrochloride (35 mg, 0.074 mmol), paraformaldehyde (10 mg), NaBH₄ (6 mg, 0.15 mmol) and trifluoroethanol (2 mL) were used to obtain the title compound (8 mg, 23%).
¹H NMR (400 MHz, DMSO-*d*₆) *δ* 13.18 (s, 1H), 9.74 (s, 1H), 8.98 (s, 1H), 8.16 (s, 1H), 7.88 (s, 1H), 7.42 (s, 1H), 7.30 (d, *J =* 10.4 Hz, 1H), 6.99 (d, *J =* 9.3 Hz, 1H), 4.57 (d, *J =* 5.9 Hz, 2H), 3.85 (s, 3H), 3.04 (s, 1H), 2.91 (d, *J* = 14.3 Hz, 2H), 2.24 (s, 3H), 2.09 - 1.88 (m, 6H).
LC-MS, *m*/*z* 449 [M+H]⁺

### Example 72

### 72.1 Ethyl 5-amino-6-((2,4-dimethoxybenzyl)amino)pyrimidine-4-carboxylate

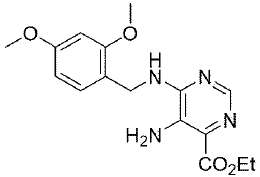

Ethyl 5-amino-2-chloro-6-((2,4-dimethoxybenzyl)amino)pyrimidine-4-carboxylate (270 mg, 0.74 mmol) was dissolved in THF/EtOH (9 mL/9 mL), ammonium formate (464 mg, 7.36 mmol) and 10% palladium on carbon (Pd/C; 100 mg) were added, and the mixture was stirred at 60°C. After 30 minutes, the solid of the reaction mixture was removed by filtration, and the filtrate was concentrated to obtain the title compound (163 mg, 67%).
LC-MS, *m*/*z* 333 [M+H]⁺

### 72.2 5-Amino-6-((2,4-dimethoxybenzyl)amino)pyrimidin-4-carboxylic acid

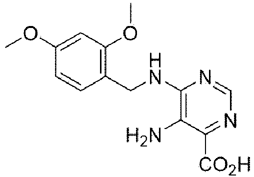

Ethyl 5-amino-6-((2,4-dimethoxybenzyl)amino)pyrimidine-4-carboxylate (130 mg, 0.39 mmol) was dissolved in THF/EtOH (1.6 mL/3 mL), 2.0 M NaOH (1.02 mL) was added, and the mixture was stirred at room temperature. After 1 h, the reaction mixture was cooled to 0°C, acidified to pH 5 with 3 M HCl, and the resulting solid was filtered to obtain the title compound (88 mg, 74%).
LC-MS, *m*/*z* 305 [M+H]⁺

### 72.3 5-Amino-6-((2,4-dimethoxybenzyl)amino)-N-(3-fluoro-5-(1-methyl-1H-pyrazol-4-yl)benzyl)pyrimidine-4-carboxamide

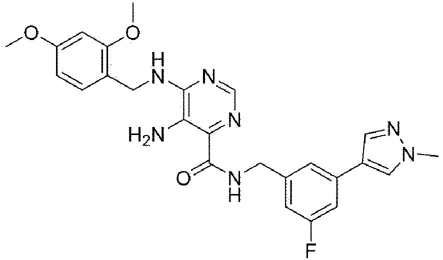

(3-Fluoro-5-(1-methyl-1H-pyrazol-4-yl)phenyl)methanamine (65 mg, 0.31 mmol), T3P (50 wt.% solution in EtOAc) (512 mg, 0.80 mmol) and DIPEA (116 µL, 0.68 mmol) were added to a solution of 5-amino-6-((2,4-dimethoxybenzyl)amino)pyrimidine-4-carboxylic acid (80 mg, 0.26 mmol) and DMF (5 mL), and the mixture was stirred at 50 °C. After stirring overnight, H₂O was added to the reaction mixture, and extracted three times with EtOAc. The organic layer was dried over MgSO₄ and concentrated, and the obtained residue was purified by silica gel column chromatography (0-5% MeOH in DCM) to obtain the title compound (46 mg, 36%).
LC-MS, *m*/*z* 492 [M+H]⁺

### 72.4 5,6-Diamino-N-(3-fluoro-5-(1-methyl-1H-pyrazol-4-yl)benzyl)pyrimidine-4-carboxamide

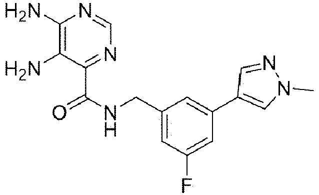

5-Amino-6-((2,4-dimethoxybenzyl)amino)-N-(3-fluoro-5-(1-methyl-1H-pyrazol-4-yl)benzyl)pyrimidine-4-carboxamide (230 mg, 0.47 mmol) was dissolved in CH₂Cl₂ (5 mL) solution, TFA (2 mL) was added, and the mixture was stirred at room temperature. After 12 h, the reaction mixture was concentrated, neutralized with aq. NaOH, and extracted with EtOAc. The organic layer was dried over MgSO₄ and concentrated, and the obtained residue was purified by silica gel column chromatography (0-5% MeOH in DCM) to obtain the title compound (130 mg, 81%).
LC-MS, *m*/*z* 342 [M+H]⁺

### 72.5 Benzyl ((6-((3-fluoro-5-(1-methyl-1H-pyrazol-4-yl)benzyl)carbamoyl)-7H-purin-8-yl)methyl)carbamate

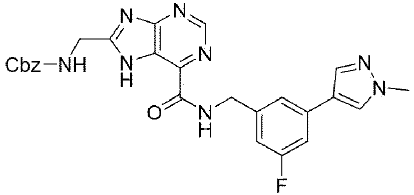

To a solution of 5,6-diamino-N-(3-fluoro-5-(1-methyl-1H-pyrazol-4-yl)benzyl)pyrimidine-4- carboxamide (160 mg, 0.46 mmol) in DMF (10 mL) were added ((benzyloxy)carbonyl)glycine (96 mg, 0.46 mmol), T3P (50 wt. % solution in EtOAc, 927 mg, 1.45 mmol) and DIPEA (234 µL, 1.37 mmol), and the mixture was stirred at 150 °C using a microwave. After 1 h, the reaction mixture was concentrated, diluted with CH₂Cl₂, and washed with H₂O. The organic layer was dried over MgSO₄ and concentrated, and the resulting residue was purified by silica gel column chromatography (0-5% MeOH in DCM) to obtain the title compound (65 mg, 27%).
¹H NMR (400 MHz, CD₃OD) *δ* 8.97 (s, 1H), 7.95 (s, 1H), 7.80 (s, 1H), 7.40 (s, 1H), 7.34 - 7.31 (m, 4H), 7.19 (d, *J* = 12 Hz, 1H), 6.98 (t, *J* = 8.0 Hz, 2H), 5.10 (s, 2H), 4.67 (s, 2H), 4.66(s, 2H), 3.88 (s, 3H).
LC-MS, *m*/*z* 515 [M+H]⁺

### Example 73

### 8-(Aminomethyl)-N-(3-fluoro-5-(1-methyl-1H-pyrazol-4-yl)benzyl)-7H-purine-6-carboxamide

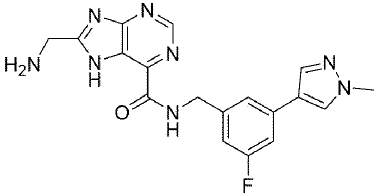

Benzyl ((6-((3-fluoro-5-(1-methyl-1H-pyrazol-4-yl)benzyl)carbamoyl)-7H-purin- 8-yl)methyl)carbamate (60 mg, 0.12 mmol) was dissolved in dioxane (2.5 mL), and HCl (35-37%, 1.2 mL) was added. The mixture was heated and stirred at 80 °C for 1 hour. The reaction mixture was concentrated and recrystallized from toluene, and the resulting solid was filtered. The solid was dried to obtain the title compound (45 mg, 90%).
¹H NMR (400 MHz, CD₃OD) *δ* 9.10 (s, 1H), 8.23 (s, 1H), 8.13 (s, 1H), 7.46 (s, 1H), 7.26 (d, *J* = 12 Hz, 1H), 7.06 (d, *J* = 8.0 Hz, 1H), 4.70 (s, 2H), 4.60 (s, 2H), 3.99 (s, 3H).
LC-MS, *m*/*z* [M+H]⁺

### Example 74

### 74.1 Methyl 7H-purine-6-carboxylate

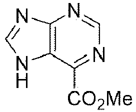

By the same method as in Preparation example 1.2, 7H-purine-6-carbonitrile (80 mg, 0.55 mmol) and TMSCl (760 µL) were used to obtain the title compound (63 mg, 64%).
LC-MS, *m*/*z* 247 [M+H]⁺

### 74.2 7H-purine-6-carboxylic acid

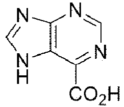

By the same method as in Preparation example 1.3, using methyl 7H-purine-6-carboxylate (60 mg, 0.34 mmol) and 2 M NaOH (850 µL), the title compound (45 mg, 81%) was obtained.
LC-MS, *m*/*z* 165 [M+H]⁺

### 74.3 N-(3-fluoro-5-(1-methyl-1H-pyrazol-4-yl)benzyl)-7H-purine-6-carboxamide

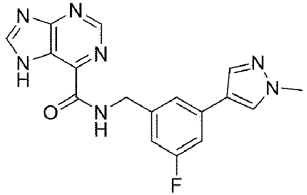

By the same method as in Example 1.3, 7H-purine-6-carboxylic acid (40 mg, 0.17 mmol), (3-fluoro-5-(1-methyl-1H-pyrazol-4-yl)phenyl)methanamine (55 mg, 0.21 mmol), T3P (50 wt.% solution in EtOAc, 335 mg, 1.06 mmol), and DIPEA (76 µL, 0.44 mmol) were used to obtain the title compound (17 mg, 28%).
¹H NMR (400 MHz, DMSO-*d*₆) δ13.45 (s, 1H),9.78 (s, 1H), 9.05 (s, 1H), 8.73 (s, 1H), 8.14 (s, 1H), 7.86 (s, 1H), 7.40 (s, 1H), 7.30 (d, J = 12 Hz, 1H), 6.99 (d, J = 12 Hz, 1H), 4.57 (d, J = 8.0 Hz, 2H), 3.83 (s, 3H).
LC-MS, *m*/*z* 352 [M+H]⁺

### Example 75

### Benzyl 3-(6-((3-fluoro-5-(1-methyl-1H-pyrazol-4-yl)benzyl)carbamoyl)-7H-purin-8-yl)pyrrolidine-1-carboxylate

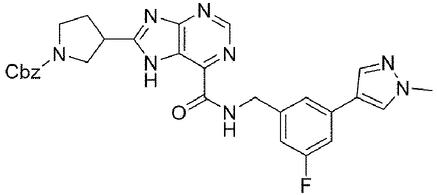

By the same method as in Example 72.5, 5,6-diamino-N-(3-fluoro-5-(1- methyl-1H-pyrazol-4-yl)benzyl)pyrimidine-4-carboxamide (50 mg, 0.15 mmol), 1-((benzyloxy)carbonyl)pyrrolidine-3-carboxylic acid (37 mg, 0.15 mmol), T3P (50 wt. % solution in EtOAc, 305 mg, 0.48 mmol) and DIPEA (66 µL, 0.39 mmol) were used to obtain the title compound (45 mg, 54%).
¹H NMR (400 MHz, CD₃OD) *δ* 8.98 (s, 1H), 7.96 (s, 1H), 7.81 (s, 1H), 7.41 (s, 1H), 7.35 - 7.31 (m, 5H), 7.20 (d, *J* = 12 Hz, 1H), 7.00 (d, *J* = 12 Hz, 1H), 5.13 (d, J = 8.0 Hz, 2H), 4.67 (s, 2H), 3.89 (s, 3H), 3.71 (m, 1H), 3.56 (m, 4H), 2.44 (m, 2H).
LC-MS *m*/*z* 555 [M+H]⁺

### Example 76

### N-(3-fluoro-5-(1-methyl-1H-pyrazol-4-yl)benzyl)-8-(pyrrolidin-3-yl)-7H-purine-6-carboxamide hydrochloride

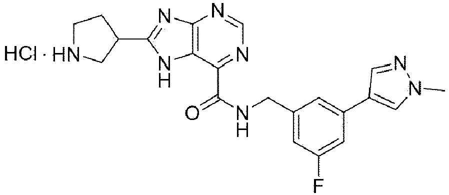

By the same method as in Example 73, benzyl 3-(6-((3-fluoro-5-(1-methyl-1H-pyrazol-4-yl)benzyl)carbamoyl)-7H-purin-8-yl)pyrrolidine-1-carboxylate (Example 108) (30 mg, 0.058 mmol) and HCl (35-37%, 540 µL) were used to obtain the title compound (15 mg, 57%).
¹H NMR (400 MHz, CD₃OD) *δ* 8.82 (s, 1H), 7.97 (s, 1H), 7.82 (s, 1H), 7.43 (s, 1H), 7.20 (d, *J =* 8.0 Hz, 1H), 7.02 (d, *J* = 8.0 Hz, 1H), 4.69 (s, 2H), 3.90 (s, 3H), 3.81 (m, 1H), 3.53 - 3.45 (m, 2H), 3.19 - 3.15 (m, 2H), 2.41 - 2.31 (m, 2H).
LC-MS, *m*/*z* 421 [M+H]⁺

### Example 77

### 8-(6,6-Difluorobicyclo[3.1.0]hexan-3-yl)-N-(3-fluoro-5-(1-methyl-1H-pyrazol-4-yl)benzyl)-7H-purine-6-carboxamide

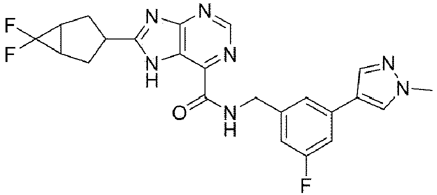

By the same method as in Example 72.5, 5,6-diamino-N-(3-fluoro-5-(1-methyl-1H-pyrazol-4-yl)benzyl)pyrimidine-4-carboxamide (50 mg, 0.15 mmol), 6,6-difluorobicyclo[3.1.0]hexane-3-carboxylic acid (37 mg, 0.15 mmol), T3P (50 wt. % solution in EtOAc, 305 mg, 0.48 mmol), and DIPEA (66 µL, 0.39 mmol) were used to obtain the title compound (7 mg, 10%).
¹H NMR (400 MHz, CD₃OD) *δ* 8.97 (s, 1H), 7.97 (s, 1H), 7.81 (s, 1H), 7.41 (s, 1H), 7.20 (d, J = 8 Hz, 1H), 7.00 (d, J = 12 Hz, 1H), 4.67 (s, 2H), 3.90 (s, 3H), 3.63 (m, 1H), 2.24 (m, 4H), 2.06 (m, 2H).
LC-MS, *m*/*z* 468 [M+H]⁺

### Example 78

### 8-(Bicyclo[3.1.0]hexan-3-yl)-N-(3-fluoro-5-(1-methyl-1H-pyrazol-4-yl)benzyl)-7H-purine-6-carboxamide

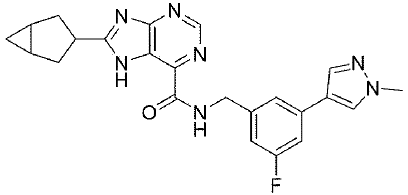

By the same method as in Example 72.5, 5,6-diamino-N-(3-fluoro-5-(1-methyl-1H-pyrazol-4-yl)benzyl)pyrimidine-4-carboxamide (50 mg, 0.15 mmol), bicyclo[3.1.0]hexane-3-carboxylic acid (19 mg, 0.15 mmol), T3P (50 wt. % solution in EtOAc, 305 mg, 0.48 mmol) and DIPEA (66 µL, 0.39 mmol) were used to obtain the title compound (3.3 mg, 5%).
¹H NMR (400 MHz, CD₃OD) *δ* 8.95 (s, 1H), 7.97 (s, 1H), 7.81 (s, 1H), 7.40 (s, 1H), 7.19 (d, J = 8 Hz, 1H), 6.99 (d, J = 8 Hz, 1H), 4.67 (s, 2H), 3.90 (s, 3H), 3.17 (m, 1H), 2.27 (m, 4H), 1.47 (m, 2H), 0.47 - 0.40 (m, 2H).
LC-MS *m*/*z* 432 [M+H]⁺

### Example 79

### N-(3-fluoro-5-(1-methyl-1H-pyrazol-4-yl)benzyl)-8-(1-methylpyrrolidin-3-yl)-7H-purine-6-carboxamide

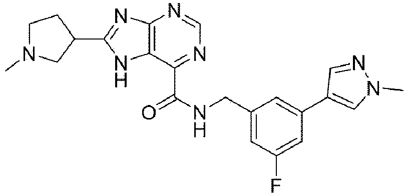

By the same method as in Example 63, N-(3-fluoro-5-(1-methyl-1H-pyrazol-4-yl)benzyl)-8-(pyrrolidin-3-yl)-7H-purine-6-carboxamide hydrochloride (15 mg, 0.036 mmol), paraformaldehyde (5 mg), NaBH₄ (3 mg, 0.075 mmol) and trifluoroethanol (1 mL) were used to obtain the title compound (7 mg, 45%).
¹H NMR (400 MHz, CD₃OD) *δ* 8.95 (s, 1H), 7.98 (s, 1H), 7.81 (s, 1H), 7.42 (s, 1H), 7.21 (d, *J* = 12 Hz, 1H), 7.00 (d, *J* = 8.0 Hz, 1H), 4.71 (s, 2H), 4.07 (m, 1H), 3.90 (s, 3H), 3.65 - 3.57 (m, 2H), 3.38 - 3.34 (m, 2H), 2.85 (s, 3H), 2.63 (m, 1H), 2.40 (m, 1H).
LC-MS, *m*/*z* 435 [M+H]⁺

### Example 80

### 80.1 8-(3,3-Difluorocyclopentyl)-7H-purine-6-carboxylic acid

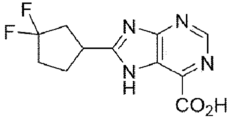

The title compound (260 mg, 78%) was obtained as a white solid by the same method as in Preparation example 1.
¹H NMR (400 MHz, CD₃OD) *δ* 9.02 (s, 1H), 3.81 (m, 1H), 2.88 - 2.62 (m, 2H), 2.40 - 2.34 (m, 2H), 2.28 - 2.24 (m, 2H).
LC-MS, *m*/*z* 269 [M+H]⁺

### 80.2 8-(3,3-Difluorocyclopentyl)-N-(3-fluoro-5-(1-(4-fluorophenyl)-1H-pyrazol-4-yl)benzyl)-7H-purine-6-carboxamide

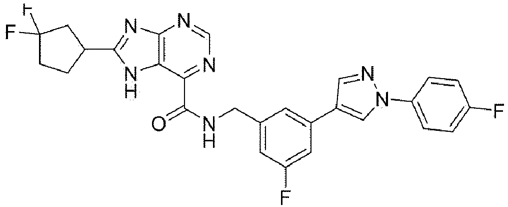

By the same method as in Example 1.3, using 8-(3,3-difluorocyclopentyl)-7H-purine-6-carboxylic acid (100 mg, 0.37 mmol), (3-fluoro-5-(1-(4-Fluorophenyl)-1H-pyrazol-4-yl)phenyl)methanamine (106 mg), T3P (50 wt.% solution in EtOAc, 356 mg, 1.12 mmol) and DIPEA (217 mg, 1.68 mmol), the title compound (115 mg, 57%) was obtained.
¹H NMR (400 MHz, CD₃OD) *δ* 8.99 (s, 1H), 8.61 (s, 1H), 8.08 (s, 1H), 7.83 - 7.75 (m, 2H), 7.53 (s, 1H), 7.32 (d, *J* = 10 Hz, 1H), 7.24 (t, *J* = 8.4 Hz, 2H), 7.05 (d, *J* = 9.2 Hz, 1H), 4.71 (s, 2H), 3.84 - 3.73 (m, 1H), 2.71 - 2.60 (m, 2H), 2.4 - 2.33 (m, 2H), 2.30 - 2.18 (m, 2H).
LC-MS, *m*/*z* 536 [M+H]⁺

**Example 81**

### 8-(3,3-Difluorocyclopentyl)-N-(3-fluoro-5-(1-(3-fluorophenyl)-1H-pyrazol-4-yl)benzyl)-7H-purine-6-carboxamide

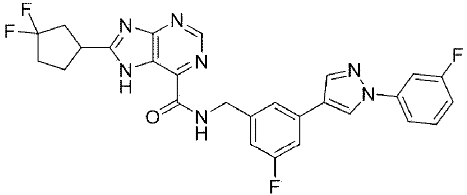

By the same method as in Example 1.3, 8-(3,3-difluorocyclopentyl)-7H-purine-6-carboxylic acid (10 mg, 0.037 mmol), (3-fluoro-5-(1-(3-fluorophenyl)-1H-pyrazol-4-yl)phenyl)methanamine hydrochloride (12 mg), T3P (50 wt.% solution in EtOAc, 67 µL, 0.11 mmol) and DIPEA (29 µL, 0.17 mmol) were used to obtain the title compound (18 mg, 43%).
¹H NMR (400 MHz, DMSO-*d*₆) *δ* 13.34 (s, 1H), 9.82 (s, 1H), 9.13 (s, 1H), 9.01 (s, 1H), 8.29 (s, 1H), 7.76 (d, J = 8.9 Hz, 2H), 7.62 - 7.54 (m, J = 10.1 Hz, 2H), 7.49 (d, J = 9.9 Hz, 1H), 7.19 (t, J = 7.8 Hz, 1H), 7.09 (d, J = 9.1 Hz, 1H), 4.62 (d, J = 6.3 Hz, 2H), 3.80 (mzz, 1H), 2.69 - 2.58 (m, 2H), 2.37 - 2.27 (m, 2H), 2.23 - 2.09 (m, 2H).
LC-MS, *m*/*z* 536 [M+H]⁺

### Example 82

### 8-(3,3-Difluorocyclopentyl)-N-(3-fluoro-5-(1-(6-fluoropyridin-3-yl)-1H-pyrazol-4-yl)benzyl)-7H-purine-6-carboxamide

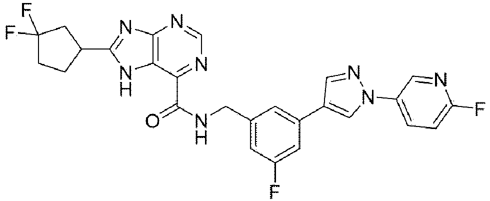

By the same method as in Example 1.3, 8-(3,3-difluorocyclopentyl)-7H-purine-6-carboxylic acid (10 mg, 0.037 mmol), (3-fluoro-5-(1-(6-fluoropyridin-3-yl)-1H-pyrazol-4-yl)phenyl)methanamine hydrochloride (12 mg), T3P (50 wt.% solution in EtOAc, 67 µL, 0.11 mmol) and DIPEA (29 µL, 0.17 mmol) were used to obtain the title compound (11 mg, 54%).
¹H NMR (400 MHz, DMSO-*d*₆) *δ* 13.34 (s, 1H), 9.83 (s, 1H), 9.12 (s, 1H), 9.01 (s, 1H), 8.77 (s, 1H), 8.46 (t, J = 6.5 Hz, 1H), 8.33 (s, 1H), 7.59 (s, 1H), 7.48 (d, J = 10.0 Hz, 1H), 7.41 (dd, J = 8.9, 2.8 Hz, 1H), 7.10 (d, J = 8.9 Hz, 1H), 4.62 (d, J = 6.2 Hz, 2H), 3.85 - 3.76 (m, 1H), 2.68 - 2.59 (m, 2H), 2.37 - 2.27 (m, 2H), 2.23 - 2.09 (m, 2H).
LC-MS, *m*/*z* 537 [M+H]⁺

### Example 83

### 8-(3,3-Difluorocyclopentyl)-N-(3-fluoro-5-(1-(pyridin-3-yl)-1H-pyrazol-4-yl)benzyl)-7H-purine-6-carboxamide

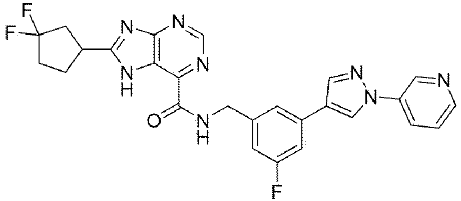

By the same method as in Example 1.3, 8-(3,3-difluorocyclopentyl)-7H-purine-6-carboxylic acid (21 mg, 0.079 mmol), (3-fluoro-5-(1-(pyridin-3-yl)-1H-pyrazol-4-yl)phenyl)methanamine hydrochloride (24 mg), T3P (50 wt.% solution in EtOAc, 140 µL, 0.24 mmol) and DIPEA (60 µL, 0.36 mmol) were used to obtain the title compound (31 mg, 75%).
¹H NMR (400 MHz, DMSO-*d*₆) *δ* 13.33 (s, 1H), 9.80 (t, *J* = 5.7 Hz, 1H), 9.17 (s, 2H), 9.02 (s, 1H), 8.58 (s, 1H), 8.34 (s, 1H), 8.30 (d, *J =* 8.0 Hz, 1H), 7.68 - 7.56 (m, 2H), 7.50 (d, *J* = 9.5 Hz, 1H), 7.10 (d, *J* = 9.5 Hz, 1H), 4.62 (d, *J* = 6.0 Hz, 2H), 3.86 - 3.77 (m, 1H), 2.70 - 2.58 (m, 2H), 2.39 - 2.27 (m, 2H), 2.24 - 2.08 (m, 2H).
LC-MS, *m*/*z* 519 [M+H]⁺

### Example 84

### 8-(3,3-Difluorocyclopentyl)-N-(3-fluoro-5-(1-(4-fluorophenyl)-1H-pyrazol-4-yl)benzyl)-7H-purine-6-carboxamide (E1, E2)

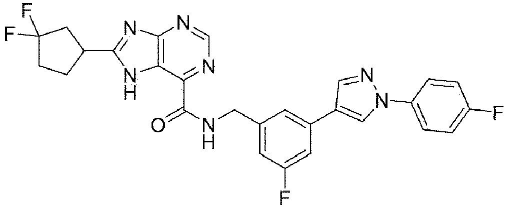

8-(3,3-difluorocyclopentyl)-N-(3-fluoro-5-(1-(4-fluorophenyl)-1H-pyrazol-4-yl)benzyl)-7H-purine-6-carboxamide (100 mg) obtained in the above Example 80 was separated by SFC (column: Daicel Chiralpak AD, 250×50 mm ID 10 µm, mobile phase: A for CO₂ and B for 2-propanol (0.1% NH₃H₂O), Gradient: B% = 45% isocratic elution mode), and the fractions containing each enantiomer were concentrated and lyophilized to obtain the title compounds E1 (36 mg) and E2 (36 mg).

E1 (Retention time: 2.04 min, Column: Daicel Chiralpak AD-3, 50×4.6 mm id., 3 µm, Mobile phase: A for CO₂ and B for 2-propanol (0.1% isopropylamine, v/v), Mobile phase: B% from 5% to 50% in 2 min)
¹H NMR (400 MHz, DMSO-*d*₆) *δ* 13.33 (s, 1H), 9.80 (s, 1H), 9.03 (s, 1H), 9.01 (s, 1H), 8.24 (s, 1H), 7.94 - 7.86 (m, 2H), 7.58 (s, 1H), 7.47 (d, J = 10.1 Hz, 1H), 7.39 (t, J = 8.8 Hz, 2H), 7.07 (d, J = 9.5 Hz, 1H), 4.61 (d, J = 6.2 Hz, 2H), 3.88 - 3.74 (m, 1H), 2.73 - 2.55 (m, 2H), 2.41 - 2.26 (m, 2H), 2.25 - 2.07 (m, 2H).
LC-MS, *m*/*z* 536 [M+H]⁺

E2 (Retention time: 2.29 min, Column: Daicel Chiralpak AD-3, 50×4.6 mm id., 3µm, Mobile phase: A for CO₂ and B for 2-propanol (0.1% isopropylamine, v/v), Mobile phase: B% from 5% to 50% in 2 min)
¹H NMR (400 MHz, DMSO-*d*₆) δ13.33 (s, 1H), 9.80 (s, 1H), 9.03 (s, 1H), 9.01 (s, 1H), 8.24 (s, 1H), 7.94 - 7.86 (m, 2H), 7.58 (s, 1H), 7.47 (d, J = 10.2 Hz, 1H), 7.39 (t, J = 8.8 Hz, 2H), 7.07 (d, J = 9.3 Hz, 1H), 4.61 (d, J = 6.2 Hz, 2H), 3.87 - 3.71 (m, 1H), 2.73 - 2.55 (m, 2H), 2.40 - 2.25 (m, 2H), 2.24 - 2.07 (m, 2H).
LC-MS, *m*/*z* 536 [M+H]⁺

### Example 85

### (R)-N-(3-fluoro-5-(1-(4-fluorophenyl)-1H-pyrazol-4-yl)benzyl)-8-(tetrahydrofuran-2-yl)-7H-purine-6-carboxamide

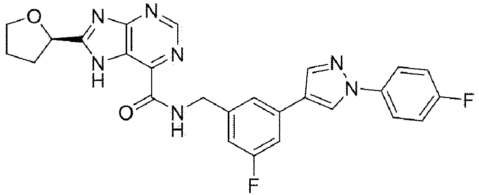

By the same method as in Example 72.5, using 5,6-diamino-N-(3-fluoro-5-(1-methyl-1H-pyrazol-4-yl)benzyl)pyrimidine-4-carboxamide (42 mg, 0.10 mmol), (R)-tetrahydrofuran-2-carboxylic acid (12 mg, 0.10 mmol), T3P (50 wt. % solution in EtOAc, 203 mg, 0.32 mmol) and DIPEA (66 µL, 0.26 mmol), the title compound (12 mg, 24%) was obtained.
¹H NMR (400 MHz, CD₃OD) *δ* 8.99 (s, 1H), 8.59 (s, 1H), 8.06 (s, 1H), 7.79 - 7.75 (m, 2H), 7.51 (s, 1H), 7.33 (d, *J =* 12 Hz, 1H), 7.22 (t, *J =* 8.0 Hz, 2H), 7.04 (d, *J =* 8.0 Hz, 1H), 5.26 (m, 1H), 4.69 (s, 2H), 4.16 - 4.06 (m, 2H), 2.51 - 2.46 (m, 1H), 2.28 - 2.23 (m, 1H), 2.09 - 2.02 (m, 2H).
LC-MS, *m*/*z* 502 [M+H]⁺

### Example 86

### (R)-N-(3-fluoro-5-(1-(4-fluorophenyl)-1H-pyrazol-4-yl)benzyl)-8-(3-oxocyclopentyl)-7H-purine-6-carboxamide

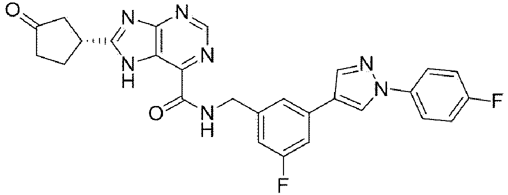

By the same method as in Example 72.5, 5,6-diamino-N-(3-fluoro-5-(1-(4-fluorophenyl)-1H-pyrazol-4-yl)benzyl)pyrimidine-4-carboxamide (30 mg, 0.071 mmol), (R)-3-oxocyclopentanecarboxylic acid (9.1 mg, 0.071 mmol), T3P (50 wt.% solution in EtOAc) (113 mg, 0.355 mmol) and DIPEA (24 µL, 0.14 mmol) were used to obtain the title compound (2.02 mg, 5.5%).
¹H NMR (400 MHz, CD₃OD) *δ* 9.01 (s, 1H), 8.61 (s, 1H), 8.07 (s, 1H), 7.81 - 7.78 (m, 2H), 7.53 (s, 1H), 7.32 (d, *J* = 9.8 Hz, 1H), 7.23 (t, *J* = 8.6 Hz, 2H), 7.05 (d, *J* = 8.9 Hz, 1H), 4.71 (s, 2H), 3.75 - 3.61 (m, 1H), 2.50 - 2.38 (m, 1H), 2.27 (m, 2H), 2.17 - 2.05 (m, 2H), 2.01 (m, 1H).
LCMS, *m*/*z* 514 [M+H]⁺

### Example 87

### 87.1 tert-Butyl 3-(1-(3-chloro-4-fluorophenyl)-1H-pyrazol-4-yl)-5-fluorobenzylcarbamate

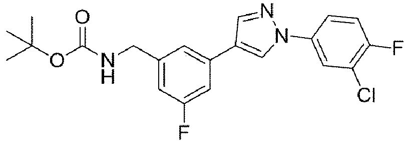

By the same method as in Example 25.3, tert-butyl 3-(1-(3-chloro-4-fluorophenyl)-1H-pyrazol-4-yl)-5-fluorobenzylcarbamate (53 mg, 0.18 mmol), 2-chloro-1-fluoro-4-iodobenzene (70 mg, 0.27 mmol), CuI (3.5 mg, 0.018 mmol), L-proline (4.2 mg, 0.037 mmol), K₂CO₃ (38 mg, 0.27 mmol) and DMSO (2 mL) were used to obtain the title compound (57 mg, 74%) as a light brown solid.
LCMS, *m*/*z* 420 [M+H]⁺

### 87.2 (3-(1-(3-Chloro-4-fluorophenyl)-1H-pyrazol-4-yl)-5-fluorophenyl)methanamine, trifluoroacetate

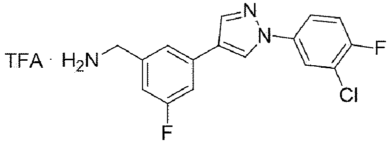

By the same method as in Example 25.4, using tert-butyl 3-(1-(3-chloro-4-fluorophenyl)-1H-pyrazol-4-yl)-5-fluorobenzylcarbamate (57 mg, 0.14 mmol), trifluoroacetic acid (0.45 mL) and DCM (1.4 mL), the title compound (58 mg, crude) was obtained.
LCMS, *m*/*z* 320 [M+H]⁺

### 87.3 N-(3-(1-(3-chloro-4-fluorophenyl)-1H-pyrazol-4-yl)-5-fluorobenzyl)-8-cyclopentyl-7H-purine-6-carboxamide

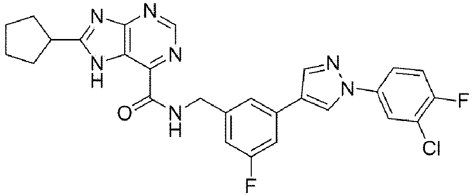

8-Cyclopentyl-7H-purine-6-carboxylic acid (26 mg, 0.11 mmol), (3-(1-(3-chloro- 4-fluorophenyl)-1H-pyrazol-4-yl)-5-fluorophenyl)methanamine, trifluoroacetate (58 mg, crude) and HOBt (18 mg, 0.14 mmol) were dissolved in ethyl acetate (2 mL), DIPEA (29 µL) and EDC (26 mg, 0.14 mmol) were added, and the mixture was stirred at room temperature overnight. Ethyl acetate and H₂O were added to the reaction mixture, extracted, and the organic layer was dried over MgSO₄ and concentrated. The obtained residue was purified by silica gel column chromatography (0-2 % MeOH in DCM) to obtain the title compound (34 mg, 2 step yield 56%) as an off-white solid.
¹H NMR (400 MHz, DMSO-*d*₆) *δ* 13.18 (s, 1H), 9.77 (s, 1H), 9.12 (s, 1H), 8.97 (s, 1H), 8.29 (s, 1H), 8.13 (dd, J = 6.4, 2.4 Hz, 1H), 7.95 - 7.87 (m, 1H), 7.68 - 7.56 (m, 2H), 7.47 (d, J = 10.0 Hz, 1H), 7.09 (d, J = 9.3 Hz, 1H), 4.61 (d, J = 6.4 Hz, 2H), 3.54 - 3.44 (m, 1H), 2.12 - 2.03 (m, 2H), 1.99 - 1.88 (m, 2H), 1.85 - 1.76 (m, 2H), 1.71 - 1.61 (m, 2H).
LCMS, *m*/*z* 534 [M+H]⁺

### Example 88

### (S)-N-(3-fluoro-5-(1-(4-fluorophenyl)-1H-pyrazol-4-yl)benzyl)-8-(3-oxocyclopentyl)-7H-purine-6-carboxamide

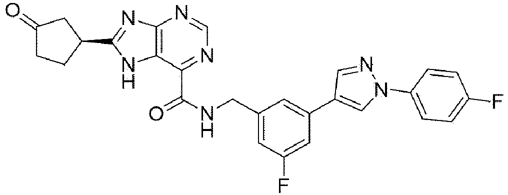

By the same method as in Example 72.5, using 5,6-diamino-N-(3-fluoro-5-(1-(4-fluorophenyl)-1H-pyrazol-4-yl)benzyl)pyrimidine-4-carboxamide (50 mg, 0.12 mmol), (S)-3-oxocyclopentanecarboxylic acid (15 mg, 0.12 mmol), T3P (50 wt. % solution in EtOAc, 203 mg, 0.32 mmol) and DIPEA (66 µL, 0.26 mmol), the title compound (30 mg, 49%) was obtained.
¹H NMR (400 MHz, CD₃OD) *δ* 8.97 (s, 1H), 8.58 (s, 1H), 8.05 (s, 1H), 7.83 - 7.75 (m, 2H), 7.51 (s, 1H), 7.30 (d, J = 8 Hz, 1H), 7.21 (t, 2H), 7.04 (d, J = 8 Hz, 1H), 4.69 (s, 2H), 3.91 (m, 1H), 2.75 (m, 2H), 2.57 - 2.27 (m, 4H).
LCMS, *m*/*z* 514 [M+H]⁺

### Example 89

### N-(3-fluoro-5-(1-(4-fluorophenyl)-1H-pyrazol-4-yl)benzyl)-8-(tetrahydrofuran-3-yl)-7H-purine-6-carboxamide

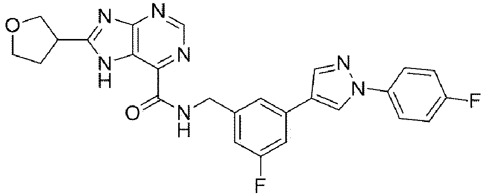

By the same method as in Example 72.5, using 5,6-diamino-N-(3-fluoro-5-(1-(4-fluorophenyl)-1H-pyrazol-4-yl)benzyl)pyrimidine-4-carboxamide (13.9 mg, 0.033 mmol), tetrahydrofuran-3-carboxylic acid (3.8 mg, 0.033 mmol), T3P (50 wt.% solution in EtOAc) (53 mg, 0.17 mmol) and DIPEA (11.2 µL, 0.66 mmol), the title compound (0.83 mg, 5%) was obtained.
¹H NMR (400 MHz, CD₃OD) *δ* 9.00 (s, 1H), 8.63 (s, 1H), 8.09 (s, 1H), 7.80 (dd, J = 9.0, 4.6 Hz, 2H), 7.55 (s, 1H), 7.33 (d, J = 10.5 Hz, 1H), 7.24 (t, J = 8.7 Hz, 2H), 7.06 (d, J = 8.7 Hz, 1H), 4.72 (s, 2H), 4.22 (t, J = 8.0 Hz, 1H), 4.14 - 4.04 (m, 2H), 4.00 - 3.84 (m, 2H), 2.51 - 2.40 (m, 2H).
LCMS, *m*/*z* 502 [M+H]⁺

### Example 90

### 90.1 (R)-tert-butyl (1-(3-bromophenyl)ethyl)carbamate

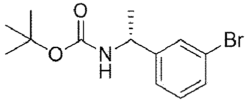

By the same method as in Example 6.1, (R)-1-(3-bromophenyl)ethanamine (200 mg, 1.1 mmol), Boc₂O (470 mg, 2.2 mmol) and DCM (5 mL) solution were used to obtain the title compound (140 mg, 95%).

¹H NMR (400 MHz, DMSO-*d*₆) *δ* 7.53 - 7.37 (m, 6H), 7.31 - 7.24 (m, 4H), 4.64 - 4.54 (m, 2H), 1.36 (s, 17H), 1.28 (d, *J* = 7.0 Hz, 6H).

### 90.2 (R)-tert-butyl (1-(3-(1-(4-fluorophenyl)-1H-pyrazol-4-yl)phenyl)ethyl)carbamate

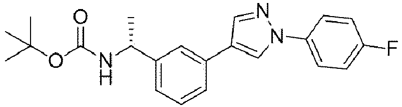

By the same method as in Example 4.1, (R)-tert-butyl (1-(3-bromophenyl)ethyl) carbamate (52 mg, 0.17 mmol), (1-(4-fluorophenyl)-1H-Pyrazol-4-yl)boronic acid (54 mg, 0.26 mmol), 2M aq. K₂CO₃ ( 0.31 mL, 0.61 mmol), PdCl₂ (dppf) (6.3 mg, 0.087 mmol) and 1,4-dioxane (0.62 mL) were used to obtain the title compound (49 mg, 74%).
LCMS, *m*/*z* 382 [M+H]⁺

### 90.3 (R)-1-(3-(1-(4-fluorophenyl)-1H-pyrazol-4-yl)phenyl)ethanamine hydrochloride salt

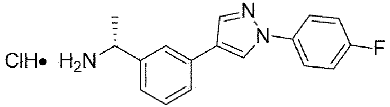

To a solution of (R)-tert-butyl (1-(3-(1-(4-fluorophenyl)-1H-pyrazol-4-yl)phenyl)ethyl)carbamate (49 mg, 0.13 mmol) in DCM (1.3 mL) was slowly added 4 M HCl (in dioxane) (400 µL), and the mixture was stirred at room temperature. After 90 min, MeOH was added to the reaction mixture, followed by concentration. This process was repeated three times to obtain the title compound (24 mg, crude) as a white solid.
LCMS, *m*/*z* 282 [M+H]⁺

### 90.4 (R)-8-cyclopentyl-N-(1-(3-(1-(4-fluorophenyl)-1H-pyrazol-4-yl)phenyl)ethyl)-7H-purine-6-carboxamide

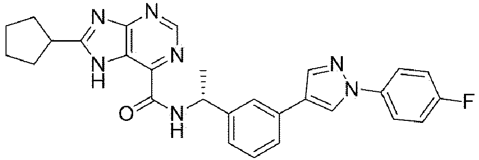

By the same method as in Example 87.3, (R)-1-(3-(1-(4-fluorophenyl)-1H-pyrazol-4-yl)phenyl)ethanamine hydrochloride salt (41 mg), 8-cyclopentyl-7H-purine-6-carboxylic acid (25 mg, 0.11 mmol), EDC (25 mg, 0.13 mmol), HOBt (17 mg, 0.13 mmol), DIPEA (54 µL, 0.32 mmol) and DCM (2 mL) were used to obtain the title compound (38 mg, 72%) as a light brown solid.
¹H NMR (400 MHz, DMSO-*d*₆) *δ* 13.12 (s, 1H), 9.40 (d, J = 5.8 Hz, 1H), 8.94 (s, 2H), 8.18 (s, 1H), 7.90 (dd, J = 8.9, 4.7 Hz, 2H), 7.81 (s, 1H), 7.57 (s, 1H), 7.42 - 7.26 (m, 4H), 5.29 - 5.19 (m, 1H), 3.51 - 3.40 (m, 1H), 2.12 - 1.97 (m, 2H), 1.95 - 1.83 (m, 2H), 1.76 (s, 2H), 1.61 (d, J = 6.8 Hz, 5H).
LCMS, *m*/*z* 496 [M+H]⁺

### Example 91

### 91.1 (S)-tert-butyl (1-(3-bromophenyl)ethyl)carbamate

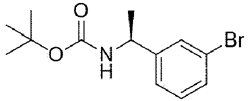

By the same method as in Example 6.1, (S)-1-(3-bromophenyl)ethanamine (100 mg, 0.50 mmol), Boc₂O (220 mg, 1.0 mmol) and DCM (3 mL) were used to obtain the title compound (140 mg, 91%).

¹H NMR (400 MHz, DMSO-*d*₆) *δ* 7.55 - 7.37 (m, 3H), 7.28 (d, *J* = 6.5 Hz, 2H), 4.64 - 4.55 (m, 1H), 1.36 (s, 8H), 1.28 (d, *J* = 7.0 Hz, 3H).

### 91.2 (S)-tert-butyl (1-(3-(1-(4-fluorophenyl)-1H-pyrazol-4-yl)phenyl)ethyl)carbamate

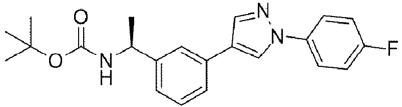

By the same method as in Example 4.1 above, (S)-tert-butyl (1-(3-bromophenyl)ethyl) carbamate (47 mg, 0.16 mmol), (1-(4-fluorophenyl)-1H -Pyrazol-4-yl)boronic acid (49 mg, 0.24 mmol), PdCl₂ (dppf) (6.0 mg, 0.0077 mmol), 2M aq. K₂CO₃ (0.28 mL, 0.55 mmol) and 1,4-dioxane (0.55 mL) were used to obtain the title compound (49 mg, 74%).
LCMS, *m*/*z* 382 [M+H]⁺

### 91.3 (S)-1-(3-(1-(4-fluorophenyl)-1H-pyrazol-4-yl)phenyl)ethanamine hydrochloride salt

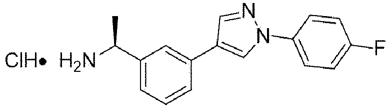

By the same method as in Example 90.3, (S)-tert-butyl (1-(3-(1-(4-fluorophenyl)-1H-pyrazol-4-yl)phenyl)ethyl)carbamate (60 mg, 0.16 mmol), 4 M HCl (in dioxane) (800 µL) and DCM (1.6 mL) were used to obtain the title compound (50 mg, crude).
LCMS, *m*/*z* 282 [M+H]⁺

### 91.4 (S)-8-cyclopentyl-N-(1-(3-(1-(4-fluorophenyl)-1H-pyrazol-4-yl)phenyl)ethyl)-7H-purine-6-carboxamide

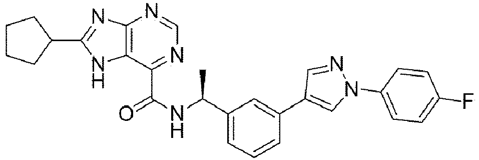

By the same method as in Example 87.3, (S)-1-(3-(1-(4-fluorophenyl)-1H-pyrazol-4-yl)phenyl)ethanamine hydrochloride salt (50 mg, 0.16 mmol), 8-cyclopentyl-7H-purine-6-carboxylic acid (30 mg, 0.13 mmol), EDC (30 mg, 0.16 mmol), HOBt (21 mg, 0.16 mmol), DIPEA (68 µL, 0.39 mmol) and DCM (2 mL) were used to obtain the title compound (37 mg, 57%) as a light brown solid.
¹H NMR (400 MHz, DMSO-*d*₆) *δ* 13.15 (s, 1H), 9.43 (d, J = 7.8 Hz, 1H), 8.97 (s, 2H), 8.20 (s, 1H), 7.92 (dd, J = 9.0, 4.7 Hz, 2H), 7.83 (s, 1H), 7.59 (s, 1H), 7.47 - 7.30 (m, 4H), 5.31 - 5.21 (m, 1H), 3.51 - 3.43 (m, 1H), 2.13 - 2.00 (m, 2H), 1.98 - 1.85 (m, 2H), 1.79 (s, 2H), 1.64 (d, J = 6.9 Hz, 5H).
LCMS, *m*/*z* 496 [M+H]⁺

### Example 92

### 8-(3,3-Difluorocyclobutyl)-N-(3-fluoro-5-(1-(4-fluorophenyl)-1H-pyrazol-4-yl)benzyl)-7H-purine-6-carboxamide

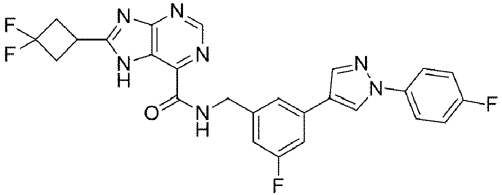

By the same method as in Example 72.5, using 5,6-diamino-N-(3-fluoro-5-(1-(4-fluorophenyl)-1H-pyrazol-4-yl)benzyl)pyrimidine-4-carboxamide (40 mg, 0.095 mmol), 3,3-difluorocyclobutanecarboxylic acid (12.9 mg, 0.095 mmol), T3P (50 wt.% solution in EtOAc) (151 mg, 0.475 mmol), DIPEA (32 µL, 0.19 mmol) and 1,4-dioxane (1 mL), the title compound (14.9 mg, 30%) was obtained as an off-white solid.
¹H NMR (400 MHz, DMSO-*d*₆) *δ* 13.40 (s, 1H), 9.81 (s, 1H), 9.03 (s, 2H), 8.24 (s, 1H), 7.91 (dd, J = 9.1, 4.7 Hz, 2H), 7.58 (s, 1H), 7.47 (d, J = 10.3 Hz, 1H), 7.39 (t, J = 8.8 Hz, 2H), 7.07 (d, J = 9.5 Hz, 1H), 4.61 (d, J = 6.2 Hz, 2H), 3.87 - 3.74 (m, 1H), 3.18 - 2.99 (m, 4H).
LCMS, *m*/*z* 522 [M+H]⁺

### Example 93

### 2-Cyclopentyl-N-(3-fluoro-5-(1-(4-fluorophenyl)-1H-pyrazol-4-yl)benzyl)-3H-imidazo[4,5-c]pyridine-4-carboxamide

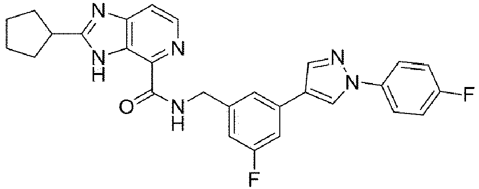

By the same method as in Example 27.3, (3-fluoro-5-(1-(4-fluorophenyl)-1H-pyrazol-4-yl)phenyl)methanamine, trifluoroacetate (36 mg, 0.11 mmol), 8-cyclopentyl-7H-purine-6-carboxylic acid (22 mg, 0.09 mmol), T3P (50 wt.% solution in EtOAc, 168 mg, 0.53 mmol), DIPEA (38 µL, 0.22 mmol) and DMF (3 mL) were used to obtain the title compound (23 mg, 51%).
¹H NMR (400 MHz, CD₃OD) *δ* 8.57 (s, 1H), 8.37 (d, J = 4 Hz, 1H), 8.05 (s, 1H), 7.78 - 7.75 (m, 2H), 7.69 (s, 1H), 7.51 (s, 1H), 7.30 (d, J = 12 Hz, 1H), 7.21 (t, 2H), 7.04 (d, J = 12 Hz, 1H), 4.69 (s, 2H), 3.44 (m, 1H), 2.06 (m, 2H), 1.96 (m, 2H), 1.88 (m, 2H), 1.73 (m, 2H).
LCMS, m/z 499 [M+H]+

### Example 94

### 94.1 tert-Butyl 3-bromophenethylcarbamate

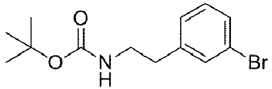

By the same method as in Example 6.1, 2-(3-bromophenyl)ethanamine (200 mg, 1.0 mmol), Boc₂O (440 mg, 2.0 mmol) and DCM (3 mL) were used to obtain the title compound (290 mg, 96%).

¹H NMR (400 MHz, DMSO-*d*₆) *δ* 7.39 (d, *J* = 6.3 Hz, 2H), 7.28 - 7.18 (m, 2H), 6.88 (s, 1H), 3.18 - 3.10 (m, 2H), 2.69 (t, *J* = 7.0 Hz, 2H), 1.35 (s, 9H).

### 94.2 tert-Butyl 3-(1-(4-fluorophenyl)-1H-pyrazol-4-yl)phenethylcarbamate

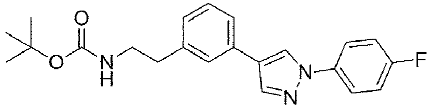

By the same method as in Example 4.1, tert-butyl 3-bromophenethylcarbamate (54 mg, 0.18 mmol), (1-(4-fluorophenyl)-1H-pyrazol-4-yl)boronic acid (56 mg, 0.27 mmol), PdCl₂ (dppf) (6.6 mg, 0.0090 mmol), 2 M aq. K₂CO₃ (0.31 mL, 0.63 mmol) and 1,4-dioxane (0.62 mL) were used to obtain the title compound (38 mg, 55%).
LCMS, *m*/*z* 382 [M+H]⁺

### 94.3 2-(3-(1-(4-Fluorophenyl)-1H-pyrazol-4-yl)phenyl)ethanamine hydrochloride salt

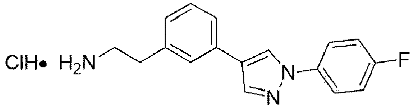

By the same method as in Example 90.3, tert-butyl 3-(1-(4-fluorophenyl) -1H-pyrazol-4-yl)phenethylcarbamate (38 mg, 0.10 mmol), 4 M HCl (in dioxane) (500 µL) and DCM (1 mL) were used to obtain the title compound (50 mg, crude).
LCMS, *m*/*z* 282 [M+H]⁺

### 94.4 8-Cyclopentyl-N-(3-(1-(4-fluorophenyl)-1H-pyrazol-4-yl)phenethyl)-7H-purine-6-carboxamide

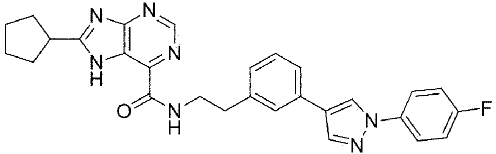

By the same method as in Example 87.3, (2-(3-(1-(4-fluorophenyl)-1H-pyrazol-4-yl)phenyl)ethanamine hydrochloride salt (32 mg, 0.10 mmol), 8-cyclopentyl-7H-purine-6-carboxylic acid (19 mg, 0.083 mmol), EDC (19 mg, 0.10 mmol), HOBt (13 mg, 0.10 mmol), DIPEA (42 µL, 0.25 mmol) and DCM (2 mL) were used to obtain the title compound (33 mg, 80%).
¹H NMR (400 MHz, DMSO-*d*₆) *δ* 13.17 (s, 1H), 9.23 (s, 1H), 8.94 (d, J = 7.3 Hz, 2H), 8.17 (s, 1H), 7.97 - 7.85 (m, 2H), 7.62 (s, 1H), 7.56 (d, J = 7.6 Hz, 1H), 7.43 - 7.31 (m, 3H), 7.16 (d, J = 7.6 Hz, 1H), 3.74 - 3.65 (m, 2H), 3.55 - 3.46 (m, 1H), 2.98 (t, J = 7.3 Hz, 2H), 2.14 - 2.02 (m, 2H), 1.98 - 1.87 (m, 2H), 1.86 - 1.75 (m, 2H), 1.72 - 1.60 (m, 2H).
LCMS, *m*/*z* 496 [M+H]⁺

### Example 95

### 95.1 tert-Butyl 3-bromo-2-fluorobenzylcarbamate

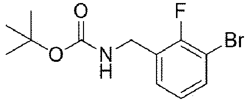

By the same method as in Example 6.1, (3-bromo-2-fluorophenyl)methanamine (200 mg, 0.98 mmol), Boc₂O (430 mg, 2.0 mmol) and DCM (3 mL) were used to obtain the title compound (290 mg, 97%).

¹H NMR (400 MHz, DMSO-*d*₆) *δ* 7.58 (t, *J* = 7.1 Hz, 1H), 7.47 (t, *J* = 5.4 Hz, 1H), 7.30 (t, *J* = 6.9 Hz, 1H), 7.14 (t, *J* = 7.8 Hz, 1H), 4.19 (d, *J* = 5.8 Hz, 2H), 1.56 - 1.21 (m, 9H).

### 95.2 tert-Butyl 2-fluoro-3-(1-(4-fluorophenyl)-1H-pyrazol-4-yl)benzylcarbamate

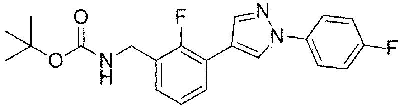

By the same method as in Example 4.1, using tert-butyl 3-bromo-2-fluorobenzylcarbamate (54 mg, 0.18 mmol), (1-(4-fluorophenyl)-1H-pyrazol-4-yl)boronic acid (55 mg, 0.27 mmol), PdCl₂ (dppf) (6.4 mg, 0.0088 mmol), 2 M aq. K₂CO₃ (0.31 mL, 0.62 mmol) and 1,4-dioxane (0.62 mL), the title compound (50 mg, 73%) was obtained.
LCMS, *m*/*z* 386 [M+H]⁺

### 95.3 2-Fluoro-3-(1-(4-fluorophenyl)-1H-pyrazol-4-yl)phenyl)methanamine hydrochloride salt

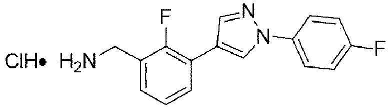

By the same method as in Example 90.3, tert-butyl 2-fluoro-3-(1-(4-fluorophenyl)-1H-pyrazol-4-yl)benzylcarbamate (50 mg, 0.13 mmol), 4 M HCl (in dioxane) (700 µL) and DCM (1.3 mL) were used to obtain the title compound (42 mg, crude).
LCMS, *m*/*z* 286 [M+H]⁺

### 95.4 8-Cyclopentyl-N-(2-fluoro-3-(1-(4-fluorophenyl)-1H-pyrazol-4-yl)benzyl)-7H-purine-6-carboxamide

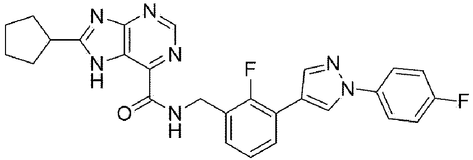

By the same method as in Example 87.3, (2-fluoro-3-(1-(4-fluorophenyl) -1H-pyrazol-4-yl)phenyl)methanamine hydrochloride salt (42 mg, 0.13 mmol), 8-cyclopentyl-7H-purine-6-carboxylic acid (25 mg, 0.11 mmol), EDC (25 mg, 0.13 mmol), HOBt (18 mg, 0.13 mmol), DIPEA (66 µL, 0.39 mmol) and DCM (2 mL) were used to obtain the title compound (35 mg, 65%).
¹H NMR (400 MHz, DMSO-*d*₆) *δ* 13.20 (s, 1H), 9.72 (s, 1H), 8.98 (s, 1H), 8.93 (s, 1H), 8.22 (s, 1H), 7.96 (dd, J = 9.0, 4.8 Hz, 2H), 7.74 (t, J = 7.3 Hz, 1H), 7.39 (t, J = 8.8 Hz, 2H), 7.33 (t, J = 7.0 Hz, 1H), 7.22 (t, J = 7.7 Hz, 1H), 4.70 (d, J = 6.1 Hz, 2H), 3.53 - 3.46 (m, 1H), 2.13 - 2.03 (m, 2H), 1.98 - 1.88 (m, 2H), 1.86 - 1.75 (m, 2H), 1.72 - 1.61 (m, 2H).
LCMS, *m*/*z* 500 [M+H]⁺

### Example 96

### 96.1 tert-Butyl 4-fluoro-3-(1-(4-fluorophenyl)-1H-pyrazol-4-yl)benzylcarbamate

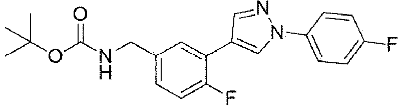

By the same method as in Example 4.1, tert-butyl 3-bromo-4-fluorobenzylcarbamate (44 mg, 0.14 mmol), (1-(4-fluorophenyl)-1H-pyrazol-4-yl)boronic acid (45 mg, 0.22 mmol), PdCl₂ (dppf) (5.3 mg, 0.0073 mmol), 2 M aq. K₂CO₃ (0.26 mL, 0.52 mmol) and 1,4-dioxane (0.52 mL) were used to obtain the title compound (37 mg, 66%).
LCMS, *m*/*z* 386 [M+H]⁺

### 96.2 (4-Fluoro-3-(1-(4-fluorophenyl)-1H-pyrazol-4-yl)phenyl)methanamine hydrochloride salt

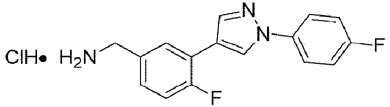

By the same method as in Example 90.3, tert-butyl 4-fluoro-3-(1-(4-fluorophenyl)-1H-pyrazol-4-yl)benzylcarbamate (37 mg, 0.096 mmol), 4 M HCl (in dioxane) (500 µL) and DCM (1 mL) were used to obtain the title compound (31 mg, crude).
LCMS, *m*/*z* 2856 [M+H]⁺

### 96.3 8-Cyclopentyl-N-(4-fluoro-3-(1-(4-fluorophenyl)-1H-pyrazol-4-yl)benzyl)-7H-purine-6-carboxamide

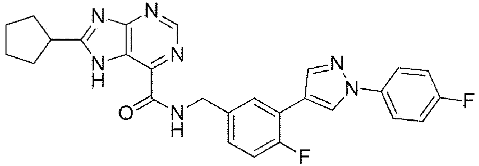

By the same method as in Example 87.3, (4-fluoro-3-(1-(4-fluorophenyl) -1H-pyrazol-4-yl)phenyl)methanamine hydrochloride salt (31 mg, 0.096 mmol), 8-cyclopentyl-7H-purine-6-carboxylic acid (19 mg, 0.080 mmol), EDC (18 mg, 0.096 mmol), HOBt (13 mg, 0.096 mmol), DIPEA (41 µL, 0.24 mmol) and DCM (2 mL) were used to obtain the title compound (26 mg, 64%).
¹H NMR (400 MHz, DMSO-*d*₆) *δ* 13.17 (s, 1H), 9.74 (s, 1H), 8.96 (s, 1H), 8.87 (s, 1H), 8.17 (s, 1H), 7.95 (dd, J = 9.0, 4.7 Hz, 2H), 7.84 (d, J = 6.0 Hz, 1H), 7.38 (t, J = 8.8 Hz, 2H), 7.34 - 7.23 (m, 2H), 4.59 (d, J = 6.1 Hz, 2H), 3.55 - 3.44 (m, 1H), 2.13 - 2.02 (m, 2H), 1.99 - 1.88 (m, 2H), 1.80 (s, 2H), 1.71 - 1.60 (m, 2H).
LCMS, *m*/*z* 500 [M+H]⁺

### Example 97

### 8-(3,3-Dimethylcyclobutyl)-N-(3-fluoro-5-(1-(4-fluorophenyl)-1H-pyrazol-4-yl)benzyl)-7H-purine-6-carboxamide

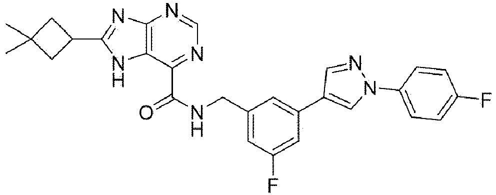

By the same method as in Example 72.5, using 5,6-diamino-N-(3-fluoro-5-(1-(4-fluorophenyl)-1H-pyrazol-4-yl)benzyl)pyrimidine-4-carboxamide (22 mg, 0.052 mmol), 3,3-dimethylcyclobutanecarboxylic acid (7 mg, 0.052 mmol), 1,4-dioxane (1 mL), T3P (50 wt.% solution (in EtOAc), 155 µL, 0.26 mmol) and DIPEA (18 µL, 0.10 mmol), the title compound (9.25 mg, 34.5%) was obtained as a yellow solid.
¹H NMR (400 MHz, DMSO-*d*₆) *δ* 13.10 (s, 1H), 9.76 (s, 1H), 9.03 (s, 1H), 8.98 (s, 1H), 8.25 (d, J = 7.3 Hz, 1H), 7.91 (dd, J = 9.0, 4.7 Hz, 2H), 7.58 (s, 1H), 7.47 (d, J = 9.5 Hz, 1H), 7.39 (t, J = 8.8 Hz, 2H), 7.07 (d, J = 9.3 Hz, 1H), 4.61 (d, J = 6.1 Hz, 2H), 3.94 - 3.81 (m, 1H), 2.28 (t, J = 10.2 Hz, 2H), 2.15 (t, J = 10.2 Hz, 2H), 1.24 (s, 3H), 1.15 (s, 3H).
LC-MS, m/z 514 [M+H]⁺

### Example 98

### 8-(6,6-Difluorospiro[3.3]heptan-2-yl)-N-(3-fluoro-5-(1-(4-fluorophenyl)-1H-pyrazol-4-yl)benzyl)-7H-purine-6-carboxamide

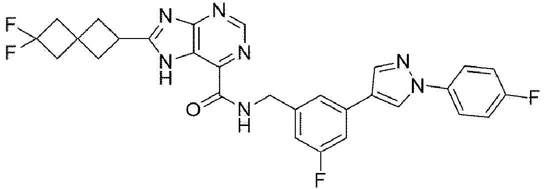

By the same method as in Example 72.5, using 5,6-diamino-N-(3-fluoro-5-(1-(4-fluorophenyl)-1H-pyrazol-4-yl)benzyl)pyrimidine-4-carboxamide (19.8 mg, 0.047 mmol), 6,6-difluorospiro[3.3]heptane-2-carboxylic acid (8.3 mg, 0.047 mmol), 1,4-dioxane (1 mL), T3P (50 wt.% solution (in EtOAc), 140 µL, 0.24 mmol) and DIPEA (16 µL, 0.094 mmol), the title compound (5 mg, 18%) was obtained as a pale yellow solid.
¹H NMR (400 MHz, CD₃OD) *δ* 8.97 (s, 1H), 8.59 (s, 1H), 8.06 (s, 1H), 7.82 - 7.74 (m, 2H), 7.51 (s, 1H), 7.30 (d, J = 9.8 Hz, 1H), 7.22 (t, J = 8.7 Hz, 2H), 7.03 (d, J = 9.4 Hz, 1H), 4.69 (s, 2H), 3.90 - 3.79 (m, 1H), 2.81 -2.52 (m, 8H).
LC-MS, m/z 562 [M+H]⁺

### Example 99

### 8-(Bicyclo[1.1.1]pentan-1-yl)-N-(3-fluoro-5-(1-(4-fluorophenyl)-1H-pyrazol-4-yl)benzyl)-7H-purine-6-carboxamide

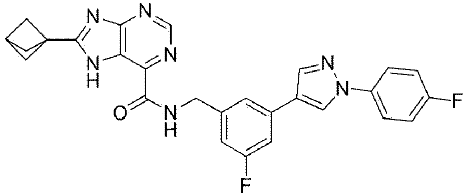

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), bicyclo[1.1.1]pentane-1-carboxylic acid (5.3 mg, 0.048 mmol), 1,4-dioxane (1 mL), T3P (50 wt.% solution (in EtOAc), 142 µL, 0.24 mmol) and DIPEA (16 µL, 0.095 mmol), the title compound (14.15 mg, 59.9%) was obtained as a yellow solid.
¹H NMR (400 MHz, DMSO-*d*₆) *δ* 13.36 (s, 1H), 9.77 (s, 1H), 9.10 - 8.94 (m, 2H), 8.24 (s, 1H), 7.97 - 7.82 (m, 2H), 7.58 (s, 1H), 7.47 (d, *J* = 9.7 Hz, 1H), 7.43 - 7.33 (m, 2H), 7.07 (d, *J* = 9.9 Hz, 1H), 4.61 (s, 2H), 2.61 - 2.55 (m, 1H), 2.31 (s, 6H).
LC-MS, m/z 498 [M+H]⁺

### Example 100

### N-(3-fluoro-5-(1-(4-fluorophenyl)-1H-pyrazol-4-yl)benzyl)-8-(spiro[3.3]heptan-2-yl)-7H-purine-6-carboxamide

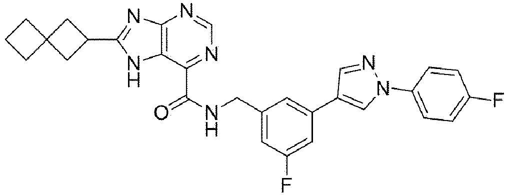

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), spiro[3.3]heptane-2-carboxylic acid (7 mg, 0.048 mmol), 1,4-dioxane (1 mL), T3P (50 wt.% solution (in EtOAc), 142 µL, 0.24 mmol) and DIPEA (16 µL, 0.095 mmol), the title compound (14.15 mg, 59.9%) was obtained as a pale yellow solid.
¹H NMR (400 MHz, CD₃OD) *δ* 8.94 (s, 1H), 8.57 (s, 1H), 8.04 (s, 1H), 7.81 - 7.72 (m, 2H), 7.50 (s, 1H), 7.28 (d, J = 9.8 Hz, 1H), 7.21 (t, J = 8.7 Hz, 2H), 7.02 (d, J = 9.2 Hz, 1H), 4.68 (s, 2H), 3.78 - 3.66 (m, 1H), 2.55 - 2.43 (m, 4H), 2.24 - 2.12 (m, 2H), 2.02 - 1.99 (d, J = 7.6 Hz, 2H), 1.90 - 1.82 (m, 2H).
LC-MS, m/z 526 [M+H]⁺

### Example 101

### N-(3-fluoro-5-(1-(4-fluorophenyl)-1H-pyrazol-4-yl)benzyl)-8-(3-fluorobicyclo[1.1.1]pentan-1-yl)-7H-purine-6-carboxamide

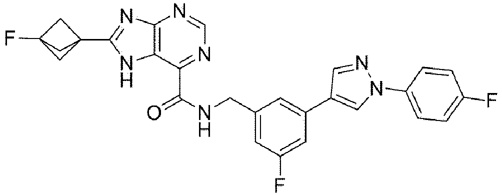

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), 3-fluorobicyclo[1.1.1]pentane-1-carboxylic acid (6.2 mg, 0.048 mmol), 1,4-dioxane (1 mL), T3P (50 wt.% solution (in EtOAc), 142 µL, 0.24 mmol) and DIPEA (16 µL, 0.095 mmol), the title compound (8.43 mg, 34.4%) was obtained as an off-white solid.
¹H NMR (400 MHz, CD₃OD) *δ* 8.99 (s, 1H), 8.58 (s, 1H), 8.05 (s, 1H), 7.83 - 7.73 (m, 2H), 7.51 (s, 1H), 7.29 (d, J = 9.8 Hz, 1H), 7.22 (t, J = 8.7 Hz, 2H), 7.03 (d, J = 9.4 Hz, 1H), 4.70 (s, 2H), 2.64 (d, J = 2.2 Hz, 6H).
LC-MS, m/z 516 [M+H]⁺

### Example 102

### N-(3-Fluoro-5-(1-(4-fluorophenyl)-1H-pyrazol-4-yl)benzyl)-8-(3-fluorocyclobutyl)-7H-purine-6-carboxamide

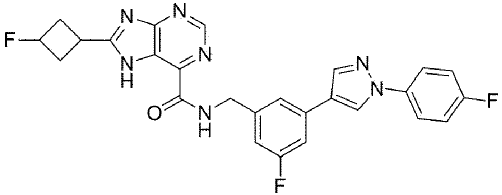

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), 3-fluorocyclobutanecarboxylic acid (5.6 mg, 0.048 mmol), 1,4-dioxane (1 mL), T3P (50 wt.% solution (in EtOAc), 142 µL, 0.24 mmol) and DIPEA (16 µL, 0.095 mmol), the title compounds were prepared as diastereomers as A (3.18 mg, 13.3%) and B (5.29 mg, 22%), respectively.

**Diastereoisomer A** (Retention time: 2.363 min., Column: XSELECT CSH C18 3.0 x 50 mm, 3.5 µm, Mobile phase: A is water (0.01% formic acid v/v) and B is acetonitrile (0.01% formic acid v/v), Gradient: from 30% to 100% of B% for 4 min) / TLC, R_{f}=0.6 (4% MeOH in DCM), eluted 4 times)
¹H NMR (400 MHz, CD₃OD) *δ* 8.98 (s, 1H), 8.60 (s, 1H), 8.07 (s, 1H), 7.79 (dd, J = 9.0, 4.6 Hz, 2H), 7.52 (s, 1H), 7.31 (d, J = 9.9 Hz, 1H), 7.23 (t, J = 8.7 Hz, 2H), 7.04 (d, J = 9.4 Hz, 1H), 5.53 - 5.29 (m, 1H), 4.70 (s, 2H), 3.96 (s, 1H), 2.93 - 2.69 (m, 4H).
LC-MS, m/z 504 [M+H]⁺

**Diastereoisomer B** (Retention time: 2.309 min., Column: XSELECT CSH C18 3.0 x 50 mm, 3.5 um, Mobile phase: A is water (0.01% formic acid v/v) and B is acetonitrile (0.01% formic acid v/v), gradient: B% 30% to 100%, for 4 min) / TLC, R_{f}=0.55 (4% MeOH (in DCM), eluted 4 times)
¹H NMR (400 MHz, CD₃OD) *δ* 8.98 (s, 1H), 8.59 (s, 1H), 8.06 (s, 1H), 7.78 (dd, J = 8.7, 4.5 Hz, 2H), 7.51 (s, 1H), 7.30 (d, J = 9.8 Hz, 1H), 7.22 (t, J = 8.5 Hz, 2H), 7.03 (d, J = 9.1 Hz, 1H), 5.21 - 4.98 (m, 1H), 4.70 (s, 2H), 3.47 - 3.36 (m, 1H), 2.88 (s, 2H), 2.78 - 2.64 (m, 2H).
LC-MS, m/z 504 [M+H]⁺

### Example 103

### Methyl 3-(6-((3-fluoro-5-(1-(4-fluorophenyl)-1H-pyrazol-4-yl)benzyl)carbamoyl)-7H-purin-8-yl)cyclobutanecarboxylate

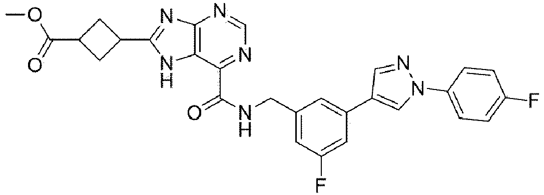

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), 3-(methoxycarbonyl)cyclobutanecarboxylic acid (7.5 mg, 0.048 mmol), 1,4-dioxane (1 mL), T3P (50 wt.% solution (in EtOAc), 142 µL, 0.24 mmol) and DIPEA (16 µL, 0.095 mmol), the title compounds were prepared as diastereomers as A (4.05 mg, 15.7%) and B (4.05 mg, 15.7%), respectively.

**Diastereoisomer A** (Retention time: 2.308 min., Column: XSELECT CSH C18 3.0 x 50 mm, 3.5 µm, Mobile phase: A is water (0.01% formic acid v/v) and B is acetonitrile (0.01% formic acid v/v), Gradient: from 30% to 100% of B for 4 min) / TLC, R_{f}=0.51 (4% MeOH in DCM), eluted 4 times)
¹H NMR (400 MHz, CD₃OD) *δ* 8.97 (s, 1H), 8.58 (s, 1H), 8.05 (s, 1H), 7.77 (dd, J = 8.6, 4.6 Hz, 2H), 7.51 (s, 1H), 7.29 (d, J = 9.4 Hz, 1H), 7.22 (t, J = 8.5 Hz, 2H), 7.03 (d, J = 9.3 Hz, 1H), 4.69 (s, 2H), 4.06 - 3.95 (m, 1H), 3.80 - 3.66 (m, 3H), 3.46 - 3.35 (m, 1H), 2.82 - 2.68 (m, 4H).
LC-MS, m/z 544 [M+H]⁺

**Diastereoisomer B** (Retention time: 2.274 min., Column: XSELECT CSH C18 3.0 x 50 mm, 3.5 um, Mobile phase: A is water (0.01% formic acid v/v) and B is acetonitrile (0.01% formic acid v/ v), gradient: B% 30% to 100%, for 4 min) / TLC, R_{f}=0.47 (4% MeOH (in DCM), eluted 4 times)
¹H NMR (400 MHz, CD₃OD) *δ* 8.97 (s, 1H), 8.60 (s, 1H), 8.06 (s, 1H), 7.78 (dd, J = 8.7, 4.7 Hz, 2H), 7.52 (s, 1H), 7.31 (d, J = 9.5 Hz, 1H), 7.23 (t, J = 8.6 Hz, 2H), 7.03 (d, J = 9.0 Hz, 1H), 4.70 (s, 2H), 3.94 - 3.80 (m, 1H), 3.77 - 3.63 (m, 3H), 3.35 - 3.33 (m, 1H), 2.87 - 2.63 (m, 4H).
LC-MS, m/z 544 [M+H]⁺

### Example 104

### 104.1 tert-Butyl 3-bromo-5-chlorobenzylcarbamate

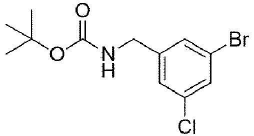

By the same method as in Example 6.1, using (3-bromo-5-chlorophenyl)methanamine (226.6 mg, 1.03 mmol), (Boc)₂O (30% (in THF), 1.6 mL, 2.06 mmol) and DCM (3.4 mL), the title compound (329 mg, 99%) was obtained as a colorless clear oil.

¹H NMR (400 MHz, CDCl₃) *δ* 7.41 (s, 1H), 7.32 (s, 1H), 7.21 (s, 1H), 1.47 (s, 9H).

### 104.2 tert-Butyl 3-chloro-5-(1H-pyrazol-4-yl)benzylcarbamate

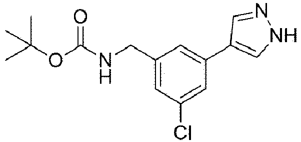

By the same method as in Example 25.2, tert-butyl 3-bromo-5-chlorobenzylcarbamate (329 mg, 1.03 mmol), 4-(4,4,5,5-tetramethyl-1,3,2-dioxaborolan-2-yl)-1H-pyrazole (299 mg, 1.54 mmol), Na₂CO₃ (381.24 mg, 3.60 mmol), Pd(PPh₃)₄ (59.4 g, 0.051 mmol), DMF (4.9 mL) and H₂O (2 mL) were used to obtain the title compound (197 mg, 62.3%) as a colorless clear oil.
LC-MS, m/z 308 [M+H]⁺

### 104.3 tert-Butyl 3-chloro-5-(1-(4-fluorophenyl)-1H-pyrazol-4-yl)benzylcarbamate

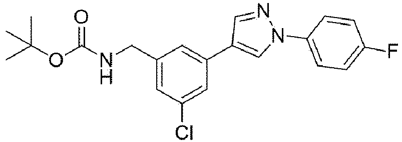

By the same method as in Example 25.3, using *tert*-butyl 3-chloro-5-(1 *H* -pyrazol-4-yl)benzylcarbamate (34.29 mg, 0.11 mmol), 1-fluoro-4-iodobenzene (37 mg, 0.17 mmol), CuI (2.1 mg, 0.011 mmol), L-proline (3 mg, 0.022 mmol), K₂CO₃ (23 mg, 0.17 mmol) and DMSO (1 mL), the title compound (25 mg, 56%) was obtained as an off-white solid.
LC-MS, m/z 402 [M+H]⁺

### 104.4 (3-Chloro-5-(1-(4-fluorophenyl)-1H-pyrazol-4-yl)phenyl)methanamine, trifluoroacetate salt

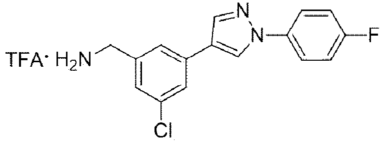

By the same method as in Example 25.4, using *tert*-butyl 3-chloro-5-(1-(4-fluorophenyl)-1*H*-pyrazol-4-yl)benzylcarbamate (25 mg, 0.14 mmol), TFA (0.4 mL) and DCM (1.2 mL), the title compound (25.6 mg, crude) was obtained.
LC-MS, m/z 302 [M+H]⁺

### 104.5 N-(3-chloro-5-(1-(4-fluorophenyl)-1H-pyrazol-4-yl)benzyl)-8-cyclopentyl-7H-purine-6-carboxamide]

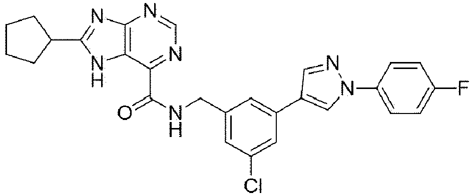

By the same method as in Example 87.3, using (3-chloro-5-(1-(4-fluorophenyl)-1H-pyrazol-4-yl)phenyl)methanamine, trifluoroacetate salt (25.6 mg, 0.062 mmol), 8-cyclopentyl-7H-purine-6-carboxylic acid (14.5 mg, 0.062 mmol), EDC (14.3 mg, 0.075 mmol), HOBt (10 mg, 0.075 mmol), DIPEA (42 µL, 0.25 mmol) and THF (1.5 mL), the title compound (15.0 mg, 47%) was obtained as a pale yellow solid.
¹H NMR (400 MHz, DMSO-*d*₆) *δ* 13.18 (s, 1H), 9.80 (s, 1H), 9.07 (s, 1H), 8.97 (s, 1H), 8.26 (s, 1H), 7.91 (dd, *J =* 9.0, 4.7 Hz, 2H), 7.71 (d, *J* = 6.6 Hz, 2H), 7.39 (t, *J* = 8.8 Hz, 2H), 7.31 (s, 1H), 4.60 (d, *J* = 6.2 Hz, 2H), 3.56 - 3.43 (m, 1H), 2.13 - 2.03 (m, 2H), 2.00 - 1.88 (m, 2H), 1.88 - 1.73 (m, 2H), 1.72 - 1.59 (m, 2H).
LC-MS, m/z 516 [M+H]⁺

### Example 105

### 105.1 tert-Butyl (3-bromo-5-fluorophenyl)carbamate

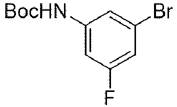

To a solution of 3-bromo-5-fluoroaniline (200 mg, 1.1 mmol) in DCM (5 mL) were added (Boc)₂0 (200 mg, 0.93 mmol) and TEA (220 *µ* L, 1.6 mmol). The mixture was stirred at 50 °C overnight. After completion of the reaction, the reaction mixture was concentrated, and the obtained residue was purified by silica gel column chromatography (0-10% EtOAc, in hexane) to obtain the title compound (170 mg, 54%).
LC-MS, *m*/*z* 291 [M+H]⁺

### 105.2 tert-Butyl (3-fluoro-5-(1-(4-fluorophenyl)-1H-pyrazol-4-yl)phenyl)carbamate

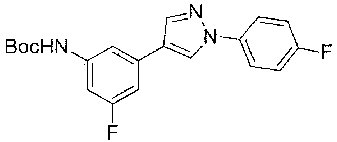

By the same method as in Example 4.1, using tert-butyl (3-bromo-5-fluorophenyl) carbamate (170 mg, 0.57 mmol), (1-(4-fluorophenyl)-1H-pyrazol-4-yl)boronic acid (180 mg, 0.86 mmol), PdCl₂ (dppf) (21 mg, 0.029 mmol), 2 M aq. K₂CO₃(1 mL) and 1,4-dioxane (2 mL), the title compound (160 mg, 77%) was obtained as a white solid.
LC-MS, *m*/*z* 372 [M+H]⁺

### 105.3 3-Fluoro-5-(1-(4-fluorophenyl)-1H-pyrazol-4-yl)aniline

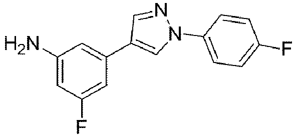

By the same method as in Example 58.2, using tert-butyl (3-fluoro-5-(1-(4-fluorophenyl)-1H-pyrazol-4-yl)carbamate (80 mg, 0.22 mmol), 4 M HCl (in dioxane) (1 mL) and DCM (2 mL), the title compound (58 mg, crude) was obtained as a pale yellow solid.
LC-MS, *m*/*z* 272 [M+H]⁺

### 105.4 8-Cyclopentyl-N-(3-fluoro-5-(1-(4-fluorophenyl)-1H-pyrazol-4-yl)phenyl)-7H-purine-6-carboxamide

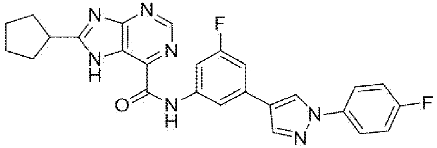

By the same method as in Example 87.3, using 3-fluoro-5-(1-(4-fluorophenyl)-1H-pyrazol-4-yl)aniline (58 mg, 0.22 mmol), 8-cyclopentyl-7H-purine-6-carboxylic acid (50 mg, 0.22 mmol), EDC (49 mg, 0.26 mmol), HOBt (35 mg, 0.26 mmol), DIPEA (110 µL, 0.65 mmol) and THF (2 mL), the title compound (4 mg, 4%) was obtained as a light yellow solid.
¹H NMR (400 MHz, DMSO-*d*₆) *δ* 13.36 (s, 1H), 11.13 (s, 1H), 9.09 - 8.99 (m, 2H), 8.20 (s, 1H), 8.13 (s, 1H), 7.93 (dd, *J* = 9.1, 4.7 Hz, 2H), 7.84 (d, *J* = 11.0 Hz, 1H), 7.41 (t, *J =* 8.7 Hz, 3H), 3.59 - 3.51 (m, 1H), 2.17 - 2.08 (m, 2H), 2.01 - 1.93 (m, 2H), 1.88 - 1.78 (m, 2H), 1.73 - 1.65 (m, 2H).
LC-MS, *m*/*z* 486 [M+H]⁺

### Example 106

### 3-(6-((3-Fluoro-5-(1-(4-fluorophenyl)-1H-pyrazol-4-yl)benzyl)carbamoyl)-7H-purin-8-yl)cyclobutanecarboxylic acid

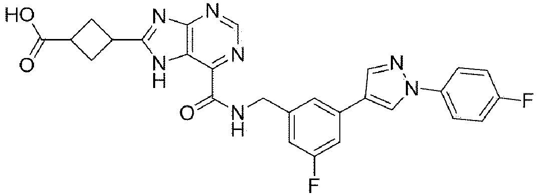

Methyl 3-(6-((3-fluoro-5-(1-(4-fluorophenyl)-1H-pyrazol-4-yl)benzyl)carbamoyl)-7H-purin-8-yl)cyclobutanecarboxylate (18.69 mg, 0.034 mmol) was dissolved in MeOH (1 mL), 0.1 M aq. NaOH (2 mL) was added, and the mixture was stirred at room temperature. After 3 h, the reaction mixture was diluted with H₂O, acidified (pH 4~5), and extracted with EtOAc. The organic layer was dried over MgSO₄ and concentrated. The obtained residue was purified by silica gel column chromatography (0-10% MeOH in DCM) to obtain the title compound prepared as diastereomers (4.68 mg, yield 25.7%, mixture of diastereomers) as a light brown solid.
¹H NMR (400 MH, CD₃OD+CDCl₃, mixture of diastereomers) *δ* 8.97 (s, 1H), 8.94 (s, 1H), 8.55 (s, 1H), 8.48 (s, 1H), 8.04 (s, 1H), 7.98 (s, 1H), 7.80 - 7.70 (m, 4H), 7.51 (s, 1H), 7.40 (s, 1H), 7.28 (d, J = 9.8 Hz, 1H), 7.26 - 7.13 (m, 5H), 7.04 (d, J = 9.5 Hz, 1H), 6.95 (d, J = 9.1 Hz, 1H), 4.74 (s, 2H), 4.71 (s, 2H), 4.06 - 3.95 (m, 1H), 3.90 - 3.80 (m, 1H), 3.30 - 3.24 (m, 1H), 3.25 - 3.16 (m, 1H), 2.98 - 2.81 (m, 2H), 2.77 (t, J = 7.8 Hz, 4H), 2.71 - 2.59 (m, 2H).
LC-MS, m/z 530 [M+H]⁺

### Example 107

### 107.1 2-Chloro-3-nitroisonicotinic acid

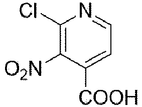

Methyl 2-chloro-3-nitroisonicotinate (100 mg, 0.46 mmol) was dissolved in MeOH (9 mL), 0.1 M aq. NaOH (17 mL) was added, and the mixture was stirred at room temperature overnight. The reaction mixture was acidified (pH 4~5) with 1 M aq. HCl, extracted with EtOAc, and the organic layer was dried over MgSO₄. The organic layer was concentrated to obtain the title compound (88.24 mg, 94.4%) as a white solid.
LC-MS, m/z 201 [M-H]⁻

### 107.2 2-Chloro-N-(3-fluoro-5-(1-(4-fluorophenyl)-1H-pyrazol-4-yl)benzyl)-3-nitroisonicotinamide

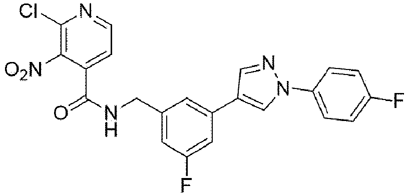

2-Chloro-3-nitroisonicotinic acid (88.24 mg, 0.44 mmol), (3-fluoro-5-(1- (4-fluorophenyl)-1H-pyrazol-4-yl)phenyl)methanamine (124.3 mg, 0.44 mmol), DIPEA (96 µL, 0.57 mmol) and HATU (199 mg, 0.52 mmol) were dissolved in THF (4 mL) and stirred at room temperature overnight. The reaction mixture was diluted with H₂O and extracted with EtOAc. The organic layer was dried over MgSO₄ and concentrated to obtain the title compound (189 mg, crude) as an off-white solid.
LC-MS, m/z 470 [M+H]⁺

### 107.3 2-((2,4-Dimethoxybenzyl)amino)-N-(3-fluoro-5-(1-(4-fluorophenyl)-1H-pyrazol-4-yl)benzyl)-3-nitroisonicotinamide

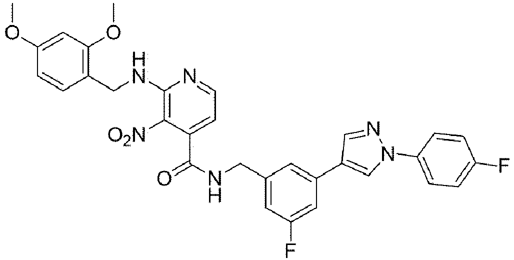

2-Chloro-*N*-(3-fluoro-5-(1-(4-fluorophenyl)-1*H*-pyrazol-4-yl)benzyl)-3-nitroisonicotinamide (189 mg, _{0.40} mmol), (2,4-dimethoxyphenyl)methanamine (81 mg, 0.48 mmol) and Cs₂CO₃ ( ₁₇₀ mg, 0.52 mmol) were dissolved in 1,4-dioxane (6 mL) and stirred at room temperature overnight. The reaction mixture was diluted with H₂O and extracted with EtOAc. The organic layer was dried over MgSO₄ and concentrated to obtain the title compound (196.4 mg, crude) as a yellow solid.
LC-MS, m/z 601 [M+H]⁺

### 107.4 3-Amino-N-(3-chloro-5-(1-(4-fluorophenyl)-1H-pyrazol-4-yl)benzyl)-2-((2,4-dimethoxybenzyl)amino)isonicotinamide

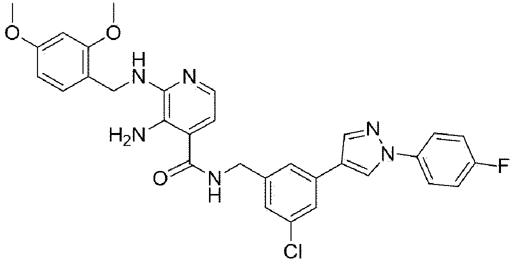

By the same method as in Example 61.2, a mixture of 2-((2,4-dimethoxybenzyl)amino)-*N*-(3-fluoro-5-(1-(4-fluorophenyl)-1*H*-pyrazol-4-yl)benzyl)-3-nitroisonicotinamide (196.4 mg, 0.33 mmol), SnCl₂ (310 mg, 1.64 mmol) and EtOH (10 mL) were used to obtain the title compound (51 mg, 54.7%).
LC-MS, m/z 571 [M+H]⁺

### 107.5 2,3-Diamino-N-(3-fluoro-5-(1-(4-fluorophenyl)-1H-pyrazol-4-yl)benzyl)isonicotinamide

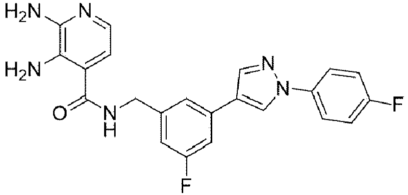

By the same method as in Example 61.5, 3-amino-N-(3-chloro-5-(1-(4-fluorophenyl)-1H-pyrazol-4-yl)benzyl)-2-((2,4-dimethoxybenzyl)amino)isonicotinamide (46 mg, 0.087 mmol), TFA (0.54 mL) and DCM (1.6 mL) were used to obtain the title compound (20.54 mg, 60.6%).
LC-MS, m/z 421 [M+H]⁺

### 107.6 2-Cyclopentyl-N-(3-fluoro-5-(1-(4-fluorophenyl)-1H-pyrazol-4-yl)benzyl)-1H-imidazo [4,5-b] pyridine-7-carboxamide

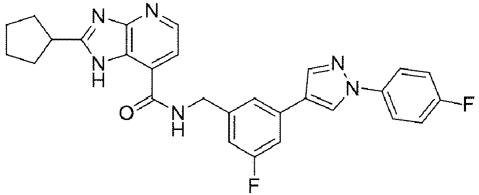

By the same method as in Example 72.5, using 2,3-diamino-N-(3-fluoro-5-(1-(4-fluorophenyl)-1H-pyrazol-4-yl)benzyl)isonicotinamide (20.54 mg, 0.049 mmol), cyclopentanecarboxylic acid (6 mg, 0.049 mmol), T3P (50 wt.% solution (in EtOAc), 146 µL, 0.24 mmol), DIPEA (17 µL, 0.098 mmol) and 1,4-dioxane (1.5 mL), the title compound (2.13 mg, 8.7%) was obtained.
¹H NMR (400 MHz, CDCl₃+CD₃OD) *δ* 9.42 - 8.34 (m, 2H), 8.01 (s, 1H), 7.84 (d, J = 5.1 Hz, 1H), 7.73 (dd, J = 8.9, 4.4 Hz, 2H), 7.49 (s, 1H), 7.30 - 7.18 (m, 3H), 7.10 (d, J = 8.9 Hz, 1H), 4.81 (s, 2H), 3.42 - 3.35 (m, 1H), 2.21 - 2.09 (m, 2H), 2.06 -1.92 (m, 2H), 1.87 - 1.75 (m, 2H), 1.75 - 1.64 (m, 2H).
LC-MS, m/z 499 [M+H]⁺

### Example 108

### 108.1 tert-Butyl 4-bromobenzylcarbamate

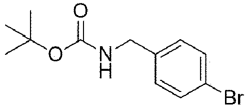

By the same method as in Example 6.1, using (4-bromophenyl)methanamine (100 mg, 0.54 mmol), (Boc)₂O (30% (in THF), 421 µL, 0.55 mmol), TEA (113 µL, 0.81 mmol) and DCM (1 mL), the title compound (149 mg, 96.9%) was obtained as a white solid.

¹H NMR (400 MHz, CDCl₃) *δ* 7.45 (d, J = 8.3 Hz, 2H), 7.16 (d, J = 8.2 Hz, 2H), 4.85 (broad, 1H), 4.26 (d, J = 5.4 Hz, 2H), 1.46 (s, 9H).

### 108.2 tert-Butyl 4-(1-(4-fluorophenyl)-1H-pyrazol-4-yl)benzylcarbamate

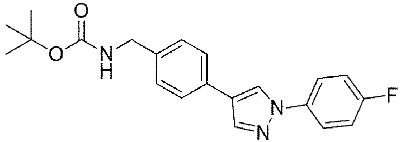

By the same method as in Example 4.1, using tert-butyl 4-bromobenzylcarbamate (144 mg, 0.50 mmol), (1-(4-fluorophenyl)-1H-pyrazol-4-yl)boronic acid (156 mg, 0.76 mmol), PdCl₂ (dppf) (7.2 mg, 0.0099 mmol), 2M K₂CO₃ (0.9 mL, 1.76 mmol) and 1,4-dioxane (3.5 mL), the title compound (131 mg, 70.9%) was obtained as a white solid.
LC-MS, m/z 368 [M+H]⁺

### 108.3 (4-(1-(4-Fluorophenyl)-1H-pyrazol-4-yl)phenyl)methanamine trifluoroacetate

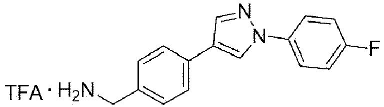

By the same method as in Example 25.4, using tert-butyl 4-(1-(4-fluorophenyl)-1H-pyrazol-4-yl)benzylcarbamate (50.38 mg, 0.14 mmol), TFA (0.5 mL) and DCM (1.4 mL), the title compound (63 mg, crude) was obtained.
LC-MS, m/z 268 [M+H]⁺

### 108.4 8-Cyclopentyl-N-(4-(1-(4-fluorophenyl)-1H-pyrazol-4-yl)benzyl)-7H-purine-6-carboxamide

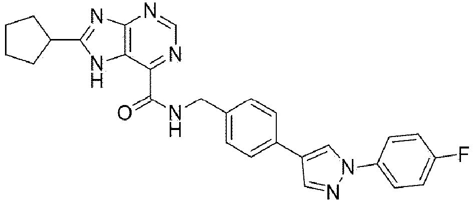

By the same method as in Example 87.3, (4-(1-(4-fluorophenyl)-1H-pyrazol-4-yl)phenyl)methanamine trifluoroacetate (52 mg, 0.14 mmol), 8-cyclopentyl-7H-purine-6-carboxylic acid (31.8 mg, 0.14 mmol), EDC (31.5 mg, 0.16 mmol), HOBt (22 mg, 0.16 mmol), DIPEA (70 µL, 0.41 mmol) and THF (1.4 mL) were used to obtain the title compound (36.25 mg, 54.95%) as a pale yellow solid.
¹H NMR (400 MHz, DMSO-*d*₆) *δ* 13.18 (s, 1H), 9.72 (t, J = 6.2 Hz, 1H), 8.95 (d, J = 9.3 Hz, 2H), 8.18 (s, 1H), 7.90 (dd, J = 9.0, 4.8 Hz, 2H), 7.66 (d, J = 8.0 Hz, 2H), 7.46 - 7.32 (m, 4H), 4.56 (d, J = 6.2 Hz, 2H), 3.57 - 3.44 (m, 1H), 2.15 - 2.03 (m, 2H), 2.10 - 1.86 (m, 2H), 1.85 - 1.74 (m, 2H), 1.73 - 1.59 (m, 2H).
LC-MS, m/z 482 [M+H]⁺

### Example 109

### 109.1 3-Fluoro-4-(1-(4-fluorophenyl)-1H-pyrazol-4-yl)benzonitrile

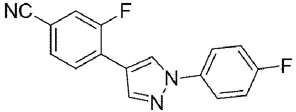

By the same method as in Example 4.1, 4-bromo-3-fluorobenzonitrile (100 mg, 0.50 mmol), (1-(4-fluorophenyl)-1H-pyrazol-4-yl)boronic acid (155 mg, 0.75 mmol), PdCl₂ (dppf) (18.3 mg, 0.025 mmol), 2M K₂CO₃ (0.9 mL, 1.76 mmol) and 1,4-dioxane (3.5 mL) were used to obtain the title compound (116 mg, 82.6%) as a white solid.
¹H NMR (400 MHz, CDCl₃) *δ* 8.33 (d, J = 1.8 Hz, 1H), 8.11 (s, 1H), 7.77 - 7.67 (m, 3H), 7.54 - 7.43 (m, 2H), 7.24 - 7.15 (m, 2H).
LC-MS, m/z 282 [M+H]⁺

### 109.2 (3-Fluoro-4-(1-(4-fluorophenyl)-1H-pyrazol-4-yl)phenyl)methanamine

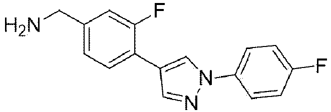

By the same method as in Example 7.2, using 3-fluoro-4-(1-(4-fluorophenyl)-1H-pyrazol-4-yl)benzonitrile (46.2 mg, 0.16 mmol), LAH (2 M (in THF), 0.16 mL, 0.36 mmol) and THF (2 mL), the title compound (20.34 mg, 40%) was obtained as a yellow solid.
LC-MS, m/z 286 [M+H]⁺

### 109.3 8-Cyclopentyl-N-(3-fluoro-4-(1-(4-fluorophenyl)-1H-pyrazol-4-yl)benzyl)-7H-purine-6-carboxamide

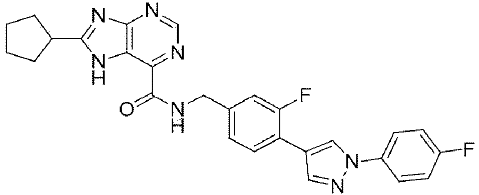

By the same method as in Example 87.3, (3-fluoro-4-(1-(4-fluorophenyl)-1H-pyrazol-4-yl)phenyl)methanamine (20.34 mg, 0.071 mmol), 8-cyclopentyl-7H-purine-6-carboxylic acid (16.6 mg, 0.071 mmol), EDC (16.4 mg, 0.086 mmol), HOBt (12 mg, 0.086 mmol), DIPEA (37 µL, 0.21 mmol) and THF (1.4 mL) were used to obtain the title compound (14.1 mg, 39.6%) as a pale yellow solid.
¹H NMR (400 MHz, DMSO-*d*₆) *δ* 13.17 (s, 1H), 9.78 (t, J = 6.2 Hz, 1H), 8.97 (s, 1H), 8.86 (s, 1H), 8.16 (s, 1H), 7.93 (dd, J = 9.0, 4.8 Hz, 2H), 7.78 (t, J = 8.0 Hz, 1H), 7.37 (t, J = 8.8 Hz, 2H), 7.33 - 7.25 (m, 2H), 4.58 (d, J = 6.3 Hz, 2H), 3.56 - 3.44 (m, 1H), 2.14 - 2.02 (m, 2H), 2.00 - 1.88 (m, 2H), 1.86 - 1.75 (m, 2H), 1.72 - 1.60 (m, 2H).
LC-MS, m/z 500 [M+H]⁺

### Example 110

### N-(3-Fluoro-5-(1-(4-fluorophenyl)-1H-pyrazol-4-yl)benzyl)-8-(3-fluorocyclopentyl)-7H-purine-6-carboxamide

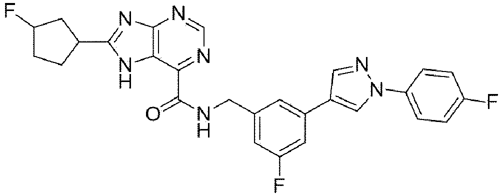

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), 3-fluorocyclopentanecarboxylic acid (6.3 mg, 0.048 mmol), T3P (50 wt.% solution in EtOAc, 142 µL, 0.24 mmol), DIPEA (16 µL, 0.095 mmol) and 1,4-dioxane (1 mL), the title compounds were prepared as diastereomers as A (1.2 mg, 4.9%) and B (1.2 mg, 4.9%), respectively.

**Diastereoisomer A** (Retention time: 2.44 min., Column: XSELECT CSH C18 3.0 x 50 mm, 3.5 µm, Mobile phase: A is water (0.01% formic acid v/v) and B is acetonitrile (0.01% formic acid v/v), Gradient: from 30% to 100% of B for 4 min) / TLC, R_{f}=0.49 (2% MeOH in DCM), eluted 4 times)
¹H NMR (400 MHz, CDCl₃) *δ* 10.63 (s, 1H), 9.05 (s, 1H), 8.48 (s, 1H), 8.10 (s, 1H), 7.96 (s, 1H), 7.68 (dd, J = 9.0, 4.6 Hz, 2H), 7.33 (s, 1H), 7.22 - 7.13 (m, 3H), 7.00 (d, J = 9.4 Hz, 1H), 5.47 - 5.27 (m, 1H), 4.74 (d, J = 6.1 Hz, 2H), 3.80 - 3.67 (m, 1H), 2.64 - 1.98 (m, 6H).
LC-MS, m/z 518 [M+H]⁺

**Diastereoisomer B** (Retention time: 2.36 min., Column: XSELECT CSH C18 3.0 x 50 mm, 3.5 um, Mobile phase: A is water (0.01% formic acid v/v) and B is acetonitrile (0.01% formic acid v/ v), gradient: B% 30% to 100%, 4 min) / TLC, R_{f} =0.47 (2% MeOH (in DCM), eluted 4 times)
¹H NMR (400 MHz, CDCl₃) *δ* 10.59 (s, 1H), 9.05 (s, 1H), 8.42 (s, 1H), 8.10 (s, 1H), 7.96 (s, 1H), 7.73 - 7.64 (m, 2H), 7.33 (s, 1H), 7.22 - 7.14 (m, 3H), 7.00 (d, J = 8.6 Hz, 1H), 5.50 - 5.27 (m, 1H), 4.74 (d, J = 6.3 Hz, 2H), 3.83 (m, 1H), 2.59 - 1.94 (m, 6H).
LC-MS, m/z 518 [M+H]⁺

### Example 111

### N-(3-fluoro-5-(1-(4-fluorophenyl)-1H-pyrazol-4-yl)benzyl)-8-(spiro[2.3]hexan-5-yl)-7H-purine-6-carboxamide

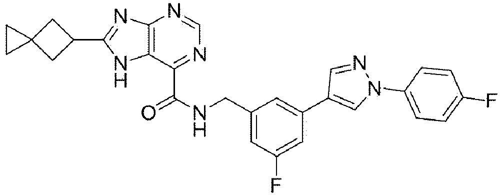

By the same method as in Example 72.5, 5,6-diamino-N-(3-fluoro-5-(1-(4-fluorophenyl)-1H-pyrazol-4-yl)benzyl)pyrimidine-4-carboxamide (20 mg, 0.048 mmol), spiro[2.3]hexane-5-carboxylic acid (6 mg, 0.048 mmol), T3P (50 wt.% solution in EtOAc, 142 µL, 0.24 mmol), DIPEA (16 µL, 0.095 mmol) and 1,4-dioxane (2 mL) were used to obtain the title compound (1.6 mg, 4.8%) as an off-white solid.
¹H NMR (400 MHz, CD₃OD) *δ* 8.98 (s, 1H), 8.62 (s, 1H), 8.08 (s, 1H), 7.80 (dd, J = 7.7, 5.0 Hz, 2H), 7.55 (s, 1H), 7.33 (d, J = 9.2 Hz, 1H), 7.24 (t, J = 7.8 Hz, 2H), 7.05 (d, J = 9.8 Hz, 1H), 4.72 (s, 2H), 4.17 - 4.03 (m, 1H), 2.90 - 2.75 (m, 2H), 2.57 - 2.44 (m, 2H), 0.67 - 0.43 (m, 4H).
LC-MS, m/z 512 [M+H]⁺

### Example 112

### 112.1 tert-Butyl 3-fluoro-5-(1-(4-fluorophenyl)-1H-pyrazol-4-yl)benzyl (methyl)carbamate

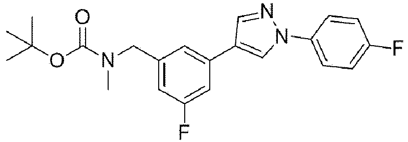

tert-Butyl 3-fluoro-5-(1-(4-fluorophenyl)-1H-pyrazol-4-yl)benzylcarbamate (100 mg, 0.26 mmol) was dissolved in THF (2.5 mL), and NaH (60% suspension in mineral oil) (7 mg, 0.29 mmol) was slowly added at 0 °C. The reaction temperature was raised to room temperature, and the mixture was stirred for 20 minutes. MeI (19 µL, 0.31 mmol) was added, and the mixture was stirred at 45 °C overnight. The reaction mixture was quenched with H₂O, extracted with EtOAc, and the organic layer was dried over MgSO₄. The concentrated residue was purified by silica gel column chromatography (0-15% EtOAc in hexane) to btain the title compound (18.89 mg, 18%).
LC-MS, m/z 400 [M+H]⁺

### 112.2 1-(3-Fluoro-5-(1-(4-fluorophenyl)-1H-pyrazol-4-yl)phenyl)-N-methylmethanamine trifluoroacetate

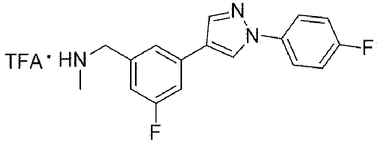

By the same method as in Example 25.4, using tert-butyl 3-fluoro-5-(1-(4-fluorophenyl)-1H-pyrazol-4-yl)benzyl(methyl)carbamate (8 mg, 0.02 mmol), TFA (0.3 mL) and DCM (1 mL), the title compound (8.27 mg, crude) was obtained.
LC-MS, m/z 300 [M+H]⁺

### 112.3 8-Cyclopentyl-N-(3-fluoro-5-(1-(4-fluorophenyl)-1H-pyrazol-4-yl)benzyl)-N-methyl-7H-purine-6-carboxamide

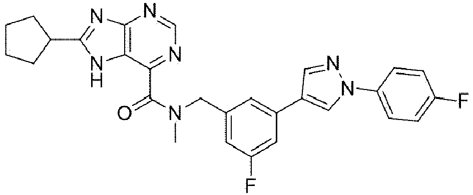

By the same method as in Example 87.3, using 1-(3-fluoro-5-(1-(4-fluorophenyl)-1H-pyrazol-4-yl)phenyl)-N-methylmethanamine trifluoroacetate (8.27 mg, 0.02 mmol), 8-cyclopentyl-7H-purine-6-carboxylic acid (5 mg, 0.02 mmol), EDC (5 mg, 0.024 mmol), HOBt (3.24 mg, 0.024 mmol), DIPEA (11 µL, 0.06 mmol) and THF (2 mL), the title compound (8.48 mg, 82.6%, rotamer mixture) was obtained as a white solid.
¹H NMR (400 MHz, DMSO-*d*₆, rotamer mixture 50/50) *δ* 13.41 (s, 1H), 9.07 (s, 0.5H), 8.98 (s, 0.5H), 8.87 (d, J = 10.8 Hz, 1H), 8.32 (s, 0.5H), 8.20 (s, 0.5H), 7.99 - 7.86 (m, 2H), 7.60 (s, 0.5H), 7.54 (d, J = 9.6 Hz, 0.5H), 7.50 - 7.33 (m, 3H), 7.19 (s, 0.5H), 7.08 (s, 0.5H), 4.85 (s, 1H), 4.62 - 4.33 (m, 1H), 3.47 - 3.25 (m, 1H), 3.04 (s, 1.5H), 2.87 (s, 1.5H), 2.15 - 1.54 (m, 8H).
LC-MS, m/z 514 [M+H]⁺

### Example 113

### 8-(Bicyclo[3.1.0]hexan-3-yl)-N-(3-fluoro-5-(1-(4-fluorophenyl)-1H-pyrazol-4-yl)benzyl)-7H-purine-6-carboxamide

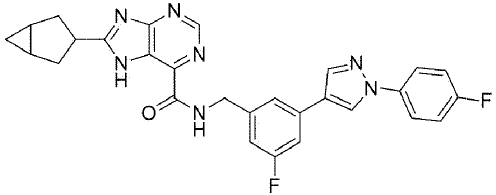

By the same method as in Example 72.5, using 5,6-diamino-N-(3-fluoro-5-(1-(4-fluorophenyl)-1H-pyrazol-4-yl)benzyl)pyrimidine-4-carboxamide (30 mg, 0.071 mmol), bicyclo[3.1.0]hexane-3-carboxylic acid (9 mg, 0.071 mmol), T3P (50 wt.% solution in EtOAc, 212 µL, 0.36 mmol), DIPEA (24 µL, 0.14 mmol) and 1,4-dioxane (2 mL), the title compound (2.4 mg, 6.6%) was obtained as an off-white solid.
¹H NMR (400 MHz, CD₃OD) *δ* 8.96 (s, 1H), 8.62 (s, 1H), 8.08 (s, 1H), 7.79 (dd, J = 8.9, 4.7 Hz, 2H), 7.53 (s, 1H), 7.32 (d, J = 10.3 Hz, 1H), 7.24 (t, J = 8.7 Hz, 2H), 7.04 (d, J = 9.1 Hz, 1H), 4.70 (s, 2H), 3.23 - 3.11 (m, 1H), 2.26 (d, J = 8.9 Hz, 2H), 2.07 - 1.87 (m, 2H), 1.36 - 1.27 (m, 2H), 0.50 - 0.43 (m, 1H), 0.42 - 0.11 (m, 1H).
LC-MS, m/z 512 [M+H]⁺

### Example 114

### 8-(Bicyclo[2.2.1]heptan-2-yl)-N-(3-fluoro-5-(1-(4-fluorophenyl)-1H-pyrazol-4-yl)benzyl)-7H-purine-6-carboxamide

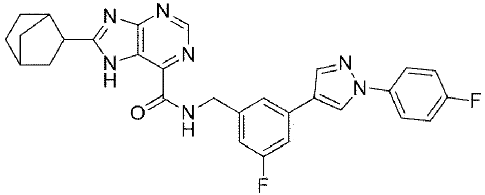

By the same method as in Example 72.5, using 5,6-diamino-N-(3-fluoro-5-(1-(4-fluorophenyl)-1H-pyrazol-4-yl)benzyl)pyrimidine-4-carboxamide (20 mg, 0.048 mmol), bicyclo[2.2.1]heptane-2-carboxylic acid (7 mg, 0.048 mmol), T3P (50 wt.% solution in EtOAc, 141 µL, 0.24 mmol), DIPEA (16 µL, 0.095 mmol) and 1,4-dioxane (1.5 mL), the title compound (3.16 mg, 12.7%) was obtained as an off-white solid.
¹H NMR (400 MHz, CD₃OD) *δ* 9.01 - 8.91 (m, J = 6.8 Hz, 1H), 8.61 (s, 1H), 7.79 (dd, J = 9.0, 4.6 Hz, 2H), 7.53 (s, 1H), 7.32 (d, J = 9.9 Hz, 1H), 7.23 (t, J = 8.7 Hz, 2H), 7.05 (d, J = 9.4 Hz, 1H), 4.71 (s, 2H), 3.21 - 3.10 (m, 1H), 2.56 (s, 1H), 2.41 (s, 1H), 2.26 - 2.16 (m, 1H), 1.86 - 1.17 (m, 8H).
LC-MS, m/z 526 [M+H]⁺

### Example 115

### 8-(Bicyclo[2.1.1]hexan-1-yl)-N-(3-fluoro-5-(1-(4-fluorophenyl)-1H-pyrazol-4-yl)benzyl)-7H-purine-6-carboxamide

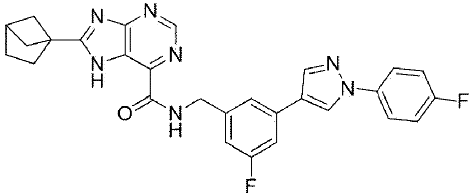

By the same method as in Example 72.5, 5,6-diamino-N-(3-fluoro-5-(1-(4-fluorophenyl)-1H-pyrazol-4-yl)benzyl)pyrimidine-4-carboxamide (30 mg, 0.071 mmol), bicyclo[2.1.1]hexane-1-carboxylic acid (9 mg, 0.071 mmol), T3P (50 wt.% solution in EtOAc, 212 µL, 0.36 mmol), DIPEA (24 µL, 0.14 mmol) and 1,4-dioxane (2 mL) were used to obtain the title compound (1.1 mg, 3%) as an off-white solid.
¹H NMR (400 MHz, CD₃OD) *δ* 8.99 (s, 1H), 8.64 (s, 1H), 8.09 (s, 1H), 7.81 (dd, J = 9.0, 4.6 Hz, 2H), 7.55 (s, 1H), 7.34 (d, J = 9.0 Hz, 1H), 7.25 (t, J = 8.7 Hz, 2H), 7.07 (d, J = 9.9 Hz, 1H), 4.72 (s, 2H), 2.63 (s, 1H), 2.21 - 1.89 (m, 7H), 1.71 - 1.55 (m, 2H).
LC-MS, m/z 512 [M+H]⁺

### Example 116

### 116.1 tert-Butyl 3-azido-5-fluorobenzylcarbamate

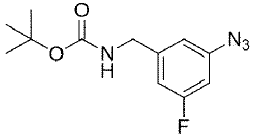

tert-Butyl 3-bromo-5-fluorobenzylcarbamate (300 mg, 0.99 mmol), NaN₃ (128 mg, 1.97 mmol), CuI (38 mg, 0.20 mmol), trans-N,N'dimethylcyclohexane-1,2-diamine (47 µL, 0.30 mmol), and sodium ascorbate (20 mg, 0.099 mmol) were dissolved in EtOH (7 mL)/H₂O (3 mL), and the mixture was stirred at 100 °C overnight. The reaction mixture was diluted with H₂O, extracted with EtOAc, and the organic layer was dried over Na₂SO₄. The organic layer was concentrated to obtain the title compound (263 mg, 99%).

### 116.2 tert-Butyl 3-fluoro-5-(4-(4-fluorophenyl)-1H-1,2,3-triazol-1-yl)benzylcarbamate

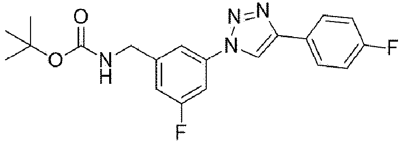

tert-Butyl 3-azido-5-fluorobenzylcarbamate (263 mg, 0.99 mmol), 1-ethynyl-4-fluorobenzylcarbamate (119 mg, 0.99 mmol), a 10% aqueous solution of CuSO₄·5H₂O (767 µL, 0.30 mmol), and sodium ascorbate (59 mg, 0.30 mmol) were dissolved in t-BuOH (5 mL)/H₂O (5 mL), and the mixture was stirred at room temperature overnight. The reaction mixture was diluted with H₂O, extracted with EtOAc, and the organic layer was dried over MgSO₄. The organic layer was concentrated, and the obtained residue was purified by silica gel column chromatography (0-20% EtOAc in hexane) to obtain the title compound (222.7 mg, 57.6%).

¹H NMR (400 MHz, CDCl₃) *δ* 8.14 (s, 1H), 7.92 - 7.85 (m, 2H), 7.54 (s, 1H), 7.46 (d, J = 8.3 Hz, 1H), 7.17 (t, J = 8.5 Hz, 2H), 7.11 (d, J = 8.8 Hz, 1H), 5.05 (s, 1H), 4.42 (d, J = 5.9 Hz, 2H), 1.48 (s, 9H).

### 116.3 (3-Fluoro-5-(4-(4-fluorophenyl)-1H-1,2,3-triazol-1-yl)phenyl)methanamine trifluoroacetate

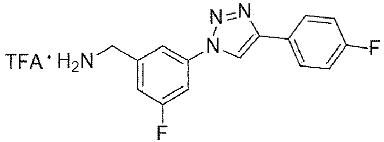

By the same method as in Example 25.4, tert-butyl 3-fluoro-5-(4-(4-fluorophenyl)-1H-1,2,3-triazol-1-yl)benzylcarbamate (33.32 mg, 0.086 mmol), TFA (0.3 mL) and DCM (1 mL) were used to obtain the title compound (34.4 mg, crude).
LC-MS, m/z 287 [M+H]⁺

### 116.4 8-Cyclopentyl-N-(3-fluoro-5-(4-(4-fluorophenyl)-1H-1,2,3-triazol-1-yl)benzyl)-7H-purine-6-carboxamide

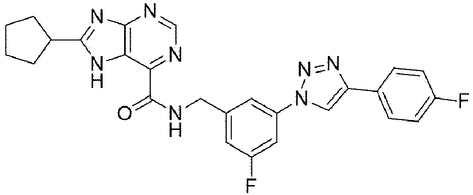

By the same method as in Example 87.3, (3-fluoro-5-(4-(4-fluorophenyl)-1H-1,2,3-triazol-1-yl)phenyl)methanamine trifluoroacetate (34.4 mg, 0.086 mmol), 8-cyclopentyl-7H-purine-6-carboxylic acid (20 mg, 0.086 mmol), EDC (20 mg, 0.10 mmol), HOBt (14 mg, 0.10 mmol), DIPEA (44 µL, 0.26 mmol) and THF (2 mL) were used to obtain the title compound (29.57 mg, 68.7%) as a pale yellow solid.
¹H NMR (400 MHz, DMSO-*d*₆) *δ* 13.18 (s, 1H), 9.90 (s, 1H), 9.32 (s, 1H), 8.98 (s, 1H), 7.95 (dd, J = 8.6, 5.6 Hz, 2H), 7.90 (s, 1H), 7.75 (d, J = 9.5 Hz, 1H), 7.42 - 7.30 (m, 3H), 4.68 (d, J = 6.2 Hz, 2H), 3.55 - 3.43 (m, 1H), 2.13 - 2.02 (m, 2H), 1.99 - 1.86 (m, 2H), 1.85 - 1.74 (m, 2H), 1.70 - 1.59 (m, 2H).
LC-MS, m/z 501 [M+H]⁺

### Example 117

### 117.1 tert-Butyl 3-fluoro-5-(1-(thiophen-3-yl)-1H-pyrazol-4-yl)benzylcarbamate

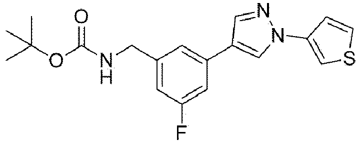

By the same method as in Example 25.3, using tert-butyl 3-fluoro-5-(1H-pyrazol-4-yl)benzylcarbamate (201.53 mg, 0.69 mmol), 3-iodothiophene (218.4 mg, 1.04 mmol), CuI (13 mg, 0.069 mmol), L-proline (16 mg, 0.14 mmol), K₂CO₃ (144 mg, 1.04 mmol) and DMSO (6 mL), the title compound (186.19 mg, 72.3%) was obtained as a yellow solid.
LC-MS, m/z 374 [M+H]⁺

### 117.2 (3-Fluoro-5-(1-(thiophen-3-yl)-1H-pyrazol-4-yl)phenyl)methanamine trifluoroacetate

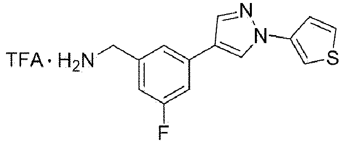

By the same method as in Example 25.4, using tert-butyl 3-fluoro-5-(1-(thiophen-3-yl)-1H-pyrazol-4-yl)benzylcarbamate (144.45 mg, 0.39 mmol), TFA (1.3 mL) and DCM (4 mL), the title compound (150 mg, crude) was obtained.
LC-MS, m/z 274 [M+H]⁺

### 117.3 8-Cyclopentyl-N-(3-fluoro-5-(1-(thiophen-3-yl)-1H-pyrazol-4-yl)benzyl)-7H-purine-6-carboxamide

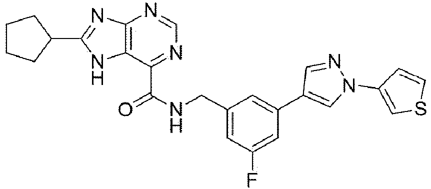

By the same method as in Example 87.3, using (3-fluoro-5-(1-(thiophen-3-yl)-1H-pyrazol-4-yl)phenyl)methanamine trifluoroacetate (150 mg, 0.39 mmol), 8-cyclopentyl-7H-purine-6-carboxylic acid (89.8 mg, 0.39 mmol), EDC (89 mg, 0.46 mmol), HOBt (63 mg, 0.46 mmol), DIPEA (197 µL, 1.16 mmol) and THF (4 mL), the title compound (137.3 mg, 72.8%) was obtained as a yellow solid.
¹H NMR (400 MHz, DMSO-*d*₆) *δ* 13.17 (s, 1H), 9.78 (t, J = 6.4 Hz, 1H), 8.97 (s, 1H), 8.91 (s, 1H), 8.18 (s, 1H), 7.76 - 7.69 (m, 2H), 7.61 - 7.53 (m, 2H), 7.44 (d, J = 10.0 Hz, 1H), 7.06 (d, J = 9.3 Hz, 1H), 4.60 (d, J = 6.1 Hz, 2H), 3.55 - 3.43 (m, 1H), 2.15 - 2.02 (m, 2H), 1.98 - 1.87 (m, 2H), 1.86 - 1.72 (m, 2H), 1.75 - 1.57 (m, 2H).
LC-MS, m/z 488 [M+H]⁺

### Example 118

### 118.1 3-Fluoro-5-((1-methyl-1H-pyrazol-3-yl)amino)benzonitrile

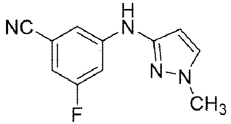

By the same method as in Example 10.1, 3-bromo-5-fluorobenzonitrile (1 g, 5.00 mmol), 1-methyl-1H-pyrazol-3-amine (434 µL, 5.00 mmol), Pd₂(dba)₃ (91.6 mg, 0.100 mmol), Xantphos (145 mg, 0.250 mmol), K₂CO₃ (2.07 g, 15.0 mmol) and dioxane (31 mL) were used to obtain the title compound (0.364 g, 33.7%) as a yellow solid.
¹H NMR (400 MHz, CDCl₃) *δ* 7.28 (d, *J* = 2.0 Hz, 1H), 7.22 - 7.13 (m, 2H), 6.78 (d, *J* = 7.9 Hz, 1H), 6.12 (s, 1H), 5.91 (d, *J* = 2.2 Hz, 1H), 3.84 (s, 3H).
LC-MS, m/z 217 [M+H]⁺

### 118.2 N-(3-(aminomethyl)-5-fluorophenyl)-1-methyl-1H-pyrazol-3-amine

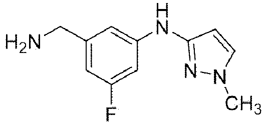

3-Fluoro-5-((1-methyl-1H-pyrazol-3-yl)amino)benzonitrile (0.36 g, 1.66 mmol) was dissolved in THF (17 mL), LAH solution (2.0 M (in THF); 1.7 mL) was added at 0 °C and the mixture was stirred at room temperature overnight. The reaction mixture was concentrated, cooled to 0 °C, quenched by adding MeOH and diluted with EtOAc. The organic layer was washed with brine, dried over Na₂SO₄ and concentrated to obtain the title compound (321 mg, 87.5%) as a yellow oil.
¹H NMR (400 MHz, DMSO-*d6*) *δ* 8.60 (s, 1H), 7.48 (d, *J =* 2.0 Hz, 1H), 7.16 (d, *J =* 12.3 Hz, 1H), 6.85 (s, 1H), 6.46 (d, *J* = 9.9 Hz, 1H), 5.75 (d, *J =* 2.2 Hz, 2H), 3.72 (s, 3H).
LC-MS, m/z 221 [M+H]⁺

### 118.3 8-Cyclopentyl-N-(3-fluoro-5-((1-methyl-1H-pyrazol-3-yl)amino)benzyl)-7H-purine-6-carboxamide

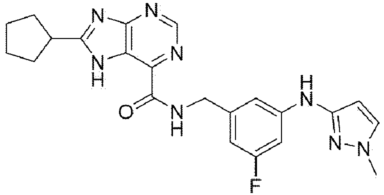

By the same method as in Example 87.3, using N-(3-(aminomethyl)-5-fluorophenyl)-1-methyl-1H-pyrazol-3-amine (0.306 g, 1.39 mmol), 8-cyclopentyl-7H-purine-6-carboxylic acid (0.323 g, 1.39 mmol), HOBt (0.320 g, 1.67 mmol), EDC (0.320 g, 1.67 mmol) and THF (32 mL), the title compound (0.155 g, 25.6%) was obtained as a yellow solid.
¹H NMR (400 MHz, DMSO-*d6*) *δ* 13.15 (bs, 1H), 9.67 (t, J = 5.0 Hz, 1H), 8.94 (s, 1H), 8.67 (s, 1H), 7.45 (d, J = 2.0 Hz, 1H), 7.21 (d, J = 12.3 Hz, 1H), 6.92 (s, 1H), 6.48 (d, J = 8.9 Hz, 1H), 5.70 (d, J = 2.2 Hz, 1H), 4.46 (d, J = 6.1 Hz, 2H), 3.64 (s, 3H), 2.05 (s, 2H), 1.93 (d, J = 7.9 Hz, 2H), 1.79 (s, 2H), 1.68 - 1.62 (m, 2H).
LC-MS, m/z 435 [M+H]⁺

### Example 119

### 119.1 tert-Butyl 3-fluoro-5-((trimethylsilyl)ethynyl)benzylcarbamate

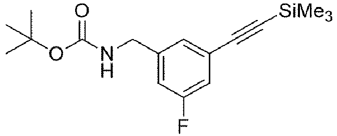

A mixture of tert-butyl 3-bromo-5-fluorobenzylcarbamate (100 mg, 0.33 mmol), TMSA (52 mg, 0.53 mmol), CuI (3 mg, 0.016 mmol), Pd(PPh₃)₄ (19 mg, 0.016 mmol and Et₃N (3 mL) was stirred at 110 °C overnight. The reaction mixture was diluted with H₂O, extracted with DCM, and the organic layer was dried over MgSO₄. The concentrated residue was purified by silica gel column chromatography (0-20% EtOAc in hexane) to obtain the title compound (42.8 mg, 40.5%) as a yellow oil.

¹H NMR (400 MHz, CDCl₃) *δ* 7.16 (s, 1H), 7.05 (d, J = 8.4 Hz, 2H), 6.96 (d, J = 9.0 Hz, 2H), 4.86 (broad, 1H), 4.28 (d, J = 5.4 Hz, 2H), 1.46 (s, 9H), 0.24 (s, 9H).

### 119.2 tert-Butyl 3-ethynyl-5-fluorobenzylcarbamate

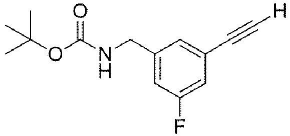

tert-Butyl 3-fluoro-5-((trimethylsilyl)ethynyl)benzylcarbamate (40 mg, 0.12 mmol) was dissolved in THF (2 mL) and then TBAF (1 M in THF, 0.19 mL, 0.19 mmol) was added, and stirred at room temperature for 30 minutes. The reaction mixture was concentrated, and the resulting residue was purified by silica gel column chromatography (hexane) to obtain the title compound (31 mg, 99%).

### 119.3 tert-Butyl 3-fluoro-5-(1-(4-fluorophenyl)-1H-1,2,3-triazol-4-yl)benzylcarbamate

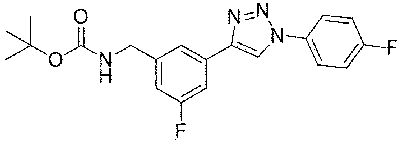

A mixture of tert-butyl 3-ethynyl-5-fluorobenzylcarbamate (31 mg, 0.12 mmol), 1-azido-4-fluorobenzene (17 mg, 0.12 mmol), a 10% aqueous solution of CuSO₄·5H₂O (192 µL, 0.074 mmol), sodium ascorbate (14.8 mg, 0.074 mmol) and t-BuOH (2 mL)/H₂O (2 mL) was stirred at room temperature overnight. The reaction mixture was diluted with H₂O, extracted with EtOAc, and the organic layer was dried over MgSO₄. The concentrated resulting residue was purified by silica gel column chromatography (0-2% MeOH in DCM) to obtain the title compound (15 mg, 31.2%).
LC-MS, m/z 387 [M+H]⁺

### 119.4 (3-Fluoro-5-(1-(4-fluorophenyl)-1H-1,2,3-triazol-4-yl)phenyl)methanamine trifluoroacetate

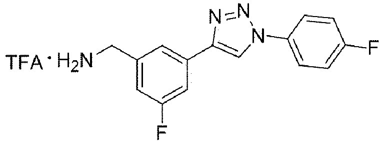

By the same method as in Example 25.4, using tert-butyl 3-fluoro-5-(1-(4-fluorophenyl)-1H-1,2,3-triazol-4-yl)benzylcarbamate (14 mg, 0.036 mmol), TFA (0.6 mL) and DCM (2 mL), the title compound (14.4 mg, crude) was obtained.
LC-MS, m/z 287 [M+H]⁺

### 119.5 8-Cyclopentyl-N-(3-fluoro-5-(1-(4-fluorophenyl)-1H-1,2,3-triazol-4-yl)benzyl)-7H-purine-6-carboxamide

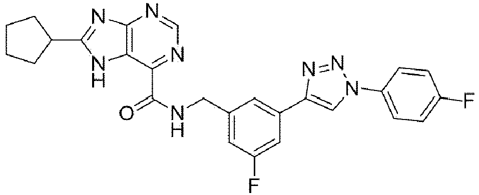

By the same method as in Example 87.3, (3-fluoro-5-(1-(4-fluorophenyl)-1H-1,2,3-triazol-4-yl)phenyl)methanamine trifluoroacetate (14.4 mg, 0.036 mmol), 8-cyclopentyl-7H-purine-6-carboxylic acid (8.4 mg, 0.036 mmol), EDC (8.3 mg, 0.043 mmol), HOBt (6 mg, 0.043 mmol), DIPEA (18 µL, 0.11 mmol) and THF (2 mL) were used to obtain the title compound (14.27 mg, 79.2%) as a yellow solid.
¹H NMR (400 MHz, CD₃OD) *δ* 8.96 (s, 1H), 8.92 (s, 1H), 7.91 (dd, J = 8.9, 4.7 Hz, 2H), 7.80 (s, 1H), 7.56 (d, J = 9.7 Hz, 1H), 7.34 (t, J = 8.6 Hz, 2H), 7.18 (d, J = 9.2 Hz, 1H), 4.75 (s, 2H), 3.55 - 3.42 (m, 1H), 2.27 - 2.12 (m, 2H), 2.10 - 1.98 (m, 2H), 1.96 - 1.84 (m, 2H), 1.84 - 1.70 (m, 2H).
LC-MS, m/z 501 [M+H]⁺

### Example 120

### 120.1 tert-Butyl (3-((1H-pyrazol-3-yl)amino)-5-fluorobenzyl)carbamate

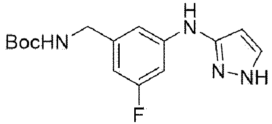

By the same method as in Example 10.1, using tert-butyl (3-bromo-5-fluorobenzyl)carbamate (50 mg, 0.164 mmol), tert-butyl 3-amino-1H-pyrazole-1-carboxylate (30 mg, 0.164 mmol), Pd₂(dba)₃ (2.9 mg, 0.0032 mmol), Xantphos (5.02 mg, 0.0084 mmol), K₂CO₃ (53 mg, 0.38 mmol) and dioxane (1.05 mL), the title compound (10.5 mg, 21%) was obtained as an off-white solid.
¹HNMR (400 MHz, CDCl₃) *δ* 7.48 (d, *J =* 2.3 Hz, 1H), 6.88 (d, *J =* 11.2 Hz, 1H), 6.78 (s, 1H), 6.47 (d, *J =* 8.8 Hz, 1H), 6.30 (s, 1H), 6.06 (d, *J =* 2.4 Hz, 1H), 4.87 (s, 1H), 4.24 (d, *J* = 6.0 Hz, 2H), 1.46 (s, 9H).
LC-MS, m/z 307 [M+H]⁺

### 120.2 N-(3-(aminomethyl)-5-fluorophenyl)-1H-pyrazol-3-amine trifluoroacetate

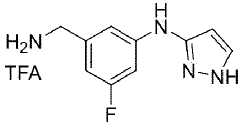

By the same method as in Example 25.4, using tert-butyl (3-((1H-pyrazol-3-yl)amino)-5-fluorobenzyl)carbamate (10 mg, 0.033 mmol), trifluoroacetic acid (0.06 mL), and DCM (0.33 mL), the title compound (17 mg, crude) was obtained as a yellow solid.
LC-MS, m/z 207 [M+H]⁺

### 120.3 N-(3-((1H-pyrazol-3-yl)amino)-5-fluorobenzyl)-8-cyclopentyl-7H-purine-6-carboxamide

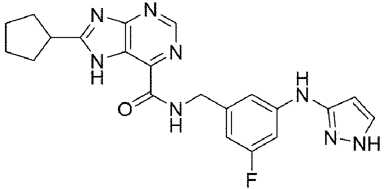

By the same method as in Example 1.3, 8-cyclopentyl-7H-purine-6-carboxylic acid (7.95 mg, 0.034 mmol), N-(3-(aminomethyl)-5-fluorophenyl)-1H-pyrazol-3-amine trifluoroacetate (17 mg, 0.033 mmol), T3P (propylphosphonic anhydride, 50 wt.% solution (in DMF), 16 mg) and DMF (0.65 mL) were used to obtain the title compound (5 mg, 36%) as a yellow solid.
¹H NMR (400 MHz, DMSO-d₆) *δ* 13.18 (s, 1H), 12.03 (s, 1H), 9.70 (s, 1H), 8.96 (s, 1H), 8.69 (s, 1H), 7.55 (s, 1H), 7.26 (d*, J =* 12.3 Hz, 1H), 6.93 (s, 1H), 6.49 (d, *J =* 9.6 Hz, 1H), 5.79 (s, 1H), 4.47 (d, *J =* 6.3 Hz, 2H), 3.55 - 3.42 (m, 1H), 2.14 - 2.01 (m, 2H), 1.98 - 1.86 (m, 2H), 1.86 - 1.73 (m, 2H), 1.73 - 1.59 (m, 2H).
LC-MS, m/z 421 [M+H]⁺

### Example 121

### 121.1 3-Fluoro-5-(isoxazol-3-ylamino)benzonitrile

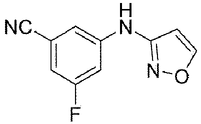

3-Fluoro-5-(4,4,5,5-tetramethyl-1,3,2-dioxaborolan-2-yl)benzonitrile (30 mg, 0.121 mmol), isoxazol-3-amine (20 mg, 0.243 mmol), Cu(OAc)₂ (22 mg, 0.121 mmol) and TEA (34 µL, 0.243 mmol) were dissolved in ACN/EtOH (115 µL/6 µL) and stirred at 80 °C for 1 h. The reaction mixture was filtered through a pad of Celite and washed several times with EtOAc. The filtrate was concentrated, and the obtained residue was purified by silica gel column chromatography (0-25% EtOAc in hexane) to obtain the title compound (11 mg, 41.8%) as a white solid.
¹H NMR (400 MHz, DMSO-*d6*) *δ* 9.92 (s, 1H), 8.70 (d, *J =* 1.7 Hz, 1H), 7.60 (d, *J*= 1.7 Hz, 1H), 7.57 (s, 1H), 7.31 (d, *J =* 7.8 Hz, 1H), 6.29 (d, *J =* 1.7 Hz, 1H).
LC-MS, m/z 202 [M-H]⁻

### 121.2 N-(3-(aminomethyl)-5-fluorophenyl)isoxazol-3-amine

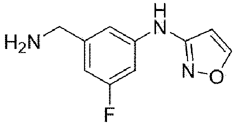

3-Fluoro-5-(isoxazol-3-ylamino)benzonitrile (11 mg, 0.054 mmol) was dissolved in THF (0.53 mL), LAH solution (2.0 M (in THF); 53 µL) was added at 0 °C, and the mixture was stirred at room temperature overnight. The reaction mixture was cooled to 0 °C again, quenched by adding H₂O, and stirred for 30 min. The resulting solid was filtered and dried in vacuo to obtain the title compound (9.6 mg, 87.2%) as a yellow solid.
LC-MS, m/z 206 [M-H]⁻

### 121.3 8-Cyclopentyl-N-(3-fluoro-5-(isoxazol-3-ylamino)benzyl)-7H-purine-6-carboxamide]

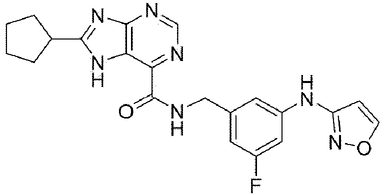

By the same method as in Example 1.3, using N-(3-(aminomethyl)-5-fluorophenyl)isoxazol-3-amine (9.6 mg, 0.046 mmol), 8-cyclopentyl-7H-purine-6-carboxylic acid (10.8 mg, 0.046 mmol), T3P (50 wt.% solution in DMF, 81 µL, 0.139 mmol), DIPEA (32 µL, 0.185 mmol) and DMF (0.28 mL), the title compound (3.8 mg, 18.4%) was obtained as a yellow solid.
¹H NMR (400 MHz, CD₃OD) *δ* 8.96 (s, 1H), 8.32 (d, J = 1.7 Hz, 1H), 7.23 (d, J = 11.1 Hz, 1H), 7.16 (s, 1H), 6.68 (d, J = 9.2 Hz, 1H), 6.10 (d, J = 1.7 Hz, 1H), 4.65 (s, 2H), 3.53 - 3.45 (m, 1H), 2.20 (d, J = 4.9 Hz, 2H), 2.05 - 2.00 (m, 2H), 1.91 (d, J = 8.5 Hz, 2H), 1.77 (dd, J = 6.9, 4.5 Hz, 2H).
LC-MS, m/z 422 [M+H]⁺

### Example 122

### 122.1 tert-Butyl (3-((4-cyanophenyl)amino)-5-fluorobenzyl)carbamate

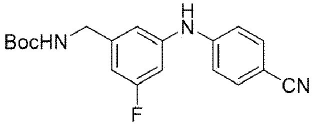

By the same method as in Example 10.1, tert-butyl 3-bromo-5-fluorobenzylcarbamate (0.5 g, 1.64 mmol), 4-aminobenzonitrile (0.194 g, 1.64 mmol), Pd₂(dba)₃ (30.1 mg, 0.033 mmol), Xantphos (47.6 mg, 0.082 mmol), K₂CO₃ (0.523 g, 3.78 mmol) and dioxane (10.3 mL) were used to obtain the title compound (0.364 g, 33.7%) as a yellow solid.

¹H NMR (400 MHz, DMSO-*d6*) *δ* 9.07 (s, 1H), 7.60 (d, *J =* 8.7 Hz, 2H), 7.42 (t, *J =* 6.2 Hz, 1H), 7.12 (d, *J =* 8.8 Hz, 2H), 6.87 (s, 1H), 6.80 (d, *J=* 10.9 Hz, 1H), 6.63 (d, *J* = 9.7 Hz, 1H), 4.08 (d, *J =* 6.1 Hz, 2H), 1.37 (s, 9H).

### 122.2 4-((3-(Aminomethyl)-5-fluorophenyl)amino)benzonitrile trifluoroacetate

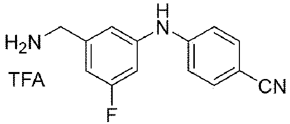

By the same method as in Example 25.4, tert-butyl (3-((4-cyanophenyl)amino)-5-fluorobenzyl)carbamate (0.154 g, 0.451 mmol), TFA (1.5 mL) and DCM (4.5 mL) were used to obtain the title compound (0.123 mg, 78.4%, crude) as a yellow oil.

¹H NMR (400 MHz, DMSO-*d6*) *δ* 9.16 (s, 1H), 8.02 (bs, 2H), 7.64 (d, *J* = 8.8 Hz, 2H), 7.18 (d, *J =* 8.8 Hz, 2H), 7.12 (s, 1H), 6.91 (d, *J =* 10.4 Hz, 2H), 4.00 (s, 2H).

### 122.3 N-(3-((4-cyanophenyl)amino)-5-fluorobenzyl)-8-cyclopentyl-7H-purine-6-carboxamide

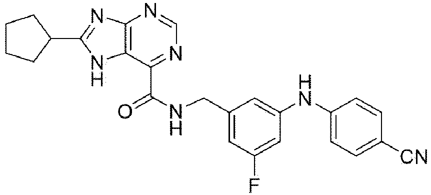

By the same method as in Example 87.3, 8-cyclopentyl-7H-purine-6-carboxylic acid (82 mg, 0.354 mmol), 4-((3-(aminomethyl)-5-fluorophenyl)amino)benzonitrile trifluoroacetate (0.120 g, 0.354 mmol), HOBt (57.3 mg, 0.424 mmol), EDC (81.4 mg, 0.424 mmol), DIPEA (180 µL, 1.06 mmol) and THF (8.2 mL) were used to obtain the title compound (0.128 g, 79.5%) as a yellow solid.
¹H NMR (400 MHz, DMSO-*d6*) *δ* 13.16 (s, 1H), 9.76 (s, 1H), 9.06 (s, 1H), 8.94 (s, 1H), 7.56 (d, J = 8.6 Hz, 2H), 7.10 (d, J = 8.8 Hz, 2H), 6.98 (s, 1H), 6.86 - 6.76 (m, 2H), 4.51 (d, J = 6.4 Hz, 2H), 3.47 (s, 1H), 2.05 (s, 2H), 1.91 (s, 2H), 1.79 (s, 2H), 1.64 (s, 2H).
LC-MS, m/z 456 [M+H]⁺

### Example 123

### 123.1 tert-Butyl 3-(1-(4-acetylphenyl)-1H-pyrazol-4-yl)-5-fluorobenzylcarbamate

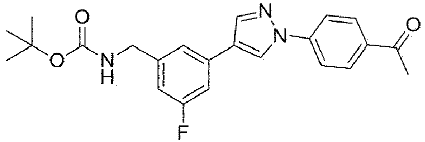

By the same method as in Example 25.3, tert-butyl 3-fluoro-5-(1H-pyrazol-4-yl)benzylcarbamate (30 mg, 0.103 mmol), 1-(4-iodophenyl)ethanone (38 mg, 0.15 mmol), CuI (2 mg, 0.010 mmol), L-proline (2.4 mg, 0.021 mmol), K₂CO₃ (21.3 mg, 0.15 mmol) and DMSO (1 mL) were used to obtain the title compound (30.89 mg, 73%) as an off-white solid.
LC-MS, m/z 410 [M+H]⁺

### 123.2 1-(4-(4-(3-(aminomethyl)-5-fluorophenyl)-1H-pyrazol-1-yl)phenyl)ethanone trifluoroacetate

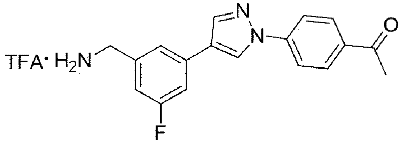

By the same method as in Example 25.4, using tert-butyl 3-(1-(4-acetylphenyl)-1H-pyrazol-4-yl)-5-fluorobenzylcarbamate (27.19 mg, 0.066 mmol), TFA (0.3 mL) and DCM (1 mL), the title compound (28 mg, crude) was obtained.
LC-MS, m/z 310 [M+H]⁺

### 123.3 N-(3-(1-(4-acetylphenyl)-1H-pyrazol-4-yl)-5-fluorobenzyl)-8-cyclopentyl-7H-purine-6-carboxamide

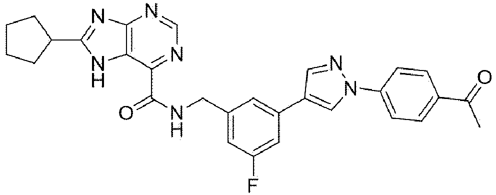

By the same method as in Example 87.3, using 1-(4-(4-(3-(aminomethyl)-5-fluorophenyl)-1H-pyrazol-1-yl)phenyl)ethanone, trifluoroacetate salt (28 mg, 0.066 mmol), 8-cyclopentyl-7H-purine-6-carboxylic acid (15.3 mg, 0.066 mmol), EDC (15.2 mg, 0.079 mmol), HOBt (10.7 mg, 0.079 mmol), DIPEA (34 µL, 0.20 mmol) and THF (2 mL), the title compound (24.7 mg, 71.5%) was obtained as a yellow solid.
¹H NMR (400 MHz, DMSO-*d*₆) *δ* 13.18 (s, 1H), 9.79 (t, J = 6.4 Hz, 1H), 9.22 (s, 1H), 8.97 (s, 1H), 8.34 (s, 1H), 8.12 (d, J = 8.7 Hz, 2H), 8.03 (d, J = 8.5 Hz, 2H), 7.62 (s, 1H), 7.52 (d, J = 9.8 Hz, 1H), 7.09 (d, J = 8.7 Hz, 1H), 4.61 (d, J = 6.2 Hz, 2H), 3.57 - 3.43 (m, 1H), 2.61 (s, 3H), 2.14 - 2.01 (m, 2H), 2.00 - 1.86 (m, 2H), 1.86 - 1.72 (m, 2H), 1.72 - 1.57 (m, 2H).
LC-MS, m/z 524 [M+H]⁺

### Example 124

### 124.1 tert-Butyl 3-fluoro-5-(1-(furan-3-yl)-1H-pyrazol-4-yl)benzylcarbamate

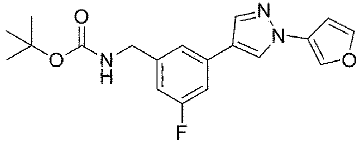

By the same method as in Example 25.3, tert-butyl 3-fluoro-5-(1H-pyrazol-4-yl)benzylcarbamate (100 mg, 0.34 mmol), 3-bromofuran (75.5 mg, 0.51 mmol), CuI (6.5 mg, 0.034 mmol), L-proline (8 mg, 0.069 mmol), K₂CO₃ (70 mg, 0.51 mmol) and DMSO (3 mL) were used to obtain the title compound (32.29 mg, 26.3%) as a colorless clear oil.
LC-MS, m/z 358 [M+H]⁺

### 124.2 (3-Fluoro-5-(1-(furan-3-yl)-1H-pyrazol-4-yl)phenyl)methanamine trifluoroacetate

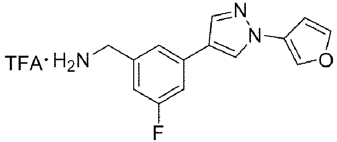

By the same method as in Example 25.4, using tert-butyl 3-fluoro-5-(1-(furan-3-yl)-1H-pyrazol-4-yl)benzylcarbamate (32.29 mg, 0.066 mmol), TFA (0.3 mL) and DCM (1 mL), the title compound (33 mg, crude) was obtained.
LC-MS, m/z 258 [M+H]⁺

### 124.3 8-Cyclopentyl-N-(3-fluoro-5-(1-(furan-3-yl)-1H-pyrazol-4-yl)benzyl)-7H-purine-6-carboxamide

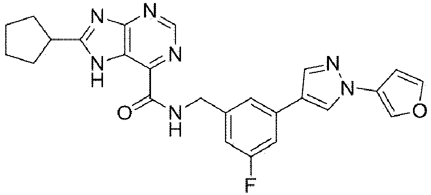

By the same method as in Example 87.3, (3-fluoro-5-(1-(furan-3-yl)-1H-pyrazol-4-yl)phenyl)methanamine, trifluoroacetate salt (33 mg, 0.09 mmol), 8-cyclopentyl-7H-purine-6-carboxylic acid (20.9 mg, 0.09 mmol), EDC (20.7 mg, 0.11 mmol), HOBt (14.6 mg, 0.11 mmol), DIPEA (46 µL, 0.27 mmol) and THF (2 mL) were used to obtain the title compound (27.15 mg, 63.98%) as an off-white solid.
¹H NMR (400 MHz, DMSO-*d*₆) *δ* 13.17 (s, 1H), 9.77 (s, 1H), 8.97 (s, 1H), 8.77 (s, 1H), 8.17 (s, 2H), 7.79 (s, 1H), 7.54 (s, 1H), 7.42 (d, J = 10.3 Hz, 1H), 7.11 - 6.99 (m, 2H), 4.59 (d, J = 6.3 Hz, 2H), 3.59 - 3.42 (m, 1H), 2.16 - 2.01 (m, 2H), 1.98 - 1.85 (m, 2H), 1.86 - 1.74 (m, 2H), 1.72 - 1.58 (m, 2H).
LC-MS, m/z 472 [M+H]⁺

### Example 125

### 125.1 tert-Butyl 3-(1'-benzyl-1'H-[1,4'-bipyrazol]-4-yl)-5-fluorobenzylcarbamate

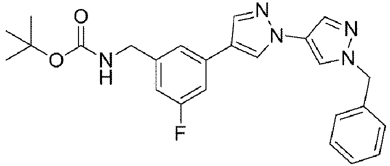

By the same method as in Example 25.3, using tert-butyl 3-fluoro-5-(1H-pyrazol-4-yl)benzylcarbamate (100 mg, 0.34 mmol), 1-benzyl-4-bromo-1H-pyrazole (122 mg, 0.52 mmol), CuI (6.5 mg, 0.034 mmol), L-proline (8 mg, 0.069 mmol), K₂CO₃ (71.2 mg, 0.51 mmol) and DMSO (3 mL), the title compound (25.92 mg, 16.9%) was obtained as a pale yellow solid.
LC-MS, m/z 448 [M+H]⁺

### 125.2 (3-(1'-Benzyl-1'H-[1,4'-bipyrazol]-4-yl)-5-fluorophenyl)methanamine trifluoroacetate

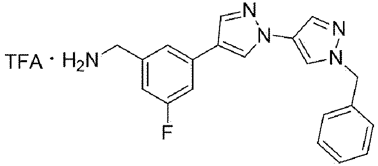

By the same method as in Example 25.4, using tert-butyl 3-(1'-benzyl-1'H-[1,4'-bipyrazol]-4-yl)-5-fluorobenzylcarbamate (24 mg, 0.054 mmol), TFA (0.3 mL) and DCM (1 mL), the title compound (25 mg, crude) was obtained.
LC-MS, m/z 348 [M+H]⁺

### 125.3 N-(3-(1'-benzyl-1'H-[1,4'-bipyrazol]-4-yl)-5-fluorobenzyl)-8-cyclopentyl-7H-purine-6-carboxamide

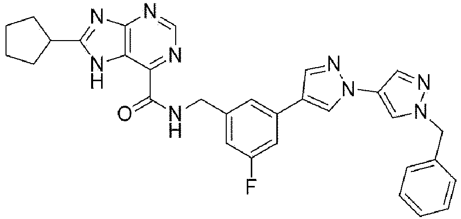

By the same method as in Example 87.3, (3-(1'-benzyl-1'H-[1,4'-bipyrazol]-4-yl)-5-fluorophenyl)methanamine trifluoroacetate (25 mg, 0.054 mmol), 8-cyclopentyl-7H-purine-6-carboxylic acid (12.54 mg, 0.054 mmol), EDC (12.4 mg, 0.065 mmol), HOBt (9 mg, 0.065 mmol), DIPEA (28 µL, 0.16 mmol) and THF (2 mL) were used to obtain the title compound (18.08 mg, 59.6%) as an off-white solid.
¹H NMR (400 MHz, CD₃OD) *δ* 8.95 (s, 1H), 8.39 (s, 1H), 8.08 (s, 1H), 8.00 (s, 1H), 7.87 (s, 1H), 7.49 (s, 1H), 7.41 - 7.23 (m, 6H), 7.03 (d, J = 9.6 Hz, 1H), 5.36 (s, 2H), 4.69 (s, 2H), 3.55 - 3.41 (m, 1H), 2.27 - 2.13 (m, 2H), 2.08 - 1.97 (m, 2H), 1.97 - 1.83 (m, 2H), 1.83 - 1.68 (m, 2H).
LC-MS, m/z 562 [M+H]⁺

### Example 126

### N-(3-((4-carbamoylphenyl)amino)-5-fluorobenzyl)-8-cyclopentyl-7H-purine-6-carboxamide

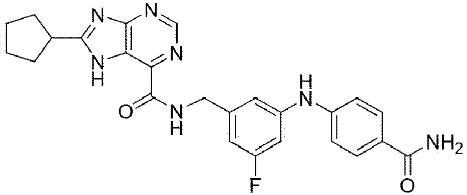

N-(3-((4-cyanophenyl)amino)-5-fluorobenzyl)-8-cyclopentyl-7H-purine-6-carboxamide (50 mg, 0.110 mmol) and K₂CO₃ (30.3 mg, 0.220 mmol) were dissolved in DMSO (0.46 mL) at 0 °C, and H₂O₂ (35 % (in H-₂O), 0.16 mL) was added. After stirring at room temperature for 2 hours, H₂O was added to the reaction mixture and stirred for 1 more hour at room temperature. The resulting solid was filtered to obtain the title compound (40 mg, 77.0%) as a white solid.
¹H NMR (400 MHz, DMSO-*d6*) *δ* 9.76 (s, 2H), 8.92 (s, 1H), 8.76 - 8.69 (m, 1H), 7.74 - 7.70 (m, 2H), 7.05 (d, *J =* 8.7 Hz, 2H), 6.94 (s, 1H), 6.76 (d, *J* = 11.0 Hz, 1H), 6.68 (d, *J =* 8.4 Hz, 1H), 4.49 (d, *J =* 6.2 Hz, 2H), 3.46 (s, 1H), 2.04 (s, 2H), 1.91 (dd, *J =* 11.1, 7.1 Hz, 2H), 1.78 (s, 2H), 1.67 - 1.60 (m, 2H).
LC-MS, m/z 474 [M+H]⁺

### Example 127

### 127.1 Ethyl 4-((3-(((tert-butoxycarbonyl)amino)methyl)-5-fluorophenyl)amino)benzoate

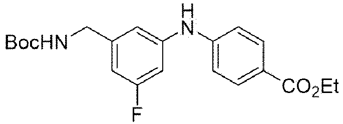

By the same method as in Example 10.1, tert-butyl 3-bromo-5-fluorobenzylcarbamate (0.5 g, 1.64 mmol), ethyl 4-aminobenzoate (0.271 g, 1.64 mmol), Pd₂(dba)₃ (30.1 mg, 0.033 mmol), Xantphos (47.6 mg, 0.082 mmol), K₂CO₃ (0.523 g, 3.78 mmol) and dioxane (10.3 mL) were used to obtain the title compound (0.212 g, 32.3%) as a yellow solid.

¹H NMR (400 MHz, CD₃OD) δ7.87 (d, *J =* 8.8 Hz, 2H), 7.09 (d, *J =* 8.8 Hz, 2H), 6.92 (s, 1H), 6.74 (d, *J =* 10.5 Hz, 1H), 6.58 (d, *J =* 9.2 Hz, 1H), 4.30 (q, *J =* 7.1 Hz, 2H), 4.18 (s, 2H), 1.44 (s, 9H), 1.36 (d, *J=* 7.1 Hz, 3H).

### 127.2 Ethyl 4-((3-(aminomethyl)-5-fluorophenyl)amino)benzoate trifluoroacetate

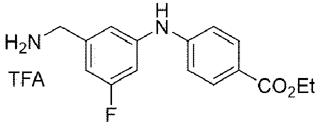

By the same method as in Example 25.4, tert-butyl 3-(1'-benzyl-1'H-[1,4'-bipyrazol]-4-yl)-5-fluorobenzylcarbamate (0.2 g), 0.451 mmol), TFA (1.7 mL) and DCM (5.1 mL) were used to obtain the title compound (0.138 g, 69.4%).

### 127.3 Ethyl 4-((3-((8-cyclopentyl-7H-purine-6-carboxamido)methyl)-5-fluorophenyl)amino)benzoate

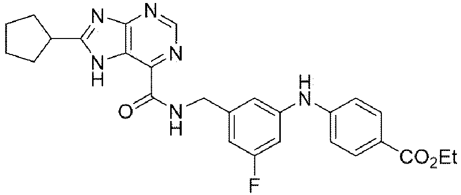

By the same method as in Example 87.3, 8-cyclopentyl-7H-purine-6-carboxylic acid (82.4 mg, 0.355 mmol), ethyl 4-((3-(aminomethyl)-5-fluorophenyl)amino)benzoate trifluoroacetate (0.137 g, 0.355 mmol), HOBt (57.5 mg, 0.426 mmol), EDC (81.5 mg, 0.426 mmol), DIPEA (181 µL, 1.06 mmol) and THF (8.2 mL) were used to obtain the title compound (86.2 mg, 48.4%) as a yellow solid.
¹H NMR (400 MHz, CD₃OD) *δ* 8.95 (s, 1H), 7.81 (d, *J =* 8.8 Hz, 2H), 7.10 - 7.03 (m, 3H), 6.78 (d, *J =* 10.9 Hz, 1H), 6.73 (d, *J =* 9.5 Hz, 1H), 4.63 (s, 2H), 4.30 (q, *J =* 7.1 Hz, 2H), 3.53 - 3.43 (m, 1H), 2.20 (d, *J =* 7.9 Hz, 2H), 2.06 - 1.99 (m, 2H), 1.94 - 1.88 (m, 2H), 1.80 - 1.72 (m, 2H), 1.35 (t, *J =* 7.1 Hz, 3H).
LC-MS, m/z 503 [M+H]⁺

### Example 128

### 128.1 tert-Butyl 3-fluoro-5-(1'-methyl-1'H-[1,4'-bipyrazol]-4-yl)benzylcarbamate

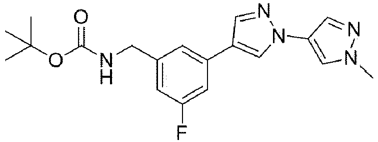

By the same method as in Example 25.3, tert-butyl 3-fluoro-5-(1H-pyrazol-4-yl)benzylcarbamate (100 mg, 0.34 mmol), 4-iodo-1-methyl-1H-pyrazole (107 mg, 0.51 mmol), CuI (6.5 mg, 0.034 mmol), L-proline (8 mg, 0.069 mmol), K₂CO₃ (71.2 mg, 0.51 mmol) and DMSO (3 mL) were used to obtain the title compound (87.14 mg, 68%) as a colorless clear oil.
LC-MS, m/z 372 [M+H]⁺

### 128.2 (3-Fluoro-5-(1'-methyl-1'H-[1,4'-bipyrazol]-4-yl)phenyl)methanamine trifluoroacetate

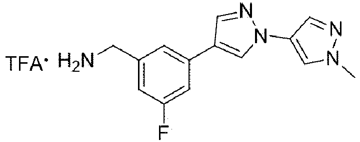

By the same method as in Example 25.4, using tert-butyl 3-fluoro-5-(1'-methyl-1'H-[1,4'-bipyrazol]-4-yl)benzylcarbamate (40 mg, 0.22 mmol), TFA (0.35 mL) and DCM (1 mL), the title compound (41 mg, crude) was obtained.
LC-MS, m/z 272 [M+H]⁺

### 128.3 8-Cyclopentyl-N-(3-fluoro-5-(1'-methyl-1'H-[1,4'-bipyrazol]-4-yl)benzyl)-7H-purine-6-carboxamide

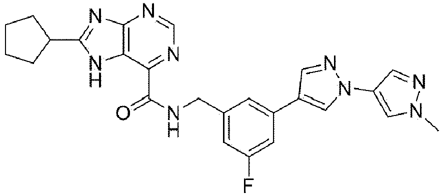

By the same method as in Example 87.3, (3-fluoro-5-(1'-methyl-1'H-[1,4'-bipyrazol]-4-yl)phenyl)methanamine trifluoroacetate (41 mg, 0.11 mmol), 8-cyclopentyl-7H-purine-6-carboxylic acid (25 mg, 0.11 mmol), EDC (25 mg, 0.13 mmol), HOBt (17.5 mg, 0.13 mmol), DIPEA (55.3 µL, 0.32 mmol) and THF (2 mL) were used to obtain the title compound (15.15 mg, 29%) as an off-white solid.
¹H NMR (400 MHz, DMSO-*d*₆) *δ* 13.17 (s, 1H), 9.77 (s, 1H), 8.96 (s, 1H), 8.68 (s, 1H), 8.13 (d, J = 12.4 Hz, 2H), 7.82 (s, 1H), 7.52 (s, 1H), 7.40 (d, J = 10.2 Hz, 1H), 7.04 (d, J = 9.5 Hz, 1H), 4.59 (d, J = 5.5 Hz, 2H), 3.88 (s, 3H), 3.54 - 3.43 (m, 1H), 2.14 - 1.02 (m, 2H), 2.00 - 1.86 (m, 2H), 1.86 - 1.73 (m, 2H), 1.71 - 1.62 (m, 2H).
LC-MS, m/z 486 [M+H]⁺

### Example 129

### 129.1 3-Fluoro-5-((1-methylpiperidin-4-yl)amino)benzonitrile

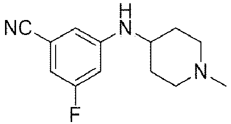

By the same method as in Example 10.1, 3-bromo-5-fluorobenzonitrile (100 mg, 0.50 mmol), 1-methylpiperidin-4-amine (68 mg, 0.60 mmol), BINAP (10 mg, 0.016 mmol), Pd₂(dba)₃ (8.0 mg, 0.009 mmol), sodium tert-butoxide (58 mg, 0.60 mmol and toluene (1 mL) were used and stirred at 120°C to obtain the title compound (39 mg, 33%) as a white solid.
¹H NMR (400 MHz, CD₃OD) *δ* 6.70 (s, 1H), 6.61 - 6.57 (m, 2H), 2.86 (d, *J =* 8 Hz, 2H), 2.30 (s, 3H), 2.26 - 2.20 (m, 2H), 2.01 (d, *J =* 8 Hz, 2H), 1.52 - 1.49 (m, 2H).
LC-MS, m/z 234 [M+H]⁺

### 129.2 N-(3-(aminomethyl)-5-fluorophenyl)-1-methylpiperidin-4-amine

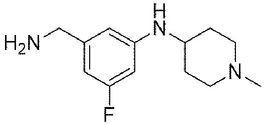

By the same method as in Example 1.2, 3-fluoro-5-((1-methylpiperidin-4-yl)amino)benzonitrile (35 mg, 0.15 mmol) and LAH solution (2.0 M in THF) ); 0.15 mL) were used to obtain the title compound (30 mg, 84%) as a yellow syrup.
LC-MS, m/z 238 [M+H]⁺

### 129.3 8-Cyclopentyl-N-(3-fluoro-5-((1-methylpiperidin-4-yl)amino)benzyl)-7H-purine-6-carboxamide

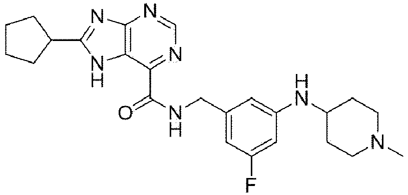

By the same method as in Example 87.3, 8-cyclopentyl-7H-purine-6-carboxylic acid (26 mg, 0.11 mmol), N-(3-(aminomethyl)-5-fluorophenyl)-1-methylpiperidin-4-amine (30 mg, 0.13 mmol), HOBt (23 mg, 0.17 mmol), EDC (26 mg, 0.14 mmol), DIPEA (52 µL, 0.30 mmol) and THF (1 mL) were used to obtain the title compound (14 mg, 27%) as a white solid.
¹H NMR (400 MHz, CD₃OD) *δ* 8.94 (s, 1H), 6.44 (s, 1H), 6.34 (d, J = 12 Hz, 1H), 6.24 (d, J = 12 Hz, 1H), 4.54 (s, 2H), 3.50 - 3.45 (m, 1H), 2.98 - 2.95 (m, 2H), 2.44 - 2.35 (m, 6H), 2.18 - 2.14 (m, 2H), 2.04 - 2.01 (m, 4H), 1.97 - 1.91 (m, 2H), 1.78 - 1.75 (m, 2H), 1.56 - 1.50 (m, 2H).
LC-MS, m/z 452 [M+H]⁺

### Example 130

### 130.1 3-Fluoro-5-(thiophen-3-ylamino)benzonitrile

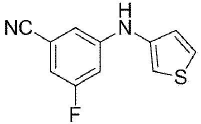

By the same method as in Example 10.1, 3-bromo-5-fluorobenzonitrile (100 mg, 0.50 mmol), thiophene-3-amine oxalate (113 mg, 0.60 mm), Cs₂CO₃ (489 mg, 1.5 mmol), BINAP (10 mg, 0.016 mmol), Pd₂(dba)₃ (8.0 mg, 0.009 mmol) and dioxane (2 mL) were used and the reaction was conducted at 100°C to obtain the title compound (70 mg, 64%) as a yellow solid.
¹H NMR (400 MHz, CD₃OD) δ 7.41 - 7.39 (t, *J* = 4 Hz, 1H), 6.99 (s, 1H), 6.95 (s, 1H), 6.94 - 6.90 (m, 2H), 6.79 (d, *J* = 8 Hz, 1H).
LC-MS, m/z 219 [M+H]+

### 130.2 N-(3-(aminomethyl)-5-fluorophenyl)thiophen-3-amine

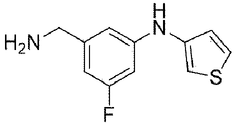

By the same method as in Example 1.2, 3-fluoro-5-((1-methylpiperidin-4-yl)amino)benzonitrile (70 mg, 0.32 mmol) and LAH solution (2.0 M (in THF); 0.32 mL) were used to obtain the title compound (50 mg, 70%) as a yellow syrup.
LC-MS, m/z 223 [M+H]+

### 130.3 8-Cyclopentyl-N-(3-fluoro-5-(thiophen-3-ylamino)benzyl)-7H-purine-6-carboxamide

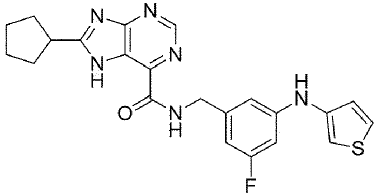

By the same method as in Example 1.3, 8-cyclopentyl-7H-purine-6-carboxylic acid (47 mg, 0.20 mmol), N-(3-(aminomethyl)-5-fluorophenyl)thiophene-3-amine (50 mg, 0.22 mmol), T3P (50 wt. % solution (in EtOAc), 393 mg) and DIPEA (138 µL) were used to obtain the title compound (24 mg, 27%) as a white solid.
¹H NMR (400 MHz, dmso-d₆) δ 13.19 (s, 1H), 9.74 - 9.72 (m, 1H), 8.97 (s, 1H), 8.58 (s, 1H), 7.44 -7.42 (m, 1H), 6.91 - 6.83 (m, 3H), 6.63 (d, *J =* 12 Hz, 1H), 6.55 (d, *J =* 8 Hz, 1H), 4.50 (d, *J* = 8 Hz, 2H), 3.53 - 3.49 (m, 1H), 2.10 - 2.05 (m, 2H), 1.96 - 1.89 (m, 2H), 1.84 - 1.78 (m, 2H), 1.70 - 1.65 (m, 2H).
LC-MS, m/z 437 [M+H]+

### Example 131

### 131.1 (3-Fluoro-5-(4-(4-fluorophenyl)piperazin-1-yl)phenyl)methanamine

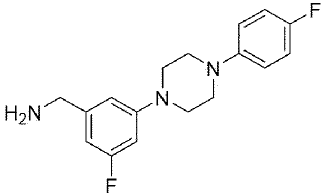

By the same method as in Example 8.1, using 1-(4-fluorophenyl)piperazine (150 mg, 0.83 mmol), tert-butyl (3-bromo-5-fluorobenzyl)carbamate (241 mg, 0.79 mmol), Pd₂(dba)₃ (16.8 mg, 0.018 mmol), BINAP (20.7 mg, 0.033 mmol), NaO^{t}Bu (160 mg, 1.66 mmol) and toluene (1.5 mL), the title compound (35 mg, 11%) was obtained as a brown viscous syrup.
¹H NMR (400 MHz, CDCl₃) *δ* 7.00 - 6.90 (m, 4H), 6.71 (s, 1H), 6.57 - 6.48 (m, 2H), 3.84 (s, 2H), 3.37 - 3.26 (m, 4H), 3.25 - 3.19 (m, 4H), 2.37 (br s, NH₂, 2H).
LC-MS, m/z 304 [M+H]⁺

### 131.2 8-Cyclopentyl-N-(3-fluoro-5-(4-(4-fluorophenyl)piperazin-1-yl)benzyl)-7H-purine-6-carboxamide

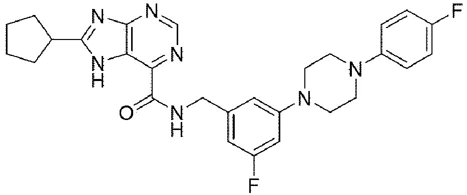

By the same method as in Example 1.3, using 8-cyclopentyl-7H-purine-6-carboxylic acid (27.3 mg, 0.12 mmol), (3-fluoro-5-(4-(4-fluorophenyl)piperazin-1-yl)phenyl)methanamine (34 mg, 0.11 mmol), T3P (propylphosphonic anhydride, 50 wt.% solution (in DMF), 23 mg), DIPEA (N,N-diisopropylethylamine, 76 µL) and DMF (2.3 mL), the title compound (5 mg, 9%) was obtained as an off-white solid.
¹H NMR (400 MHz, CDCl₃) *δ* 10.37 (s, 1H), 9.02 (s, 1H), 8.47 - 8.29 (m, 1H), 7.03 - 6.87 (m, 4H), 6.71 (s, 1H), 6.58 (d, *J =* 10.4 Hz, 2H), 4.64 (d, *J =* 6.2 Hz, 2H), 3.48 - 3.38 (m, 1H), 3.38 - 3.29 (m, 4H), 3.27 - 3.18 (m, 4H), 2.30 - 2.17 (m, 2H), 2.05 (td, *J =* 15.6, 7.8 Hz, 2H), 1.97 - 1.85 (m, 2H), 1.83 - 1.72 (m, 2H).
LC-MS, m/z 518 [M+H]⁺

### Example 132

### 132.1 tert-Butyl (3-fluoro-5-(1H-tetrazol-5-yl)benzyl)carbamate

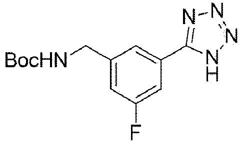

tert-Butyl (3-cyano-5-fluorobenzyl)carbamate (80 mg, 0.32 mmol), NaN₃ (42 mg, 0.64 mmol) and NH₄Cl (34 mg, 0.64 mmol) were dissolved in DMF (3 mL) and stirred at 140 °C overnight. After the reaction was completed, the mixture was diluted with a small amount of H₂O, acidified with 1 N HCl (pH 5), and extracted three times with EA. The organic layer was dried over MgSO₄ and concentrated to obtain the title compound (100 mg, crude) as a light brown solid.
LC-MS, *m*/*z* 294 [M+H]⁺

### 132.2 tert-Butyl (3-fluoro-5-(5-(4-fluorophenyl)-1,3,4-oxadiazol-2-yl)benzyl)carbamate

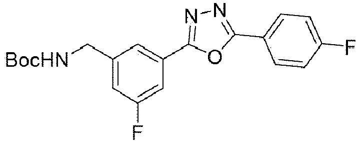

A mixture of tert-butyl (3-fluoro-5-(1H-tetrazol-5-yl)benzyl)carbamate (86 mg, 0.29 mmol), 4-fluorobenzoic acid (97 mg, 0.35 mmol), 1 M DCC (in DCM) (350 µL, 0.35 mmol) and 1,2-dichloroethane (3 mL) was stirred at 100 °C for 24 h. After the reaction was completed, the mixture was diluted with a small amount of H₂O and extracted three times with EA. The organic layer was dried over MgSO₄ and concentrated, and the obtained residue was purified by silica gel column chromatography (0-20% EtOAc in hexane) to obtain the title compound (74 mg, 65%) as a white solid.
LC-MS, *m*/*z* 388 [M+H]⁺

### 132.3 (3-Fluoro-5-(5-(4-fluorophenyl)-1,3,4-oxadiazol-2-yl)phenyl)methanamine trifluoroacetate

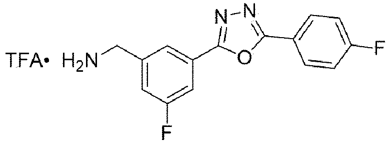

By the same method as in Example 25.4, tert-butyl (3-fluoro-5-(5-(4- fluorophenyl)-1,3,4-oxadiazol-2-yl)benzyl)carbamate (74 mg, 0.19 mmol), trifluoroacetic acid (1 mL) and DCM (2 ml) were used to obtain the title compound (98 mg, crude) as a light brown oil.
LC-MS, *m*/*z* 288 [M+H]⁺

### 132.4 8-Cyclopentyl-N-(3-fluoro-5-(5-(4-fluorophenyl)-1,3,4-oxadiazol-2-yl)benzyl)-7H-purine-6-carboxamide

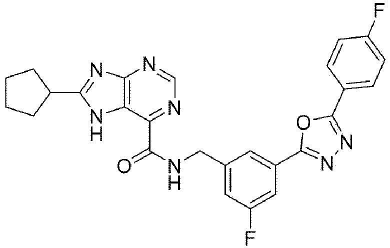

By the same method as in Example 87.3, using (3-fluoro-5-(5-(4-fluorophenyl)-1,3,4-oxadiazol-2-yl)phenyl)methanamine trifluoroacetate (98 mg, 0.24 mmol), 8-cyclopentyl-7H-purine-6-carboxylic acid (47 mg, 0.20 mmol), EDC (47 mg, 0.24 mmol), HOBt (33 mg, 0.24 mmol), DIPEA (170 *µ* L, 1.0 mmol) and THF (2 mL), the title compound (38 mg, 37%) was obtained as an off-white solid.
¹H NMR (400 MHz, DMSO-*d*₆) *δ* 13.20 (s, 1H), 9.93 (t, J = 6.1 Hz, 1H), 8.99 (s, 1H), 8.21 (dd, J = 8.7, 5.3 Hz, 2H), 8.05 (s, 1H), 7.87 (d, J = 8.5 Hz, 1H), 7.57 - 7.42 (m, 3H), 4.68 (d, J = 6.0 Hz, 2H), 3.54 - 3.46 (m, 1H), 2.13 - 2.03 (m, 2H), 1.99 - 1.88 (m, 2H), 1.87 - 1.75 (m, 2H), 1.72 - 1.60 (m, 2H).
LC-MS, *m*/*z* 502 [M+H]⁺

### Example 133

### 4-((3-((8-Cyclopentyl-7H-purine-6-carboxamido)methyl)-5-fluorophenyl)amino)benzoic acid

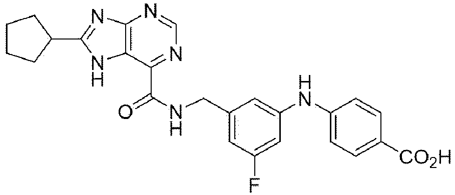

Ethyl 4-((3-((8-cyclopentyl-7H-purine-6-carboxamido)methyl)-5-fluorophenyl)amino)benzoate (35 mg, 0.070 mmol) was dissolved in MeOH/THF (141 µL/1.4 mL), and LiOH (8.3 mg) dissolved in H₂O (141 µL) was added and stirred at room temperature overnight. The reaction mixture was acidified with 1 M HCl, extracted with EtOAc, and the organic layer was dried over Na₂SO₄. The concentrated residue was purified by silica gel column chromatography MPLC (5-15% MeOH in DCM) to obtain the title compound (16.2 mg, 49.0%) as a yellow solid.
¹H NMR (400 MHz, CD₃OD) δ8.96 (s, 1H), 7.83 (d, *J =* 8.8 Hz, 2H), 7.08 (d, *J =* 8.8 Hz, 2H), 7.04 (s, 1H), 6.78 (d, *J =* 11.0 Hz, 1H), 6.72 (d, *J =* 9.4 Hz, 1H), 4.64 (s, 2H), 3.52 - 3.45 (m, 1H), 2.19 (s, 2H), 2.03 (s, 2H), 1.92 (s, 2H), 1.77 (s, 2H).
LC-MS, m/z 475 [M+H]⁺

### Example 134

### 134.1 3-((1,3,4-Oxadiazol-2-yl)amino)-5-fluorobenzonitrile

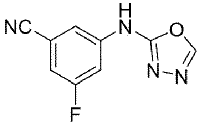

3-Cyano-5-fluorophenylboronic acid (0.2 g, 1.21 mmol) was dissolved in DCM (4.9 mL), and 1,3,4-oxadiazol-2-amine (0.206 g, 2.43 mmol), Cu(OAc)₂ (0.220 g, 1.21 mmol) and TEA (338 µL, 2.43 mmol) were added. After stirring at room temperature under air conditions for 8 h, the reaction mixture was filtered through a pad of Celite and washed several times with EtOAc. The filtrate was concentrated, and the obtained residue was purified by silica gel column chromatography (0-5% MeOH in DCM) to obtain the title compound (59.1 mg, 23.9%) as a white solid.
¹H NMR (400 MHz, CD₃OD) δ8.56 (s, 1H), 8.23 (s, 1H), 7.79 - 7.73 (m, 1H), 7.71 (s, 1H), 7.19 (d, *J =* 7.1 Hz, 1H).
LC-MS, m/z 205 [M+H]⁺

### 134.2 N-(3-(aminomethyl)-5-fluorophenyl)-1,3,4-oxadiazol-2-amine

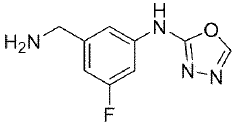

3-((1,3,4-Oxadiazol-2-yl)amino)-5-fluorobenzonitrile (59 mg, 0.289 mmol) was dissolved in THF (2.9 mL), LAH solution (2.0 M (in THF); 0.29 mL) was added at 0 °C and the mixture was stirred at room temperature overnight. The reaction mixture was cooled to 0 °C, quenched by adding H₂O, filtered through a pad of Celite, and washed several times with EtOAc. The filtrate was washed with H₂O, and the organic layer was dried over Na₂SO₄ and concentrated. The title compound (31.4 mg, 87.5%) was obtained as a yellow solid and used for the next reaction without purification.
LC-MS, m/z 209 [M+H]⁺

### 134.3 N-(3-((1,3,4-oxadiazol-2-yl)amino)-5-fluorobenzyl)-8-cyclopentyl-7H-purine-6-carboxamide

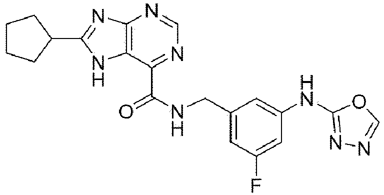

By the same method as in Example 87.3, 8-cyclopentyl-7H-purine-6-carboxylic acid (34.6 mg, 0.149 mmol), N-(3-(aminomethyl)-5-fluorophenyl)-1,3,4-oxadiazol-2-amine (31 mg, 0.149 mmol), HOBt (24.1 mg, 0.179 mmol), EDC (81.5 mg, 0.179 mmol), DIPEA (76 µL, 0.447 mmol) and THF (3.5 mL) were used to obtain the title compound (1.4 mg, 2.2%) as a yellow solid.
¹H NMR (400 MHz, CD₃OD) δ8.95 (s, 1H), 8.48 (s, 1H), 7.36 (d, *J =* 10.3 Hz, 1H), 7.31 (s, 1H), 6.83 (d, *J=* 9.6 Hz, 1H), 4.67 (s, 2H), 3.51 - 3.46 (m, 1H), 2.21 - 2.17 (m, 2H), 2.06 - 2.01 (m, 2H), 1.91 (d, *J =* 5.8 Hz, 2H), 1.77 (dd, *J =* 6.7, 4.9 Hz, 2H).
LC-MS, m/z 423 [M+H]⁺

### Example 135

### 8-Cyclopentyl-N-(3-(cyclopentylamino)-5-fluorobenzyl)-7H-purine-6-carboxamide

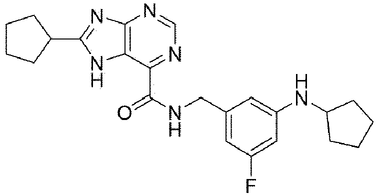

By the same method as in Example 1.3, 8-cyclopentyl-7H-purine-6-carboxylic acid (19 mg, 0.082 mmol), 3-(aminomethyl)-N-cyclopentyl-5-fluoroaniline (19 mg, 0.091 mmol), T3P (50 wt.% solution (in EtOAc), 159 mg) and DIPEA (56 µL) were used to obtain the title compound (13 mg, 38%) as a white solid.
¹H NMR (400 MHz, CDCl₃) δ 8.94 (s, 1H), 6.42 (s, 1H), 6.31 (d, *J =* 12 Hz, 1H), 6.20 (d, *J =* 12 Hz, 1H), 4.54 (s, 2H), 3.71 - 3.68 (m, 1H), 3.50 - 3.46 (m, 1H), 2.20 - 2.18 (m, 2H), 2.04 - 1.91 (m, 6H), 1.78 - 1.65 (m, 4H), 1.50 - 1.42 (m, 4H).
LC-MS, m/z 423 [M+H]+

### Example 136

### 136.1 3-((1,3,4-Thiadiazol-2-yl)amino)-5-fluorobenzonitrile

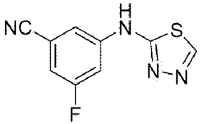

By the same method as in Example 121.1, using 3-fluoro-5-(4,4,5,5-tetramethyl-1,3,2-dioxaborolan-2-yl)benzonitrile (30 mg, 0.12 mmol), 1,3,4-thiadiazol-2-amine (25 mg, 0.24 mmol), Cu(OAc)₂ (22 mg, 0.12 mmol), TEA (34 uL, 0.52 mmol) and acetonitrile (115 uL)/ethanol (6 uL), the title compound (10 mg, 38%) was obtained as a white solid.
¹H NMR (400 MHz, CD₃OD) δ 8.11 (s, 1H), 7.60 (s, 1H), 7.46 (d, *J=* 12 Hz, 1H), 7.30 (d, *J* = 8 Hz, 1H).
LC-MS, m/z 221 [M+H]+

### 136.2 N-(3-(aminomethyl)-5-fluorophenyl)-1,3,4-thiadiazol-2-amine

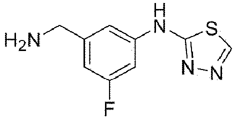

By the same method as in Example 1.2, 3-((1,3,4-thiadiazol-2-yl)amino)-5-fluorobenzonitrile (10 mg, 0.045 mmol) and LAH solution (2.0 M (in THF); 0.05 mL) were used to obtain the title compound (7 mg, 69%) as a yellow syrup.
LC-MS, m/z 225 [M+H]+

### 136.3 N-(3-((1,3,4-thiadiazol-2-yl)amino)-5-fluorobenzyl)-8-cyclopentyl-7H-purine-6-carboxamide

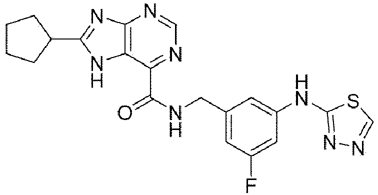

By the same method as in Example 1.3, 8-cyclopentyl-7H-purine-6-carboxylic acid (7 mg, 0.037 mmol), N-(3-(aminomethyl)-5-fluorophenyl)-1,3,4-thiadiazol-2-amine (7 mg, 0.03 mmol), T3P (50 wt.% solution (in EtOAc), 64 mg) and DIPEA (20 µL) were used to obtain the title compound (1.7 mg, 13%) as a white solid.
¹H NMR (400 MHz, CD₃OD) δ 8.96 (s, 1H), 8.78 (s, 1H), 7.53 (d, *J* = 12 Hz, 1H), 7.33 (s, 1H), 6.83 (d, *J =* 8 Hz, 1H), 4.67 (s, 2H), 3.51 - 3.47 (m, 1H), 2.25 - 2.17 (m, 2H), 2.06 - 1.99 (m, 2H), 1.94 - 1.88 (m, 2H), 1.80 - 1.74 (m, 2H).
LC-MS, m/z 439 [M+H]+

### Example 137

### 137.1 tert-Butyl (3-fluoro-5-(4-(4-fluorophenyl)-1,4-diazepan-1-yl)benzyl)carbamate

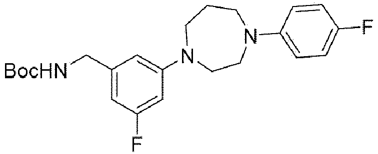

By the same method as in Example 8.1, using 1-(4-fluorophenyl)-1,4-diazepane (42 mg, 0.22 mmol), tert-butyl (3-bromo-5-fluorobenzyl)carbamate (62.6 mg, 0.21 mmol), Pd₂(dba)₃ (4.2 mg, 0.0045 mmol), BINAP (5.2 mg, 0.0.0082 mmol), NaO^{t}Bu (39.6 mg, 0.412 mmol) and toluene (1.03 mL), the title compound (16 mg, 19%) was obtained as a yellow solid.
¹H NMR (400 MHz, CDCl₃) *δ* 6.94 (t, *J* = 8.7 Hz, 2H), 6.64 (dd, *J* = 9.0, 4.2 Hz, 2H), 6.39 (s, 1H), 6.29 (d, *J* = 9.7 Hz, 2H), 4.82 (s, 1H), 4.22 (d, *J* = 5.5 Hz, 2H), 3.67 - 3.54 (m, 4H), 3.40 (dd, *J* = 11.3, 5.8 Hz, 4H), 2.09 (p, *J* = 6.3 Hz, 2H), 1.47 (s, 9H).
LC-MS, m/z 418 [M+H]⁺

### 137.2 (3-Fluoro-5-(4-(4-fluorophenyl)-1,4-diazepan-1-yl)phenyl)methanamine trifluoroacetate

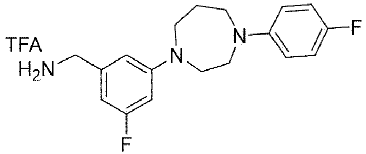

By the same method as in Example 25.4, using tert-butyl (3-fluoro-5-(4-(4-fluorophenyl)-1,4-diazepan-1-yl)benzyl)carbamate (15.5 mg, 0.037 mmol), trifluoroacetic acid (57 µL) and DCM (0.74 mL), the title compound (21.8 mg, crude) was obtained as a yellow solid.
LC-MS, m/z 318 [M+H]⁺

### 137.3 8-Cyclopentyl-N-(3-fluoro-5-(4-(4-fluorophenyl)-1,4-diazepan-1-yl)benzyl)-7H-purine-6-carboxamide

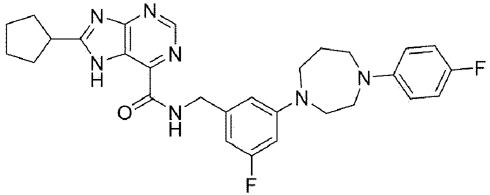

By the same method as in Example 1.3, using 8-cyclopentyl-7H-purine-6-carboxylic acid (9.1 mg, 0.039 mmol), (3-fluoro-5-(4-(4-fluorophenyl)-1,4-diazepan-1-yl)phenyl)methanamine trifluoroacetate (21.8 mg, crude, 0.037 mmol), T3P (propylphosphonic anhydride, 50 wt.% solution (in DMF), 16 mg), DIPEA (N,N-diisopropylethylamine, 32 µL) and DMF (0.74 mL), the title compound (3.1 mg, 16%) was obtained as an off-white solid.
¹H NMR (400 MHz, CDCl₃) *δ* 10.42 (s, 1H), 9.02 (s, 1H), 8.36 (s, 1H), 6.93 (t, *J =* 8.7 Hz, 2H), 6.70 - 6.57 (m, 2H), 6.45 (s, 1H), 6.36 (dd, *J* = 21.1, 10.4 Hz, 2H), 4.61 (d, *J* = 6.1 Hz, 2H), 3.67 - 3.57 (m, 4H), 3.46 - 3.35 (m, 5H), 2.30 - 2.18 (m, 2H), 2.13 - 1.98 (m, 4H), 1.97 - 1.85 (m, 2H), 1.83 - 1.71 (m, 2H).
LC-MS, m/z 532 [M+H]⁺

### Example 138

### 138.1 tert-Butyl 3-(1-cyclopentyl-1H-pyrazol-4-yl)-5-fluorobenzylcarbamate

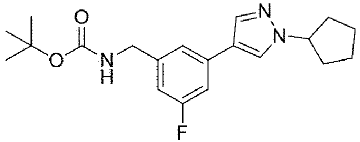

By the same method as in Example 4.1, using tert-butyl 3-bromo-5-fluorobenzylcarbamate (101.5 mg, 0.33 mmol), 1-cyclopentyl-4-(4,4,5,5-tetramethyl-1,3,2-dioxaborolan-2-yl)-1H-pyrazole (131.2 mg, 0.50 mmol), PdCl₂ (dppf) (12.2 mg, 0.017 mmol), 2M K₂CO₃ (0.6 mL, 1.17 mmol) and 1,4-dioxane (2 mL), the title compound (77.94 mg, 65%) was obtained as a colorless clear oil.
LC-MS, m/z 360 [M+H]⁺

### 138.2 (3-(1-Cyclopentyl-1H-pyrazol-4-yl)-5-fluorophenyl)methanamine trifluoroacetate

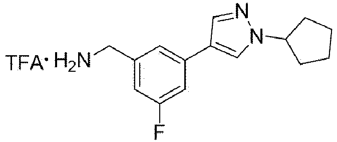

By the same method as in Example 25.4, using tert-butyl 3-(1-cyclopentyl-1H-pyrazol-4-yl)-5-fluorobenzylcarbamate (77.94 mg, 0.22 mmol), TFA (0.7 mL) and DCM (2 mL), the title compound (81 mg, crude) was obtained.
LC-MS, m/z 260 [M+H]⁺

### 138.3 8-Cyclopentyl-N-(3-(1-cyclopentyl-1H-pyrazol-4-yl)-5-fluorobenzyl)-7H-purine-6-carboxamide

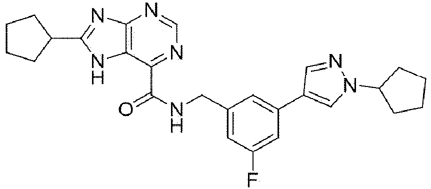

By the same method as in Example 87.3, (3-(1-cyclopentyl-1H-pyrazol-4-yl)-5-fluorophenyl)methanamine trifluoroacetate (81 mg, 0.22 mmol), 8-cyclopentyl-7H-purine-6-carboxylic acid (50.3 mg, 0.22 mmol), EDC (50 mg, 0.26 mmol), HOBt (35 mg, 0.26 mmol), DIPEA (111 µL, 0.65 mmol) and THF (2 mL) were used to obtain the title compound (54.52 mg, 53%) as a pale yellow solid.
¹H NMR (400 MHz, , DMSO-*d*₆) *δ* 13.17 (s, 1H), 9.74 (s, 1H), 8.96 (s, 1H), 8.26 (s, 1H), 7.88 (s, 1H), 7.45 (s, 1H), 7.33 (d, J = 10.1 Hz, 1H), 6.98 (d, J = 9.4 Hz, 1H), 4.68 (dt, J = 13.8, 6.9 Hz, 1H), 4.56 (d, J = 6.2 Hz, 2H), 3.56 - 3.41 (m, 1H), 2.16 - 2.01 (m, 4H), 2.00 - 1.98 (m, 4H), 1.86 - 1.73 (m, 4H), 1.73 - 1.57 (m, 4H).
LC-MS, m/z 474 [M+H]⁺

### Example 139

### 139.1 tert-Butyl 3-(1-cyclohexyl-1H-pyrazol-4-yl)-5-fluorobenzylcarbamate

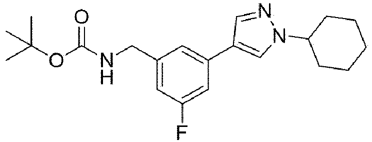

By the same method as in Example 4.1, using tert-butyl 3-bromo-5-fluorobenzylcarbamate (101.73 mg, 0.33 mmol), 1-cyclohexyl-4-(4,4,5,5-tetramethyl-1,3,2-dioxaborolan-2-yl)-1H-pyrazole (138.6 mg, 0.50 mmol), PdCl₂ (dppf) (12.2 mg, 0.017 mmol), 2M K₂CO₃ (0.6 mL, 1.17 mmol) and 1,4-dioxane (2 mL), the title compound (107.22 mg, 85.8%) was obtained as a yellow oil.
LC-MS, m/z 374 [M+H]⁺

### 139.2 (3-(1-Cyclohexyl-1H-pyrazol-4-yl)-5-fluorophenyl)methanaminetrifluoroacetate

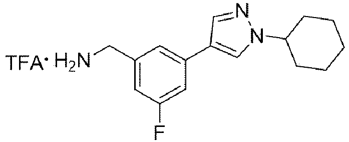

By the same method as in Example 25.4, using tert-butyl 3-(1-cyclohexyl-1H-pyrazol-4-yl)-5-fluorobenzylcarbamate (53.6 mg, 0.14 mmol), TFA (0.7 mL) and DCM (2 mL), the title compound (56 mg, crude) was obtained.
LC-MS, m/z 274 [M+H]⁺

### 139.3 N-(3-(1-cyclohexyl-1H-pyrazol-4-yl)-5-fluorobenzyl)-8-cyclopentyl-7H-purine-6-carboxamide

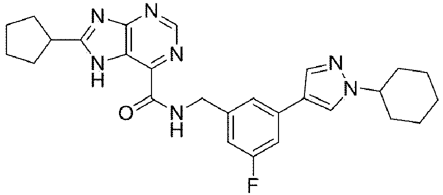

By the same method as in Example 87.3, (3-(1-cyclohexyl-1H-pyrazol-4-yl)-5-fluorophenyl)methanamine trifluoroacetate (56 mg, 0.14 mmol), 8-cyclopentyl-7H-purine-6-carboxylic acid (33.3 mg, 0.14 mmol), EDC (33 mg, 0.17 mmol), HOBt (23 mg, 0.17 mmol), DIPEA (74 µL, 0.43 mmol) and THF (2 mL) were used to obtain the title compound (39.38 mg, 56.3%) as a yellow solid.
¹H NMR (400 MHz, , DMSO-*d*₆) *δ* 13.17 (s, 1H), 9.74 (s, 1H), 8.96 (s, 1H), 8.25 (s, 1H), 7.87 (s, 1H), 7.44 (s, 1H), 7.32 (d, J = 9.8 Hz, 1H), 6.98 (d, J = 9.6 Hz, 1H), 4.56 (d, J = 6.4 Hz, 2H), 4.12 (t, J = 11.4 Hz, 1H), 3.57 - 3.42 (m, 1H), 2.15 - 1.99 (m, 4H), 1.99 - 1.87 (m, 2H), 1.86 - 1.76 (m, 4H), 1.75 - 1.57 (m, 4H), 1.49 - 1.29 (m, 2H), 1.28 - 1.12 (m, 2H).
LC-MS, m/z 488 [M+H]⁺

### Example 140

### 140.1 tert-Butyl 3-fluoro-5-(1-(tetrahydrofuran-3-yl)-1H-pyrazol-4-yl)benzylcarbamate

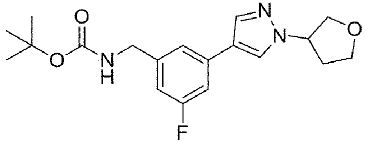

By the same method as in Example 4.1, tert-butyl 3-bromo-5-fluorobenzylcarbamate (40 mg, 0.13 mmol), 1-(tetrahydrofuran-3-yl)-4-(4,4,5,5-tetramethyl-1,3,2-dioxaborolan-2-yl)-1H-pyrazole (52 mg, 0.20 mmol), PdCl₂ (dppf) (4.8 mg, 0.0066 mmol), 2M K₂CO₃ (0.23 mL, 0.46 mmol) and 1,4-dioxane (1.5 mL) were used to obtain the title compound (14.82 mg, 31%) as a colorless clear oil.
LC-MS, m/z 362 [M+H]⁺

### 140.2 (3-Fluoro-5-(1-(tetrahydrofuran-3-yl)-1H-pyrazol-4-yl)phenyl)methanamine trifluoroacetate

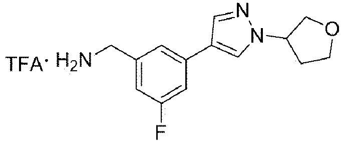

By the same method as in Example 25.4, using tert-butyl 3-fluoro-5-(1-(tetrahydrofuran -3-yl)-1H-pyrazol-4-yl)benzylcarbamate (14.82 mg, 0.041 mmol), TFA (0.6 mL) and DCM (2 mL), the title compound (15 mg, crude) was obtained.
LC-MS, m/z 262 [M+H]⁺

### 140.3 8-Cyclopentyl-N-(3-fluoro-5-(1-(tetrahydrofuran-3-yl)-1H-pyrazol-4-yl)benzyl)-7H-purine-6-carboxamide

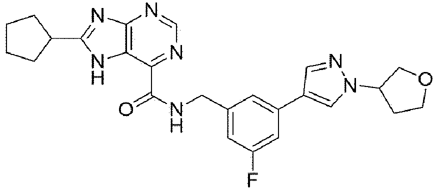

By the same method as in Example 87.3, (3-fluoro-5-(1-(tetrahydrofuran-3-yl)-1H-pyrazol-4-yl)phenyl)methanamine trifluoroacetate (14.82 mg, 0.041 mmol), 8-cyclopentyl-7H-purine-6-carboxylic acid (9.5 mg, 0.041 mmol), EDC (9.5 mg, 0.049 mmol), HOBt (7 mg, 0.049 mmol), DIPEA (21 µL, 0.12 mmol) and THF (2 mL) were used to obtain the title compound (11.82 mg, 60.6%) as an off-white solid.
¹H NMR (400 MHz, CD₃OD) *δ* 8.96 (s, 1H), 8.08 (s, 1H), 7.86 (s, 1H), 7.44 (s, 1H), 7.22 (d, J = 9.5 Hz, 1H), 7.00 (d, J = 9.6 Hz, 1H), 5.10 - 4.98 (m, 1H), 4.68 (s, 2H), 4.12 (dd, J = 15.3, 7.8 Hz, 1H), 4.04 (d, J = 4.7 Hz, 2H), 3.91 (dd, J = 14.2, 8.4 Hz, 1H), 3.58 - 3.41 (m, 1H), 2.60 - 2.41 (m, 1H), 2.41 - 2.28 (m, 1H), 2.26 - 2.13 (m, 2H), 2.09 - 1.98 (m, 2H), 1.98 - 1.85 (m, 2H), 1.85 - 1.67 (m, 2H).
LC-MS, m/z 476 [M+H]⁺

### Example 141

### 141.1 tert-Butyl 3-fluoro-5-(1-(thiazol-4-yl)-1H-pyrazol-4-yl)benzylcarbamate

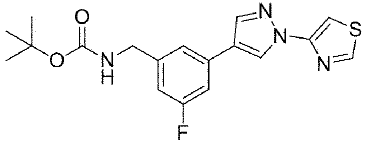

By the same method as in Example 25.3, using tert-butyl 3-fluoro-5-(1H-pyrazol-4-yl)benzylcarbamate (101.95 mg, 0.35 mmol), 4-bromothiazole (86.1 mg, 0.52 mmol), CuI (6.7 mg, 0.035 mmol), L-proline (8 mg, 0.069 mmol), K₂CO₃ (72.5 mg, 0.52 mmol) and DMSO (3 mL), the title compound (58 mg, 44%) was obtained as a light yellow solid.
LC-MS, m/z 375 [M+H]⁺

### 141.2 (3-Fluoro-5-(1-(thiazol-4-yl)-1H-pyrazol-4-yl)phenyl)methanamine, trifluoroacetate

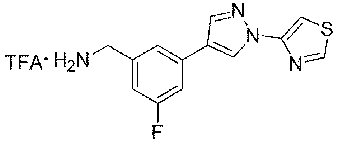

By the same method as in Example 25.4, using tert-butyl 3-fluoro-5-(1-(thiazol-4-yl)-1H-pyrazol-4-yl)benzylcarbamate (55.22 mg, 0.15 mmol), TFA (0.5 mL) and DCM (1.5 mL), the title compound (57.3 mg, crude) was obtained.
LC-MS, m/z 275 [M+H]⁺

### 141.3 8-Cyclopentyl-N-(3-fluoro-5-(1-(thiazol-4-yl)-1H-pyrazol-4-yl)benzyl)-7H-purine-6-carboxamide

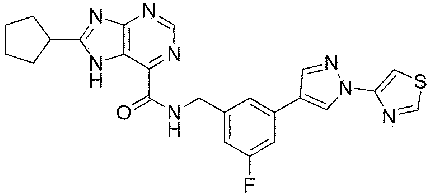

By the same method as in Example 87.3, (3-fluoro-5-(1-(thiazol-4-yl)-1H-pyrazol-4-yl)phenyl)methanamine trifluoroacetate (57.3 mg, 0.15 mmol), 8-cyclopentyl-7H-purine-6-carboxylic acid (34.3 mg, 0.15 mmol), EDC (34 mg, 0.18 mmol), HOBt (24 mg, 0.18 mmol), DIPEA (75 µL, 0.44 mmol) and THF (2 mL) were used to obtain the title compound (57.46 mg, 79.7%) as a light yellow solid.
¹H NMR (400 MHz, DMSO-*d*₆) *δ* 13.18 (s, 1H), 9.77 (t, J = 6.6 Hz, 1H), 9.22 (d, J = 2.0 Hz, 1H), 8.97 (s, 1H), 8.91 (s, 1H), 8.28 (s, 1H), 7.77 (d, J = 2.1 Hz, 1H), 7.65 (s, 1H), 7.54 (d, J = 9.7 Hz, 1H), 7.06 (d, J = 9.3 Hz, 1H), 4.60 (d, J = 6.0 Hz, 2H), 3.57 - 3.43 (m, 1H), 2.16 - 2.03 (m, 2H), 1.99 - 1.87 (m, 2H), 1.87 - 1.74 (m, 2H), 1.73 - 1.57 (m, 2H).
LC-MS, m/z 489 [M+H]⁺

### Example 142

### 142.1 tert-Butyl (3-fluoro-5-((1-methyl-1H-pyrrol-3-yl)amino)benzyl)carbamate

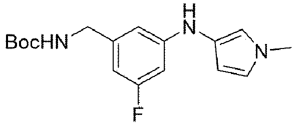

By the same method as in Example 87.3, using tert-butyl 3-bromo-5-fluorobenzylcarbamate (0.268 g, 0.881 mmol), 1-methyl-1H-pyrrol-3-amine hydrochloride (0.129 g, 0.881 mmol), (±)BINAP (54.9 mg, 0.088 mmol), Pd₂(dba)₃ (80.8 mg, 0.088 mmol), sodium tert-butoxide (0.212 g, 2.20 mmol) and toluene (4.9 mL), the title compound (53.3 mg, 18.9%) was obtained as a yellow solid.
¹H NMR (400 MHz, DMSO-*d6*) *δ* 7.59 (s, 1H), 7.30 (t, *J =* 5.4 Hz, 1H), 6.57 (d, *J*= 10.9 Hz, 2H), 6.45 (s, 1H), 6.30 (d, *J=* 11.4 Hz, 1H), 6.15 (d, *J =* 9.0 Hz, 1H), 5.81 (s, 1H), 4.08 (dd, *J* = 10.6, 5.4 Hz, 1H), 3.95 (d, *J =* 6.2 Hz, 2H), 3.55 (s, 3H), 1.37 (s, 9H).
LC-MS, m/z 320 [M+H]⁺

### 142.2 N-(3-(aminomethyl)-5-fluorophenyl)-1-methyl-1H-pyrrol-3-amine hydrochloride

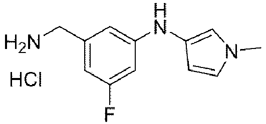

By the same method as in Example 90.3, tert-butyl (3-fluoro-5-((1-methyl-1H-pyrrol-3-yl)amino)benzyl)carbamate (20 mg, 0.063 mmol) and 4 M HCl (in dioxane) (0.31 mL) were used to obtain the title compound (12 mg, 74.9%) as a brown solid, which was used in the next reaction without separation and purification.
¹H NMR (400 MHz, DMSO-*d6*) *δ* 8.16 (s, 2H), 6.62 (d, *J =* 5.8 Hz, 3H), 6.45 (d, *J =* 10.2 Hz, 2H), 5.85 (s, 1H), 3.86 (d, *J =* 5.8 Hz, 2H), 3.55 (d, *J =* 5.3 Hz, 3H).
LC-MS, m/z 220 [M+H]⁺

### 142.3 8-Cyclopentyl-N-(3-fluoro-5-((1-methyl-1H-pyrrol-3-yl)amino)benzyl)-7H-purine-6-carboxamide

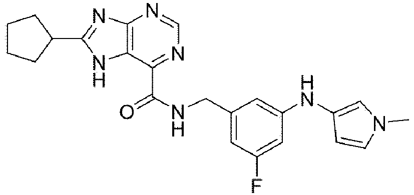

By the same method as in Example 87.3, N-(3-(aminomethyl)-5-fluorophenyl)-1-methyl-1H-pyrrol-3-amine hydrochloride (12 mg, 0.047 mmol), 8-cyclopentyl-7H-purine-6-carboxylic acid (10.9 mg, 0.047 mmol), HOBt (7.6 mg, 0.056 mmol), EDC (10.8 mg, 0.056 mmol), DIPEA (170 µL, 0.141 mmol) and THF (1.1 mL) were used to obtain the title compound (2.6 mg, 12.8%) as a yellow solid.
¹H NMR (400 MHz, CD₃OD) *δ* 8.95 (s, 1H), 6.60 (s, 1H), 6.47 (s, 1H), 6.34 (t, J = 11.5 Hz, 3H), 5.86 (d, J = 2.8 Hz, 1H), 4.54 (s, 2H), 3.55 (s, 3H), 3.52 - 3.47 (m, 1H), 2.20 (d, J = 8.0 Hz, 2H), 2.05 - 2.00 (m, 2H), 1.94 - 1.90 (m, 2H), 1.80 - 1.75 (m, 2H).
LC-MS, m/z 434 [M+H]⁺

### Example 143

### 143.1 3-Iodothiophene 1-oxide

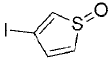

3-Iodothiophene (500 mg, 2.38 mmol) was dissolved in DCE (12 mL), 3-chloroperbenzoic acid (1.3 g, 5.95 mmol) was added, and stirred at 90 °C for 2 hours. The reaction mixture was concentrated. The obtained residue was purified by silica gel column chromatography (hexane) to obtain the title compound (116 mg, 23%) as a red oil.

### 143.2 tert-Butyl 3-fluoro-5-(1-(1-oxidothiophen-3-yl)-1H-pyrazol-4-yl)benzylcarbamate

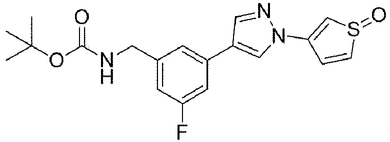

By the same method as in Example 25.3, tert-butyl 3-fluoro-5-(1H-pyrazol-4-yl)benzylcarbamate (99 mg, 0.34 mmol), 3-iodothiophene 1-oxide (115 mg, 0.51 mmol), CuI (6.5 mg, 0.034 mmol), L-proline (8 mg, 0.068 mmol), K₂CO₃ (70 mg, 0.51 mmol) and DMSO (3.5 mL) were used to obtain the title compound (29.0 mg, 23%) as a brown solid.
LC-MS, m/z 390 [M+H]⁺

### 143.3 3-(4-(3-(Aminomethyl)-5-fluorophenyl)-1H-pyrazol-1-yl)thiophene 1-oxide trifluoroacetate

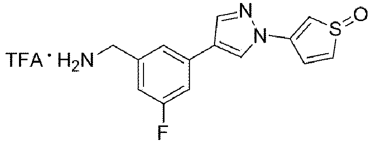

By the same method as in Example 25.4, using tert-butyl 3-fluoro-5-(1-(1-oxidothiophen -3-yl)-1H-pyrazol-4-yl)benzylcarbamate (28.08 mg, 0.072 mmol), TFA (0.3 mL) and DCM (1 mL), the title compound (29 mg, crude) was obtained.
LC-MS, m/z 290 [M+H]⁺

### 143.4 8-Cyclopentyl-N-(3-fluoro-5-(1-(1-oxidothiophen-3-yl)-1H-pyrazol-4-yl)benzyl)-7H-purine-6-carboxamide

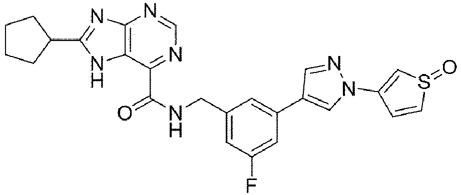

By the same method as in Example 87.3, using 3-(4-(3-(aminomethyl)-5-fluorophenyl)-1H-pyrazol-1-yl)thiophene 1-oxide trifluoroacetate (29 mg, 0.072 mmol), 8-cyclopentyl-7H-purine-6-carboxylic acid (16.7 mg, 0.072 mmol), EDC (16.6 mg, 0.087 mmol), HOBt (11.7 mg, 0.087 mmol), DIPEA (37 µL, 0.22 mmol) and THF (2 mL), the title compound (9.7 mg, 27%) was obtained as a white solid.
¹H NMR (400 MHz, DMSO-*d*₆) *δ* 13.16 (s, 1H), 9.77 (s, 1H), 8.96 (s, 1H), 8.22 (s, 1H), 8.11 (s, 1H), 7.48 (s, 1H), 7.38 (d, J = 11.0 Hz, 1H), 7.04 (d, J = 9.1 Hz, 1H), 5.75 (s, 1H), 5.50 (d, J = 13.4 Hz, 1H), 5.28 (d, J = 13.2 Hz, 1H), 4.58 (d, J = 5.9 Hz, 2H), 3.50 - 3.44 (m, 1H), 2.14 - 2.02 (m, 2H), 2.02 - 1.87 (m, 2H), 1.86 - 1.74 (m, 2H), 1.72 - 1.61 (m, 2H).
LC-MS, m/z 504 [M+H]⁺

### Example 144

### 144.1 3-Fluoro-5-((1-methyl-1H-pyrazol-4-yl)amino)benzonitrile

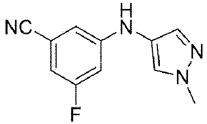

By the same method as in Example 10.1, 3-bromo-5-fluorobenzonitrile (1 g, 5.00 mmol), 1-methyl-1H-pyrazol-4-amine (426 µL, 5.00 mm), then Pd₂(dba)₃ (91.6 mg, 0.100 mmol), Xantphos (145 mg, 0.250 mmol), K₂CO₃ (1.59 g, 11.5 mmol), and dioxane (31 mL) were used to obtain the title compound (0.545 g, 50.4%) as a white solid.
¹H NMR (400 MHz, DMSO-*d6*) *δ* 8.24 (s, 1H), 7.77 (s, 1H), 7.35 (s, 1H), 6.92 (d,*J* = 7.4 Hz, 1H), 6.84 (s, 1H), 6.74 (d, *J=* 11.8 Hz, 1H), 3.80 (s, 3H).
LC-MS, m/z 217 [M+H]⁺

### 144.2 N-(3-(Aminomethyl)-5-fluorophenyl)-1-methyl-1H-pyrazol-4-amine

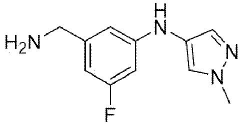

By the same method as in Example 7.2 above, 3-fluoro-5-((1-methyl-1H-pyrazol-4-yl)amino)benzonitrile (0.541 g, 2.50 mmol), LAH solution (2.0 M (THF (2.5 mL) and THF (25 mL) were used to obtain the title compound (0.518 g, 94.1%) as a brown solid.
¹H NMR (400 MHz, DMSO-*d6*) *δ* 7.70 (s, 1H), 7.64 (s, 1H), 7.28 (s, 1H), 6.51 (s, 1H), 6.37 (d, *J* = 9.3 Hz, 1H), 6.28 (d, *J =* 11.8 Hz, 1H), 3.78 (s, 3H), 3.56 (s, 2H).
LC-MS, m/z 221 [M+H]⁺

### 144.3 8-(3,3-Difluorocyclopentyl)-N-(3-fluoro-5-((1-methyl-1H-pyrazol-4-yl)amino)benzyl)-7H-purine-6-carboxamide

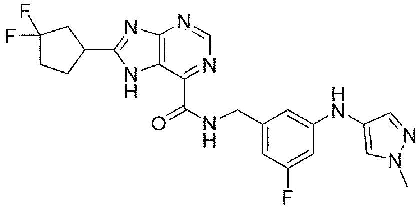

By the same method as in Example 87.3, using N-(3-(aminomethyl)-5-fluorophenyl)-1-methyl-1H-pyrazol-4-amine (49.3 mg, 0.224 mmol), 8-(3,3-difluorocyclopentyl)-7H-purine-6-carboxylic acid (60 mg, 0.224 mmol), EDC (51.5 mg, 0.268 mmol), HOBt (36.3 mg, 0.268 mmol) and THF (5.2 mL), the title compound (29 mg, 27.6%) was obtained as a yellow solid.
¹H NMR (400 MHz, DMSO-*d6*) *δ* 13.34 (s, 1H), 9.71 (s, 1H), 9.01 (s, 1H), 7.80 (s, 1H), 7.63 (s, 1H), 7.29 (d, *J =* 0.5 Hz, 1H), 6.59 (s, 1H), 6.42 (d, *J* = 9.4 Hz, 1H), 6.34 (dt, *J =* 11.8, 2.2 Hz, 1H), 4.44 (d, *J =* 6.3 Hz, 2H), 3.81 (dd, *J =* 8.7, 5.9 Hz, 1H), 3.77 (s, 3H), 2.70 - 2.59 (m, 2H), 2.36 - 2.27 (m, 2H), 2.26 - 2.09 (m, 2H).
LC-MS, m/z 471 [M+H]⁺

### Example 145

### 145.1 tert-Butyl 3-fluoro-5-(1-phenyl-d5-1H-pyrazol-4-yl)benzylcarbamate

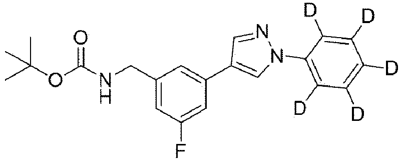

By the same method as in Example 25.3, tert-butyl 3-fluoro-5-(1H-pyrazol-4-yl)benzylcarbamate (30 mg, 0.103 mmol), bromobenzene-*d*₅ (25 mg, 0.15 mmol), CuI (2 mg, 0.010 mmol), L-proline (2.5 mg, 0.021 mmol), K₂CO₃ (21.3 mg, 0.15 mmol) and DMSO (2 mL) were used to obtain the title compound (7.27 mg, 18.95%) as a colorless clear oil.
LC-MS, m/z 373 [M+H]⁺

### 145.2 (3-Fluoro-5-(1-phenyl-d5-1H-pyrazol-4-yl)phenyl)methanamine trifluoroacetate

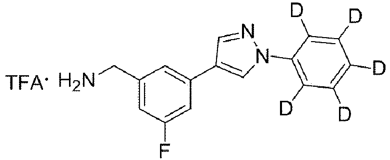

By the same method as in Example 25.4, using tert-butyl 3-fluoro-5-(1-phenyl-*d*₅-1H-pyrazol-4-yl)benzylcarbamate (7.27 mg, 0.02 mmol), TFA (0.2 mL) and DCM (0.6 mL), the title compound (8 mg, crude) was obtained.
LC-MS, m/z 275 [M+H]⁺

### 145.3 8-Cyclopentyl-N-(3-fluoro-5-(1-phenyl-d5-1H-pyrazol-4-yl)benzyl)-7H-purine-6-carboxamide

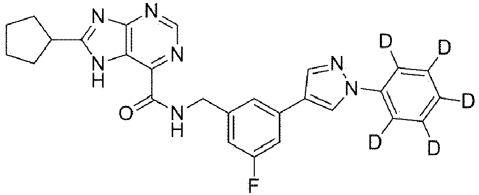

By the same method as in Example 87.3, (3-Fluoro-5-(1-phenyl-*d*₅-1H-pyrazol-4-yl)phenyl)methanamine, trifluoroacetate salt (8 mg, 0.02 mmol), 8-cyclopentyl-7H-purine-6-carboxylic acid (4.5 mg, 0.02 mmol), EDC (4.5 mg, 0.023 mmol), HOBt (3.2 mg, 0.023 mmol), DIPEA (10 µL, 0.059 mmol) and THF (2 mL) were used to obtain the title compound (7.24 mg, 76.3%) as an off-white solid.
¹H NMR (400 MHz, DMSO-*d*₆) *δ* 13.17 (s, 1H), 9.77 (t, J = 5.6 Hz, 1H), 9.06 (s, 1H), 8.97 (s, 1H), 8.24 (s, 1H), 7.61 (s, 1H), 7.49 (d, J = 9.9 Hz, 1H), 7.07 (d, J = 9.3 Hz, 1H), 4.61 (d, J = 6.4 Hz, 2H), 3.58 - 3.43 (m, 1H), 2.15 - 2.01 (m, 2H), 2.02 - 1.87 (m, 2H), 1.97 - 1.75 (s, 2H), 1.73 - 1.61 (s, 2H).
LC-MS, m/z 487 [M+H]⁺

### Example 146

### 146.1 tert-Butyl 3-fluoro-5-(3-methyl-1H-pyrazol-4-yl)benzylcarbamate

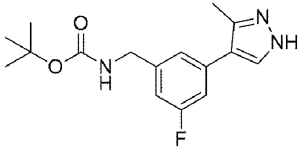

By the same method as in Example 25.2, tert-butyl 3-bromo-5-fluorobenzylcarbamate (244.75 mg, 0.8 mmol), 3-methyl-4-(4,4,5,5-tetramethyl-1,3,2-dioxaborolan-2-yl)-1H-pyrazole (251 mg, 1.21 mmol), Na₂CO₃ (298.5 mg, 2.82 mmol), Pd(PPh₃)₄ (46.3 mg, 0.04 mmol) and DMF (4 mL)/H₂O (1.7 mL) were used to obtain the title compound (191.97 mg, 78%) as a colorless clear oil.
LC-MS, m/z 306 [M+H]⁺

### 146.2 tert-Butyl 3-fluoro-5-(1-(4-fluorophenyl)-3-methyl-1H-pyrazol-4-yl)benzylcarbamate

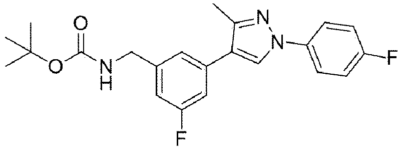

By the same method as in Example 25.3, tert-butyl 3-fluoro-5-(3-methyl-1H-pyrazol-4-yl)benzylcarbamate (28.39 mg, 0.093 mmol), 1-fluoro-4-iodobenzene (31 mg, 0.14 mmol), CuI (1.8 mg, 0.0093 mmol), L-proline (2 mg, 0.019 mmol), K₂CO₃ (19.2 mg, 0.14 mmol) and DMSO (2 mL) were used to obtain the title compound (18.41 mg, 49.6%) as a colorless clear oil. (TLC, R_{f}=0.32, elution condition Hex:EA=8:2)
LC-MS, m/z 400 [M+H]⁺

### 146.3 (3-Fluoro-5-(1-(4-fluorophenyl)-3-methyl-1H-pyrazol-4-yl)phenyl)methanamine trifluoroacetate

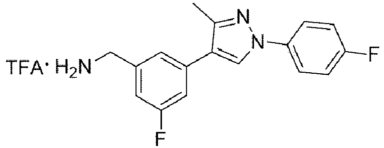

By the same method as in Example 25.4, using tert-butyl 3-fluoro-5-(1-(4-fluorophenyl)-3-methyl-1H-pyrazol-4-yl)benzylcarbamate (16.41 mg, 0.041 mmol), TFA (0.6 mL) and DCM (1.8 mL), the title compound (17 mg, crude) was obtained.
LC-MS, m/z 275 [M+H]⁺

### 146.4 8-Cyclopentyl-N-(3-fluoro-5-(1-(4-fluorophenyl)-3-methyl-1H-pyrazol-4-yl)benzyl)-7H-purine-6-carboxamide

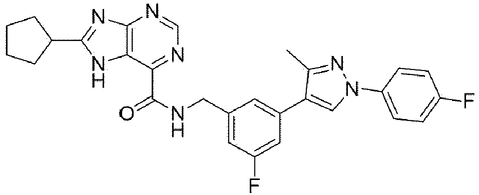

By the same method as in Example 87.3, using (3-fluoro-5-(1-(4-fluorophenyl)-3-methyl-1H-pyrazol-4-yl)phenyl)methanamine trifluoroacetate (17 mg, 0.041 mmol), 8-cyclopentyl-7H-purine-6-carboxylic acid (9.5 mg, 0.041 mmol), EDC (9.4 mg, 0.049 mmol), HOBt (6.65 mg, 0.049 mmol), DIPEA (21 µL, 0.12 mmol) and THF (2 mL), the title compound (10.1 mg, 48%) was obtained as a yellow solid.
¹H NMR (400 MHz, DMSO-*d*₆) *δ* 13.16 (s, 1H), 9.80 (s, 1H), 8.96 (s, 1H), 8.77 (s, 1H), 7.91 - 7.81 (m, 2H), 7.43 (s, 1H), 7.35 (t, J = 8.8 Hz, 2H), 7.29 (d, J = 10.1 Hz, 1H), 7.12 (d, J = 9.2 Hz, 1H), 4.61 (d, J = 5.9 Hz, 2H), 3.55 - 3.45 (m, 1H), 2.39 (s, 3H), 2.15 - 2.01 (m, 2H), 1.01 - 1.87 (m, 2H), 1.86 - 1.75 (m, 2H), 1.71 - 1.61 (m, 2H).
LC-MS, m/z 514 [M+H]⁺

### Example 147

### 147.1 tert-Butyl 3-fluoro-5-(1-(4-fluorophenyl)-5-methyl-1H-pyrazol-4-yl)benzylcarbamate

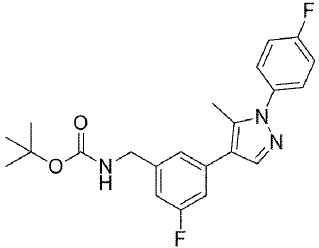

By the same method as in Example 25.3, tert-butyl 3-fluoro-5-(3-methyl-1H-pyrazol-4-yl)benzylcarbamate (127.25 mg, 0.42 mmol), 1-fluoro-4-iodobenzene (139 mg, 0.63 mmol), CuI (8 mg, 0.042 mmol), L-proline (10 mg, 0.083 mmol), K₂CO₃ (87 mg, 0.63 mmol) and DMSO (4 mL) were used to obtain the title compound (18.64 mg, 11.2%). (TLC, R_{f} =0.22, elution condition Hex:EA=8:2)
LC-MS, *m*/*z* 400 [M+H]⁺

### 147.2 3-Fluoro-5-(1-(4-fluorophenyl)-5-methyl-1H-pyrazol-4-yl)benzylcarbamate trifluoroacetate

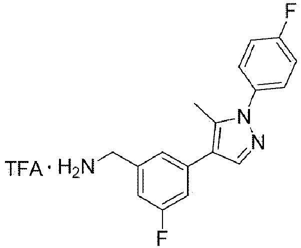

By the same method as in Example 25.4, using tert-butyl 3-fluoro-5-(1- (4-fluorophenyl)-5-methyl-1H-pyrazol-4-yl)benzylcarbamate (16.64 mg, 0.042 mmol), TFA (0.5 mL) and DCM (1.5 mL), the title compound (18 mg, crude) was obtained.
LC-MS, *m*/*z* 300 [M+H]⁺

### 147.3 8-Cyclopentyl-N-(3-fluoro-5-(1-(4-fluorophenyl)-5-methyl-1H-pyrazol-4-yl)benzyl)-7H-purine-6-carboxamide

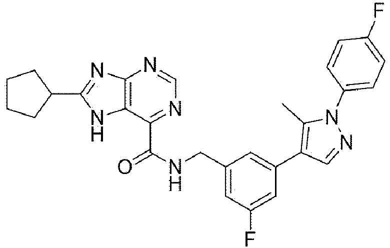

By the same method as in Example 87.3, using (3-fluoro-5-(1-(4-fluorophenyl)-3-methyl-1H-pyrazol-4-yl)phenyl)methanamine trifluoroacetate (17 mg, 0.041 mmol), 8-cyclopentyl-7H-purine-6-carboxylic acid (9.5 mg, 0.041 mmol), EDC (9.4 mg, 0.049 mmol), HOBt (6.65 mg, 0.049 mmol), DIPEA (21 µL, 0.12 mmol) and THF (2 mL), the title compound (10.1 mg, 48%) was obtained as a yellow solid.
¹H NMR (400 MHz, DMSO-*d*₆) *δ* 13.16 (s, 1H), 9.80 (t, J = 6.3 Hz, 1H), 8.96 (s, 1H), 7.91 (s, 1H), 7.63 - 7.54 (m, 2H), 7.43 - 7.36 (m, 2H), 7.36 (s, 1H), 7.21 (d, J = 9.8 Hz, 1H), 7.12 (d, J = 9.6 Hz, 1H), 4.61 (d, J = 6.3 Hz, 2H), 3.57 - 3.48 (m, 1H), 2.37 (s, 3H), 2.13 - 2.00 (m, 2H), 2.02 - 1.85 (m, 2H), 1.86 - 1.74 (m, 2H), 1.73 - 1.60 (m, 2H).
LC-MS, m/z 514 [M+H]+

### Example 148

### 8-(3,3-Difluorocyclopentyl)-N-(3-fluoro-5-(1-(phenyl-d5)-1H-pyrazol-4-yl)benzyl)-7H-purine-6-carboxamide

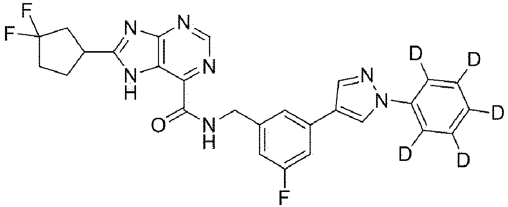

By the same method as in Example 87.3, (3-fluoro-5-(1-phenyl-d5-1H-pyrazol-4-yl)phenyl)methanamine trifluoroacetate (44 mg, 0.11 mmol), 8-(3,3-difluorocyclopentyl)-7H-purine-6-carboxylic acid (30.6 mg, 0.11 mmol), EDC (26.2 mg, 0.14 mmol), HOBt (18.5 mg, 0.14 mmol), DIPEA (58 µL, 0.34 mmol) and THF (2 mL) were used to obtain the title compound (42 mg, 70.3%).
¹H NMR (400 MHz, DMSO-*d*₆) *δ* 13.33 (s, 1H), 9.80 (t, J = 6.3 Hz, 1H), 9.06 (s, 1H), 9.01 (s, 1H), 8.24 (s, 1H), 7.59 (s, 1H), 7.48 (d, J = 10.0 Hz, 1H), 7.06 (d, J = 9.5 Hz, 1H), 4.61 (d, J = 6.2 Hz, 2H), 3.88 - 3.75 (m, 1H), 2.60 - 2.57 (m, 2H), 2.39 - 2.26 (m, 2H), 2.25 - 2.07 (m, 2H).
LC-MS, *m*/*z* 523 [M+H]⁺

### Example 149

### 149.1 3-Fluoro-5-(4-(4-fluorophenyl)-1H-pyrazol-1-yl)benzonitrile

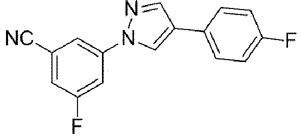

4-(4-Fluorophenyl)-1H-pyrazole (75 mg, 0.46 mmol) was dissolved in DCM (2.5 mL), (3-cyano-5-fluorophenyl)boronic acid (114 mg, 0.69 mmol), pyridine (0.11 mL, 1.38 mmol), and Cu(OAc)₂ (125.2 mg, 0.69 mmol) were added, and the mixture was stirred at room temperature for 20 h. The reaction mixture was diluted with EtOAc, washed with H₂O, and the organic layer was dried over MgSO₄ and concentrated. The obtained residue was purified by silica gel column chromatography (0-10% EtOAc in hexane) to obtain the title compound (7 mg, 5.4%).

¹H NMR (400 MHz, DMSO-*d*₆) *δ* 9.16 (s, 1H), 8.33 (s, 1H), 8.25 (s, 1H), 8.13 (d, J = 10.3 Hz, 1H), 7.82 (d, J = 8.2 Hz, 1H), 7.75 (dd, J = 8.5, 5.5 Hz, 2H), 7.29 (t, J = 8.8 Hz, 2H).

### 149.2 (3-Fluoro-5-(4-(4-fluorophenyl)-1H-pyrazol-1-yl)phenyl)methanamine

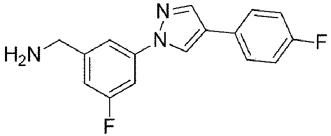

By the same method as in Example 7.2 above, 3-fluoro-5-(4-(4-fluorophenyl)-1H-pyrazol-1-yl)benzonitrile (5.2 mg, 0.019 mmol), LAH (2 M (in THF), 0.019 mL, 0.37 mmol) and THF (1.5 mL) were used to obtain the title compound (7 mg, crude) as a yellow solid.
LC-MS, m/z 286 [M+H]+

### 149.3 8-Cyclopentyl-N-(3-fluoro-5-(4-(4-fluorophenyl)-1H-pyrazol-1-yl)benzyl)-7H-purine-6-carboxamide

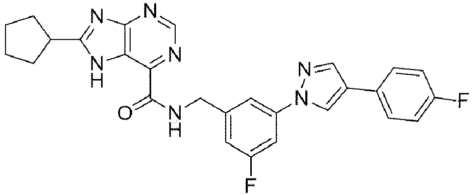

By the same method as in Example 87.3, using (3-fluoro-5-(4-(4-fluorophenyl)-1H-pyrazol-1-yl)phenyl)methanamine (7 mg, 0.019 mmol), 8-cyclopentyl-7H-purine-6-carboxylic acid (6 mg, 0.025 mmol), EDC (6 mg, 0.03 mmol), HOBt (4.1 mg, 0.03 mmol), DIPEA (13 µL, 0.075 mmol) and THF (2 mL), the title compound (1.88 mg, 15%) was obtained.
¹H NMR (400 MHz, CDCl₃) *δ* 10.42 (s, 1H), 9.03 (s, 1H), 8.52 (s, 1H), 8.09 (s, 1H), 7.93 (s, 1H), 7.58 (s, 1H), 7.55 - 1.45 (m, 2H), 7.41 (d, J = 9.5 Hz, 1H), 7.10 (t, J = 8.6 Hz, 2H), 7.04 (d, J = 8.5 Hz, 1H), 4.76 (d, J = 6.3 Hz, 2H), 3.49 - 3.36 (m, 1H), 2.30 - 2.16 (m, 2H), 2.12 - 1.98 (m, 2H), 1.97 - 1.85 (m, 2H), 1.85 - 1.72 (m, 2H).
LC-MS, *m*/*z* 500 [M+H]⁺

### Example 150

### 150.1 Methyl 2-((tert-butoxycarbonyl)amino)-3-(4-(4-(3-(((tert-butoxycarbonyl)amino)methyl)-5-fluorophenyl)-1H-pyrazol-1-yl)phenyl)propanoate

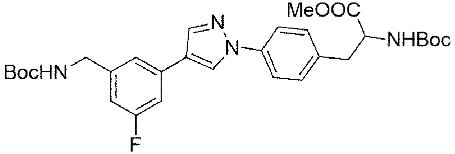

By the same method as in Example 25.3, using tert-butyl 3-fluoro-5-(1H-pyrazol-4-yl)benzylcarbamate (50 mg, 0.17 mmol), methyl 2-((tert-butoxycarbonyl)amino)-3-(4-iodophenyl)propanoate (104 mg, 0.26 mmol), CuI (3.2 mg, 0.017 mmol), L-proline (4 mg, 0.034 mmol), K₂CO₃ (36 mg, 0.26 mmol) and DMSO (2 mL), the title compound (51.5 mg, 51%) was obtained as an off-white solid.

¹H NMR (400 MHz, CDCl₃) *δ* 8.13 (s, 1H), 7.95 (s, 1H), 7.65 (d, J = 7.2 Hz, 2H), 7.25 - 7.19 (m, 3H), 7.13 (d, J = 9.7 Hz, 1H), 6.89 (d, J = 8.9 Hz, 1H), 5.01 (s, 1H), 4.93 (s, 1H), 4.61 (s, 1H), 4.34 (s, 2H), 3.73 (s, 3H), 3.24 - 3.03 (m, 2H), 1.48 (s, 9H), 1.43 (s, 9H).

### 150.2 Methyl 2-amino-3-(4-(4-(3-(aminomethyl)-5-fluorophenyl)-1H-pyrazol-1-yl)phenyl)propanoate dihydrochloride

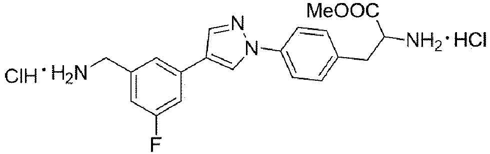

By the same method as in Example 90.3, methyl 2-((tert-butoxycarbonyl)amino)-3-(4-(4-(3-(((tert-butoxycarbonyl)amino)methyl)-5-fluorophenyl)- 1H-pyrazol-1-yl)phenyl)propanoate (20.55 mg, 0.035 mmol), 4 M HCl (in dioxane) (0.2 mL) and DCM (0.6 mL) were used to obtain the title compound (16 mg, crude).
LC-MS, *m*/*z* 369 [M+H]⁺

### 150.3 Methyl 2-(8-cyclopentyl-7H-purine-6-carboxamido)-3-(4-(4-(3-((8-cyclopentyl-7H-purine-6-carboxamido)methyl)-5-fluorophenyl)-1H-pyrazol-1-yl)phenyl)propanoate

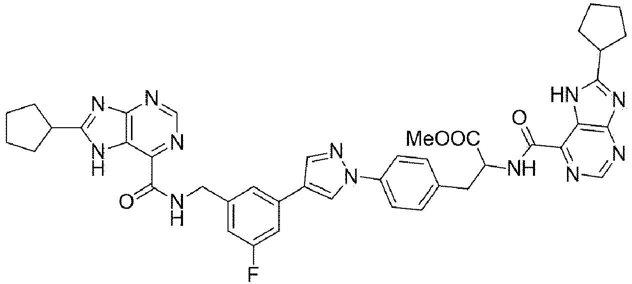

By the same method as in Example 87.3, methyl 2-amino-3-(4-(4-(3-(aminomethyl)-5-fluorophenyl)-1H-pyrazol-1-yl)phenyl)propanoate dihydrochloride (16 mg, 0.035 mmol), 8-cyclopentyl-7H-purine-6-carboxylic acid (8.13 mg, 0.035 mmol), EDC (8 mg, 0.042 mmol), HOBt (5.7 mg, 0.042 mmol), DIPEA (18 µL, 0.105 mmol) and THF (2 mL) were used to obtain the title compound (8.61 mg, 31%).
¹H NMR (400 MHz, CDCl₃) *δ* 10.42 (s, 1H), 10.30 (s, 1H), 9.03 (s, 2H), 8.46 (d, J = 8.0 Hz, 2H), 8.12 (s, 1H), 7.95 (s, 1H), 7.65 (d, J = 8.4 Hz, 2H), 7.31 (d, J = 8.0 Hz, 3H), 7.16 (d, J = 9.3 Hz, 1H), 6.99 (d, J = 8.6 Hz, 1H), 5.08 (dd, J = 14.1, 7.0 Hz, 1H), 4.72 (d, J = 6.3 Hz, 2H), 3.80 (s, 3H), 3.50 - 3.20 (m, 4H), 2.30 - 2.14 (m, 4H), 2.13 - 1.98 (m, 4H), 1.98 - 1.84 (m, 4H), 1.83 - 1.71 (m, 4H).
LC-MS, *m*/*z* 797 [M+H]⁺

### Example 151

### 151.1 3-Fluoro-5-((1-(methyl-d3)-1H-pyrazol-4-yl)amino)benzonitrile

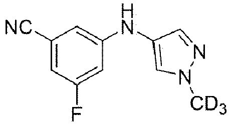

By the same method as in Example 10.1, 3-bromo-5-fluorobenzonitrile (0.14 g, 0.700 mmol), 1-(methyl-d3)-1H-pyrazol-4-amine (70 mg, 0.700 mmol), Pd₂(dba)₃ (12.9 mg, 0.014 mmol), Xantphos (145 mg, 0.035 mmol), and dioxane (4.4 mL) were used to obtain the title compound (0.137 g, 89.3%) as a red solid.
¹H NMR (400 MHz, DMSO-d6) *δ* 8.27 (s, 1H), 7.79 (s, 1H), 7.38 (d, *J =* 0.6 Hz, 1H), 6.99 - 6.91 (m, 1H), 6.89 - 6.84 (m, 1H), 6.77 (dt, *J =* 11.9, 2.3 Hz, 1H).
LC-MS, m/z 220 [M+H]⁺

### 151.2 N-(3-(aminomethyl)-5-fluorophenyl)-1-(methyl-d3)-1H-pyrazol-4-amine

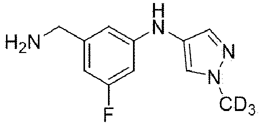

By the same method as in Example 7.2, 3-fluoro-5-((1-(methyl-*d*₃)-1H-pyrazol-4-yl)amino)benzonitrile (0.135 g, 0.616 mmol), LAH solution (2.0 M (in THF); 0.62 mL) and THF (6.2 mL) were used to obtain the title compound (0.150 g, quant.) as a red solid, which was used in the next reaction without purification.
¹H NMR (400 MHz, DMSO-d6) *δ* 7.78 (s, 1H), 7.72 (s, 1H), 7.36 (d, *J =* 0.6 Hz, 1H), 6.60 (s, 1H), 6.45 (d, *J* = 9.9 Hz, 1H), 6.36 (dt, *J =* 11.8, 2.2 Hz, 1H), 3.65 (s, 2H).
LC-MS, m/z 224 [M+H]⁺

### 151.3 8-Cyclopentyl-N-(3-fluoro-5-((1-(methyl-d3)-1H-pyrazol-4-yl)amino)benzyl)-7H-purine-6-carboxamide

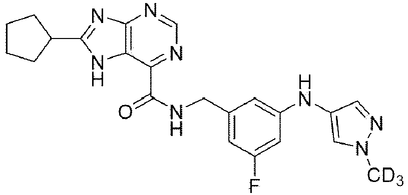

By the same method as in Example 87.3, using N-(3-(aminomethyl)-5-fluorophenyl)-1-(methyl-*d*₃)-1H-pyrazol-4-amine (60 mg, 0.269 mmol), 8-cyclopentyl-7H-purine-6-carboxylic acid (62 mg, 0.269 mmol), EDC (61.4 mg, 0.322 mmol), HOBt (43.3 mg, 0.322 mmol) and THF (6.2 mL), the title compound (72 mg, 61.6%) was obtained as a red solid.
¹H NMR (400 MHz, DMSO-d6) *δ* 13.18 (s, 1H), 9.68 (s, 1H), 8.96 (s, 1H), 7.80 (s, 1H), 7.63 (s, 1H), 7.29 (d, *J* = 0.7 Hz, 1H), 6.59 (s, 1H), 6.42 (d, *J* = 9.4 Hz, 1H), 6.34 (dt, *J* = 11.6, 2.1 Hz, 1H), 4.44 (d, *J* = 6.4 Hz, 2H), 3.55 - 3.45 (m, 1H), 2.09 (dt, *J* = 19.6, 5.7 Hz, 2H), 1.94 (dd, *J =* 12.1, 6.4 Hz, 2H), 1.82 (t, *J* = 8.5 Hz, 2H), 1.67 (dd, *J =* 7.0, 4.5 Hz, 2H).
LC-MS, m/z 438 [M+H]⁺

### Example 152

### 8-(3,3-Difluorocyclopentyl)-N-(3-fluoro-5-((1-(methyl-d3)-1H-pyrazol-4-yl)amino)benzyl)-7H-purine-6-carboxamide

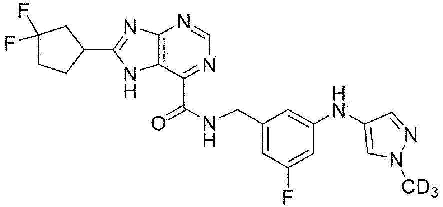

By the same method as in Example 87.3, using N-(3-(aminomethyl)-5-fluorophenyl)-1- (methyl-*d*₃)-1H-pyrazol-4-amine (88.5 mg, 0.396 mmol), 8-(3,3-difluorocyclopentyl)-7H-purine-6-carboxylic acid (106 mg, 0.396 mmol), EDC (91.1 mg, 0.476 mmol), HOBt (64.2 mg, 0.476 mmol), DIPEA (202 µL, 1.19 mmol) and THF (9.2 mL), the title compound (65 mg, 34.7%) was obtained as a light red solid.
¹H NMR (400 MHz, DMSO-*d6*) *δ* 13.34 (s, 1H), 9.72 (s, 1H), 9.00 (s, 1H), 7.80 (s, 1H), 7.63 (s, 1H), 7.29 (s, 1H), 6.59 (s, 1H), 6.42 (d, *J* = 9.3 Hz, 1H), 6.34 (dt, *J =* 11.8, 2.1 Hz, 1H), 4.44 (d, *J =* 6.3 Hz, 2H), 3.86 - 3.75 (m, 1H), 2.70 - 2.61 (m, 2H), 2.36 - 2.28 (m, 2H), 2.24 - 2.12 (m, 2H).
LC-MS, m/z 474 [M+H]⁺

### Example 153

### 8-(Cyclopentyl-2,2,3,3,4,4,5,5-d8)-N-(3-fluoro-5-(1-(4-fluorophenyl)-1H-pyrazol-4-yl)benzyl)-7H-purine-6-carboxamide

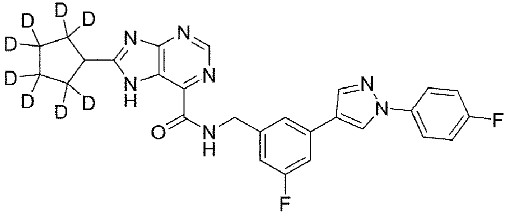

By the same method as in Example 72.5, 5,6-diamino-N-(3-fluoro-5-(1-(4-fluorophenyl)-1H-pyrazol-4-yl)benzyl)pyrimidine-4-carboxamide (80 mg, 0.1894 mmol), (cyclopentane-*d*₈)carboxylic acid (28 mg, 0.23 mmol), T3P (50 wt.% solution (in EtOAc), 0.57 mL, 0.95 mmol), DIPEA (64 µL, 0.38 mmol) and 1,4-dioxane (3 mL) were used to obtain the title compound (4.5 mg, 4.7%) as an off-white solid.
¹H NMR (400 MHz, CD₃OD) δ8.97 (s, 1H), 8.62 (s, 1H), 8.09 (s, 1H), 7.84 - 7.76 (m, 2H), 7.54 (s, 1H), 7.33 (d, J = 9.9 Hz, 1H), 7.29 - 7.19 (m, 2H), 7.05 (d, J = 8.9 Hz, 1H), 4.71 (s, 2H), 3.46 (s, 1H).
LC-MS, *m*/*z* 508 [M+H]⁺

### Experimental Example 1. Cell Culture Method

ARPE-19 cells (Human retinal pigment epithelial cells, ATCC CRL-2302) were cultured in DMEM/F12 (LM002-08, Welgene) medium containing 10% fetal bovine serum (FBS, 16000-044, Gibco) in an incubator at 37°C with 5% carbon dioxide. When the cells grew sufficiently, they were cultured in experimental culture dishes for protein isolation and medium supernatant acquisition.

Cells were detached from the culture dish using TrypLE^{™} Express (Gibco). After centrifugation at 1000 rpm for 3 minutes, the supernatant was removed. The cells were counted and seeded into new culture dishes. Normoxic conditions were maintained by culturing the cells in a 37°C incubator with 5% carbon dioxide, and hypoxic conditions were maintained by culturing the cells in a hypoxic incubator with 1% oxygen, 5% carbon dioxide, and 94% nitrogen gas.

### Experimental Example 2. Analysis of HIF-1α protein expression (Western blot)

In a disease of macular degeneration, hypoxia is a very important condition, and since the expression of downstream genes is regulated by HIF-1α, a transcription factor that is stabilized in hypoxic conditions, the following experiment was conducted to confirm this.

Mixed hypoxic condition was used for HIF-1α stability experiments, in which hypoxia was induced with 1% oxygen and the stability of HIF-1α was enhanced by treatment with cobalt chloride (CoCl₂).

The compounds of each Example were diluted to 0.1 nM, 1 nM, 3 nM, 10 nM, 30 nM, 100 nM, 300 nM, 1 µM, and 10 µM in DMEM/F12 medium without fetal bovine serum, pretreated for 16 hours or 2 hours, and cultured for 1 hour or 24 hours in a mixed hypoxic condition. The cell medium was discarded, and protein lysates were obtained by rapidly treating with protein lysis buffer (RIPA Lysis and Extraction buffer, Thermo Fisher Scientific). LDS (4X Bolt ^{™} LDS Sample buffer) and reducing agent (10X Bolt ^{™} Sample Reducing Agent) were added together to the protein lysate, and heated at 70°C for 10 minutes. The lysate was separated by electrophoresis on a 4-12% Bis-Tris gel, transferred to a PVDF membrane, and blocked with 3% BSA (Albumin, Bovine, Fraction V, MPbio). The membrane was incubated overnight at 4°C with a mixture of anti-HIF-1α (ab179483, abcam) and anti-b-actin (MA515739, Invitrogen) antibodies at a ratio of 1:4000. The following day, the membrane was treated with a peroxidase-conjugated secondary antibody for 1 hour at room temperature, and the target protein was expressed using an ECL solution (Clarity ^{™} Western ECL substrate, Bio-rad) and quantified.

Based on the results of the expression level of HIF-1α in cells treated with compounds diluted from 10 µM to 0.1 nM, the concentration range of the compound at which the expression level of HIF-1α was reduced by 50% or less compared to the untreated control group was derived.

As a result of the experiment, it was confirmed that in most of the tested Examples, the expression level of HIF-1α was suppressed by less than 50% at a concentration of less than 100 nM (see Table 1 below).

As a result of the experiment, in the groups treated with the compounds of Examples 11, 12, 31, 37, 42, 45, 47, 50, 80, 82, 83 and 84, the expression level of VEGF was suppressed to less than 50% at concentrations of less than 100 nM, confirming that these compounds suppress the expression of VEGF even in small amounts (see Table 1 below).

### Experimental Example 3. Evaluation of VEGF Protein Secretion Level

The expression of the downstream gene, angiogenesis inducer VEGF, is regulated by HIF-1α, a major transcription factor under hypoxic conditions, and the level of secreted VEGF protein can be quantitatively confirmed through ELISA (Enzyme Linked Immunosorbent Assay, Human VEGF Quantikine ELISA Kit, R&D systems). For this purpose, the following experiment was conducted.

The compounds of each Example were diluted to 0.1 nM, 1 nM, 3 nM, 10 nM, 30 nM, 100 nM, 300 nM, 1 µM and 10 µM in DMEM/F12 medium containing 1% fetal bovine serum, pretreated for 2 hours, and cultured for 24 hours under hypoxic conditions to obtain the cell medium supernatant. The supernatant was centrifuged at 1000 rpm for 3 minutes, and a specific amount was treated with a VEGF ELISA assay kit and the absorbance value was measured at 450 nm to quantitatively confirm the level of VEGF protein (see Table 1 below).

Based on the results of secreted VEGF protein expression levels in cell media treated with compounds diluted at concentrations ranging from 10 µM to 0.1 nM, the concentration range of the compound at which the secreted VEGF protein level was reduced by 50% or less compared to the untreated control group was derived.

As a result of the experiment, in the groups treated with the compounds of Examples 11, 12, 31, 37, 42, 45, 47, 50, 80, 82, 83 and 84, the secreted VEGF protein level was suppressed to less than 50% at concentrations of less than 100 nM, confirming that these compounds suppress the expression of VEGF even in small amounts (see Table 1 below).

**[Table 1]**

| Example | IC₅₀ | |
|---|---|---|
| | HIF-1α Hypoxia (1 hour) | VEGF |
| 1 | ++++ | +++ |
| 2 | ++++ | +++ |
| 3 | ++++ | +++ |
| 4 | +++ | ++ |
| 5 | +++ | ++ |
| 6 | +++ | - |
| 7 | ++++ | +++ |
| 8 | ++++ | +++ |
| 9 | ++++ | +++ |
| 10 | ++++ | +++ |
| 11 | ++++ | ++++ |
| 12 | ++++ | ++++ |
| 13 | ++++ | +++ |
| 14 | +++ | - |
| 15 | ++++ | +++ |
| 16 | ++++ | +++ |
| 17 | ++++ | +++ |
| 18 | ++++ | +++ |
| 19 | ++++ | +++ |
| 20 | ++++ | +++ |
| 21 | ++++ | +++ |
| 22 | +++ | - |
| 23 | ++++ | +++ |
| 24 | ++++ | +++ |
| 25 | ++++ | +++ |
| 26 | ++++ | +++ |
| 27 | ++++ | +++ |
| 28 | ++++ | +++ |
| 29 | ++++ | +++ |
| 30 | ++++ | +++ |
| 31 | ++++ | ++++ |
| 32 | ++++ | +++ |
| 33 | ++++ | +++ |
| 34 | ++++ | +++ |
| 35 | ++++ | +++ |
| 36 | ++++ | +++ |
| 37 | ++++* | ++++ |
| 38 | ++++ | +++ |
| 39 | +++ | +++ |
| 40 | ++++* | +++ |
| 41 | ++++ | +++ |
| 42 | ++++* | ++++ |
| 43 | ++++ | +++ |
| 44 | ++++ | +++ |
| 45 | ++++* | ++++ |
| 46 | ++++* | +++ |
| 47 | ++++* | ++++ |
| 48 | ++++ | ++ |
| 49 | ++++ | ++ |
| 50 | ++++* | ++++ |
| 51 | ++++ | +++ |
| 52 | ++++ | +++ |
| 53 | ++++* | +++ |
| 54 | ++++ | +++ |
| 55 | +++ | - |
| 56 | ++++* | +++ |
| 57 | ++++ * | +++ |
| 58 | ++++* | +++ |
| 59 | +++* | +++ |
| 60 | ++++ * | +++ |
| 61 | +++ | - |
| 62 | ++ | - |
| 63 | +++ | ++ |
| 64 | ++ | - |
| 65 | ++ | - |
| 66 | ++++ | ++ |
| 67 | ++ | - |
| 68 | ++ | - |
| 69 | +++ | - |
| 70 | +++ | - |
| 71 | ++ | - |
| 72 | ++ | - |
| 73 | ++ | - |
| 74 | ++ | - |
| 75 | +++ | - |
| 76 | ++ | - |
| 77 | +++ | ++ |
| 78 | ++++* | +++ |
| 79 | ++ | - |
| 80 | ++++* | ++++ |
| 81 | ++++* | +++ |
| 82 | ++++ * | ++++ |
| 83 | ++++* | ++++ |
| 84(E1) | ++++* | ++++ |
| 84(E2) | ++++* | ++++ |
| 85 | ++++* | ++ |
| 86 | +++* | +++ |
| 87 | ++++* | +++ |
| 88 | +++* | +++ |
| 89 | +++* | - |
| 90 | +++* | - |
| 91 | ++* | - |
| 92 | +++* | +++ |
| 93 | ++++* | +++ |
| 94 | +++* | - |
| 95 | +++* | +++ |
| 96 | +++* | - |
| 97 | +++* | +++ |
| 98 | ++* | + |
| 99 | ++* | + |
| 100 | ++* | ++ |
| 101 | +++* | ++ |
| 102(A) | ++* | + |
| 102(B) | +++* | +++ |
| 103(A) | +* | + |
| 103(B) | +* | + |
| 104 | ++++* | +++ |
| 105 | +* | + |
| 106 | +* | + |
| 107 | +* | + |
| 108 | +* | + |
| 109 | +* | + |
| 110(A) | ++++* | +++ |
| 110(B) | ++++* | +++ |
| 111 | ++++* | +++ |
| 112 | +* | + |
| 113 | ++++* | +++ |
| 114 | +++* | +++ |
| 115 | +++* | +++ |
| 116 | +++* | + |
| 117 | ++++* | +++ |
| 118 | ++++* | +++ |
| 119 | +++* | + |
| 120 | +++* | + |
| 121 | +++* | + |
| 122 | ++++* | ++++ |
| 123 | ++++* | ++++ |
| 124 | ++++* | +++ |
| 125 | ++++* | +++ |
| 126 | ++++* | ++++ |
| 127 | +++* | + |
| 128 | ++++* | ++++ |
| 129 | ++* | + |
| 130 | ++++* | +++ |
| 131 | ++* | + |
| 132 | ++* | + |
| 133 | +++* | + |
| 134 | +++* | + |
| 135 | +++* | + |
| 136 | +++* | + |
| 137 | +++* | + |
| 138 | ++++* | +++ |
| 139 | ++++* | ++++ |
| 140 | ++++* | + |
| 141 | ++++* | ++++ |
| 142 | +++* | + |
| 143 | ++* | + |
| 144 | ++++* | +++ |
| 145 | ++++* | +++ |
| 146 | +++* | +++ |
| 147 | +++* | +++ |
| 148 | ++++* | +++ |
| 149 | +++* | + |
| 150 | +++* | + |
| 151 | ++++* | +++ |
| 152 | ++++* | +++ |
| 153 | ++++* | +++ |

| | | |
|---|---|---|
| * HIF-1α, Hypoxia(24h)++++: <100 nM +++: 100 nM~1 µM ++: 1 µM~10 µM +: >10 µM | | |

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. The scope of the invention is indicated by the following patent claims. The meaning and scope of the patent claims and all modifications or variations derived from their equivalents are considered to be falling within the scope of the invention.

## Claims

1. A compound of the following Formula I, a hydrate thereof, a solvate thereof, a prodrug thereof, an isomer thereof, or a pharmaceutically acceptable salt thereof, wherein: 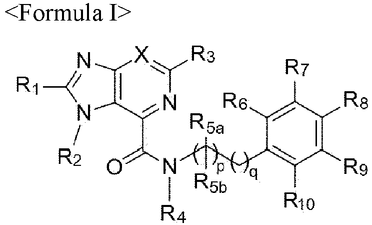
X is C or N,
R₁ is hydrogen, amino-C₁₋₃ alkyl, cycloalkyl of 1 to 2 rings of C₃₋₈ (wherein hydrogen is protium or deuterium), or Rₛ, R₁ is substituted with one or more independent R₁ₐ or not,
wherein R₁ₐ is C₁₋₅ alkyl, halogen, oxo, or -C(O)OR_{1b}, and R_{1b} is hydrogen, C₁₋₃ alkyl, or arylalkyl,
R₂ is hydrogen, C₁₋₃ alkyl or triC₁₋₃ alkyl-silyl-C₁₋₃ alkoxy-C₁₋₃ alkyl,
R₃ is hydrogen, C₁₋₃ alkyl or halogen,
R₄ is hydrogen or C₁₋₃ alkyl,
p and q are independently integers 0 or 1,
when p is 1, R₅ₐ and R_{5b} is independently hydrogen or C₁₋₃ alkyl, or R₅ₐ and R_{5b} are connected to each other to form a C₃₋₆ ring,
R₆ is hydrogen or halogen,
R₇ is hydrogen, halogen, Rₛ, Rᵤ, -NH-aryl, -NH-cycloC₃₋₇alkyl, -NHRₛ, -NHRᵤ or - NHC(O)Rᵤ,
R₈ is hydrogen, halogen, or a 4- to 6-membered heteroaryl having 1 to 3 of N,
R₇ and R₈ are independently substituted with one or more independent Rₘ or not,
Rₘ is C₁₋₃ alkyl (wherein hydrogen is protium or deuterium), C₃₋₁₀ cycloalkyl, C₁₋₃ alkoxy, sulfonyl, amino, halogen, cyano, haloalkyl, carbamoyl, carboxyl, aryl (wherein hydrogen is protium or deuterium), arylalkyl, Rᵤ, or Rₛ, Rₘ is substituted with one or more independent Rₙ or not,
Rₙ is C₁₋₃ alkyl, C₁₋₃ alkylsulfonyl, C₁₋₃ alkoxy, halogen, cyano, haloalkyl, carbamoyl, carboxyl, acetyl, aryl, arylalkyl, oxido, or alkylcarboxyl-(8-cycloalkyl-7H-carboxamido)-alkyl,
R₉ and R₁₀ are independently hydrogen or halogen,
Rₛ is a 4- to 8-membered saturated heterocycloalkyl having 1 to 2 heteroatoms selected from N and O,
Rᵤ is a 4- to 6-membered heteroaryl having 1 to 3 heteroatoms selected from N, O and S.

2. The compound, a hydrate thereof, a solvate thereof, a prodrug thereof, an isomer thereof, or a pharmaceutically acceptable salt thereof of Claim 1,
wherein R₁ is hydrogen, aminomethyl, cyclobutyl, cyclopentyl (wherein hydrogen is protium or deuterium), spiro[2.3]hexanyl, spiro[3.3]heptanyl, bicyclo[1.1.1]pentanyl, bicyclo[3.1.0]hexanyl, bicyclo[2.1.1]hexanyl, bicyclo[2.2.1]heptanyl, pyrrolidinyl, tetrahydrofuranyl, or piperidinyl, and
R₁ₐ is methyl, propyl, isopropyl, fluoro, oxo, -C(O)OH, -C(O)O-methyl, or -C(O)O-benzyl.

3. The compound, a hydrate thereof, a solvate thereof, a prodrug thereof, an isomer thereof, or a pharmaceutically acceptable salt thereof of Claim 1, wherein R₂ is hydrogen, methyl or trimethyl-silyl-ethoxy-methyl.

4. The compound, a hydrate thereof, a solvate thereof, a prodrug thereof, an isomer thereof, or a pharmaceutically acceptable salt thereof of Claim 1, wherein R₃ is hydrogen, methyl, ethyl or chloro.

5. The compound, a hydrate thereof, a solvate thereof, a prodrug thereof, an isomer thereof, or a pharmaceutically acceptable salt thereof of Claim 1, wherein R₄ is hydrogen or methyl.

6. The compound, a hydrate thereof, a solvate thereof, a prodrug thereof, an isomer thereof, or a pharmaceutically acceptable salt thereof of Claim 1, wherein R₅ₐ and R_{5b} are independently hydrogen or methyl, or R₅ₐ and R_{5b} are connected to each other to form cyclopropyl.

7. The compound, a hydrate thereof, a solvate thereof, a prodrug thereof, an isomer thereof, or a pharmaceutically acceptable salt thereof of Claim 1, wherein R₆ is hydrogen or fluoro.

8. The compound, a hydrate thereof, a solvate thereof, a prodrug thereof, an isomer thereof, or a pharmaceutically acceptable salt thereof of Claim 1, wherein R₇ is hydrogen, Br, piperazinyl, diazepanyl, pyrrolyl, furanyl, thiophenyl, pyrazolyl, isothiazolyl, triazolyl, oxadiazolyl, -NH-phenyl, -NH-cyclopentyl, -NH-piperidinyl, -NH-pyrrolyl, -NH-thiophenyl, - NH-pyrazolyl, -NH-isoxazolyl, -NH-oxadiazolyl, -NH-thiadiazolyl, -NH-pyridinyl, or - NHC(O)-pyrazolyl.

9. The compound, a hydrate thereof, a solvate thereof, a prodrug thereof, an isomer thereof, or a pharmaceutically acceptable salt thereof of Claim 1, wherein R₈ is hydrogen, fluoro, chloro, or pyrazolyl.

10. The compound, a hydrate thereof, a solvate thereof, a prodrug thereof, an isomer thereof, or a pharmaceutically acceptable salt thereof of Claim 1,
wherein Rₘ is methyl (wherein hydrogen is protium or deuterium), methoxy, sulfonyl, amino, fluoro, chloro, cyano, trifluoromethyl, difluoroethyl, trifluoroethyl, carbamoyl, carboxyl, cyclopentyl, cyclohexyl, phenyl (wherein hydrogen is protium or deuterium), benzyl, thiophenyl, furanyl, pyrazolyl, thiazolyl, pyridinyl, pyrimidinyl, or tetrahydrofuranyl,
Rₙ is methyl, ethyl, methylsulfonyl, methoxy, fluoro, chloro, iodo, bromo, cyano, trifluoromethyl, carbamoyl, carboxyl, acetyl, phenyl, benzyl, oxido, or methylcarboxyl-(8-cyclopentyl-7H-purine-6-carboxamido)-ethyl.

11. The compound, a hydrate thereof, a solvate thereof, a prodrug thereof, an isomer thereof, or a pharmaceutically acceptable salt thereof of Claim 1, wherein R₉ is hydrogen, fluoro or chloro.

12. The compound, a hydrate thereof, a solvate thereof, a prodrug thereof, an isomer thereof, or a pharmaceutically acceptable salt thereof of Claim 1, wherein R₁₀ is hydrogen or fluoro.

13. The compound, a hydrate thereof, a solvate thereof, a prodrug thereof, an isomer thereof, or a pharmaceutically acceptable salt thereof of Claim 1, wherein the compound represented by the Formula I is any one selected from the group consisting of:
[1] 8-cyclopentyl-N-(3-fluoro-5-(1-(trifluoromethyl)-1H-pyrazol-4-yl)benzyl)-7H-purine-6-carboxamide,
[2] 8-cyclopentyl-N-(3-(1-(2,2-difluoroethyl)-1H-pyrazol-4-yl)-5-fluorobenzyl)-7H-purine-6-carboxamide,
[3] 8-cyclopentyl-N-(3-fluoro-5-(1-(2,2,2-trifluoroethyl)-1H-pyrazol-4-yl)benzyl)-7H-purine-6-carboxamide,
[4] (S)-8-cyclopentyl-N-(1-(3-(1-methyl-1H-pyrazol-4-yl)phenyl)ethyl)-7H-purine-6-carboxamide,
[5] 8-cyclopentyl-N-(3-fluoro-5-(1-methyl-1H-pyrazole-4-carboxamido)benzyl)-7H-purine-6-carboxamide,
[6] 8-cyclopentyl-N-(1-(3-(1-methyl-1H-pyrazol-4-yl)phenyl)cyclopropyl)-7H-purine-6-carboxamide,
[7] 8-cyclopentyl-N-(3-fluoro-5-(1-phenyl-1H-pyrazol-4-yl)benzyl)-7H-purine-6-carboxamide,
[8] 8-cyclopentyl-N-(3-fluoro-5-((1-methyl-1H-pyrazol-4-yl)amino)benzyl)-7H-purine-6-carboxamide,
[9] N-(3-(1-benzyl-1H-pyrazol-4-yl)-5-fluorobenzyl)-8-cyclopentyl-7H-purine-6-carboxamide,
[10] 8-cyclopentyl-N-(3-fluoro-5-(pyridin-3-ylamino)benzyl)-7H-purine-6-carboxamide,
[11] 8-cyclopentyl-N-(3-fluoro-5-(1H-pyrazol-4-yl)benzyl)-7H-purine-6-carboxamide,
[12] 8-cyclopentyl-N-(3-fluoro-5-(1-(pyridin-4-yl)-1H-pyrazol-4-yl)benzyl)-7H-purine-6-carboxamide,
[13] 8-cyclopentyl-N-(3-fluorobenzyl)-7H-purine-6-carboxamide,
[14] N-(3-bromo-5-fluorobenzyl)-8-cyclopentyl-7H-purine-6-carboxamide,
[15] 8-cyclopentyl-N-(3-fluoro-5-(phenylamino)benzyl)-7H-purine-6-carboxamide,
[16] 8-cyclopentyl-N-(3-fluoro-5-(pyridin-4-ylamino)benzyl)-7H-purine-6-carboxamide,
[17] 8-cyclopentyl-N-(3-fluoro-5-((3-(trifluoromethyl)phenyl)amino)benzyl)-7H-purine-6-carboxamide,
[18] 8-cyclopentyl-N-(3-((4-(dimethylamino)phenyl)amino)-5-fluorobenzyl)-7H-purine-6-carboxamide,
[19] 8-cyclopentyl-N-(3-fluoro-5-((4-(methylsulfonyl)phenyl)amino)benzyl)-7H-purine-6-carboxamide,
[20] N-(3-((4-chloro-2-fluorophenyl)amino)-5-fluorobenzyl)-8-cyclopentyl-7H-purine-6-carboxamide,
[21] 8-cyclopentyl-N-(3-fluoro-5-((4-fluorophenyl)amino)benzyl)-7H-purine-6-carboxamide,
[22] N-(3-(1-(3-cyanophenyl)-1H-pyrazol-4-yl)-5-fluorobenzyl)-8-cyclopentyl-7-((2-(trimethylsilyl)ethoxy)methyl)-7H-purine-6-carboxamide,
[23] N-(3-(1-(3-cyanophenyl)-1H-pyrazol-4-yl)-5-fluorobenzyl)-8-cyclopentyl-7H-purine-6-carboxamide,
[24] 8-cyclopentyl-N-(3-fluoro-5-(1-(4-(methylsulfonyl)benzyl)-1H-pyrazol-4-yl)benzyl)-7H-purine-6-carboxamide,
[25] 8-cyclopentyl-N-(3-fluoro-5-(1-(4-methoxyphenyl)-1H-pyrazol-4-yl)benzyl)-7H-purine-6-carboxamide,
[26] 8-cyclopentyl-N-(3-fluoro-5-(1-(4-(trifluoromethyl)phenyl)-1H-pyrazol-4-yl)benzyl)-7H-purine-6-carboxamide,
[27] 8-cyclopentyl-N-(3-fluoro-5-(1-(4-fluorophenyl)-1H-pyrazol-4-yl)benzyl)-7H-purine-6-carboxamide,
[28] 8-cyclopentyl-N-(3-fluoro-5-(1-(4-(methylsulfonyl)phenyl)-1H-pyrazol-4-yl)benzyl)-7H-purine-6-carboxamide,
[29] 8-cyclopentyl-N-(3-fluoro-5-(1-(3-(trifluoromethyl)benzyl)-1H-pyrazol-4-yl)benzyl)-7H-purine-6-carboxamide,
[30] 8-cyclopentyl-N-(3-fluoro-5-(1-(pyridin-2-yl)-1H-pyrazol-4-yl)benzyl)-7H-purine-6-carboxamide,
[31] 8-cyclopentyl-N-(3-fluoro-5-(1-(pyridin-3-yl)-1H-pyrazol-4-yl)benzyl)-7H-purine-6-carboxamide,
[32] 8-cyclopentyl-N-(3-fluoro-5-(1-(pyrimidin-5-yl)-1H-pyrazol-4-yl)benzyl)-7H-purine-6-carboxamide,
[33] 8-cyclopentyl-N-(3-fluoro-5-((2-methoxyphenyl)amino)benzyl)-7H-purine-6-carboxamide,
[34] 8-cyclopentyl-N-(3-fluoro-5-((3-methoxyphenyl)amino)benzyl)-7H-purine-6-carboxamide,
[35] 8-cyclopentyl-N-(3-fluoro-5-((4-methoxyphenyl)amino)benzyl)-7H-purine-6-carboxamide,
[36] 8-cyclopentyl-N-(3-fluoro-5-((4-methoxyphenyl)amino)benzyl)-7-methyl-7H-purine-6-carboxamide,
[37] N-(3-(1-(3-carbamoylphenyl)-1H-pyrazol-4-yl)-5-fluorobenzyl)-8-cyclopentyl-7H-purine-6-carboxamide,
[38] 8-cyclopentyl-N-(3-fluoro-5-(isothiazol-4-yl)benzyl)-7H-purine-6-carboxamide,
[39] 8-cyclopentyl-N-(3-fluoro-5-(furan-2-yl)benzyl)-7H-purine-6-carboxamide,
[40] 8-cyclopentyl-N-(3-fluoro-5-(thiophen-3-yl)benzyl)-7H-purine-6-carboxamide,
[41] 8-cyclopentyl-N-(3-fluoro-5-((4-(trifluoromethyl)phenyl)amino)benzyl)-7H-purine-6-carboxamide,
[42] 8-cyclopentyl-N-(3-fluoro-5-(1-(2-methylpyridin-4-yl)-1H-pyrazol-4-yl)benzyl)-7H-purine-6-carboxamide,
[43] 8-cyclopentyl-N-(3-fluoro-5-(1-(6-methoxypyridin-3-yl)-1H-pyrazol-4-yl)benzyl)-7H-purine-6-carboxamide,
[44] 8-cyclopentyl-N-(3-fluoro-5-(1-(6-(trifluoromethyl)pyridin-3-yl)-1H-pyrazol-4-yl)benzyl)-7H-purine-6-carboxamide,
[45] 8-cyclopentyl-N-(3-fluoro-5-(1-(3-(methylsulfonyl)phenyl)-1H-pyrazol-4-yl)benzyl)-7H-purine-6-carboxamide,
[46] 8-cyclopentyl-N-(3-fluoro-5-(1-(3-fluorophenyl)-1H-pyrazol-4-yl)benzyl)-7H-purine-6-carboxamide,
[47] 8-cyclopentyl-N-(3-fluoro-5-(1-(6-fluoropyridin-3-yl)-1H-pyrazol-4-yl)benzyl)-7H-purine-6-carboxamide,
[48] 8-cyclopentyl-N-(3-fluoro-5-(1-(5-iodopyridin-2-yl)-1H-pyrazol-4-yl)benzyl)-7H-purine-6-carboxamide,
[49] 3-(4-(3-((8-cyclopentyl-7H-purine-6-carboxamido)methyl)-5-fluorophenyl)-1H-pyrazol-1-yl)benzoic acid,
[50] N-(3-(1-(4-cyanophenyl)-1H-pyrazol-4-yl)-5-fluorobenzyl)-8-cyclopentyl-7H-purine-6-carboxamide,
[51] 8-cyclopentyl-N-(3-fluoro-5-(1-(4-fluorophenyl)-1H-pyrazol-3-yl)benzyl)-7H-purine-6-carboxamide,
[52] 8-cyclopentyl-N-(3-fluoro-5-(1-(4-fluorophenyl)-1H-pyrrol-3-yl)benzyl)-7H-purine-6-carboxamide,
[53] N-(3-(1-(4-carbamoylphenyl)-1H-pyrazol-4-yl)-5-fluorobenzyl)-8-cyclopentyl-7H-purine-6-carboxamide,
[54] 4-(4-(3-((8-cyclopentyl-7H-purine-6-carboxamido)methyl)-5-fluorophenyl)-1H-pyrazol-1-yl)benzoic acid,
[55] 8-cyclopentyl-N-(3-fluoro-5-(1-(4-fluorophenyl)-1H-pyrazol-4-yl)benzyl)-7-methyl-7H-purine-6-carboxamide,
[56] 8-cyclopentyl-N-(2-fluoro-5-(1-(4-fluorophenyl)-1H-pyrazol-4-yl)benzyl)-7H-purine-6-carboxamide,
[57] 8-cyclopentyl-N-(3-(1-(4-fluorophenyl)-1H-pyrazol-4-yl)benzyl)-7H-purine-6-carboxamide,
[58] N-(3-(1-(4-chlorophenyl)-1H-pyrazol-4-yl)-5-fluorobenzyl)-8-cyclopentyl-7H-purine-6-carboxamide,
[59] N-(3-(1-(4-bromo-2-(methylsulfonyl)phenyl)-1H-pyrazol-4-yl)-5-fluorobenzyl)-8-cyclopentyl-7H-purine-6-carboxamide,
[60] 8-cyclopentyl-N-(3-(1-(3,4-difluorophenyl)-1H-pyrazol-4-yl)-5-fluorobenzyl)-7H-purine-6-carboxamide,
[61] 2-chloro-8-cyclopentyl-N-(3-fluoro-5-(1-(4-fluorophenyl)-1H-pyrazol-4-yl)benzyl)-7H-purine-6-carboxamide,
[62] (S)-N-(3-fluoro-5-(1-methyl-1H-pyrazol-4-yl)benzyl)-8-(pyrrolidin-2-yl)-7H-purine-6-carboxamide hydrochloride,
[63] (S)-N-(3-fluoro-5-(1-methyl-1H-pyrazol-4-yl)benzyl)-8-(1-methylpyrrolidin-2-yl)-7H-purine-6-carboxamide,
[64] benzyl (R)-2-(6-((3-fluoro-5-(1-methyl-1H-pyrazol-4-yl)benzyl)carbamoyl)-7H-purin-8-yl)pyrrolidine-1-carboxylate,
[65] (R)-N-(3-fluoro-5-(1-methyl-1H-pyrazol-4-yl)benzyl)-8-(pyrrolidin-2-yl)-7H-purine-6-carboxamide hydrochloride,
[66] (R)-N-(3-fluoro-5-(1-methyl-1H-pyrazol-4-yl)benzyl)-8-(1-methylpyrrolidin-2-yl)-7H-purine-6-carboxamide,
[67] (S)-N-(3-fluoro-5-(1-methyl-1H-pyrazol-4-yl)benzyl)-8-(1-isopropylpyrrolidin-2-yl)-7H-purine-6-carboxamide,
[68] (R)-N-(3-fluorobenzyl)-8-(1-methylpyrrolidin-2-yl)-7H-purine-6-carboxamide,
[69] benzyl 4-(6-((3-fluoro-5-(1-methyl-1H-pyrazol-4-yl)benzyl)carbamoyl)-7H-purin-8-yl)piperidine-1-carboxylate,
[70] N-(3-fluoro-5-(1-methyl-1H-pyrazol-4-yl)benzyl)-8-(piperidin-4-yl)-7H-purine-6-carboxamide hydrochloride,
[71] N-(3-fluoro-5-(1-methyl-1H-pyrazol-4-yl)benzyl)-8-(1-methylpiperidin-4-yl)-7H-purine-6-carboxamide,
[72] benzyl ((6-((3-fluoro-5-(1-methyl-1H-pyrazol-4-yl)benzyl)carbamoyl)-7H-purin-8-yl)methyl)carbamate,
[73] 8-(aminomethyl)-N-(3-fluoro-5-(1-methyl-1H-pyrazol-4-yl)benzyl)-7H-purine-6-carboxamide,
[74] N-(3-fluoro-5-(1-methyl-1H-pyrazol-4-yl)benzyl)-7H-purine-6-carboxamide,
[75] benzyl 3-(6-((3-fluoro-5-(1-methyl-1H-pyrazol-4-yl)benzyl)carbamoyl)-7H-purin-8-yl)pyrrolidine-1-carboxylate,
[76] N-(3-fluoro-5-(1-methyl-1H-pyrazol-4-yl)benzyl)-8-(pyrrolidin-3-yl)-7H-purine-6-carboxamide hydrochloride,
[77] 8-(6,6-difluorobicyclo[3.1.0]hexan-3-yl)-N-(3-fluoro-5-(1-methyl-1H-pyrazol-4-yl)benzyl)-7H-purine-6-carboxamide,
[78] 8-(bicyclo[3.1.0]hexan-3-yl)-N-(3-fluoro-5-(1-methyl-1H-pyrazol-4-yl)benzyl)-7H-purine-6-carboxamide,
[79] N-(3-fluoro-5-(1-methyl-1H-pyrazol-4-yl)benzyl)-8-(1-methylpyrrolidin-3-yl)-7H-purine-6-carboxamide,
[80] 8-(3,3-difluorocyclopentyl)-N-(3-fluoro-5-(1-(4-fluorophenyl)-1H-pyrazol-4-yl)benzyl)-7H-purine-6-carboxamide,
[81] 8-(3,3-difluorocyclopentyl)-N-(3-fluoro-5-(1-(3-fluorophenyl)-1H-pyrazol-4-yl)benzyl)-7H-purine-6-carboxamide,
[82] 8-(3,3-difluorocyclopentyl)-N-(3-fluoro-5-(1-(6-fluoropyridin-3-yl)-1H-pyrazol-4-yl)benzyl)-7H-purine-6-carboxamide,
[83] 8-(3,3-difluorocyclopentyl)-N-(3-fluoro-5-(1-(pyridin-3-yl)-1H-pyrazol-4-yl)benzyl)-7H-purine-6-carboxamide,
[84] (E1, E2) 8-(3,3-difluorocyclopentyl)-N-(3-fluoro-5-(1-(4-fluorophenyl)-1H-pyrazol-4-yl)benzyl)-7H-purine-6-carboxamide,
[85] (R)-N-(3-fluoro-5-(1-(4-fluorophenyl)-1H-pyrazol-4-yl)benzyl)-8-(tetrahydrofuran-2-yl)-7H-purine-6-carboxamide,
[86] (R)-N-(3-fluoro-5-(1-(4-fluorophenyl)-1H-pyrazol-4-yl)benzyl)-8-(3-oxocyclopentyl)-7H-purine-6-carboxamide,
[87] N-(3-(1-(3-chloro-4-fluorophenyl)-1H-pyrazol-4-yl)-5-fluorobenzyl)-8-cyclopentyl-7H-purine-6-carboxamide,
[88] (S)-N-(3-fluoro-5-(1-(4-fluorophenyl)-1H-pyrazol-4-yl)benzyl)-8-(3-oxocyclopentyl)-7H-purine-6-carboxamide,
[89] N-(3-fluoro-5-(1-(4-fluorophenyl)-1H-pyrazol-4-yl)benzyl)-8-(tetrahydrofuran-3-yl)-7H-purine-6-carboxamide,
[90] (R)-8-cyclopentyl-N-(1-(3-(1-(4-fluorophenyl)-1H-pyrazol-4-yl)phenyl)ethyl)-7H-purine-6-carboxamide,
[91] (S)-8-cyclopentyl-N-(1-(3-(1-(4-fluorophenyl)-1H-pyrazol-4-yl)phenyl)ethyl)-7H-purine-6-carboxamide
[92] 8-(3,3-difluorocyclobutyl)-N-(3-fluoro-5-(1-(4-fluorophenyl)-1H-pyrazol-4-yl)benzyl)-7H-purine-6-carboxamide,
[93] 2-cyclopentyl-N-(3-fluoro-5-(1-(4-fluorophenyl)-1H-pyrazol-4-yl)benzyl)-3H-imidazo[4,5-c]pyridine-4-carboxamide,
[94] 8-cyclopentyl-N-(3-(1-(4-fluorophenyl)-1H-pyrazol-4-yl)phenethyl)-7H-purine-6-carboxamide,
[95] 8-cyclopentyl-N-(2-fluoro-3-(1-(4-fluorophenyl)-1H-pyrazol-4-yl)benzyl)-7H-purine-6-carboxamide,
[96] 8-cyclopentyl-N-(4-fluoro-3-(1-(4-fluorophenyl)-1H-pyrazol-4-yl)benzyl)-7H-purine-6-carboxamide,
[97] 8-(3,3-dimethylcyclobutyl)-N-(3-fluoro-5-(1-(4-fluorophenyl)-1H-pyrazol-4-yl)benzyl)-7H-purine-6-carboxamide,
[98] 8-(6,6-difluorospiro[3.3]heptan-2-yl)-N-(3-fluoro-5-(1-(4-fluorophenyl)-1H-pyrazol-4-yl)benzyl)-7H-purine-6-carboxamide,
[99] 8-(bicyclo[1.1.1]pentan-1-yl)-N-(3-fluoro-5-(1-(4-fluorophenyl)-1H-pyrazol-4-yl)benzyl)-7H-purine-6-carboxamide,
[100] N-(3-fluoro-5-(1-(4-fluorophenyl)-1H-pyrazol-4-yl)benzyl)-8-(spiro[3.3]heptan-2-yl)-7H-purine-6-carboxamide,
[101] N-(3-fluoro-5-(1-(4-fluorophenyl)-1H-pyrazol-4-yl)benzyl)-8-(3-fluorobicyclo[1.1.1]pentan-1-yl)-7H-purine-6-carboxamide,
[102] (A, B) N-(3-fluoro-5-(1-(4-fluorophenyl)-1H-pyrazol-4-yl)benzyl)-8-(3-fluorocyclobutyl)-7H-purine-6-carboxamide,
[103] (A, B) methyl 3-(6-((3-fluoro-5-(1-(4-fluorophenyl)-1H-pyrazol-4-yl)benzyl)carbamoyl)-7H-purin-8-yl)cyclobutanecarboxylate,
[104] N-(3-chloro-5-(1-(4-fluorophenyl)-1H-pyrazol-4-yl)benzyl)-8-cyclopentyl-7H-purine-6-carboxamide,
[105] 8-cyclopentyl-N-(3-fluoro-5-(1-(4-fluorophenyl)-1H-pyrazol-4-yl)phenyl)-7H-purine-6-carboxamide,
[106] 3-(6-((3-fluoro-5-(1-(4-fluorophenyl)-1H-pyrazol-4-yl)benzyl)carbamoyl)-7H-purin-8-yl)cyclobutanecarboxylic acid,
[107] 2-cyclopentyl-N-(3-fluoro-5-(1-(4-fluorophenyl)-1H-pyrazol-4-yl)benzyl)-1H-imidazo[4,5-b]pyridine-7-carboxamide,
[108] 8-cyclopentyl-N-(4-(1-(4-fluorophenyl)-1H-pyrazol-4-yl)benzyl)-7H-purine-6-carboxamide,
[109] 8-cyclopentyl-N-(3-fluoro-4-(1-(4-fluorophenyl)-1H-pyrazol-4-yl)benzyl)-7H-purine-6-carboxamide,
[110] (A, B) N-(3-fluoro-5-(1-(4-fluorophenyl)-1H-pyrazol-4-yl)benzyl)-8-(3-fluorocyclopentyl)-7H-purine-6-carboxamide,
[111] N-(3-fluoro-5-(1-(4-fluorophenyl)-1H-pyrazol-4-yl)benzyl)-8-(spiro[2.3]hexan-5-yl)-7H-purine-6-carboxamide,
[112] 8-cyclopentyl-N-(3-fluoro-5-(1-(4-fluorophenyl)-1H-pyrazol-4-yl)benzyl)-N-methyl-7H-purine-6-carboxamide,
[113] 8-(bicyclo[3.1.0]hexan-3-yl)-N-(3-fluoro-5-(1-(4-fluorophenyl)-1H-pyrazol-4-yl)benzyl)-7H-purine-6-carboxamide,
[114] 8-(bicyclo[2.2.1]heptan-2-yl)-N-(3-fluoro-5-(1-(4-fluorophenyl)-1H-pyrazol-4-yl)benzyl)-7H-purine-6-carboxamide,
[115] 8-(bicyclo[2.1.1]hexan-1-yl)-N-(3-fluoro-5-(1-(4-fluorophenyl)-1H-pyrazol-4-yl)benzyl)-7H-purine-6-carboxamide,
[116] 8-cyclopentyl-N-(3-fluoro-5-(4-(4-fluorophenyl)-1H-1,2,3-triazol-1-yl)benzyl)-7H-purine-6-carboxamide,
[117] 8-cyclopentyl-N-(3-fluoro-5-(1-(thiophen-3-yl)-1H-pyrazol-4-yl)benzyl)-7H-purine-6-carboxamide,
[118] 8-cyclopentyl-N-(3-fluoro-5-((1-methyl-1H-pyrazol-3-yl)amino)benzyl)-7H-purine-6-carboxamide,
[119] 8-cyclopentyl-N-(3-fluoro-5-(1-(4-fluorophenyl)-1H-1,2,3-triazol-4-yl)benzyl)-7H-purine-6-carboxamide,
[120] N-(3-((1H-pyrazol-3-yl)amino)-5-fluorobenzyl)-8-cyclopentyl-7H-purine-6-carboxamide,
[121] 8-cyclopentyl-N-(3-fluoro-5-(isoxazol-3-ylamino)benzyl)-7H-purine-6-carboxamide,
[122] N-(3-((4-cyanophenyl)amino)-5-fluorobenzyl)-8-cyclopentyl-7H-purine-6-carboxamide,
[123] N-(3-(1-(4-acetylphenyl)-1H-pyrazol-4-yl)-5-fluorobenzyl)-8-cyclopentyl-7H-purine-6-carboxamide,
[124] 8-cyclopentyl-N-(3-fluoro-5-(1-(furan-3-yl)-1H-pyrazol-4-yl)benzyl)-7H-purine-6-carboxamide,
[125] N-(3-(1'-benzyl-1'H-[1,4'-bipyrazol]-4-yl)-5-fluorobenzyl)-8-cyclopentyl-7H-purine-6-carboxamide,
[126] N-(3-((4-carbamoylphenyl)amino)-5-fluorobenzyl)-8-cyclopentyl-7H-purine-6-carboxamide,
[127] ethyl 4-((3-((8-cyclopentyl-7H-purine-6-carboxamido)methyl)-5-fluorophenyl)amino)benzoate,
[128] 8-cyclopentyl-N-(3-fluoro-5-(1'-methyl-1'H-[1,4'-bipyrazol]-4-yl)benzyl)-7H-purine-6-carboxamide,
[129] 8-cyclopentyl-N-(3-fluoro-5-((1-methylpiperidin-4-yl)amino)benzyl)-7H-purine-6-carboxamide,
[130] 8-cyclopentyl-N-(3-fluoro-5-(thiophen-3-ylamino)benzyl)-7H-purine-6-carboxamide,
[131] 8-cyclopentyl-N-(3-fluoro-5-(4-(4-fluorophenyl)piperazin-1-yl)benzyl)-7H-purine-6-carboxamide,
[132] 8-cyclopentyl-N-(3-fluoro-5-(5-(4-fluorophenyl)-1,3,4-oxadiazol-2-yl)benzyl)-7H-purine-6-carboxamide,
[133] 4-((3-((8-cyclopentyl-7H-purine-6-carboxamido)methyl)-5-fluorophenyl)amino)benzoic acid,
[134] N-(3-((1,3,4-oxadiazol-2-yl)amino)-5-fluorobenzyl)-8-cyclopentyl-7H-purine-6-carboxamide,
[135] 8-cyclopentyl-N-(3-(cyclopentylamino)-5-fluorobenzyl)-7H-purine-6-carboxamide,
[136] N-(3-((1,3,4-thiadiazol-2-yl)amino)-5-fluorobenzyl)-8-cyclopentyl-7H-purine-6-carboxamide,
[137] 8-cyclopentyl-N-(3-fluoro-5-(4-(4-fluorophenyl)-1,4-diazepan-1-yl)benzyl)-7H-purine-6-carboxamide,
[138] 8-cyclopentyl-N-(3-(1-cyclopentyl-1H-pyrazol-4-yl)-5-fluorobenzyl)-7H-purine-6-carboxamide,
[139] N-(3-(1-cyclohexyl-1H-pyrazol-4-yl)-5-fluorobenzyl)-8-cyclopentyl-7H-purine-6-carboxamide,
[140] 8-cyclopentyl-N-(3-fluoro-5-(1-(tetrahydrofuran-3-yl)-1H-pyrazol-4-yl)benzyl)-7H-purine-6-carboxamide,
[141] 8-cyclopentyl-N-(3-fluoro-5-(1-(thiazol-4-yl)-1H-pyrazol-4-yl)benzyl)-7H-purine-6-carboxamide,
[142] 8-cyclopentyl-N-(3-fluoro-5-((1-methyl-1H-pyrrol-3-yl)amino)benzyl)-7H-purine-6-carboxamide,
[143] 8-cyclopentyl-N-(3-fluoro-5-(1-(1-oxidothiophen-3-yl)-1H-pyrazol-4-yl)benzyl)-7H-purine-6-carboxamide,
[144] 8-(3,3-difluorocyclopentyl)-N-(3-fluoro-5-((1-methyl-1H-pyrazol-4-yl)amino)benzyl)-7H-purine-6-carboxamide,
[145] 8-cyclopentyl-N-(3-fluoro-5-(1-phenyl-*d*₅-1H-pyrazol-4-yl)benzyl)-7H-purine-6-carboxamide,
[146] 8-cyclopentyl-N-(3-fluoro-5-(1-(4-fluorophenyl)-3-methyl-1H-pyrazol-4-yl)benzyl)-7H-purine-6-carboxamide,
[147] 8-cyclopentyl-N-(3-fluoro-5-(1-(4-fluorophenyl)-5-methyl-1H-pyrazol-4-yl)benzyl)-7H-purine-6-carboxamide,
[148] 8-(3,3-difluorocyclopentyl)-N-(3-fluoro-5-(1-(phenyl-*d*₅)-1H-pyrazol-4-yl)benzyl)-7H-purine-6-carboxamide,
[149] 8-cyclopentyl-N-(3-fluoro-5-(4-(4-fluorophenyl)-1H-pyrazol-1-yl)benzyl)-7H-purine-6-carboxamide,
[150] methyl 2-(8-cyclopentyl-7H-purine-6-carboxamido)-3-(4-(4-(3-((8-cyclopentyl-7H-purine-6-carboxamido)methyl)-5-fluorophenyl)-1H-pyrazol-1-yl)phenyl)propanoate,
[151] 8-cyclopentyl-N-(3-fluoro-5-((1-(methyl-*d*₃)-1H-pyrazol-4-yl)amino)benzyl)-7H-purine-6-carboxamide,
[152] 8-(3,3-difluorocyclopentyl)-N-(3-fluoro-5-((1-(methyl-*d*₃)-1H-pyrazol-4-yl)amino)benzyl)-7H-purine-6-carboxamide, and
[153] 8-(cyclopentyl-2,2,3,3,4,4,5,5-*d*₈)-N-(3-fluoro-5-(1-(4-fluorophenyl)-1H-pyrazol-4-yl)benzyl)-7H-purine-6-carboxamide.

14. A pharmaceutical composition for preventing or treating a disease associated with HIF-1α(Hypoxia-inducible factor 1 alpha) or VEGF(Vascular endothelial growth factor) comprising the compound, a hydrate thereof, a solvate thereof, a prodrug thereof, an isomer thereof, or a pharmaceutically acceptable salt thereof of any one of Claims 1-13 as an active ingredient.

15. The pharmaceutical composition of Claim 14, **characterized in that** preventing or treating a disease associated with HIF-1α or VEGF is via an inhibition of angiogenesis.

16. The pharmaceutical composition of Claim 15, **characterized in that** the inhibition of angiogenesis is via inhibition of HIF-1α expression or inhibition of VEGF expression.

17. The pharmaceutical composition of Claim 14, **characterized in that** the disease associated with HIF-1α or VEGF is at least one selected from the group consisting of stroke, brainstem glioma, cerebellar astrocytoma, cerebral astrocytoma, ependymoma, liver cancer, stomach cancer, breast cancer, colon cancer, bone cancer, pancreatic cancer, head or neck cancer, ovarian cancer, rectal cancer, esophageal cancer, small intestine cancer, anal cancer, skin cancer, fallopian tube carcinoma, endometrial carcinoma, uterine cancer, vaginal carcinoma, vulvar carcinoma, Hodgkin's disease, oral cancer, prostate cancer, testicular cancer, bladder cancer, kidney cancer, ureteral cancer, spleen cell carcinoma, renal pelvic carcinoma, medulloblastoma, neuroblastoma, brain tumor, central nervous system tumor, melanoma, non-small cell lung cancer, lymphoma, non-Hodgkin's lymphoma, cancer, cervical cancer, thyroid cancer, blood cancer, renal cell carcinoma, hepatocellular carcinoma, metastatic cancer, hemangioma, pyogenic granuloma, Kaposi's sarcoma, hemangioendothelioma, solid tumor, follicular cyst, endometriosis, uterine sclerosis, ovarian hypertension, ovarian hyperactivity, uterine dysfunction, warts, scar keloids, allergic edema, atherosclerosis, peritoneal sclerosis, cardiac dysfunction, amyotrophic lateral sclerosis, obesity, rheumatoid arthritis, synovitis, bone and cartilage destruction, malignant fibrous histiocytoma of bone, osteomyelitis, pannus growth, liver fibrosis, pulmonary fibrosis, fibrosis, NASH (non-alcoholic steato hepatitis), pneumonia, asthma, rhinitis, pulmonary hypertension, ischemic acute kidney injury, glomerulonephritis, diabetic nephropathy, malignant nephrosclerosis, thrombotic microvascular lesions, organ transplant rejection, glomerulopathy, inflammatory neurodegenerative diseases, nicotine addiction-related vascular diseases, chronic kidney disease, retinopathy of prematurity, diabetic retinopathy, corneal transplant rejection, ischemic retinopathy, erythroderma, proliferative retinopathy, psoriasis, hemophilic joints, keloids, wound granulation, vascular adhesion, autoimmune diseases, angiofibromas, retrolental fibroplasia, cataracts, glaucoma, macular degeneration, age-related macular degeneration, corneal neovascularization, retinal neovascularization, choroidal neovascularization, intraocular neovascularization, neovascular glaucoma, neovascular macular degeneration, retinal artery occlusion, retinal vein occlusion, retinocytoma, visual pathway and hypothalamic glioma, rhabdomyosarcoma, histosarcoma, inflammation, corneal ulcer, proliferative vitreoretinopathy, cirrhosis, asthma, periodontal disease, allergic dermatitis, Alzheimer's disease, Parkinson's disease, Huntington's disease, thyroiditis, Graves' orbitopathy, leukomalacia, leukemia, acute lymphoblastic leukemia, chronic lymphocytic leukemia, chronic myelogenous leukemia, acute myeloid leukemia, multiple myeloma, inflammatory bowel disease, Crohn's disease, ulcerative colitis, and Behcet's enteritis.

18. The pharmaceutical composition of Claim 14, **characterized in that** the disease associated with HIF-1α or VEGF is a neovascular eye disease.

19. The pharmaceutical composition of Claim 18, **characterized in that** the neovascular eye disease is at least one selected from the group consisting of corneal neovascularization, retinal neovascularization, choroidal neovascularization, intraocular neovascularization, neovascular glaucoma, proliferative diabetic retinopathy, neovascular macular degeneration, and retinopathy of prematurity.

20. The pharmaceutical composition of Claim 18, **characterized in that** the neovascular eye disease is macular degeneration.

21. A formulation comprising the compound, a hydrate thereof, a solvate thereof, a prodrug thereof, an isomer thereof, or a pharmaceutically acceptable salt thereof of any one of Claims 1-13, and a pharmaceutically acceptable additive.
